# EUROPEAN PATENT SPECIFICATION

(11) **EP 3 826 666 B1**
(45) Date of publication and mention of the grant of the patent: **25.11.2026**
(21) Application number: 19841403.9
(22) Date of filing: 26.07.2019
(51) Int. Cl.: A61K 38/53, A61K 39/395, A61K 45/06, A61P 21/00, C12N 9/00, C12N 9/96

(54) **HISTIDYL-TRNA SYNTHETASE POLYPEPTIDE FOR USE IN TREATING NEUROPILIN-2 ASSOCIATED CANCER**
HISTIDYL-TRNA-SYNTHETASE-POLYPEPTID ZUR ANWENDUNG BEI DER BEHANDLUNG VON NEUROPILIN-2-ASSOZIIERTEM KREBS
POLYPEPTIDE D'HISTIDYL-TRNA SYNTHÉTASE DESTINÉ AU TRAITEMENT DU CANCER ASSOCIÉ À LA NEUROPILINE-2

(30) Priority: 26.07.2018 US 201862703757 P; 06.12.2018 US 201862776208 P; 01.02.2019 US 201962800035 P; 17.05.2019 US 201962849440 P
(43) Date of publication of application: 02.06.2021
(73) Proprietor: aTyr Pharma, Inc., San Diego, CA 92121 (US)
(72) Inventor: BURKART, Christoph, San Diego, California 92122 (US); OGILVIE, Kathleen M., San Diego, California 92024 (US); PAZ, Suzanne, San Marcos, California 92078 (US); ROSENGREN, Sanna, Encinitas, California 92024 (US); DATTA, Kaustubh, Omaha, Nebraska 68135 (US); DUTTA, Samikshan, Omaha, Nebraska 68131 (US)
(74) Representative: Grund, Martin
(86) International application number: PCT/US2019/043755
(87) International publication number: WO 2020/023918

(56) References cited:
- WO-A2-2018/102589
- US-A1- 2013 280 230
- US-A1- 2014 349 369
- US-B2- 8 753 638
- US-B2- 9 328 340
- YASUOKA HIRONAO ET AL: "Neuropilin-2 expression in breast cancer: correlation with lymph node metastasis, poor prognosis, and regulation of CXCR4 expression", BMC CANCER, BIOMED CENTRAL, LONDON, GB, vol. 9, no. 1, 7 July 2009 (2009-07-07), pages 220, XP021057598, ISSN: 1471-2407, DOI: 10.1186/1471-2407-9-220
- CAUNT MARESA ET AL: "Blocking neuropilin-2 function inhibits tumor cell metastasis", CANCER CELL, CELL PRESS, US, vol. 13, no. 4, 1 April 2008 (2008-04-01), pages 331 - 342, XP002478224, ISSN: 1535-6108, DOI: 10.1016/J.CCR.2008.01.029
- OGILVIE ET AL.: "Preclinical Characterization of ATYR1923 (iMod.Fc), an Immune-Modulatory Therapeutic With Potentially Broad Application in Interstitial Lung Diseases", POWER POINT ATYR1923, 18 May 2018 (2018-05-18), XP055680925, Retrieved from the Internet <URL:https://www.atyrpharma.com/wp-content/uploads/2019/01/Exh99.1-ATS-2018.pdf> [retrieved on 20190928]

## Description

### BACKGROUND

### Technical Field

Embodiments of the present disclosure relate to histidyl-tRNA synthetase (HRS) polypeptides for use in therapies, including standalone and combination therapies, for treating neuropilin-2 (NRP2)-associated diseases and conditions.

### Description of the Related Art

Recent research developments suggest that tRNA synthetases play important roles in cellular responses beyond their well characterized role in protein synthesis. In particular, there is a growing recognition that tRNA synthetases may participate in a range of previously unrecognized roles in responding to cellular stress, and tissue homeostasis, both within the intracellular and extracellular environments.

The Resokine family of proteins (HRS Polypeptides) are derived from the histidyl tRNA synthetase gene (HARS) via proteolysis or alternative splicing, and are important extracellular modulators of cellular activity. Extracellular HARS is readily detectable in the circulation in normal healthy volunteers, and autoantibodies to HARS (Jo-1 antibodies) have been characterized in some inflammatory myopathies (IM) and subjects with inflammatory lung disease (ILD). While the role of Jo-1 antibodies in disease progression is not yet well understood, subjects with Jo-1 antibodies tend to be less susceptible to cancer, compared to subjects with inflammatory myopathies without Jo-1 antibodies (see, e.g., Lu et al., PLOS ONE 9(4) e94128, 2014; Modan et al., Clin. Exp. Dermatol. 34(5) 561-565, 2009; and Shi et al., J. Rheum 44 (7) doi 10.3899/jrheum.161480).

Recently, significant progress has been made in elucidating the role of extracellular HARS derived proteins, including the identification of a putative cellular receptor, neuropilin-2 (NRP2, or NRP-2). Interactions of HARS with NRP2 appear to be mediated by the N-terminal region of HARS, and can lead to important changes in the cellular function of NRP2.

Accordingly, the current discovery of the Resokine/neuropilin-2 axis represents a previously unknown mechanism, which acts as a central regulator of cellular processes with direct relevance to muscular, vascular, neuronal, bone, and immune homeostasis. The deregulation of any of these processes may lead to a spectrum of diseases, which new HRS polypeptide based therapeutics may be able to address.

US 8,753,638 discloses the use of HRS splice variants for the treatment of cancer and . Yasuoka et al. (BMC Cancer 2009, 9(1): 220) and Caunt et al. (Cancer Cell 2008, 13(4): 331-342) disclose the involvement of NRP2 expression in tumor metastasis.

### BRIEF SUMMARY

The invention is defined by the appended claims.

Embodiments of the present disclosure include therapeutic compositions comprising a histidinyl-tRNA synthetase (HRS) polypeptide for use in treating a neuropilin-2 (NRP2) associated cancer in a subject in need thereof, wherein the NRP2 associated cancer expresses or overexpresses NRP2, and wherein the HRS polypeptide comprises, consists, or consists essentially of an amino acid sequence that is at least 90%, 95%, 96%, 97%, 98%, 99%, or 100% identical to a sequence selected from SEQ ID NOs: 156-171.

In some embodiments, the subject is to be selected for treatment based on having, increased extracellular fluid levels of a soluble NRP2 polypeptide (optionally selected from **Table** N1), either bound or free, relative to the levels of a healthy control or matched control standard or population of subject(s), optionally levels of about or at least about 10, 20, 30, 50, 100, 200, 300, 400, 500, 600, 700, 800, 900, 1000, 1100, 1200, 1300, 1400, 1500, 1600, 1700, 1800, 1900, 2000, 3000, 4000, 5000 pM of the soluble NRP2 polypeptide, or about 30-50, 50-100, 100-2000, 200-2000, 300-2000, 400-2000, 500-2000, 600-2000, 700-2000, 800-2000, 900-2000, 1000-2000, 2000-3000, 3000-4000, 4000-5000 pM of the soluble NRP2 polypeptide.

In some embodiments, the subject is to be selected for treatment based on having, increased extracellular fluid levels of NRP2:NRP2 ligand complexes (optionally selected from **Table N1** and **Table N2)** relative to a healthy control or matched control standard or population of subject(s), optionally levels that are about or at least about 1.5, 2, 3, 4, 5, 6, 7, 8, 9, 10, 15, 20, 30, 40, 50, 60, 70, 80, 90, 100 or more times the levels of the control or reference.

In some embodiments, the subject is to be selected for treatment based on having, increased extracellular fluid levels of HRS:NRP2 complexes (optionally selected from **Table H1** and **Table N1**) relative to a healthy control or matched control standard or population of subject(s), optionally levels that are about or at least about 1.5, 2, 3, 4, 5, 6, 7, 8, 9, 10, 15, 20, 30, 40, 50, 60, 70, 80, 90, 100 or more times the levels of the control or reference.

In some embodiments, the subject is to be selected for treatment based on having, a single nucleotide polymorphism (SNP) in an NRP2 polypeptide or an NRP2 encoding polynucleotide from the subject.

In some embodiments, the subject is to be selected for treatment based on having, a disease associated with increased levels or expression of NRP2a and/or NRP2b, or an altered ratio of NRP2a:NRP2b expression, relative to a healthy control or matched control standard or population of subject(s). In some embodiments, the levels of NRP2b are increased by about or at least about 10%, 20%, 30%, 40%, 50%, 100%, 200%, 300%, 400%, 500%, 600%, 700%, 800%, 900%, 1000% compared to a healthy control or matched control standard or population of subject(s).

In some embodiments, the healthy control or matched control standard or population of subject(s) comprises average ranges for age-matched samples of cancerous or non-cancerous cells or tissue of the same type as the cancer, which comprise specific characteristics such as drug resistance, metastatic potential, aggressiveness, genetic signature (optionally p53 mutation(s), PTEN deletion, IGFR expression), and/or expression patterns

In some embodiments, the subject in need thereof is to be selected for treatment based on having, an infection, and optionally wherein the method further comprises administering to the subject at least one antimicrobial agent, antifungal agent, and/or antihelminthic agent. In some embodiments, (a) the HRS polypeptide and (b) the antimicrobial agent, antifungal agent, and/or antihelminthic agent, are administered together as part of the same therapeutic composition. In some embodiments, (a) the HRS polypeptide and (b) the antimicrobial agent, antifungal agent, and/or antihelminthic agent, are administered as separate therapeutic compositions. In some embodiments, the antimicrobial agent, antifungal agent, and/or antihelminthic agent is selected from one or more of aminoglycosides such as Amikacin, Gentamicin, Kanamycin, Neomycin, Netilmicin, Tobramycin, Paromomycin, Streptomycin, and Spectinomycin; carbapenems such as Ertapenem, Doripenem, Imipenem/Cilastatin, and Meropenem; cephalasporins such as Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefixime, Cefdinir, Cefditoren, Cefoperazone, Cefotaxime, Cefpodoxime, Ceftazidime, Ceftibuten, Ceftizoxime, Moxalactam, Cefepime, Ceftaroline fosamil, and Ceftobiprole; glycopeptides such as Teicoplanin, Vancomycin, Telavancin, Dalbavancin, Oritavancin; lincosamines such as Clindamycin and Lincomycin; macrolides such as Azithromycin, Clarithromycin, Erythromycin, Roxithromycin, Telithromycin, and Spiramycin; penicillins such as Amoxicillin, Ampicillin, Azlocillin, Dicloxacillin, Flucloxacillin, Mezlocillin, Methicillin, Nafcillin, Oxacillin, Penicillin G, Penicillin V, Piperacillin, Penicillin G, Temocillin, and Ticarcillin; polypeptides such as Bacitracin, Colistin, and Polymyxin B; quinolones/fluoroquinolones such as Ciprofloxacin, Enoxacin, Gatifloxacin, Gemifloxacin, Levofloxacin, Lomefloxacin, Moxifloxacin, Nadifloxacin, Nalidixic acid, Norfloxacin, Ofloxacin, Trovafloxacin, Grepafloxacin, Sparfloxacin, and Temafloxacin; sulfonamides such as Mafenide, Sulfacetamide, Sulfadiazine, Silver sulfadiazine, Sulfadimethoxine, Sulfamethizole, Sulfamethoxazole, Sulfanilimide, Sulfasalazine, Sulfisoxazole, Trimethoprim-Sulfamethoxazole (Co-trimoxazole) (TMP-SMX), and Sulfonamidochrysoidine; tetracyclines such as Demeclocycline, Doxycycline, Metacycline, Minocycline, Oxytetracycline, and Tetracycline; anti-mycobacterials such as Clofazimine, Dapsone, Capreomycin, Cycloserine, Ethambutol, Ethionamide, Isoniazid, Pyrazinamide, Rifampicin (Rifampin), Rifabutin, Rifapentine, and Streptomycin; Chloramphenicol; Metronidazole; Mupirocin; Tigecycline; Tinidazole; and antihelminthic agents such as Diethylcarbamazine and Albendazole.

In some embodiments, the cancer displays NRP2-dependent growth, NRP2-dependent adhesion, NRP2-dependent migration, NRP2-dependent chemoresistance, and/or NRP2-dependent invasion. In some embodiments, the cancer is a primary cancer. In some embodiments, the cancer is a metastatic cancer, optionally a metastatic cancer that expresses NRP2a and/or NRP2b.

In some embodiments, the cancer is chemoresistant to at least one cancer therapy, for example, a cancer immunotherapy agent, a chemotherapeutic agent, a hormonal therapeutic agent, and/or a kinase inhibitor. In some instances, the cancer is chemoresistant to at least one cancer therapy prior to administering the HRS polypeptide.

In some embodiments, the HRS polypeptide modulates autophagy, efferocytosis, or phagocyte maturation in a cancer cell or cancer-associated macrophage. In specific embodiments, the HRS polypeptide modulates autophagy in the cancer cell.

In some embodiments, the cancer is selected from one or more of melanoma (e.g., metastatic melanoma), pancreatic cancer, bone cancer, prostate cancer, small cell lung cancer, non-small cell lung cancer (NSCLC), mesothelioma, leukemia (e.g., lymphocytic leukemia, chronic myelogenous leukemia, acute myeloid leukemia, relapsed acute myeloid leukemia), lymphoma, hepatoma (hepatocellular carcinoma), sarcoma, B-cell malignancy, breast cancer, ovarian cancer, colorectal cancer, glioma, glioblastoma multiforme, meningioma, pituitary adenoma, vestibular schwannoma, primary CNS lymphoma, primitive neuroectodermal tumor (medulloblastoma), kidney cancer (e.g., renal cell carcinoma), bladder cancer, uterine cancer, esophageal cancer, brain cancer, head and neck cancers, cervical cancer, testicular cancer, thyroid cancer, and stomach cancer.

In some embodiments, the metastatic cancer is selected from one or more of:
(a) a bladder cancer which has metastasized to the bone, liver, and/or lungs;
(b) a breast cancer which has metastasized to the bone, brain, liver, and/or lungs;
(c) a colorectal cancer which has metastasized to the liver, lungs, and/or peritoneum;
(d) a kidney cancer which has metastasized to the adrenal glands, bone, brain, liver, and/or lungs;
(e) a lung cancer which has metastasized to the adrenal glands, bone, brain, liver, and/or other lung sites;
(f) a melanoma which has metastasized to the bone, brain, liver, lung, and/or skin/muscle;
(g) a ovarian cancer which has metastasized to the liver, lung, and/or peritoneum;
(h) a pancreatic cancer which has metastasized to the liver, lung, and/or peritoneum;
(i) a prostate cancer which has metastasized to the adrenal glands, bone, liver, and/or lungs;
(j) a stomach cancer which has metastasized to the liver, lung, and/or peritoneum;
(l) a thyroid cancer which has metastasized to the bone, liver, and/or lungs; and
(m) a uterine cancer which has metastasized to the bone, liver, lung, peritoneum, and/or vagina.

In some embodiments, at least one additional agent selected from one or more of a cancer immunotherapy agent, a chemotherapeutic agent, a hormonal therapeutic agent, and a kinase inhibitor is to be administered to the subject. In some embodiments, the at least one HRS polypeptide and the at least one agent are to be administered separately, as separate compositions. In some embodiments, the at least one HRS polypeptide and the at least one agent are to be administered together as part of the same therapeutic composition.

In some embodiments, the cancer immunotherapy agent is selected from one or more of an immune checkpoint modulatory agent, a cancer vaccine, an oncolytic virus, a cytokine, and a cell-based immunotherapies. In some embodiments, the immune checkpoint modulatory agent is a polypeptide, optionally an antibody or antigen-binding fragment thereof or a ligand, or a small molecule. In some embodiments, the immune checkpoint modulatory agent comprises
(a) an antagonist of a inhibitory immune checkpoint molecule; or
(b) an agonist of a stimulatory immune checkpoint molecule, for example, wherein the immune checkpoint modulatory agent specifically binds to the immune checkpoint molecule.

In some embodiments, the inhibitory immune checkpoint molecule is selected from one or more of Programmed Death-Ligand 1 (PD-L1), Programmed Death 1 (PD-1), Programmed Death-Ligand 2 (PD-L2), Cytotoxic T-Lymphocyte-Associated protein 4 (CTLA-4), Indoleamine 2,3-dioxygenase (IDO), tryptophan 2,3-dioxygenase (TDO), T-cell Immunoglobulin domain and Mucin domain 3 (TIM-3), Lymphocyte Activation Gene-3 (LAG-3), V-domain Ig suppressor of T cell activation (VISTA), B and T Lymphocyte Attenuator (BTLA), CD160, Herpes Virus Entry Mediator (HVEM), and T-cell immunoreceptor with Ig and ITIM domains (TIGIT).

In some embodiments, the antagonist is a PD-L1 and/or PD-L2 antagonist optionally selected from one or more of an antibody or antigen-binding fragment or small molecule that specifically binds thereto, atezolizumab (MPDL3280A), avelumab (MSB0010718C), and durvalumab (MEDI4736), optionally wherein the cancer is selected from one or more of colorectal cancer, melanoma, breast cancer, non-small-cell lung carcinoma, bladder cancer, and renal cell carcinoma;
the antagonist is a PD-1 antagonist optionally selected from one or more of an antibody or antigen-binding fragment or small molecule that specifically binds thereto, nivolumab, pembrolizumab, MK-3475, AMP-224, AMP-514PDR001, and pidilizumab, optionally wherein the PD-1 antagonist is nivolumab and the cancer is optionally selected from one or more of Hodgkin's lymphoma, melanoma, non-small cell lung cancer, hepatocellular carcinoma, renal cell carcinoma, and ovarian cancer;
the PD-1 antagonist is pembrolizumab and the cancer is optionally selected from one or more of melanoma, non-small cell lung cancer, small cell lung cancer, head and neck cancer, and urothelial cancer;
the antagonist is a CTLA-4 antagonist optionally selected from one or more of an antibody or antigen-binding fragment or small molecule that specifically binds thereto, ipilimumab, tremelimumab, optionally wherein the cancer is selected from one or more of melanoma, prostate cancer, lung cancer, and bladder cancer;
the antagonist is an IDO antagonist optionally selected from one or more of an antibody or antigen-binding fragment or small molecule that specifically binds thereto, indoximod (NLG-8189), 1-methyl-tryptophan (1MT), β-Carboline (norharmane; 9H-pyrido[3,4-b]indole), rosmarinic acid, and epacadostat, and wherein the cancer is optionally selected from one or more of metastatic breast cancer and brain cancer optionally glioblastoma multiforme, glioma, gliosarcoma or malignant brain tumor;
the antagonist is a TDO antagonist optionally selected from one or more of an antibody or antigen-binding fragment or small molecule that specifically binds thereto, 680C91, and LM10;
the antagonist is a TIM-3 antagonist optionally selected from one or more of an antibody or antigen-binding fragment or small molecule that specifically binds thereto;
the antagonist is a LAG-3 antagonist optionally selected from one or more of an antibody or antigen-binding fragment or small molecule that specifically binds thereto, and BMS-986016;
the antagonist is a VISTA antagonist optionally selected from one or more of an antibody or antigen-binding fragment or small molecule that specifically binds thereto;
the antagonist is a BTLA, CD160, and/or HVEM antagonist optionally selected from one or more of an antibody or antigen-binding fragment or small molecule that specifically binds thereto;
the antagonist is a TIGIT antagonist optionally selected from one or more of an antibody or antigen-binding fragment or small molecule that specifically binds thereto.

In some embodiments, the stimulatory immune checkpoint molecule is selected from one or more of OX40, CD40, Glucocorticoid-Induced TNFR Family Related Gene (GITR), CD137 (4-1BB), CD27, CD28, CD226, and Herpes Virus Entry Mediator (HVEM).

In some embodiments, the agonist is an OX40 agonist optionally selected from one or more of an antibody or antigen-binding fragment or small molecule or ligand that specifically binds thereto, OX86, Fc-OX40L, and GSK3174998;
the agonist is a CD40 agonist optionally selected from one or more of an antibody or antigen-binding fragment or small molecule or ligand that specifically binds thereto, CP-870,893, dacetuzumab, Chi Lob 7/4, ADC-1013, and rhCD40L, and wherein the cancer is optionally selected from one or more of melanoma, pancreatic carcinoma, mesothelioma, and hematological cancers optionally lymphoma such as Non-Hodgkin's lymphoma;
the agonist is a GITR agonist optionally selected from one or more of an antibody or antigen-binding fragment or small molecule or ligand that specifically binds thereto, INCAGN01876, DTA-1, and MEDI1873;
the agonist is a CD137 agonist optionally selected from one or more of an antibody or antigen-binding fragment or small molecule or ligand that specifically binds thereto, utomilumab, and 4-1BB ligand;
the agonist is a CD27 agonist optionally selected from one or more of an antibody or antigen-binding fragment or small molecule or ligand that specifically binds thereto, varlilumab, and CDX-1127 (1F5);
the agonist is a CD28 agonist optionally selected from one or more of an antibody or antigen-binding fragment or small molecule or ligand that specifically binds thereto, and TAB08; and/or
the agonist is an HVEM agonist optionally selected from one or more of an antibody or antigen-binding fragment or small molecule or ligand that specifically binds thereto.

In some embodiments, the cancer vaccine is selected from one or more of Oncophage, a human papillomavirus HPV vaccine optionally Gardasil or Cervarix, a hepatitis B vaccine optionally Engerix-B, Recombivax HB, or Twinrix, and sipuleucel-T (Provenge), or comprises a cancer antigen selected from one or more of human Her2/neu, Her1/EGF receptor (EGFR), Her3, A33 antigen, B7H3, CD5, CD19, CD20, CD22, CD23 (IgE Receptor), MAGE-3, C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), guanylyl cyclase C, NY-ESO-1, p53, survivin, integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin, optionally wherein the subject has or is at risk for having a cancer that comprises the corresponding cancer antigen.

In some embodiments, the oncolytic virus selected from one or more of talimogene laherparepvec (T-VEC), coxsackievirus A21 (CAVATAK^{™}), Oncorine (H101), pelareorep (REOLYSIN^{®}), Seneca Valley virus (NTX-010), Senecavirus SVV-001, ColoAd1, SEPREHVIR (HSV-1716), CGTG-102 (Ad5/3-D24-GMCSF), GL-ONC1, MV-NIS, and DNX-2401.

In some embodiments, the cytokine selected from one or more of interferon (IFN)-α, IL-2, IL-12, IL-7, IL-21, and Granulocyte-macrophage colony-stimulating factor (GM-CSF).

In some embodiments, the cell-based immunotherapy agent comprises cancer antigen-specific T-cells, optionally ex vivo-derived T-cells. In some embodiments, the cancer antigen-specific T-cells are selected from one or more of chimeric antigen receptor (CAR)-modified T-cells, and T-cell Receptor (TCR)-modified T-cells, tumor infiltrating lymphocytes (TILs), and peptide-induced T-cells.

In some embodiments, the at least one chemotherapeutic agent is selected from one or more of an alkylating agent, an anti-metabolite, a cytotoxic antibiotic, a topoisomerase inhibitor (type 1 or type II), and an anti-microtubule agent.

In some embodiments, the alkylating agent is selected from one or more of nitrogen mustards (optionally mechlorethamine, cyclophosphamide, mustine, melphalan, chlorambucil, ifosfamide, and busulfan), nitrosoureas (optionally N-Nitroso-N-methylurea (MNU), carmustine (BCNU), lomustine (CCNU), semustine (MeCCNU), fotemustine, and streptozotocin), tetrazines (optionally dacarbazine, mitozolomide, and temozolomide), aziridines (optionally thiotepa, mytomycin, and diaziquone (AZQ)), cisplatins and derivatives thereof (optionally carboplatin and oxaliplatin), and non-classical alkylating agents (optionally procarbazine and hexamethylmelamine);
the anti-metabolite is selected from one or more of anti-folates (optionally methotrexate and pemetrexed), fluoropyrimidines (optionally 5-fluorouracil and capecitabine), deoxynucleoside analogues (optionally ancitabine, enocitabine, cytarabine, gemcitabine, decitabine, azacitidine, fludarabine, nelarabine, cladribine, clofarabine, fludarabine, and pentostatin), and thiopurines (optionally thioguanine and mercaptopurine);
the cytotoxic antibiotic is selected from one or more of anthracyclines (optionally doxorubicin, daunorubicin, epirubicin, idarubicin, pirarubicin, aclarubicin, and mitoxantrone), bleomycins, mitomycin C, mitoxantrone, and actinomycin;
the topoisomerase inhibitor is selected from one or more of camptothecin, irinotecan, topotecan, etoposide, doxorubicin, mitoxantrone, teniposide, novobiocin, merbarone, and aclarubicin; and/or
the anti-microtubule agent is selected from one or more of taxanes (optionally paclitaxel and docetaxel) and vinca alkaloids (optionally vinblastine, vincristine, vindesine, vinorelbine).

In some embodiments, the at least one hormonal therapeutic agent is a hormonal agonist or a hormonal antagonist. In some embodiments, the hormonal agonist is selected from one or more of a progestogen (progestin), a corticosteroid (optionally prednisolone, methylprednisolone, or dexamethasone), insulin like growth factors, VEGF derived angiogenic and lymphangiogenic factors (optionally VEGF-A, VEGF-A145, VEGF-A165, VEGF-C, VEGF-D, PIGF-2), fibroblast growth factor (FGF), galectin, hepatocyte growth factor (HGF), platelet derived growth factor (PDGF), transforming growth factor (TGF)-beta, an androgen, an estrogen, and a somatostatin analog. In some embodiments, the hormonal antagonist is selected from one or more of a hormone synthesis inhibitor, optionally an aromatase inhibitor or a gonadotropin-releasing hormone (GnRH) or an analog thereof, and a hormone receptor antagonist, optionally a selective estrogen receptor modulator (SERM) or an anti-androgen, or an antibody directed against a hormonal receptor, optionally cixutumumab, dalotuzumab, figitumumab, ganitumab, istiratumab, robatumumab, alacizumab pegol, bevacizumab, icrucumab, ramucirumab, fresolimumab, metelimumab, naxitamab, cetuximab, depatuxizumab mafodotin, futuximab, imgatuzumab, laprituximab emtansine, matuzumab, modotuximab, necitumumab, nimotuzumab, panitumumab, tomuzotuximab, zalutumumab, aprutumab ixadotin, bemarituzumab, olaratumab, or tovetumab.

In some embodiments, the kinase inhibitor is selected from one or more of adavosertib, afanitib, aflibercept, axitinib, bevacizumab, bosutinib, cabozantinib, cetuximab, cobimetinib, crizotinib, dasatinib, entrectinib, erdafitinib, erlotinib, fostamitinib, gefitinib, ibrutinib, imatinib, lapatinib, lenvatinib, mubritinib, nilotinib, panitumumab, pazopanib, pegaptanib, ponatinib, ranibizumab, regorafenib, ruxolitinib, sorafenib, sunitinib, SU6656, tofacitinib, trastuzumab, vandetanib, and vemuafenib. In some embodiments, the kinase inhibitor is a PI3 kinase inhibitor selected from one or more of alpelisib, buparlisib, copanlisib, CUDC-907, dactolisib, duvelisib, GNE-477, idelasib, IPI-549, LY294002, ME-401, perifosine, PI-103, pictilisib, PWT33597, RP6503, taselisib, umbralisib, voxtalisib, wortmannin, and XL147.

In some embodiments, the HRS polypeptide comprises, consists, or consists essentially of an amino acid sequence that is at least 90%, 95%, 96%, 97%, 98%, 99%, or 100% identical to SEQ ID NO: 156 (Fc-HRS(2-60) or **HRS**^{FC1}). In some embodiments, the HRS polypeptide comprises, consists, or consists essentially of an amino acid sequence that is at least 90%, 95%, 96%, 97%, 98%, 99%, or 100% identical to a sequence selected from SEQ ID NOs: 157-171.

In some embodiments, the HRS polypeptide is fused to a heterologous polypeptide. In some embodiments, the heterologous polypeptide comprises an Fc region, to form an HRS-Fc fusion polypeptide, optionally wherein the HRS-Fc fusion polypeptide comprises, consists, or consists essentially of an amino acid sequence that is at least 80%, 85%, 90%, 95%, 96%, 97%, 98%, 99%, or 100% identical to a sequence selected from **Table H8.** In some embodiments, the heterologous polypeptide comprises a cartilage oligomeric protein (COMP) polypeptide, optionally a COMP pentameric domain polypeptide, to form an HRS-COMP fusion polypeptide, optionally wherein the HRS-COMP fusion polypeptide comprises, consists, or consists essentially of an amino acid sequence that is at least 80%, 85%, 90%, 95%, 96%, 97%, 98%, 99%, or 100% identical to a sequence selected from **Table H9.**

Also included are therapeutic compositions, comprising:
(a) a histidyl-tRNA synthetase (HRS) polypeptide; and
(b) at least one additional agent selected from an antimicrobial agent, an antifungal agent, an antihelminthic agent, a cancer immunotherapy agent, a chemotherapeutic agent, a hormonal therapeutic agent, and a kinase inhibitor.

In some embodiments, the HRS polypeptide comprises, consists, or consists essentially of an amino acid sequence that is at least 90%, 95%, 96%, 97%, 98%, 99%, or 100% identical to SEQ ID NO: 156 (Fc-HRS(2-60) or **HRS**^{FC1}). In some embodiments, the HRS polypeptide comprises, consists, or consists essentially of an amino acid sequence that is at least 90%, 95%, 96%, 97%, 98%, 99%, or 100% identical to a sequence selected from SEQ ID NOs: 157-171.

In some embodiments, the HRS polypeptide is fused to a heterologous polypeptide. In some embodiments, the heterologous polypeptide comprises an Fc region, to form an HRS-Fc fusion polypeptide, optionally wherein the HRS-Fc fusion polypeptide comprises, consists, or consists essentially of an amino acid sequence that is at least 80%, 85%, 90%, 95%, 96%, 97%, 98%, 99%, or 100% identical to a sequence selected from **Table H8.** In some embodiments, the heterologous polypeptide comprises a cartilage oligomeric protein (COMP) polypeptide, optionally a COMP pentameric domain polypeptide, to form an HRS-COMP fusion polypeptide, optionally wherein the HRS-COMP fusion polypeptide comprises, consists, or consists essentially of an amino acid sequence that is at least 80%, 85%, 90%, 95%, 96%, 97%, 98%, 99%, or 100% identical to a sequence selected from **Table H9.**

In some embodiments, the antimicrobial agent, antifungal agent, and/or antihelminthic agent is selected from one or more of aminoglycosides such as Amikacin, Gentamicin, Kanamycin, Neomycin, Netilmicin, Tobramycin, Paromomycin, Streptomycin, and Spectinomycin; carbapenems such as Ertapenem, Doripenem, Imipenem/Cilastatin, and Meropenem; cephalasporins such as Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefixime, Cefdinir, Cefditoren, Cefoperazone, Cefotaxime, Cefpodoxime, Ceftazidime, Ceftibuten, Ceftizoxime, Moxalactam, Cefepime, Ceftaroline fosamil, and Ceftobiprole; glycopeptides such as Teicoplanin, Vancomycin, Telavancin, Dalbavancin, Oritavancin; lincosamines such as Clindamycin and Lincomycin; macrolides such as Azithromycin, Clarithromycin, Erythromycin, Roxithromycin, Telithromycin, and Spiramycin; penicillins such as Amoxicillin, Ampicillin, Azlocillin, Dicloxacillin, Flucloxacillin, Mezlocillin, Methicillin, Nafcillin, Oxacillin, Penicillin G, Penicillin V, Piperacillin, Penicillin G, Temocillin, and Ticarcillin; polypeptides such as Bacitracin, Colistin, and Polymyxin B; quinolones/fluoroquinolones such as Ciprofloxacin, Enoxacin, Gatifloxacin, Gemifloxacin, Levofloxacin, Lomefloxacin, Moxifloxacin, Nadifloxacin, Nalidixic acid, Norfloxacin, Ofloxacin, Trovafloxacin, Grepafloxacin, Sparfloxacin, and Temafloxacin; sulfonamides such as Mafenide, Sulfacetamide, Sulfadiazine, Silver sulfadiazine, Sulfadimethoxine, Sulfamethizole, Sulfamethoxazole, Sulfanilimide, Sulfasalazine, Sulfisoxazole, Trimethoprim-Sulfamethoxazole (Co-trimoxazole) (TMP-SMX), and Sulfonamidochrysoidine; tetracyclines such as Demeclocycline, Doxycycline, Metacycline, Minocycline, Oxytetracycline, and Tetracycline; anti-mycobacterials such as Clofazimine, Dapsone, Capreomycin, Cycloserine, Ethambutol, Ethionamide, Isoniazid, Pyrazinamide, Rifampicin (Rifampin), Rifabutin, Rifapentine, and Streptomycin; Chloramphenicol; Metronidazole; Mupirocin; Tigecycline; Tinidazole; and antihelminthic agents such as Diethylcarbamazine and Albendazole

In some embodiments, the cancer immunotherapy agent is selected from one or more of an immune checkpoint modulatory agent, a cancer vaccine, an oncolytic virus, a cytokine, and a cell-based immunotherapies.

In some embodiments, the immune checkpoint modulatory agent is a polypeptide, optionally an antibody or antigen-binding fragment thereof or a ligand, or a small molecule. In some embodiments, the immune checkpoint modulatory agent comprises
(a) an antagonist of a inhibitory immune checkpoint molecule; or
(b) an agonist of a stimulatory immune checkpoint molecule, for example, wherein the immune checkpoint modulatory agent specifically binds to the immune checkpoint molecule.

In some embodiments, the inhibitory immune checkpoint molecule is selected from one or more of Programmed Death-Ligand 1 (PD-L1), Programmed Death 1 (PD-1), Programmed Death-Ligand 2 (PD-L2), Cytotoxic T-Lymphocyte-Associated protein 4 (CTLA-4), Indoleamine 2,3-dioxygenase (IDO), tryptophan 2,3-dioxygenase (TDO), T-cell Immunoglobulin domain and Mucin domain 3 (TIM-3), Lymphocyte Activation Gene-3 (LAG-3), V-domain Ig suppressor of T cell activation (VISTA), B and T Lymphocyte Attenuator (BTLA), CD160, Herpes Virus Entry Mediator (HVEM), and T-cell immunoreceptor with Ig and ITIM domains (TIGIT).

In some embodiments, the antagonist is a PD-L1 and/or PD-L2 antagonist optionally selected from one or more of an antibody or antigen-binding fragment or small molecule that specifically binds thereto, atezolizumab (MPDL3280A), avelumab (MSB0010718C), and durvalumab (MEDI4736);
the antagonist is a PD-1 antagonist optionally selected from one or more of an antibody or antigen-binding fragment or small molecule that specifically binds thereto, nivolumab, pembrolizumab, MK-3475, AMP-224, AMP-514, PDR001, and pidilizumab;
the antagonist is a CTLA-4 antagonist optionally selected from one or more of an antibody or antigen-binding fragment or small molecule that specifically binds thereto, ipilimumab, and tremelimumab;
the antagonist is an IDO antagonist optionally selected from one or more of an antibody or antigen-binding fragment or small molecule that specifically binds thereto, indoximod (NLG-8189), 1-methyl-tryptophan (1MT), β-Carboline (norharmane; 9H-pyrido[3,4-b]indole), rosmarinic acid, and epacadostat;
the antagonist is a TDO antagonist optionally selected from one or more of an antibody or antigen-binding fragment or small molecule that specifically binds thereto, 680C91, and LM10;
the antagonist is a TIM-3 antagonist optionally selected from one or more of an antibody or antigen-binding fragment or small molecule that specifically binds thereto;
the antagonist is a LAG-3 antagonist optionally selected from one or more of an antibody or antigen-binding fragment or small molecule that specifically binds thereto, and BMS-986016;
the antagonist is a VISTA antagonist optionally selected from one or more of an antibody or antigen-binding fragment or small molecule that specifically binds thereto;
the antagonist is a BTLA, CD160, and/or HVEM antagonist optionally selected from one or more of an antibody or antigen-binding fragment or small molecule that specifically binds thereto; and/or
the antagonist is a TIGIT antagonist optionally selected from one or more of an antibody or antigen-binding fragment or small molecule that specifically binds thereto.

In some embodiments, the stimulatory immune checkpoint molecule is selected from one or more of OX40, CD40, Glucocorticoid-Induced TNFR Family Related Gene (GITR), CD137 (4-1BB), CD27, CD28, CD226, and Herpes Virus Entry Mediator (HVEM).

In some embodiments, the agonist is an OX40 agonist optionally selected from one or more of an antibody or antigen-binding fragment or small molecule or ligand that specifically binds thereto, OX86, Fc-OX40L, and GSK3174998;
the agonist is a CD40 agonist optionally selected from one or more of an antibody or antigen-binding fragment or small molecule or ligand that specifically binds thereto, CP-870,893, dacetuzumab, Chi Lob 7/4, ADC-1013, and rhCD40L;
the agonist is a GITR agonist optionally selected from one or more of an antibody or antigen-binding fragment or small molecule or ligand that specifically binds thereto, INCAGN01876, DTA-1, and MEDI1873;
the agonist is a CD137 agonist optionally selected from one or more of an antibody or antigen-binding fragment or small molecule or ligand that specifically binds thereto, utomilumab, and 4-1BB ligand;
the agonist is a CD27 agonist optionally selected from one or more of an antibody or antigen-binding fragment or small molecule or ligand that specifically binds thereto, varlilumab, and CDX-1127 (1F5);
the agonist is a CD28 agonist optionally selected from one or more of an antibody or antigen-binding fragment or small molecule or ligand that specifically binds thereto, and TAB08; and/or
the agonist is an HVEM agonist optionally selected from one or more of an antibody or antigen-binding fragment or small molecule or ligand that specifically binds thereto.

In some embodiments, the cancer vaccine is selected from one or more of Oncophage, a human papillomavirus HPV vaccine optionally Gardasil or Cervarix, a hepatitis B vaccine optionally Engerix-B, Recombivax HB, or Twinrix, and sipuleucel-T (Provenge), or comprises a cancer antigen selected from one or more of human Her2/neu, Her1/EGF receptor (EGFR), Her3, A33 antigen, B7H3, CD5, CD19, CD20, CD22, CD23 (IgE Receptor), MAGE-3, C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), guanylyl cyclase C, NY-ESO-1, p53, survivin, integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin.

In some embodiments, the oncolytic virus selected from one or more of talimogene laherparepvec (T-VEC), coxsackievirus A21 (CAVATAK^{™}), Oncorine (H101), pelareorep (REOLYSIN^{®}), Seneca Valley virus (NTX-010), Senecavirus SVV-001, ColoAd1, SEPREHVIR (HSV-1716), CGTG-102 (Ad5/3-D24-GMCSF), GL-ONC1, MV-NIS, and DNX-2401.

In some embodiments, the cytokine selected from one or more of interferon (IFN)-α, IL-2, IL-12, IL-7, IL-21, and Granulocyte-macrophage colony-stimulating factor (GM-CSF).

In some embodiments, the cell-based immunotherapy agent comprises cancer antigen-specific T-cells, optionally ex vivo-derived T-cells. In some embodiments, the cancer antigen-specific T-cells are selected from one or more of chimeric antigen receptor (CAR)-modified T-cells, and T-cell Receptor (TCR)-modified T-cells, tumor infiltrating lymphocytes (TILs), and peptide-induced T-cells.

In some embodiments, the at least one chemotherapeutic agent is selected from one or more of an alkylating agent, an anti-metabolite, a cytotoxic antibiotic, a topoisomerase inhibitor (type 1 or type II), and an anti-microtubule agent.

In some embodiments, the alkylating agent is selected from one or more of nitrogen mustards (optionally mechlorethamine, cyclophosphamide, mustine, melphalan, chlorambucil, ifosfamide , and busulfan), nitrosoureas (optionally N-Nitroso-N-methylurea (MNU), carmustine (BCNU), lomustine (CCNU), semustine (MeCCNU), fotemustine, and streptozotocin), tetrazines (optionally dacarbazine, mitozolomide, and temozolomide), aziridines (optionally thiotepa, mytomycin, and diaziquone (AZQ)), cisplatins and derivatives thereof (optionally carboplatin and oxaliplatin), and non-classical alkylating agents (optionally procarbazine and hexamethylmelamine);
the anti-metabolite is selected from one or more of anti-folates (optionally methotrexate and pemetrexed), fluoropyrimidines (optionally 5-fluorouracil and capecitabine), deoxynucleoside analogues (optionally ancitabine, enocitabine, cytarabine, gemcitabine, decitabine, azacitidine, fludarabine, nelarabine, cladribine, clofarabine, fludarabine, and pentostatin), and thiopurines (optionally thioguanine and mercaptopurine);
the cytotoxic antibiotic is selected from one or more of anthracyclines (optionally doxorubicin, daunorubicin, epirubicin, idarubicin, pirarubicin, aclarubicin, and mitoxantrone), bleomycins, mitomycin C, mitoxantrone, and actinomycin;
the topoisomerase inhibitor is selected from one or more of camptothecin, irinotecan, topotecan, etoposide, doxorubicin, mitoxantrone, teniposide, novobiocin, merbarone, and aclarubicin; and/or
the anti-microtubule agent is selected from one or more of taxanes (optionally paclitaxel and docetaxel) and vinca alkaloids (optionally vinblastine, vincristine, vindesine, vinorelbine).

In some embodiments, the at least one hormonal therapeutic agent is a hormonal agonist or a hormonal antagonist. In some embodiments, the hormonal agonist is selected from one or more of a progestogen (progestin), a corticosteroid (optionally prednisolone, methylprednisolone, or dexamethasone), insulin like growth factors, VEGF derived angiogenic and lymphangiogenic factors (optionally VEGF-A, VEGF-A145, VEGF-A165, VEGF-C, VEGF-D, PIGF-2), fibroblast growth factor (FGF), galectin, hepatocyte growth factor (HGF), platelet derived growth factor (PDGF), transforming growth factor (TGF)-beta, an androgen, an estrogen, and a somatostatin analog. In some embodiments, the hormonal antagonist is selected from one or more of a hormone synthesis inhibitor, optionally an aromatase inhibitor or a gonadotropin-releasing hormone (GnRH) or an analog thereof, and a hormone receptor antagonist, optionally a selective estrogen receptor modulator (SERM) or an anti-androgen, or an antibody directed against a hormonal receptor, optionally cixutumumab, dalotuzumab, figitumumab, ganitumab, istiratumab, robatumumab, alacizumab pegol, bevacizumab, icrucumab, ramucirumab, fresolimumab, metelimumab, naxitamab, cetuximab, depatuxizumab mafodotin, futuximab, imgatuzumab, laprituximab emtansine, matuzumab, modotuximab, necitumumab, nimotuzumab, panitumumab, tomuzotuximab, zalutumumab, aprutumab ixadotin, bemarituzumab, olaratumab, or tovetumab.

In some embodiments, the kinase inhibitor is selected from one or more of adavosertib, afanitib, aflibercept, axitinib, bevacizumab, bosutinib, cabozantinib, cetuximab, cobimetinib, crizotinib, dasatinib, entrectinib, erdafitinib, erlotinib, fostamitinib, gefitinib, ibrutinib, imatinib, lapatinib, lenvatinib, mubritinib, nilotinib, panitumumab, pazopanib, pegaptanib, ponatinib, ranibizumab, regorafenib, ruxolitinib, sorafenib, sunitinib, SU6656, tofacitinib, trastuzumab, vandetanib, and vemuafenib. In some embodiments, the kinase inhibitor is a PI3 kinase inhibitor selected from one or more of alpelisib, buparlisib, copanlisib, CUDC-907, dactolisib, duvelisib, GNE-477, idelasib, IPI-549, LY294002, ME-401, perifosine, PI-103, pictilisib, PWT33597, RP6503, taselisib, umbralisib, voxtalisib, wortmannin, and XL147.

In some embodiments, the therapeutic composition has a purity of at least about 80%, 85%, 90%, 95%, 98%, or 99% on a protein basis with respect to the HRS polypeptide, and is substantially aggregate-free. In some embodiments, the therapeutic composition is substantially endotoxin-free. In some embodiments, the therapeutic composition is a sterile, injectable solution, optionally suitable for intravenous, intramuscular, subcutaneous, or intraperitoneal administration.

Also included are patient care kits, comprising:
(a) a histidyl-tRNA synthetase (HRS) polypeptide, as described herein; and
(b) at least one additional agent selected from an antimicrobial agent, an antifungal agent, an antihelminthic agent, a cancer immunotherapy agent, a chemotherapeutic agent, a hormonal therapeutic agent, and a kinase inhibitor, as described herein.

In some embodiments, (a) and (b) are in separate therapeutic compositions. In some embodiments, (a) and (b) are in the same therapeutic composition.

Certain embodiments include an isolated fusion protein, comprising a histidyl-tRNA synthetase polypeptide fused to a cartilage oligomeric protein (COMP) polypeptide, optionally a COMP pentameric domain polypeptide, to form an HRS-COMP fusion polypeptide, optionally wherein the HRS-COMP fusion polypeptide comprises, consists, or consists essentially of an amino acid sequence that is at least 80%, 85%, 90%, 95%, 96%, 97%, 98%, 99%, or 100% identical to a sequence selected from **Table H9.**

Some embodiments include a therapeutic composition, comprising a HRS-COMP fusion protein described herein. In some embodiments, the therapeutic composition has a purity of at least about 80%, 85%, 90%, 95%, 98%, or 99% on a protein basis with respect to the HRS-COMP fusion polypeptide, and is substantially aggregate-free. In particular embodiments, the therapeutic composition is substantially endotoxin-free. In some embodiments, the therapeutic composition is a sterile, injectable solution, optionally suitable for intravenous, intramuscular, subcutaneous, or intraperitoneal administration

### BRIEF DESCRIPTION OF THE DRAWINGS

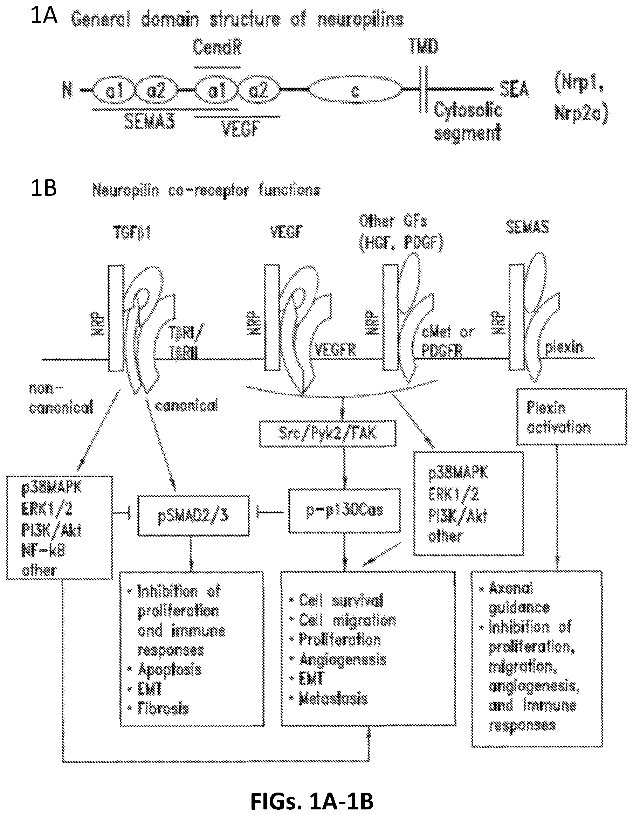

**Figures 1A-1B** illustrate the general domain structure of neuropilins (1A) and exemplary neuropilin co-receptor functions (1B).

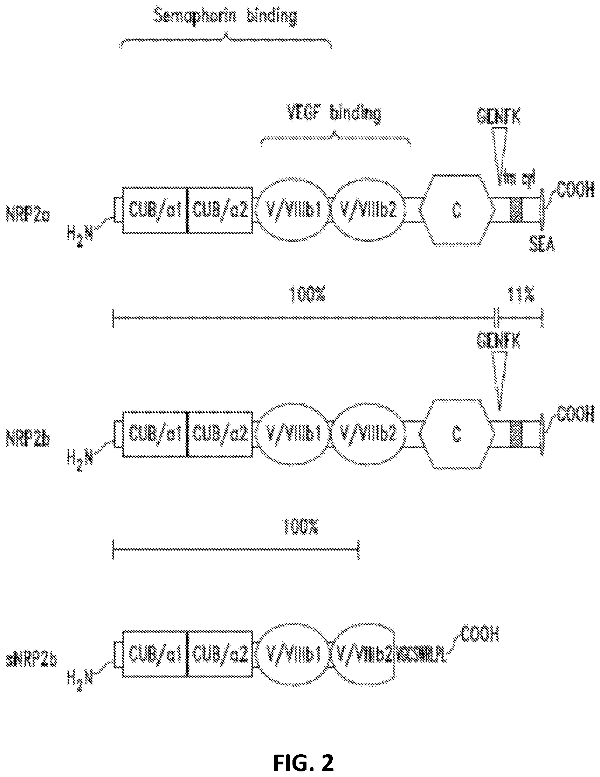

**Figure 2** illustrates the domain structure of certain NRP2 isoforms and NRP2 ligand binding domains.

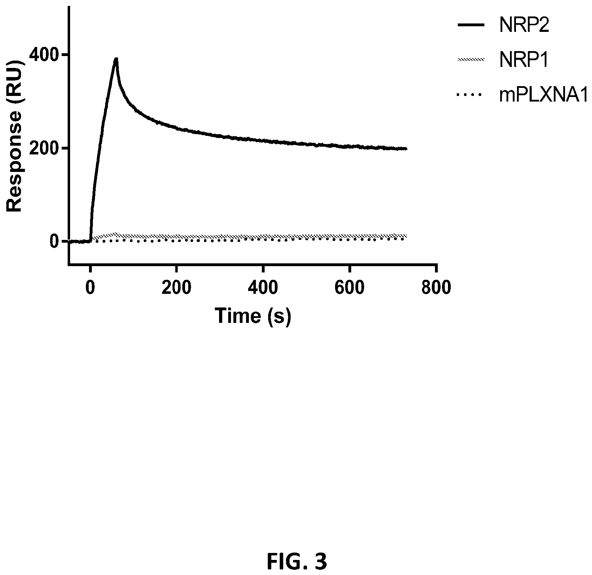

**Figure 3** shows binding of human NRP2 to Fc-HRS(2-60) on an SPR chip. 50 nM of NRP2 (solid black line), NRP1 (solid gray line) and mouse Plexin A1 (dotted line) were flowed as analytes over an SPR chip coated with immobilized Fc-HRS(2-60) .

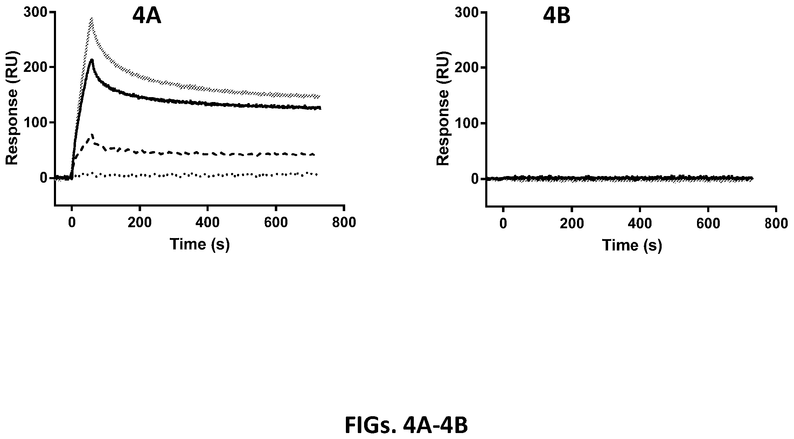

**Figures 4A-4B** shows binding of NRP2 from human, mouse, and rat to Fc-HRS(2-60) and not a truncated form of Fc-HRS(2-11). 50 nM of human NRP2 (solid black line), mouse NRP2 (dashed line), rat NRP2 (solid gray line), or NRP1 (dotted line) were flowed as analytes over an SPR chip coated with immobilized full length Fc-HRS(2-60) (4A), or a truncated form of Fc-HRS(2-11) missing 49 amino acids at the C-terminus (4B).

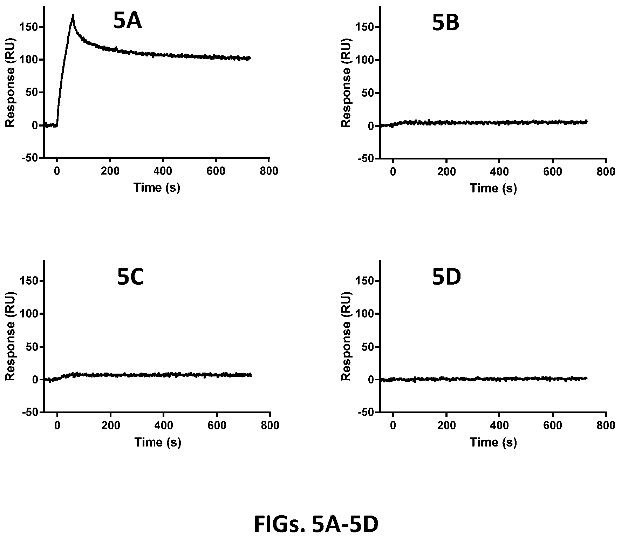

**Figures 5A-5D** show binding of human NRP2 to Fc-HRS(2-60) and t-RNA synthetases comprising domains that share homology with the WHEP domain of Fc-HRS(2-60). 20 nM of NRP2 was flowed as an analyte over SPR chip surfaces coated with immobilized Fc-HRS(2-60) (5A), GARS Fc-WHEP (5B), MARS Fc-WHEP (5C), or WARS WHEP (5D).

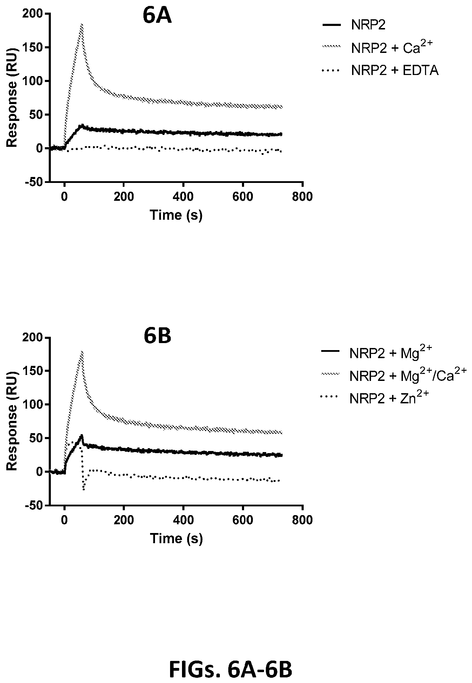

**Figures 6A-6B** show the binding of human NRP2 to Fc-HRS(2-60) on an SPR chip coated with immobilized Fc-HRS(2-60) in the presence and absence of divalent cations. The running buffer in this experiment was 50 mM HEPES, 300 mM NaCl, 0.005% Tween20, pH 7.4. For each analyte, 20 nM NRP2 was prepared in running buffer supplemented with 5 mM of either CaCl₂, EDTA (6A) or MgCl₂, MgCl₂ + CaCl₂ or ZnCl₂ (6B).

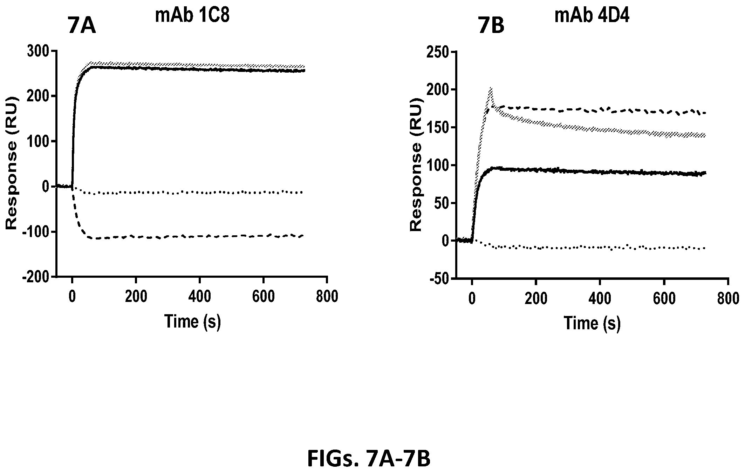

**Figures 7A-7B** show binding of a pre-formed complex of Fc-HRS(2-60) and NRP2 to 4D4 monoclonal antibody but not to the 1C8 monoclonal antibody. Monoclonal antibodies against Fc-HRS(2-60) (monoclonal antibody clones 1C8 (7A) and 4D4 (7B)) were immobilized on an SPR chip. Analytes consisted of 200 nM NRP2 (dotted line), 100 nM Fc-HRS(2-60) (solid black line), a mixture of 100 nM Fc-HRS(2-60) and 200 nM NRP2 (solid gray line), or a mixture of 100 nM Fc-HRS(2-60) and 200 nM 1C8 mAb (dashed line).

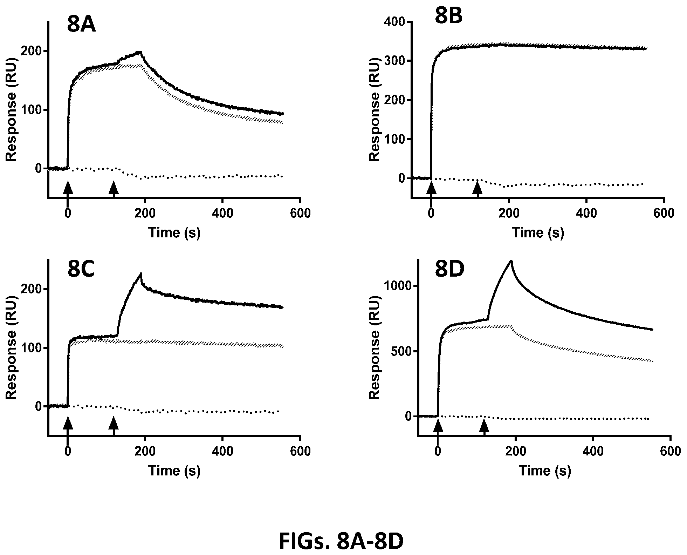

**Figures 8A-8D** show binding of NRP2 to Fc-HRS(2-60) captured by some monoclonal antibodies against Fc-HRS(2-60) but not others. Monoclonal antibodies against Fc-HRS(2-60) (Monoclonal antibody clones 12H6 (8A), 1C8 (8B), 4D4 (8C) and 13E9 (8D) were immobilized on an SPR chip. Co-injections were then carried out where one analyte is injected, immediately followed by a second analyte. Timing of the two injections is indicated by arrowheads. In each of the panels above, 2000 nM Fc-HRS(2-60) was injected as the first analyte to saturate the antibody surfaces, followed by either additional Fc-HRS(2-60) (solid gray line), or 200 nM NRP2 (solid black line). To rule out non-specific binding of NRP2 to the antibody surfaces, co-injection of buffer followed by 200 nM NRP2 were also performed (dotted line).

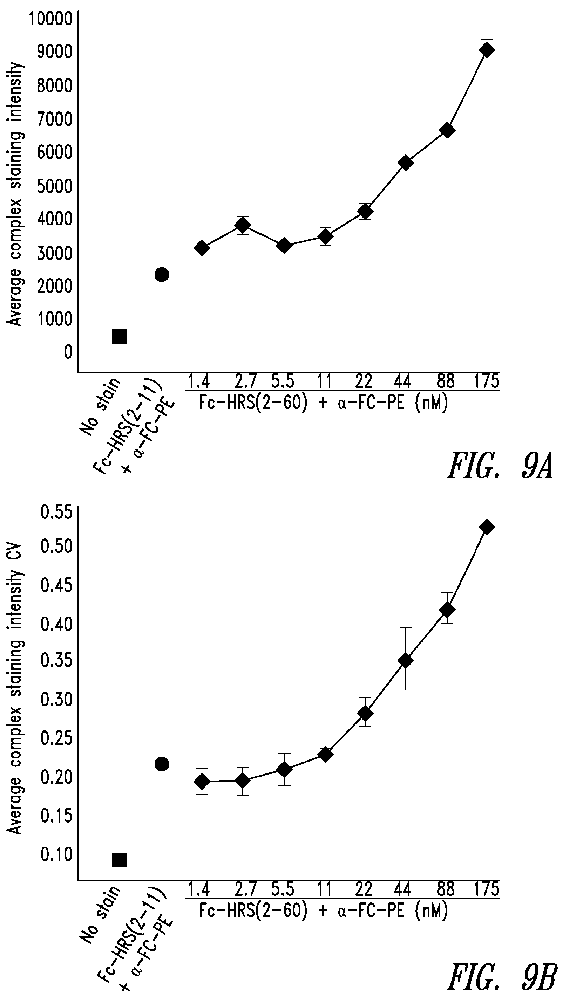

**Figures 9A-9B** show dose-dependent binding of Fc-HRS (2-60) to cells expressing a NRP2a-GFP fusion protein. Quantification of the staining intensity (9A) and staining intensity CV (9B) of Fc-HRS (2-60)/anti-Fc-PE complex on HEK293T cells overexpressing NRP2v2-GFP. Intensity values are from cells gated on high NRP2 expression (GFP Bright). Fc-HRS (2-60) was titrated in 2 fold steps and then combined with 87.5 nM of anti-Fc-PE. As a control for specificity, 175 nM Fc-HRS (2-11)/anti-Fc-PE was included.

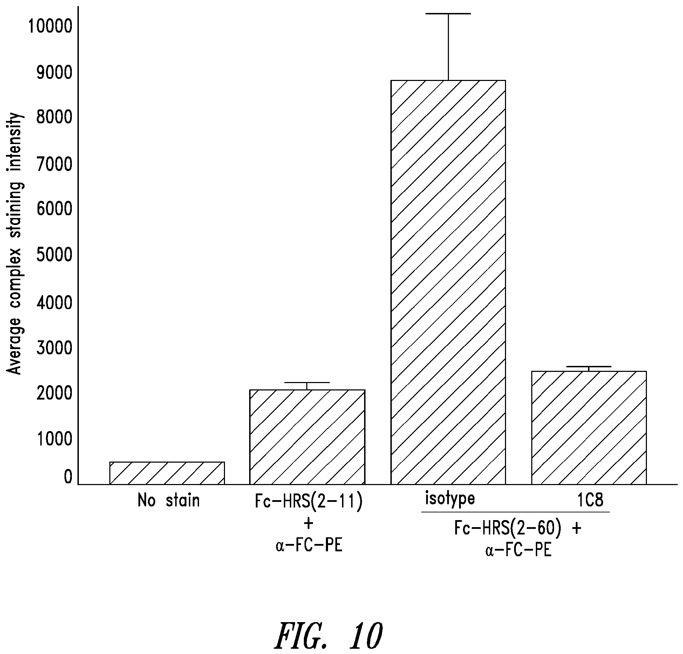

**Figure 10** shows binding inhibition of Fc-HRS (2-60) to cells expressing a NRP2a-GFP fusion protein in the presence of anti-HRS antibody clone 1C8. Quantification of the staining intensity of Fc-HRS (2-60)/anti-Fc-PE complex pre-incubated with either an isotype antibody control or anti-HRS (WHEP) clone 1C8 on HEK293T cells overexpressing NRP2v2-GFP. Intensity values are from cells gated on high NRP2 expression (GFP Bright). 175nM of Fc-HRS (2-60)/anti-Fc-PE was used. As a control for specificity, 175 nM Fc-HRS (2-11)/anti-Fc-PE was included.

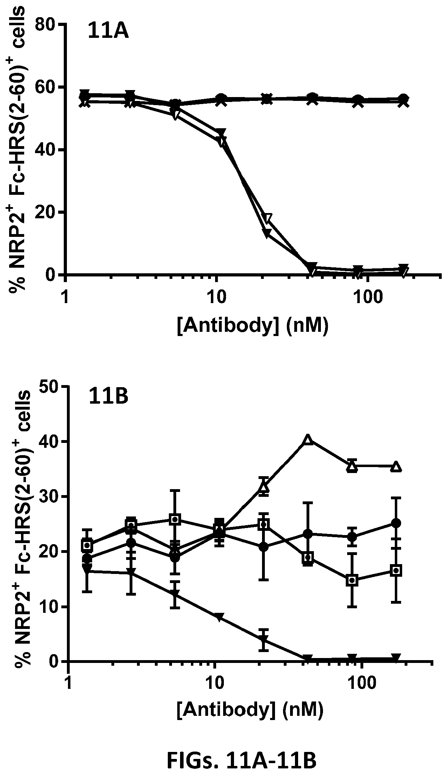

**Figures 11A-11B** show that anti-HRS antibodies from the KL31 series blocked binding of Fc-HRS(2-60) to NRP2 in a concentration-dependent manner, whereas other antibodies of the AB04 and AB13 series did not demonstrate significant blocking characteristics in this assay. Quantification of the staining of stably expressing Expi293-NRP2 cells with biotinylated Fc-HRS - streptavidin-PE using flow cytometry in the presence of various concentrations of anti-HRS antibodies. Data are from two experiments using different antibodies. Fig. 11A shows control human IgG1 (filled circles), KL31-467 (filled triangles), KL31-356 (partially filled triangles), mouse clone 13C8 (crosses), and 11B shows control human IgG1 (filled circles), AB04-425 (open triangles), AB13-288 (partially filled squares), and KL31-478 (filled triangles), which are shown as the percentage of streptavidin-PE+/NRP2+ cells in the viable singlet gate.

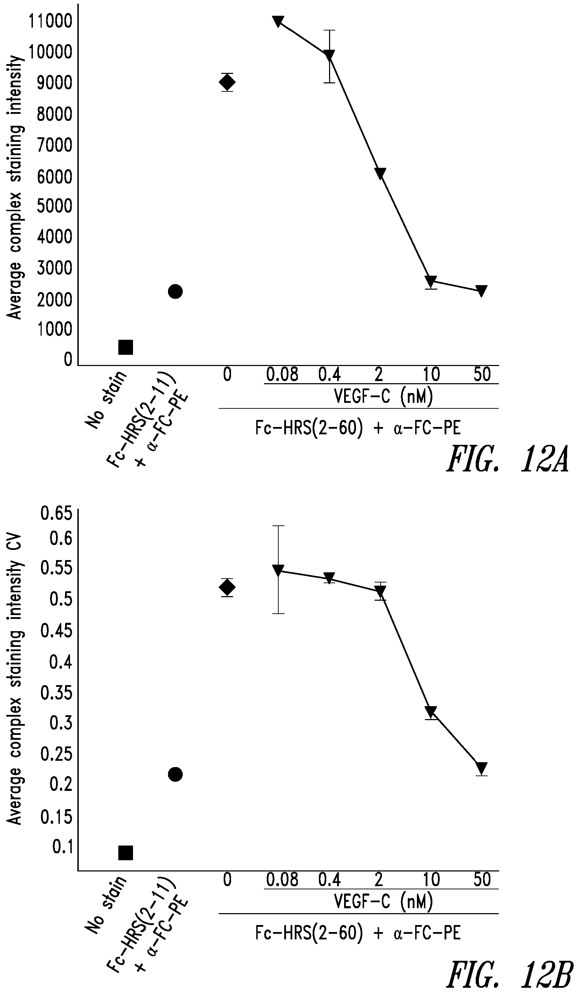

**Figures 12A-12B** show binding inhibition of Fc-HRS (2-60) to cells expressing a NRP2a-GFP fusion protein in the presence of VEGF-C. Quantification of the staining intensity of Fc-HRS (2-60)/anti-Fc-PE complex pre-incubated with different doses of VEGF-C on HEK293T cells overexpressing NRP2v2-GFP. Intensity values are from cells gated on high NRP2 expression (GFP Bright). 175nM of Fc-HRS (2-60)/anti-Fc-PE was used. As a control for specificity, 175nM Fc-HRS (2-11)/anti-Fc-PE was included.

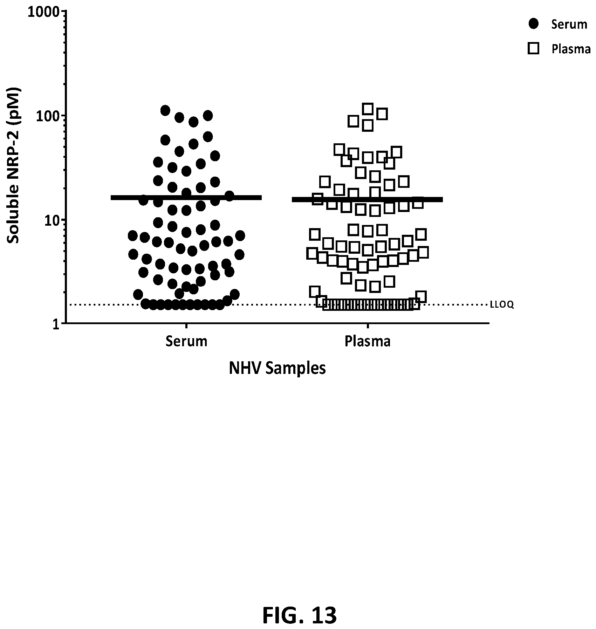

**Figure 13** shows quantification of circulating NRP2 levels in serum and plasma from normal healthy donors. Normal healthy volunteer (n=72) serum and plasma was isolated and quantified for circulating levels of NRP-2. Serum (black circles) and plasma (open squares) samples were tested in an ELISA specific for human NRP-2. Mean levels for serum (16.3 pM) and plasma (15.6 pM) were shown for all 72 samples. The limit of quantification for the NRP2 ELISA was 1.5 pM.

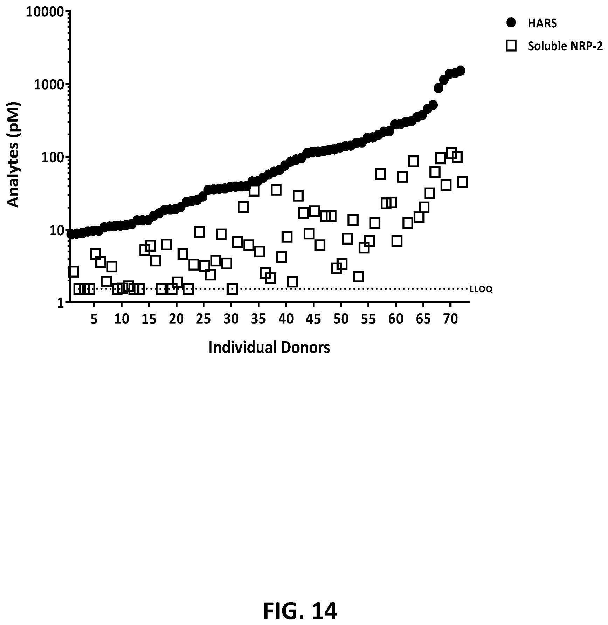

**Figure 14** shows a comparison of circulating HRS and NRP2 levels. Serum HRS (black circles) levels show a broad range in circulation within 72 normal healthy volunteers tested. Matching serum NRP2 levels from the identical donors were overlaid on the same axes. Donors with low HRS levels show low to undetectable levels of soluble NRP2 (Limit of quantification = 1.5 pM). Those donors with elevated HRS levels generally have corresponding increased levels of circulating NRP2.

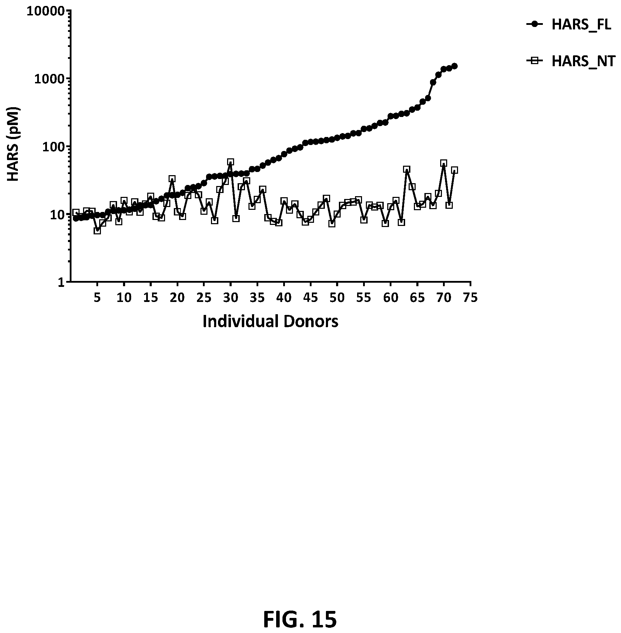

**Figure 15** shows HRS N-terminal interference in human serum from healthy donors. Normal serum from healthy donors was assayed in two separate HARS ELISAs. Samples were assayed in an ELISA to detect full length HARS (HARS_FL; black circles) as well as an ELISA directed against specifically the N-terminus (HARS_NT; open squares). The lack of correlation between these two ELISAs, as full length HARS levels increased, is referred to as N-terminal interference and may represent the presence of a cofactor, binding partner or soluble receptor to HRS.

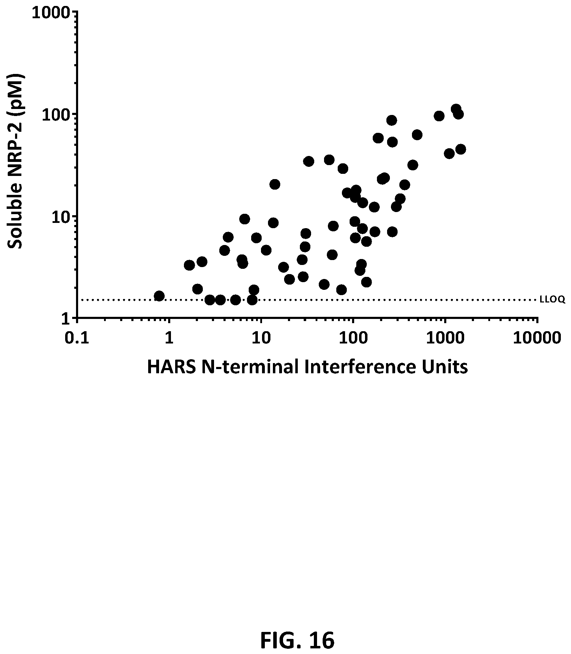

**Figure 16** shows a correlation between HARS N-terminal assay interference and soluble NRP2 levels. Normal healthy serum was analyzed for differences in detection with two HARS ELISAs and compared to circulating NRP2 levels. The difference in levels detected between the full length HARS ELISA and the N-terminal HARS ELISA was termed HARS N-terminal Interference Units. These interference units were plotted versus soluble NRP2 levels. The results show a relationship between increased N-terminal interference and soluble NRP2 in normal serum.

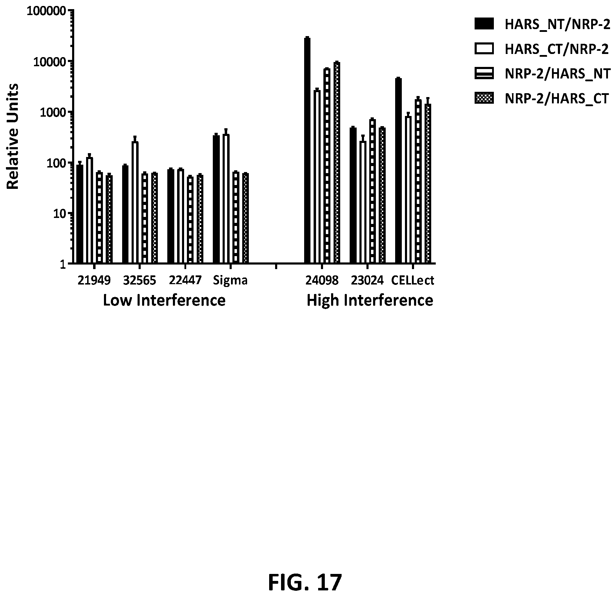

**Figure 17** shows detection of an endogenous HRS & NRP2 soluble complex. Serum samples from normal healthy donors were analyzed in multiple HRS & NRP-2 complex ELISAs. These assay formats utilized capture of circulating HRS (HARS_NT or HARS_CT) and detection with an NRP2 monoclonal antibody. Similarly the reverse format was also used whereby circulating NRP2 was captured and detection was observed with anti-HRS antibodies. In both formats, signals were elevated in the high interference samples as compared to low interference serum samples.

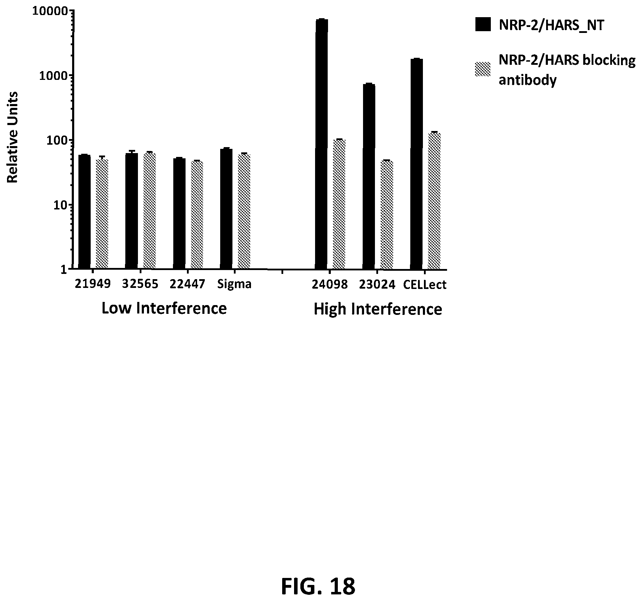

**Figure 18** shows that complexed HRS and NRP2 in high interference samples blocks detection with a site-specific HRS antibody. Serum from low and high HRS N-terminal interference samples was assayed in a HRS and NRP2 complex ELISA. Serum samples were captured with an NRP2 monoclonal antibody and detected with either of two unique HRS N-terminal monoclonal antibodies. Samples with high interference showed complex formation when detected with HRS_NT (black bars) but this signal was completely blocked with an N-terminal anti-HRS antibody (HRS blocking antibody; gray bars).

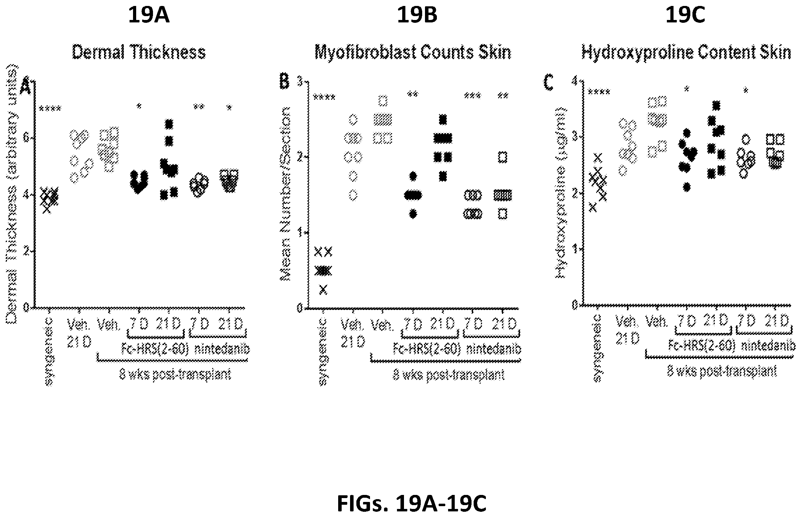

**Figures 19A-19C** shows the activity of Fc-HRS(2-60) on skin markers in a murine model of sclerodermatous chronic Graft vs. Host Disease. Figure 19A shows the impact of treatment with Vehicle, Fc-HRS(2-60) or nintedanib beginning 7 (7 D) or 21 (21 D) days after allogenic transplantation on dermal thickness. Figure 19B shows the impact of treatment with Vehicle, Fc-HRS(2-60) or nintedanib beginning 7 (7 D) or 21 (21 D) days after allogenic transplantation on the number of myofibroblasts counted in skin sections. Figure 19 C shows the impact of treatment with Vehicle, Fc-HRS(2-60) or nintedanib beginning 7 (7 D) or 21 (21 D) days after allogenic transplantation on hydroxyproline content (an indicator or collagen content) in the skin. ****p < 0.0001, ***p < 0.001, **p < 0.01, *p <0.05 Kruskal-Wallis non-parametric ANOVA followed by Dunn's multiple comparison test to Vehicle animals euthanized 8 weeks after allogenic transplant (3^{rd} group from the left).

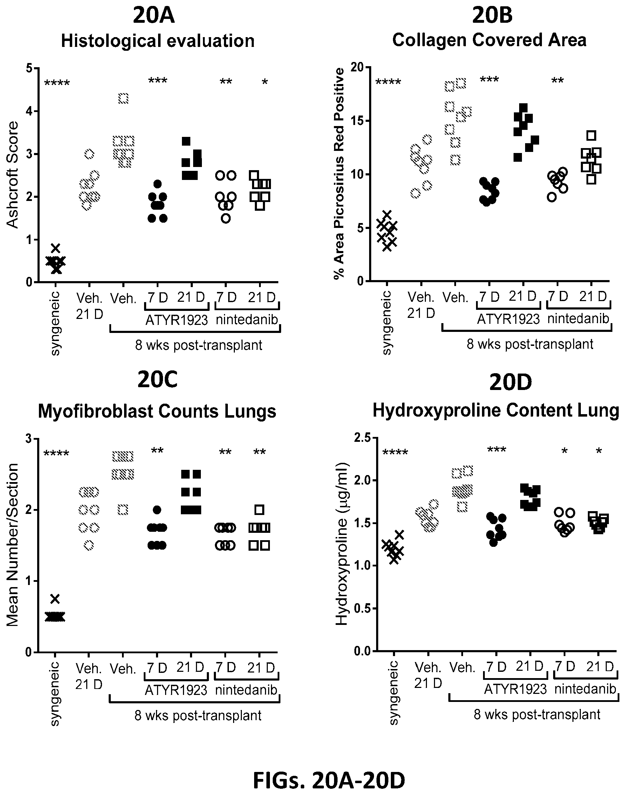

**Figures 20A-20D** show the activity of Fc-HRS(2-60) on lung markers in a murine model of sclerodermatous chronic Graft vs. Host Disease. Figure 20A shows the impact of treatment with Vehicle, Fc-HRS(2-60) or nintedanib beginning 7 (7 D) or 21 (21 D) days after allogenic transplantation on Ashcroft score. Figure 20B shows the impact of treatment with Vehicle, Fc-HRS(2-60) or nintedanib beginning 7 (7 D) or 21 (21 D) days after allogenic transplantation on the percentage of each section occupied by tissue that stains with picrosirius red, a stain that specific stains collagen fibers. Figure 20C shows the impact of treatment with Vehicle, Fc-HRS(2-60) or nintedanib beginning 7 (7 D) or 21 (21 D) days after allogenic transplantation on the number of myofibroblasts counted in lung sections. Figure 20D shows the impact of treatment with Vehicle, Fc-HRS(2-60) or nintedanib beginning 7 (7 D) or 21 (21 D) days after allogenic transplantation on hydroxyproline content (an indicator or collagen content) in the skin. ****p < 0.0001, ***p < 0.001, **p < 0.01, *p <0.05 Kruskal-Wallis non-parametric ANOVA followed by Dunn's multiple comparison test to Vehicle animals euthanized 8 weeks after allogenic transplant (3^{rd} group from the left).

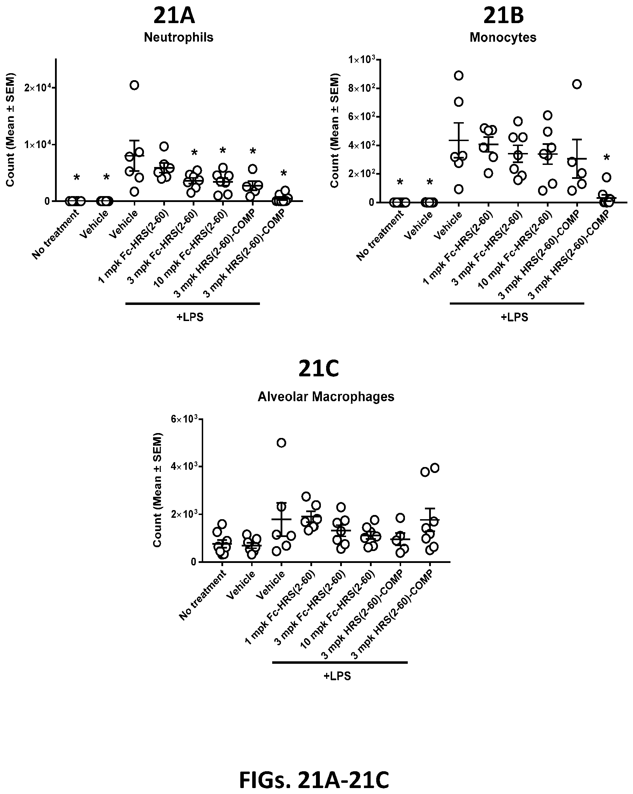

**Figures 21A-21C** show the effects of HRS(2-60) containing proteins on LPS-induced leukocyte infiltration into the mouse lung. Mice were treated intravenously with Fc-HRS(2-60) or HRS(2-60)-COMP at the doses indicated. The following day, saline or LPS (10 µg/mouse) was instilled into the airways via oropharyngeal administration, and infiltrating cells were collected 24 hours later via bronchoalveolar lavage and analyzed by flow cytometry. Groups receiving LPS are demarcated by a horizontal bar. Data from individual animals (circles) along with mean and SEM are shown. Asterisk indicates significant difference (p < 0.05) from the LPS/Vehicle group by ANOVA followed by Dunnett's post-hoc test.

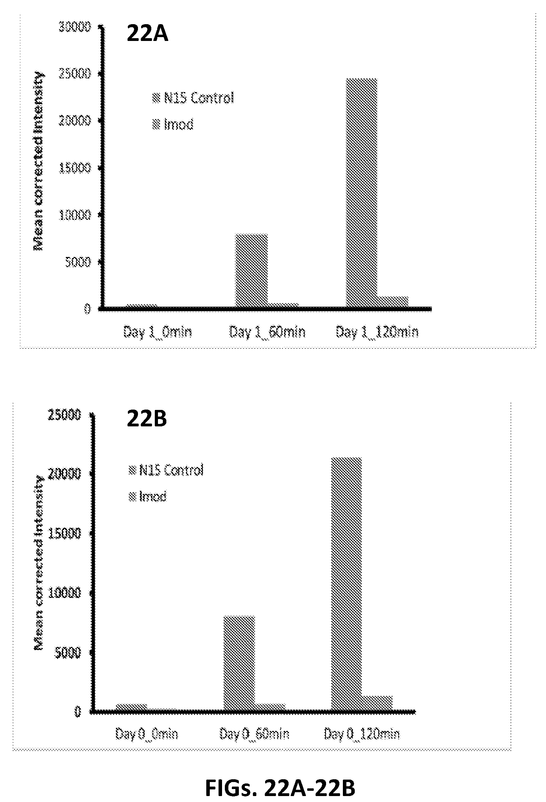

**Figures 22A-22B** show that incubation of bone marrow derived macrophages with either 100nM or 200 nM Fc-HRS(2-60) (Imod), but not the Fc control compound N15, for 5 days during monocyte differentiation resulted in a profound inhibition of phagocyte maturation, as revealed by the dramatically diminished spectral shift reported by the pH sensitive fluorescent dye pH rhodo^{™}.

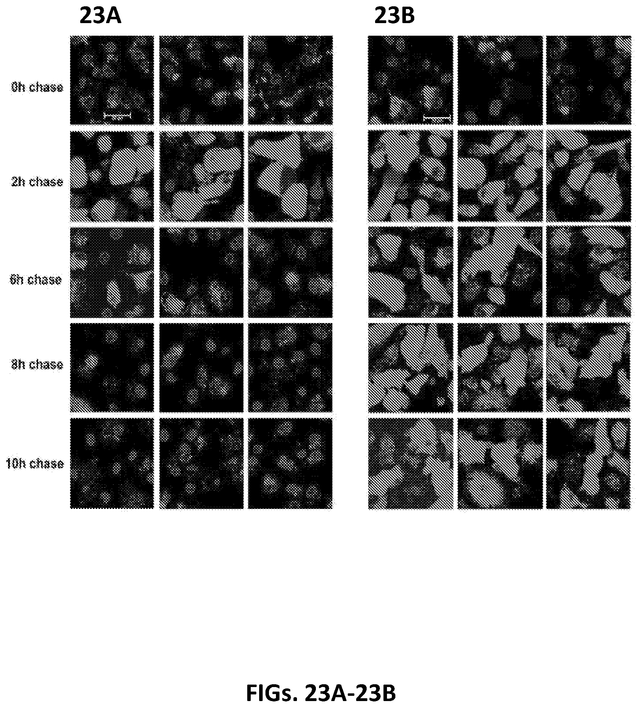

**Figures 23A-23B** show that incubation of bone marrow derived macrophages with 100nM HRS(2-60)-COMP), but not the control compound COMP, for 5 days during monocyte differentiation resulted in a profound inhibition of efferocytosis, as revealed by the dramatically diminished spectral shift reported by the pH sensitive fluorescent dye pH rhodo^{™}.

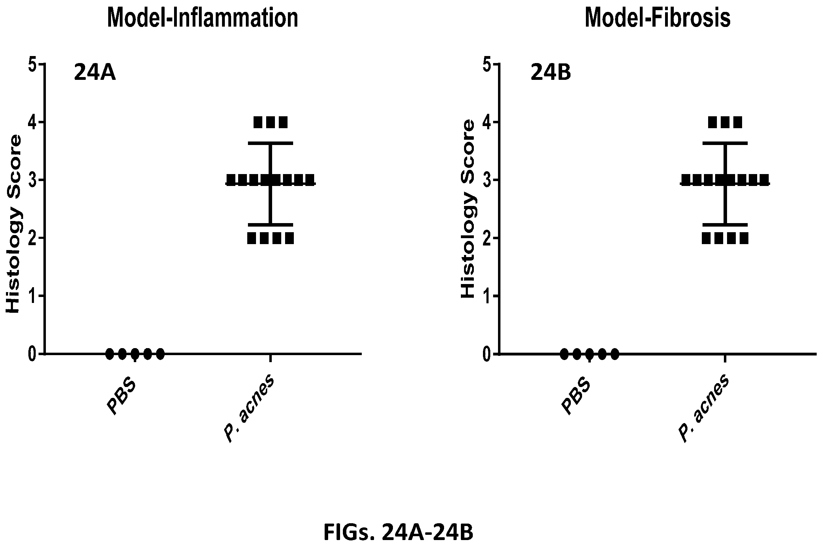

**Figures 24A-24B** show histological confirmation of model induction by presence of increased inflammation (H&E) and fibrosis (Masson's Trichrome) in mice that received *P. acnes* (group 3 and 4) as compared to mice that did not receive *P. acnes* (group 2).

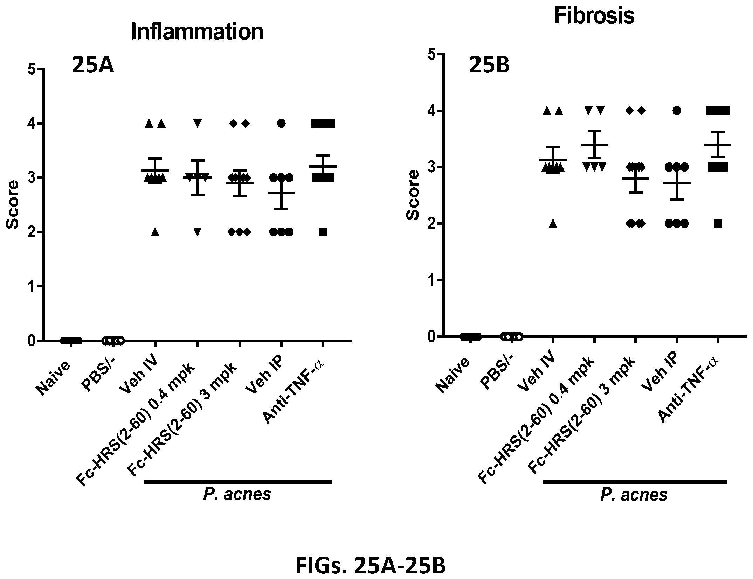

**Figures 25A-25B** show measurements of lung inflammation (25A) and fibrosis (25B) at study termination.

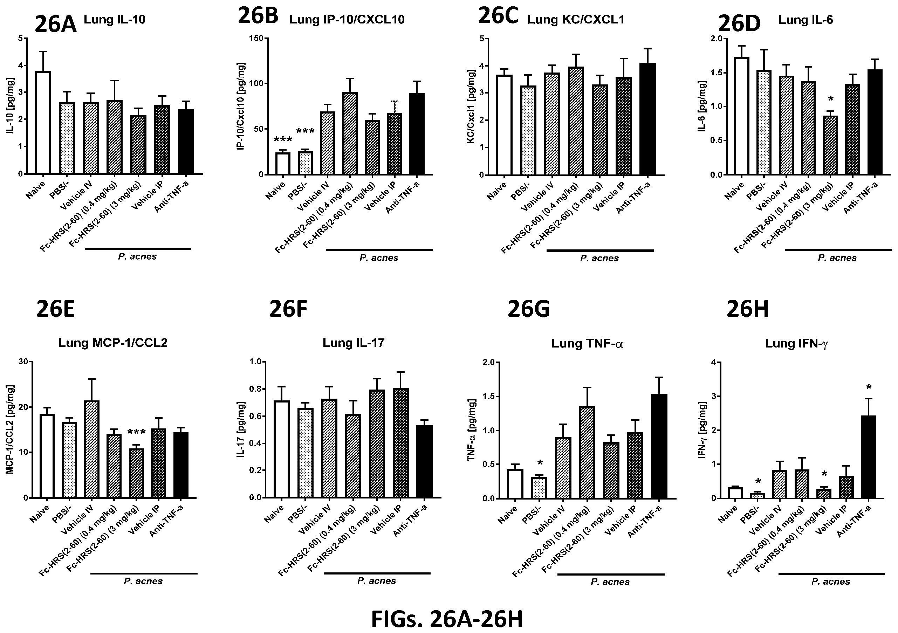

**Figures 26A-26H** show that several profibrotic cytokines in the lung were reduced in response to Fc-HRS(2-60) treatment at 3 mg/kg, as indicated.

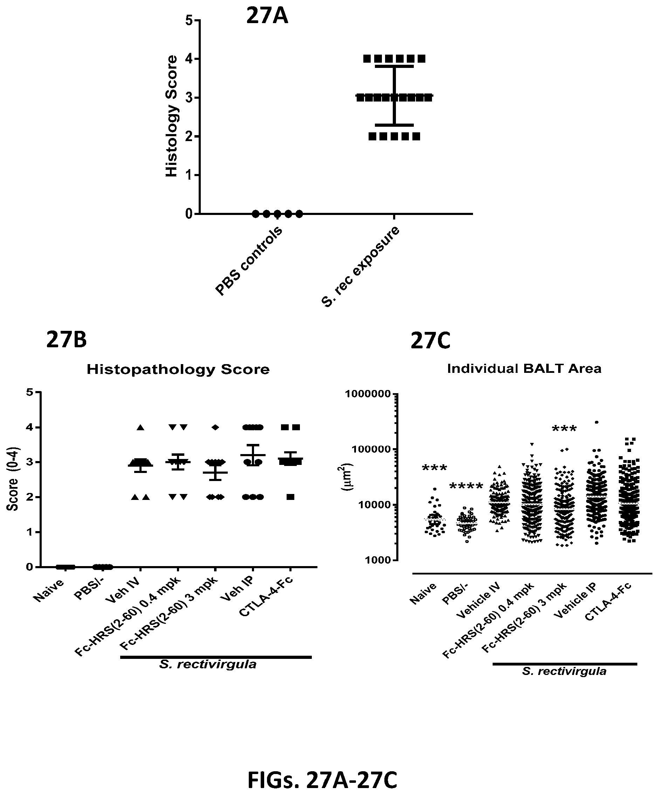

**Figure 27A** shows that mice exposed to S. *rectivirgula* in control groups (Group 3 and Group 4) had a robust and consistent multifocal chronic pneumonia compared to mice exposed to PBS in Group 2. **Figure 27B** shows histopathology scores as determined by a veterinary pathologist. **Figure 27C** shows a reduction of individual BALT area in the Fc-HRS(2-60) 3 mg/kg group following and in-depth analysis of the H&E stained lung tissue sections using the HALO platform.

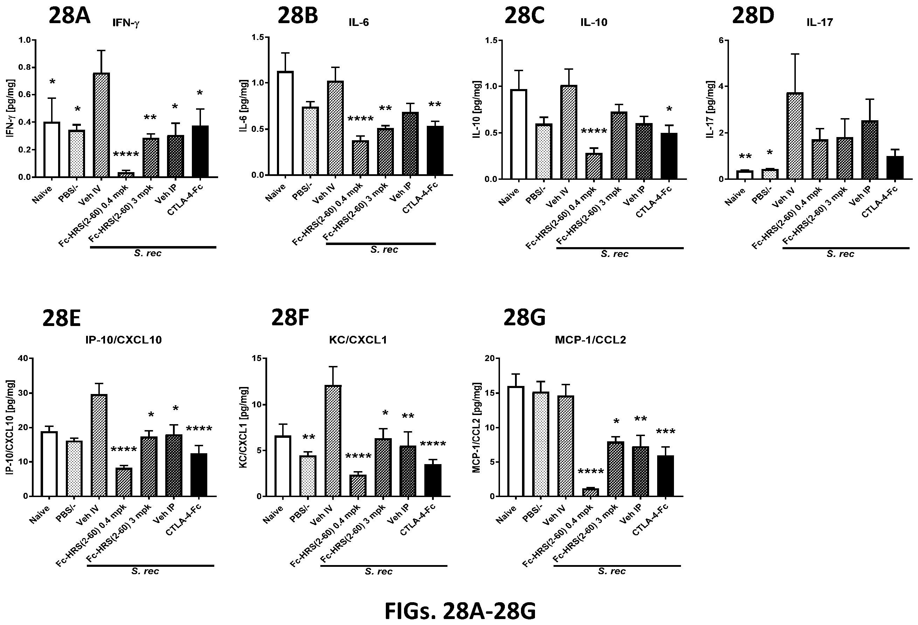

**Figures 28A-28G** show that several pro-inflammatory cytokines and chemokines were significantly reduced in presence of Fc-HRS(2-60) at both 0.4 and 3 mg/kg.

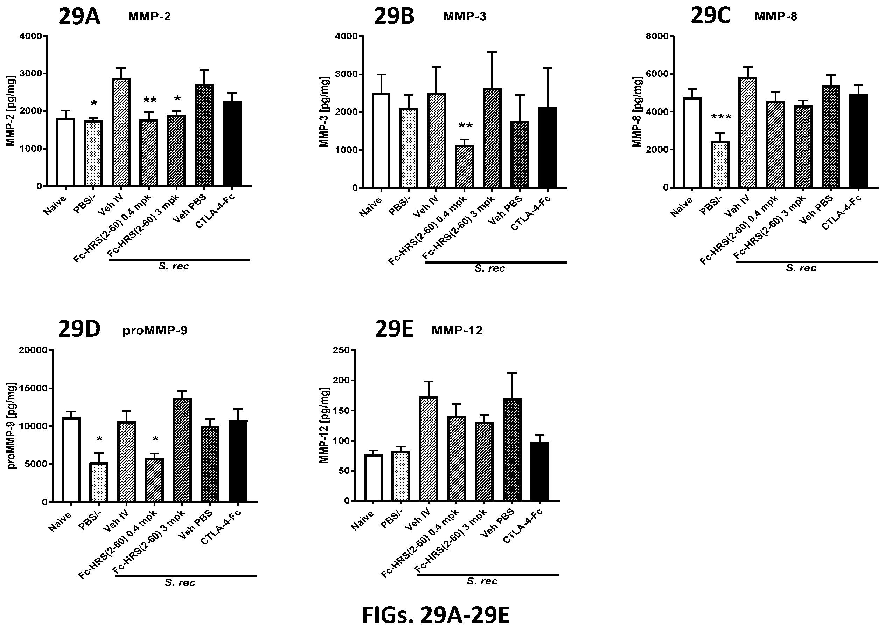

**Figures 29A-29E** show that matrix metalloproteases (MMPs) were significantly reduced in presence of Fc-HRS(2-60) at both 0.4 and 3 mg/kg.

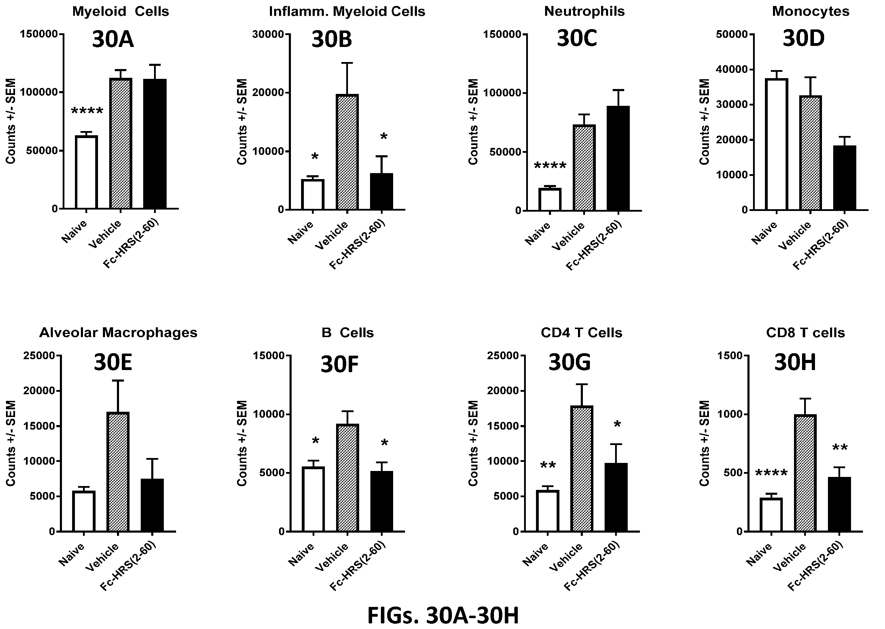

**Figures 30A-30H** show that inflammatory arthritis was successfully induced in SKG mice upon administration of 5 mg of zymosan, and that Fc-HRS(2-60) can lower the number of specific immune cells in the lungs of SKG mice; most notably B cells and T cells.

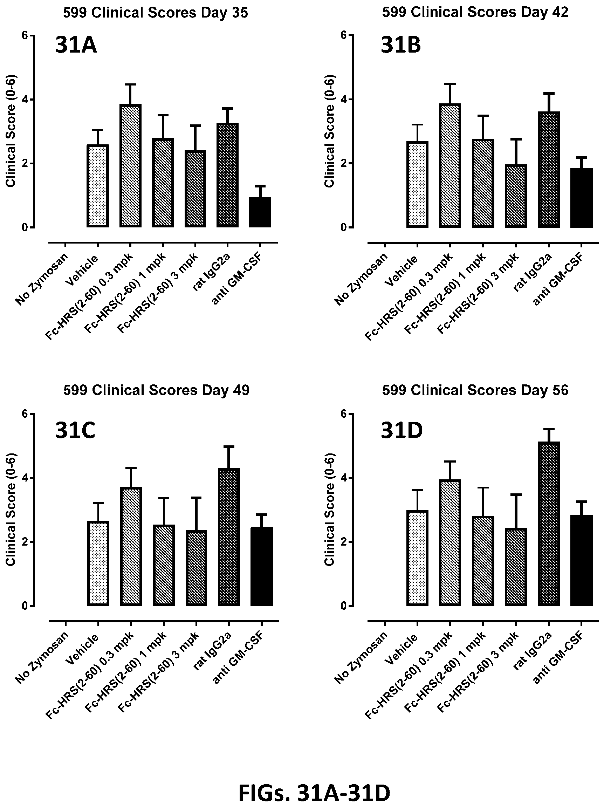

**Figures 31A-31D** show the overall clinical arthritis scores at days 35, 42, 49, and 56.

### DETAILED DESCRIPTION

The practice of the present invention will employ, unless indicated specifically to the contrary, conventional methods of molecular biology and recombinant DNA techniques within the skill of the art, many of which are described below for the purpose of illustration. Such techniques are explained fully in the literature. See, e.g., Sambrook, et al., Molecular Cloning: A Laboratory Manual (3rd Edition, 2000); DNA Cloning: A Practical Approach, vol. I & II (D. Glover, ed.); Oligonucleotide Synthesis (N. Gait, ed., 1984); Oligonucleotide Synthesis: Methods and Applications (P. Herdewijn, ed., 2004); Nucleic Acid Hybridization (B. Hames & S. Higgins, eds., 1985); Nucleic Acid Hybridization: Modern Applications (Buzdin and Lukyanov, eds., 2009); Transcription and Translation (B. Hames & S. Higgins, eds., 1984); Animal Cell Culture (R. Freshney, ed., 1986); Freshney, R.I. (2005) Culture of Animal Cells, a Manual of Basic Technique, 5th Ed. Hoboken NJ, John Wiley & Sons; B. Perbal, A Practical Guide to Molecular Cloning (3rd Edition 2010); Farrell, R., RNA Methodologies: A Laboratory Guide for Isolation and Characterization (3rd Edition 2005). Poly(ethylene glycol), Chemistry and Biological Applications, ACS, Washington, 1997; Veronese, F., and J.M. Harris, Eds., Peptide and protein PEGylation, Advanced Drug Delivery Reviews, 54(4) 453-609 (2002); Zalipsky, S., et al., "Use of functionalized Poly(Ethylene Glycols) for modification of polypeptides" in Polyethylene Glycol Chemistry: Biotechnical and Biomedical Applications.

### Definitions

Unless defined otherwise, all technical and scientific terms used herein have the same meaning as commonly understood by those of ordinary skill in the art to which the disclosure belongs. Although any methods, materials, compositions, reagents, cells, similar or equivalent similar or equivalent to those described herein can be used in the practice or testing of the subject matter of the present disclosure, preferred methods and materials are described.

For the purposes of the present disclosure, the following terms are defined below.

The articles "a" and "an" are used herein to refer to one or to more than one (i.e., to at least one) of the grammatical object of the article. By way of example, "an element" means one element or more than one element.

By "about" is meant a quantity, level, value, number, frequency, percentage, dimension, size, amount, weight or length that varies by as much as 30, 25, 20, 15, 10, 9, 8, 7, 6, 5, 4, 3, 2 or 1% to a reference quantity, level, value, number, frequency, percentage, dimension, size, amount, weight or length.

An "antagonist" or "inhibitor" refers to biological structure or chemical agent that interferes with or otherwise reduces the physiological action of another agent or molecule. In some instances, the antagonist specifically binds to the other agent or molecule. Included are full and partial antagonists.

An "agonist" refers to biological structure or chemical agent that increases or enhances the physiological action of another agent or molecule. In some instances, the agonist specifically binds to the other agent or molecule. Included are full and partial agonists.

The term "anergy" refers to the functional inactivation of a T cell, or B cell response to re-stimulation by antigen.

As used herein, the term "amino acid" is intended to mean both naturally occurring and non-naturally occurring amino acids as well as amino acid analogs and mimetics. Naturally occurring amino acids include the 20 (L)-amino acids utilized during protein biosynthesis as well as others such as 4-hydroxyproline, hydroxylysine, desmosine, isodesmosine, homocysteine, citrulline and ornithine, for example. Non-naturally occurring amino acids include, for example, (D)-amino acids, norleucine, norvaline, p-fluorophenylalanine, ethionine and the like, which are known to a person skilled in the art. Amino acid analogs include modified forms of naturally and non-naturally occurring amino acids. Such modifications can include, for example, substitution or replacement of chemical groups and moieties on the amino acid or by derivatization of the amino acid. Amino acid mimetics include, for example, organic structures which exhibit functionally similar properties such as charge and charge spacing characteristic of the reference amino acid. For example, an organic structure which mimics Arginine (Arg or R) would have a positive charge moiety located in similar molecular space and having the same degree of mobility as the e-amino group of the side chain of the naturally occurring Arg amino acid. Mimetics also include constrained structures so as to maintain optimal spacing and charge interactions of the amino acid or of the amino acid functional groups. Those skilled in the art know or can determine what structures constitute functionally equivalent amino acid analogs and amino acid mimetics.

As used herein, a subject "at risk" of developing a disease, or adverse reaction may or may not have detectable disease, or symptoms of disease, and may or may not have displayed detectable disease or symptoms of disease prior to the treatment methods described herein. "At risk" denotes that a subject has one or more risk factors, which are measurable parameters that correlate with development of a disease, as described herein and known in the art. A subject having one or more of these risk factors has a higher probability of developing disease, or an adverse reaction than a subject without one or more of these risk factor(s).

By "coding sequence" is meant any nucleic acid sequence that contributes to the code for the polypeptide product of a gene. By contrast, the term "non-coding sequence" refers to any nucleic acid sequence that does not directly contribute to the code for the polypeptide product of a gene.

The term "binding" refers to a direct association between two molecules, due to, for example, covalent, electrostatic, hydrophobic, and ionic and/or hydrogen-bond interactions, including interactions such as salt bridges and water bridges.

The term "clonal deletion" refers to the deletion (e.g., loss, or death) of auto-reactive T-cells. Clonal deletion can be achieved centrally in the thymus, or in the periphery, or both.

The term "chemoresistance" refers to the change in therapeutic sensitivity of a cancer cell population over time following exposure to cancer therapy, including resistance to at least one of a cancer immunotherapy agent, a chemotherapeutic agent, a hormonal therapeutic agent, and/or a kinase inhibitor. Eventually, chemoresistance leads to the relapse and/or metastasis of the cancer, and challenges the improvement of clinical outcome for the cancer patients. It remains the main obstacle to long term successful cancer therapy. For example, approximately 30 percent of women diagnosed with early-stage breast cancer develop chemoresistance and eventually progress to metastatic breast cancer. The molecular mechanisms of chemoresistance include the induction of transporter pumps, oncogenes, tumor suppressor genes, mitochondrial alteration, DNA repair, autophagy, epithelial-mesenchymal transition (EMT), cancer stemness, and exosome production. These mechanisms may operate via distinct mechanisms, but ultimately coordinate to prevent cell death in response to a chemotherapeutic agent. For example, the encoding proteins of oncogenes (EGFR-Akt-NF-κB) could modulate the expression of apoptosis-related genes, and thereby contribute to EMT, cell stemness, and autophagy. Autophagic cells are characterized by anti-apoptosis during chemoresistance. Accordingly, agents which reduce chemoresistance, including those that modulate autophagy, endosome maturation, phagocytosis, and/or efferocytosis, could find utility in the treatment or reduction of chemoresistant cancers.

Throughout this disclosure, unless the context requires otherwise, the words "comprise," "comprises," and "comprising" will be understood to imply the inclusion of a stated step or element or group of steps or elements but not the exclusion of any other step or element or group of steps or elements.

By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements.

The term "endotoxin free" or "substantially endotoxin free" relates generally to compositions, solvents, and/or vessels that contain at most trace amounts (e.g., amounts having no clinically adverse physiological effects to a subject) of endotoxin, and preferably undetectable amounts of endotoxin. Endotoxins are toxins associated with certain micro-organisms, such as bacteria, typically gram-negative bacteria, although endotoxins may be found in gram-positive bacteria, such as *Listeria monocytogenes.* The most prevalent endotoxins are lipopolysaccharides (LPS) or lipo-oligosaccharides (LOS) found in the outer membrane of various Gram-negative bacteria, and which represent a central pathogenic feature in the ability of these bacteria to cause disease. Small amounts of endotoxin in humans may produce fever, a lowering of the blood pressure, and activation of inflammation and coagulation, among other adverse physiological effects.

Therefore, in pharmaceutical production, it is often desirable to remove most or all traces of endotoxin from drug products and/or drug containers, because even small amounts may cause adverse effects in humans. A depyrogenation oven may be used for this purpose, as temperatures in excess of 300°C are typically required to break down most endotoxins. For instance, based on primary packaging material such as syringes or vials, the combination of a glass temperature of 250°C and a holding time of 30 minutes is often sufficient to achieve a 3 log reduction in endotoxin levels. Other methods of removing endotoxins are contemplated, including, for example, chromatography and filtration methods, as described herein and known in the art.

Endotoxins can be detected using routine techniques known in the art. For example, the Limulus Amoebocyte Lysate assay, which utilizes blood from the horseshoe crab, is a very sensitive assay for detecting presence of endotoxin. In this test, very low levels of LPS can cause detectable coagulation of the limulus lysate due a powerful enzymatic cascade that amplifies this reaction. Endotoxins can also be quantitated by enzyme-linked immunosorbent assay (ELISA). To be substantially endotoxin free, endotoxin levels may be less than about 0.001, 0.005, 0.01, 0.02, 0.03, 0.04, 0.05, 0.06, 0.08, 0.09, 0.1, 0.5, 1.0, 1.5, 2, 2.5, 3, 4, 5, 6, 7, 8, 9, or 10 EU/mg of active compound. Typically, 1 ng lipopolysaccharide (LPS) corresponds to about 1-10 EU.

As used herein, the terms "contacting a cell", "introducing" or "delivering" include delivery of the agents described herein (e.g., polypeptide agents, polynucleotide agents) into a cell by methods routine in the art, e.g., transfection (e.g., liposome, calcium-phosphate, polyethyleneimine), electroporation (e.g., nucleofection), microinjection) or administration to a subject.

The terms "cell penetrating peptide" (CPP) or "a peptide moiety which enhances cellular uptake" are used interchangeably and refer to cationic cell penetrating peptides, also called "transport peptides", "carrier peptides", or "peptide transduction domains." In some embodiments, the peptides have the capability of inducing cell (e.g., muscle cell) penetration within about or at least about 30%, 40%, 50%, 60%, 70%, 80%, 90%, or 100% of cells of a given cell culture population and allow macromolecular translocation within multiple tissues (e.g., muscle tissues) in vivo upon systemic or other form of administration. In some embodiments, the CPPs are of the formula -[(C(O)CHR'NH)ₘ]R" wherein R' is a side chain of a naturally occurring amino acid or a one- or two-carbon homolog thereof , R" is selected from Hydrogen or acyl, and m is an integer up to 50. Additional CPPs are well-known in the art and are disclosed, for example, in U.S. Application No. 2010/0016215. In some embodiments, m is an integer selected from 1 to 50 where, when m is 1, the moiety is a single amino acid or derivative thereof. Any of the polynucleotide agents (e.g., antisense, RNAi agents) described herein can be conjugated to a CPP, for example, to improve uptake into target cells, e.g., muscle cells.

The term "half maximal effective concentration" or "EC50" refers to the concentration of an agent (e.g., HRS polypeptide, or other agent) as described herein at which it induces a response halfway between the baseline and maximum after some specified exposure time; the EC50 of a graded dose response curve therefore represents the concentration of a compound at which 50% of its maximal effect is observed. EC50 also represents the plasma concentration required for obtaining 50% of a maximum effect in vivo. Similarly, the "EC90" refers to the concentration of an agent or composition at which 90% of its maximal effect is observed. The "EC90" can be calculated from the "EC50" and the Hill slope, or it can be determined from the data directly, using routine knowledge in the art. In some embodiments, the EC50 of an agent is less than about 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 40, 50, 60, 70, 80, 90, 100, 200 or 500 nM. In some embodiments, a biotherapeutic composition will have an EC50 value of about 1nM or less.

"Homology" refers to the percentage number of amino acids that are identical or constitute conservative substitutions. Homology may be determined using sequence comparison programs such as GAP (Deveraux et al., 1984, Nucleic Acids Research 12, 387-395). In this way sequences of a similar or substantially different length to those cited herein could be compared by insertion of gaps into the alignment, such gaps being determined, for example, by the comparison algorithm used by GAP.

The term "innate immune response" refers to the responses of immune cells (including myeloid derived cells, such as macrophages, neutrophils, eosinophils, granulocytes, and natural killer (NK) cells) and the associated mechanisms of modulating cytokine expression and release (e.g., interferons and interferon-signaling), inducing cell death, and inhibiting protein synthesis, which defend the host from infection by pathogens.

By "isolated" is meant material that is substantially or essentially free from components that normally accompany it in its native state. For example, an "isolated polynucleotide," "isolated oligonucleotide," or "isolated oligonucleotide" as used herein, may refer to a polynucleotide that has been purified or removed from the sequences that flank it in a naturally-occurring state, e.g., a DNA fragment that is removed from the sequences that are adjacent to the fragment in the genome. The term "isolating" as it relates to cells refers to the purification of cells (e.g., fibroblasts, lymphoblasts) from a source subject (e.g., a subject with a polynucleotide repeat disease). In the context of mRNA or protein, "isolating" refers to the recovery of mRNA or protein from a source, e.g., cells.

The term "migratory cells" refers to cells that are capable of movement from one place to another in response to a stimulus. Exemplary migratory cells include immune cells such as monocytes, Natural Killer (NK) cells, dendritic cells (immature or mature), subsets of dendritic cells including myeloid, plasmacytoid (also called lymphoid) and Langerhans cells, macrophages such as histiocytes, tissue resident macrophages such as Kupffer's cells, microglia cells in the CNS, alveolar macrophages, and peritoneal macrophages, macrophage subtypes such as M0, M1, Mox,M2a, M2b, and M2c macrophages, neutrophils, eosinophils, mast cells, basophils, B cells including plasma B cells, memory B cells, B-1 cells, and B-2 cells, CD45RO (naive T) cells, CD45RA (memory T) cells, CD4 Helper T Cells including Th1, Th2, and Tr1/Th3 cells, CD8 Cytotoxic T Cells, Regulatory T Cells, Gamma Delta T Cells, and thymocytes. Additional examples of migratory cells include fibroblasts, fibrocytes, tumor cells, and stem cells. Accordingly the term "cell migration", refers to the movement of migratory cells, and the term "modulation of cell migration" refers to the modulation of the movement of any such migratory cells.

The terms "modulate" includes to "increase" or "decrease" one or more quantifiable parameters, optionally by a defined and/or statistically significant amount. By "increase" or "increasing," "enhance" or "enhancing," or "stimulate" or "stimulating," refers generally to the ability of one or more agents or compositions to produce or cause a greater physiological response (i.e., downstream effects) in a cell or a subject relative to the response caused by either no agent/compound or a control compound. Relevant physiological or cellular responses (in vivo or in vitro) will be apparent to persons skilled in the art, and may include increases in skeletal muscle mass in a tissue or subject in need thereof. An "increased" or "enhanced" amount is typically a "statistically significant" amount, and may include an increase that is 1.1, 1.2, 2, 3, 4, 5, 6, 7, 8, 9, 10, 15, 20, 30, 40, 50 or more times (e.g., 500, 1000 times), including all integers and decimal points in between and above 1 (e.g., 1.5, 1.6, 1.7. 1.8), the amount produced by no agent/compound (the absence of an agent) or a control compound. The term "reduce" or "inhibit" may relate generally to the ability of one or more agents or compositions to "decrease" a relevant physiological or cellular response, such as expression of a target gene or a symptom of a disease or condition described herein, as measured according to routine techniques in the diagnostic art. Relevant physiological or cellular responses (in vivo or in vitro) will be apparent to persons skilled in the art, and may include reductions or improvements in the symptoms or pathology of lung inflammation or an ILD, as described herein. A "decrease" in a response may be "statistically significant" as compared to the response produced by no agent or composition or a control agent or composition, and may include a 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 55%, 60%, 65%, 70%, 75%, 80%, 85%, 90%, 95%, or 100% decrease, including all integers in between.

**In** certain embodiments, the "purity" of any given agent in a composition may be specifically defined. For instance, certain compositions may comprise an agent that is at least 80, 85, 90, 91, 92, 93, 94, 95, 96, 97, 98, 99, or 100% pure, including all decimals in between, as measured, for example and by no means limiting, by high performance liquid chromatography (HPLC), a well-known form of column chromatography used frequently in biochemistry and analytical chemistry to separate, identify, and quantify compounds.

A "lipid nanoparticle" or "solid lipid nanoparticle" refers to one or more spherical nanoparticles with an average diameter of between about 10 to about 1000 nanometers, and which comprise a solid lipid core matrix that can solubilize lipophilic molecules. The lipid core is stabilized by surfactants (e.g., emulsifiers), and can comprise one or more of triglycerides (e.g., tristearin), diglycerides (e.g., glycerol bahenate), monoglycerides (e.g., glycerol monostearate), fatty acids (e.g., stearic acid), steroids (e.g., cholesterol), and waxes (e.g., cetyl palmitate), including combinations thereof. Lipid nanoparticles are described, for example, in Petrilli et al., Curr Pharm Biotechnol. 15:847-55, 2014; and U.S. Patent Nos. 6,217,912; 6,881,421; 7,402,573; 7,404,969; 7,550,441; 7,727,969; 8,003,621; 8,691,750; 8,871,509; 9,017,726; 9,173,853; 9,220,779; 9,227,917; and 9,278,130.

The terms or "Neuropilin 2-associated disease" or "NRP2-associated disease" refer to diseases and conditions in which NRP2 activity, expression, and/or spatial distribution plays a role in the pathophysiology of that disease or condition. In some instances, NRP2 associated diseases are modulated by the HRS polypeptides of the present disclosure, for example, by altering the interaction of NRP2 with at least one NRP2 ligand, to impact NRP2 activity, signaling, expression, and/or spatial distribution. Exemplary NRP2-associated diseases and conditions include, without limitation, cancer and diseases or pathologies associated with cancer, including cancer cell growth, cancer initiation, cancer migration, cancer cell adhesion, cancer cell invasion, cancer cell chemoresistance, and cancer cell metastasis. Also included are diseases associated with inflammation and autoimmunity, including inflammatory lung diseases such as chronic hypersensitivity pneumonitis, pulmonary inflammation, and related inflammatory diseases. Also included are diseases associated with inappropriate immune cell activation or migration such as graft versus host disease (GVHD) and rheumatoid arthritis-associated interstitial lung disease (RA-ILD). Additional examples include diseases associated with lymphatic development, lymphangiogenesis, and lymphatic damage, including edema, lymphedema, secondary lymphedema, inappropriate fat absorption and deposition, excess fat deposition, and vascular permeability. Also included are diseases associated with infections, including latent infections, and diseases associated with allergic disorders/diseases and allergic responses, including chronic obstructive pulmonary disorder (COPD), neutrophilic asthma, antineutrophil cytoplasmic antibody (ANCA)-associated systemic vasculitis, systemic lupus erythematosus, rheumatoid arthritis, inflammasome-related disease(s), and skin-related neutrophil-mediated disease(s) such as pyoderma gangrenosum. Additional examples include diseases associated with granulomatous inflammatory diseases, including sarcoidosis and other pulmonary granulomatous diseased, and non-lung granulomas. Also included are fibrotic diseases such endometriosis, fibrosis, endothelial to mesenchymal transition (EMT), and wound healing, among others. Also included are diseases associated with inappropriate smooth muscle contractility and vascular smooth muscle cell migration and/or adhesion, and diseases associated with inappropriate autophagy, phagocytosis, and efferocytosis. Additional examples include neuronal diseases, including diseases associated with peripheral nervous system remodeling and pain perception. Also included are diseases associated with bone development and/or bone remodeling, and diseases associated with inappropriate migratory cell movement.

As used herein, "nucleobase" (Nu), "base pairing moiety" or "base" are used interchangeably to refer to a purine or pyrimidine base found in native DNA or RNA (uracil, thymine, adenine, cytosine, and guanine), as well as analogs of the naturally occurring purines and pyrimidines, that confer improved properties, such as binding affinity to the oligonucleotide. Exemplary analogs include hypoxanthine (the base component of the nucleoside inosine); 2, 6-diaminopurine; 5-methyl cytosine; C5-propynyl-modifed pyrimidines; 9-(aminoethoxy)phenoxazine (G-clamp) and the like.

Further examples of base pairing moieties include, but are not limited to, uracil, thymine, adenine, cytosine, guanine and hypoxanthine having their respective amino groups protected by acyl protecting groups, 2-fluorouracil, 2-fluorocytosine, 5-bromouracil, 5-iodouracil, 2,6-diaminopurine, azacytosine, pyrimidine analogs such as pseudoisocytosine and pseudouracil and other modified nucleobases such as 8-substituted purines, xanthine, or hypoxanthine (the latter two being the natural degradation products). The modified nucleobases disclosed in Chiu and Rana, RNA, 2003, 9, 1034-1048, Limbach et al. Nucleic Acids Research, 1994, 22, 2183-2196 and Revankar and Rao, Comprehensive Natural Products Chemistry, vol. 7, 313, are also contemplated.

Further examples of base pairing moieties include, but are not limited to, expanded-size nucleobases in which one or more benzene rings has been added. Nucleic base replacements described in the Glen Research catalog (www.glenresearch.com); Krueger AT et al, Acc. Chem. Res., 2007, 40, 141-150; Kool, ET, Acc. Chem. Res., 2002, 35, 936-943; Benner S.A., et al., Nat. Rev. Genet., 2005, 6, 553-543; Romesberg, F.E., et al., Curr. Opin. Chem. Biol., 2003, 7, 723-733; Hirao, I., Curr. Opin. Chem. Biol., 2006, 10, 622-627, are contemplated as useful for the synthesis of the oligonucleotides described herein. Examples of expanded-size nucleobases are shown below:

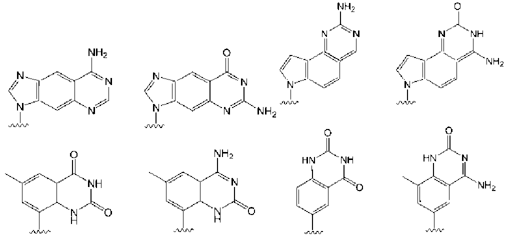

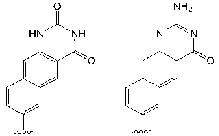

A nucleobase covalently linked to a ribose, sugar analog or morpholino comprises a nucleoside. "Nucleotides" are composed of a nucleoside together with one phosphate group. The phosphate groups covalently link adjacent nucleotides to one another to form an oligonucleotide.

The terms "polypeptide" and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers.

The term "polynucleotide" and "nucleic acid" includes mRNA, RNA, cRNA, cDNA, and DNA. The term typically refers to polymeric form of nucleotides of at least 10 bases in length, either ribonucleotides or deoxynucleotides or a modified form of either type of nucleotide. The term includes single and double stranded forms of DNA. The terms "isolated DNA" and "isolated polynucleotide" and "isolated nucleic acid" refer to a molecule that has been isolated free of total genomic DNA of a particular species. Therefore, an isolated DNA segment encoding a polypeptide refers to a DNA segment that contains one or more coding sequences yet is substantially isolated away from, or purified free from, total genomic DNA of the species from which the DNA segment is obtained. Also included are non-coding polynucleotides (e.g., primers, probes, oligonucleotides), which do not encode a polypeptide. Also included are recombinant vectors, including, for example, expression vectors, viral vectors, plasmids, cosmids, phagemids, phage, viruses, and the like.

Additional coding or non-coding sequences may, but need not, be present within a polynucleotide described herein, and a polynucleotide may, but need not, be linked to other molecules and/or support materials. Hence, a polynucleotide or expressible polynucleotides, regardless of the length of the coding sequence itself, may be combined with other sequences, for example, expression control sequences.

"Expression control sequences" include regulatory sequences of nucleic acids, or the corresponding amino acids, such as promoters, leaders, enhancers, introns, recognition motifs for RNA, or DNA binding proteins, polyadenylation signals, terminators, internal ribosome entry sites (IRES), secretion signals, subcellular localization signals, and the like, which have the ability to affect the transcription or translation, or subcellular, or cellular location of a coding sequence in a host cell. Exemplary expression control sequences are described in Goeddel; Gene Expression Technology: Methods in Enzymology 185, Academic Press, San Diego, Calif. (1990).

A "promoter" is a DNA regulatory region capable of binding RNA polymerase in a cell and initiating transcription of a downstream (3' direction) coding sequence. As used herein, the promoter sequence is bounded at its 3' terminus by the transcription initiation site and extends upstream (5' direction) to include the minimum number of bases or elements necessary to initiate transcription at levels detectable above background. A transcription initiation site (conveniently defined by mapping with nuclease S1) can be found within a promoter sequence, as well as protein binding domains (consensus sequences) responsible for the binding of RNA polymerase. Eukaryotic promoters can often, but not always, contain "TATA" boxes and "CAT" boxes. Prokaryotic promoters contain Shine- Dalgarno sequences in addition to the -10 and -35 consensus sequences.

A large number of promoters, including constitutive, inducible and repressible promoters, from a variety of different sources are well known in the art. Representative sources include for example, viral, mammalian, insect, plant, yeast, and bacterial cell types), and suitable promoters from these sources are readily available, or can be made synthetically, based on sequences publicly available on line or, for example, from depositories such as the ATCC as well as other commercial or individual sources. Promoters can be unidirectional (i.e., initiate transcription in one direction) or bidirectional (i.e., initiate transcription in either a 3' or 5' direction). Non-limiting examples of promoters include, for example, the T7 bacterial expression system, pBAD (araA) bacterial expression system, the cytomegalovirus (CMV) promoter, the SV40 promoter, the RSV promoter. Inducible promoters include the Tet system, (US Patents 5,464,758 and 5,814,618), the Ecdysone inducible system (No et al., Proc. Natl. Acad. Sci. (1996) 93 (8): 3346-3351; the T-RExTM system (Invitrogen Carlsbad, CA), LacSwitch^{®} (Stratagene, (San Diego, CA) and the Cre-ERT tamoxifen inducible recombinase system (Indra et al. Nuc. Acid. Res. (1999) 27 (22): 4324-4327; Nuc. Acid. Res. (2000) 28 (23): e99; US Patent No. 7,112,715; and Kramer & Fussenegger, Methods Mol. Biol. (2005) 308: 123-144) or any promoter known in the art suitable for expression in the desired cells.

An "expressible polynucleotide" includes a cDNA, RNA, mRNA or other polynucleotide that comprises at least one coding sequence and optionally at least one expression control sequence, for example, a transcriptional and/or translational regulatory element, and which can express an encoded polypeptide (for example, an HRS polypeptide) upon introduction into a cell, for example, a cell in a subject.

In some embodiments, the expressible polynucleotide is a modified RNA or modified mRNA polynucleotide, for example, a non-naturally occurring RNA analog. In certain embodiments, the modified RNA or mRNA polypeptide comprises one or more modified or non-natural bases, for example, a nucleotide base other than adenine (A), guanine (G), cytosine (C), thymine (T), and/or uracil (U). In some embodiments, the modified mRNA comprises one or more modified or non-natural internucleotide linkages. Expressible RNA polynucleotides for delivering an encoded therapeutic polypeptide are described, for example, in Kormann et al., Nat Biotechnol. 29:154-7, 2011; and U.S. Application Nos. 2015/0111248; 2014/0243399; 2014/0147454; and 2013/0245104.

In some embodiments, various viral vectors that can be utilized to deliver an expressible polynucleotide include adenoviral vectors, herpes virus vectors, vaccinia virus vectors, adeno-associated virus (AAV) vectors, and retroviral vectors. In some instances, the retroviral vector is a derivative of a murine or avian retrovirus, or is a lentiviral vector. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), SIV, BIV, HIV and Rous Sarcoma Virus (RSV). A number of additional retroviral vectors can incorporate multiple genes. All of these vectors can transfer or incorporate a gene for a selectable marker so that transduced cells can be identified and generated. By inserting a polypeptide sequence of interest into the viral vector, along with another gene that encodes the ligand for a receptor on a specific target cell, for example, the vector may be made target specific. Retroviral vectors can be made target specific by inserting, for example, a polynucleotide encoding a protein. Illustrative targeting may be accomplished by using an antibody to target the retroviral vector. Those of skill in the art will know of, or can readily ascertain without undue experimentation, specific polynucleotide sequences which can be inserted into the retroviral genome to allow target specific delivery of the retroviral vector.

In certain instances, the expressible polynucleotides described herein are engineered for localization within a cell, potentially within a specific compartment such as the nucleus, or are engineered for secretion from the cell or translocation to the plasma membrane of the cell. In exemplary embodiments, the expressible polynucleotides are engineered for nuclear localization.

Also included are biologically active "variants" and "fragments" of the polypeptides described herein, and the polynucleotides that encode the same. "Variants" contain one or more substitutions, additions, deletions, and/or insertions relative to a reference polypeptide or polynucleotide (see, e.g., the Tables and the Sequence Listing). A variant polypeptide or polynucleotide comprises an amino acid or polynucleotide sequence with at least about 50%, 55%, 60%, 65%, 70%, 75%, 80%, 85%, 90%, 91%, 92%, 93%, 94%, 95%, 96%, 97%, 98% , 99% or more sequence identity or similarity or homology to a reference sequence, as described herein, and substantially retains the activity of that reference sequence. Also included are sequences that consist of or differ from a reference sequences by the addition, deletion, insertion, or substitution of 1, 2, 3, 4, 5, 6, 7, 8, 9, 10, 11, 12, 13, 14, 15, 16, 17, 18, 19, 20, 30, 40, 50, 60,70, 80, 90, 100, 110, 120, 130, 140, 150 or more amino acids or nucleotides and which substantially retain the activity of that reference sequence. In certain embodiments, the additions or deletions include C-terminal and/or N-terminal additions and/or deletions.

The terms "sequence identity" or, for example, comprising a "sequence 50% identical to," as used herein, refer to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, Wis., USA) or by inspection and the best alignment (i.e., resulting in the highest percentage homology over the comparison window) generated by any of the various methods selected. Reference also may be made to the BLAST family of programs as for example disclosed by Altschul et al., Nucl. Acids Res. 25:3389, 1997.

By "statistically significant", it is meant that the result was unlikely to have occurred by chance. Statistical significance can be determined by any method known in the art. Commonly used measures of significance include the p-value, which is the frequency or probability with which the observed event would occur, if the null hypothesis were true. If the obtained p-value is smaller than the significance level, then the null hypothesis is rejected. In simple cases, the significance level is defined at a p-value of 0.05 or less.

The term "solubility" refers to the property of an agent provided herein to dissolve in a liquid solvent and form a homogeneous solution. Solubility is typically expressed as a concentration, either by mass of solute per unit volume of solvent (g of solute per kg of solvent, g per dL (100 mL), mg/ml, etc.), molarity, molality, mole fraction or other similar descriptions of concentration. The maximum equilibrium amount of solute that can dissolve per amount of solvent is the solubility of that solute in that solvent under the specified conditions, including temperature, pressure, pH, and the nature of the solvent. In certain embodiments, solubility is measured at physiological pH, or other pH, for example, at pH 5.0, pH 6.0, pH 7.0, or pH 7.4. In certain embodiments, solubility is measured in water or a physiological buffer such as PBS or NaCl (with or without NaP). In specific embodiments, solubility is measured at relatively lower pH (e.g., pH 6.0) and relatively higher salt (e.g., 500mM NaCl and 10mM NaP). In certain embodiments, solubility is measured in a biological fluid (solvent) such as blood or serum. In certain embodiments, the temperature can be about room temperature (e.g., about 20, 21, 22, 23, 24, 25°C) or about body temperature (37°C). In certain embodiments, an agent has a solubility of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 40, 50, 60, 70, 80, 90 or 100 mg/ml at room temperature or at 37°C.

A "subject" or a "subject in need thereof" includes a mammalian subject such as a human subject.

"Substantially" or "essentially" means nearly totally or completely, for instance, 95% or greater of some given quantity.

"Therapeutic response" refers to improvement of symptoms (whether or not sustained) based on the administration of the therapeutic response.

As used herein, the term "target" refers to a RNA region, and specifically, to a RNA region of a target gene described herein. The target can include coding and non-coding sequences, 5' upstream sequences, 3' downstream sequences, and other RNA sequences described herein.

The term "target sequence" refers to a portion of the target RNA against which the antisense or RNAi agent is directed, for example, the sequence to which the antisense oligonucleotide will hybridize by Watson-Crick base pairing of a complementary sequence, or the sequence that corresponds to the sense strand of the RNAi agent.

As used herein, the term "quantifying", "quantification" or other related words refer to determining the quantity, mass, or concentration in a unit volume, of a nucleic acid, polynucleotide, oligonucleotide, peptide, polypeptide, or protein.

As used herein, the terms "therapeutically effective amount", "therapeutic dose," "prophylactically effective amount," or "diagnostically effective amount" is the amount of an agent needed to elicit the desired biological response following administration. Similarly the term "antisense therapy" or "RNAi therapy" includes a therapy that maintains the average steady state concentration of an antisense or RNAi agent in the patient's plasma or other tissue compartment (e.g., muscle tissue) above the minimum effective therapeutic level.

As used herein, "treatment" of a subject (e.g. a mammal, such as a human) or a cell is any type of intervention used in an attempt to alter the natural course of the subject or cell. Treatment includes, but is not limited to, administration of a pharmaceutical composition, and may be performed either prophylactically or subsequent to the initiation of a pathologic event or contact with an etiologic agent. Also included are "prophylactic" treatments, which can be directed to reducing the rate of progression of the disease or condition being treated, delaying the onset of that disease or condition, or reducing the severity of its onset. "Treatment" or "prophylaxis" does not necessarily indicate complete eradication, cure, or prevention of the disease or condition, or associated symptoms thereof.

The term "wild-type" refers to a gene or gene product (e.g., a polypeptide) that is most frequently observed in a population and is thus arbitrarily designed the "normal" or "wild-type" form of the gene.

### Histidyl-tRNA Synthetase (HRS) Polypeptides

Certain embodiments include histidyl-tRNA synthetase polypeptides ("HRS" or "HisRS" polypeptides), including conjugates (e.g., fusion proteins, Fc conjugates, Fc fusion proteins, COMP conjugates, COMP fusion proteins), variants, and fragments thereof. Histidyl-tRNA synthetases belong to the class II tRNA synthetase family, which has three highly conserved sequence motifs. Class I and II tRNA synthetases are widely recognized as being responsible for the specific attachment of an amino acid to its cognate tRNA in a two-step reaction: the amino acid (AA) is first activated by ATP to form AA-AMP and then transferred to the acceptor end of the tRNA. The full-length histidyl-tRNA synthetases typically exist either as a cytosolic homodimer, or an alternatively spliced mitochondrial form.

Certain biological fragments or alternatively spliced isoforms of eukaryotic histidyl-tRNA synthetases, or in some contexts the intact full-length synthetase, modulate certain therapeutically relevant cell-signaling pathways, bind to one or more neuropilin polypeptides (see, for example, **Table N1),** and/or have anti-inflammatory properties. These activities, which are distinct from the classical role of tRNA synthetases in protein synthesis, are referred to herein as "non-canonical activities." For example, as provided herein, HRS polypeptides such as the N-terminal region of histidyl-tRNA synthetase (e.g., HRS 1-48, HRS 1-60) are capable, inter alia, of binding to a neuropilin polypeptide, and thereby modulating the migration, activation, and/or differentiation of inflammatory cells or migratory cells, and treating neuropilin-associated disease. In addition, certain fragments, splice variants, mutations, and/or deletions (e.g., HRS 1-60) relative to the full-length HRS polypeptide sequence confer increased activities and/or improved pharmacological properties. The sequences of certain exemplary HRS polypeptides (not part of the invention) are provided in **Table H1** below.

| **Table H1. Exemplary HRS polypeptides (not part of the invention)** | | | | |
|---|---|---|---|---|
| **Name** | **Residues** | **Sequence** | | **SEQ ID NO:** |
| FL cytosolic wild type | 1-509 | | | **1** |
| HRS (1-500) | 1-500 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKR | | 2 |
| HRS (1-501) | 1-501 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRR | | 3 |
| HRS (1-502) | 1-502 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRT | | 4 |
| HRS(1-503) | 1-503 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTG | | 5 |
| HRS (1-504) | 1-504 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQ | | 6 |
| HRS (1-505) | 1-505 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQP | | 172 |
| HisRS1^{N8} HRS (1-506) | 1-506 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQPL | | 7 |
| HRS (2-506) | 2-506 | AERAALEELVKLQGERVRGLKQQKASAELIEEEVAKLLKL KAQLGPDESKQKFVLKTPKGTRDYSPRQMAVREKVFDVII RCFKRHGAEVIDTPVFELKETLMGKYGEDSKLIYDLKDQG GELLSLRYDLTVPFARYLAMNKLTNIKRYHIAKVYRRDNP AMTRGRYREFYQCDFDIAGNFDPMIPDAECLKIMCEILSS LQIGDFLVKVNDRRILDGMFAICGVSDSKFRTICSSVDKL DKVSWEEVKNEMVGEKGLAPEVADRIGDYVQQHGGVSLVE QLLQDPKLSQNKQALEGLGDLKLLFEYLTLFGIDDKISFD LSLARGLDYYTGVIYEAVLLQTPAQAGEEPLGVGSVAAGG RYDGLVGMFDPKGRKVPCVGLSIGVERIFSIVEQRLEALE EKIRTTETQVLVASAQKKLLEERLKLVSELWDAGIKAELL YKKNPKLLNQLQYCEEAGIPLVAIIGEQELKDGVIKLRSV TSREEVDVRREDLVEEIKRRTGQPL | | 8 |
| HRS (1-507) | 1-507 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQPLC | | 9 |
| HRS (1-508) | 1-508 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQPLCI | | 10 |
| HisRS1^{N6} HRS (1-48) | 1-48 | MAERAALEELVKLQGERVRGLKQQKASAELIEEEVAKLLK LKAQLGPD | | 11 |
| | 1-80 | MAERAALEELVKLQGERVRGLKQQKASAELIEEEVAKLLK LKAQLGPDESKQKFVLKTPKGTRDYSPRQMAVREKVFDVI | | 12 |
| | 1-79 | MAERAALEELVKLQGERVRGLKQQKASAELIEEEVAKLLK LKAQLGPDESKQKFVLKTPKGTRDYSPRQMAVREKVFDV | | 13 |
| | 1-78 | MAERAALEELVKLQGERVRGLKQQKASAELIEEEVAKLLK LKAQLGPDESKQKFVLKTPKGTRDYSPRQMAVREKVFD | | 14 |
| | 1-77 | MAERAALEELVKLQGERVRGLKQQKASAELIEEEVAKLLK LKAQLGPDESKQKFVLKTPKGTRDYSPRQMAVREKVF | | 15 |
| | 1-76 | MAERAALEELVKLQGERVRGLKQQKASAELIEEEVAKLLK LKAQLGPDESKQKFVLKTPKGTRDYSPRQMAVREKV | | 16 |
| | 1-75 | MAERAALEELVKLQGERVRGLKQQKASAELIEEEVAKLLK LKAQLGPDESKQKFVLKTPKGTRDYSPRQMAVREK | | 17 |
| | 1-74 | MAERAALEELVKLQGERVRGLKQQKASAELIEEEVAKLLK LKAQLGPDESKQKFVLKTPKGTRDYSPRQMAVRE | | 18 |
| | 1-73 | MAERAALEELVKLQGERVRGLKQQKASAELIEEEVAKLLK LKAQLGPDESKQKFVLKTPKGTRDYSPRQMAVR | | 19 |
| | 1-72 | MAERAALEELVKLQGERVRGLKQQKASAELIEEEVAKLLK LKAQLGPDESKQKFVLKTPKGTRDYSPRQMAV | | 20 |
| | 1-71 | MAERAALEELVKLQGERVRGLKQQKASAELIEEEVAKLLK LKAQLGPDESKQKFVLKTPKGTRDYSPRQMA | | 21 |
| | 1-70 | MAERAALEELVKLQGERVRGLKQQKASAELIEEEVAKLLK LKAQLGPDESKQKFVLKTPKGTRDYSPRQM | | 22 |
| | 1-69 | MAERAALEELVKLQGERVRGLKQQKASAELIEEEVAKLLK LKAQLGPDESKQKFVLKTPKGTRDYSPRQ | | 23 |
| | 1-68 | MAERAALEELVKLQGERVRGLKQQKASAELIEEEVAKLLK LKAQLGPDESKQKFVLKTPKGTRDYSPR | | 24 |
| | 1-67 | MAERAALEELVKLQGERVRGLKQQKASAELIEEEVAKLLK LKAQLGPDESKQKFVLKTPKGTRDYSP | | 25 |
| | 1-66 | MAERAALEELVKLQGERVRGLKQQKASAELIEEEVAKLLK LKAQLGPDESKQKFVLKTPKGTRDYS | | 26 |
| | 1-65 | MAERAALEELVKLQGERVRGLKQQKASAELIEEEVAKLLK LKAQLGPDESKQKFVLKTPKGTRDY | | 27 |
| | 1-64 | MAERAALEELVKLQGERVRGLKQQKASAELIEEEVAKLLK LKAQLGPDESKQKFVLKTPKGTRD | | 28 |
| | 1-63 | MAERAALEELVKLQGERVRGLKQQKASAELIEEEVAKLLK LKAQLGPDESKQKFVLKTPKGTR | | 29 |
| | 1-62 | MAERAALEELVKLQGERVRGLKQQKASAELIEEEVAKLLK LKAQLGPDESKQKFVLKTPKGT | | 30 |
| | 1-61 | MAERAALEELVKLQGERVRGLKQQKASAELIEEEVAKLLK LKAQLGPDESKQKFVLKTPKG | | 31 |
| | 1-60 | MAERAALEELVKLQGERVRGLKQQKASAELIEEEVAKLLK LKAQLGPDESKQKFVLKTPK | | 32 |
| | 1-59 | MAERAALEELVKLQGERVRGLKQQKASAELIEEEVAKLLLK LKAQLGPDESKQKFVLKTP | | 33 |
| | 1-58 | MAERAALEELVKLQGERVRGLKQQKASAELIEEEVAKLLK LKAQLGPDESKQKFVLKT | | 34 |
| | 1-57 | MAERAALEELVKLQGERVRGLKQQKASAELIEEEVAKLLK LKAQLGPDESKQKFVLK | | 35 |
| | 1-56 | MAERAALEELVKLQGERVRGLKQQKASAELIEEEVAKLLK LKAQLGPDESKQKFVL | | 36 |
| | 1-55 | MAERAALEELVKLQGERVRGLKQQKASAELIEEEVAKLLK LKAQLGPDESKQKFV | | 37 |
| | 1-54 | MAERAALEELVKLQGERVRGLKQQKASAELIEEEVAKLLK LKAQLGPDESKQKF | | 38 |
| | 1-53 | MAERAALEELVKLQGERVRGLKQQKASAELIEEEVAKLLK LKAQLGPDESKQK | | 39 |
| | 1-52 | MAERAALEELVKLQGERVRGLKQQKASAELIEEEVAKLLK LKAQLGPDESKQ | | 40 |
| | 1-51 | MAERAALEELVKLQGERVRGLKQQKASAELIEEEVAKLLK LKAQLGPDESK | | 41 |
| | 1-50 | MAERAALEELVKLQGERVRGLKQQKASAELIEEEVAKLLK LKAQLGPDES | | 42 |
| | 1-49 | MAERAALEELVKLQGERVRGLKQQKASAELIEEEVAKLLK LKAQLGPDE | | 43 |
| | 1-48 | MAERAALEELVKLQGERVRGLKQQKASAELIEEEVAKLLK LKAQLGPD | | 44 |
| | 1-47 | MAERAALEELVKLQGERVRGLKQQKASAELIEEEVAKLLK LKAQLGP | | 45 |
| | 1-46 | MAERAALEELVKLQGERVRGLKQQKASAELIEEEVAKLLLK LKAQLG | | 46 |
| | 1-45 | MAERAALEELVKLQGERVRGLKQQKASAELIEEEVAKLLK LKAQL | | 47 |
| | 1-44 | MAERAALEELVKLQGERVRGLKQQKASAELIEEEVAKLLK LKAQ | | 48 |
| | 1-43 | MAERAALEELVKLQGERVRGLKQQKASAELIEEEVAKLLK LKA | | 49 |
| | 1-42 | MAERAALEELVKLQGERVRGLKQQKASAELIEEEVAKLLK LK | | 50 |
| | 1-41 | MAERAALEELVKLQGERVRGLKQQKASAELIEEEVAKLLK L | | 51 |
| | 1-40 | MAERAALEELVKLQGERVRGLKQQKASAELIEEEVAKLLK | | 52 |
| | 2-80 | AERAALEELVKLQGERVRGLKQQKASAELIEEEVAKLLKL KAQLGPDESKQKFVLKTPKGTRDYSPRQMAVREKVFDVI | | 53 |
| | 3-80 | ERAALEELVKLQGERVRGLKQQKASAELIEEEVAKLLKLK AQLGPDESKQKFVLKTPKGTRDYSPRQMAVREKVFDVI | | 54 |
| | 4-80 | RAALEELVKLQGERVRGLKQQKASAELIEEEVAKLLKLKA QLGPDESKQKFVLKTPKGTRDYSPRQMAVREKVFDVI | | 55 |
| | 5-80 | AALEELVKLQGERVRGLKQQKASAELIEEEVAKLLKLKAQ LGPDESKQKFVLKTPKGTRDYSPRQMAVREKVFDVI | | 56 |
| | 6-80 | ALEELVKLQGERVRGLKQQKASAELIEEEVAKLLKLKAQL GPDESKQKFVLKTPKGTRDYSPRQMAVREKVFDVI | | 57 |
| | 7-80 | LEELVKLQGERVRGLKQQKASAELIEEEVAKLLKLKAQLG PDESKQKFVLKTPKGTRDYSPRQMAVREKVFDVI | | 58 |
| | 8-80 | EELVKLQGERVRGLKQQKASAELIEEEVAKLLKLKAQLGP DESKQKFVLKTPKGTRDYSPRQMAVREKVFDVI | | 59 |
| | 9-80 | ELVKLQGERVRGLKQQKASAELIEEEVAKLLKLKAQLGPD ESKQKFVLKTPKGTRDYSPRQMAVREKVFDVI | | 60 |
| | 10-80 | LVKLQGERVRGLKQQKASAELIEEEVAKLLKLKAQLGPDE SKQKFVLKTPKGTRDYSPRQMAVREKVFDVI | | 61 |
| | 11-80 | VKLQGERVRGLKQQKASAELIEEEVAKLLKLKAQLGPDES KQKFVLKTPKGTRDYSPRQMAVREKVFDVI | | 62 |
| | 12-80 | KLQGERVRGLKQQKASAELIEEEVAKLLKLKAQLGPDESK QKFVLKTPKGTRDYSPRQMAVREKVFDVI | | 63 |
| | 13-80 | LQGERVRGLKQQKASAELIEEEVAKLLKLKAQLGPDESKQ KFVLKTPKGTRDYSPRQMAVREKVFDVI | | 64 |
| | 14-80 | QGERVRGLKQQKASAELIEEEVAKLLKLKAQLGPDESKQK FVLKTPKGTRDYSPRQMAVREKVFDVI | | 65 |
| | 15-80 | GERVRGLKQQKASAELIEEEVAKLLKLKAQLGPDESKQKF VLKTPKGTRDYSPRQMAVREKVFDVI | | 66 |
| | 16-80 | RVRGLKQQKASAELIEEEVAKLLKLKAQLGPDESKQKFVL KTPKGTRDYSPRQMAVREKVFDVI | | 67 |
| | 17-80 | VRGLKQQKASAELIEEEVAKLLKLKAQLGPDESKQKFVLK TPKGTRDYSPRQMAVREKVFDVI | | 68 |
| | 18-80 | RGLKQQKASAELIEEEVAKLLKLKAQLGPDESKQKFVLKT PKGTRDYSPRQMAVREKVFDVI | | 69 |
| | 19-80 | GLKQQKASAELIEEEVAKLLKLKAQLGPDESKQKFVLKTP KGTRDYSPRQMAVREKVFDVI | | 70 |
| | 20-80 | LKQQKASAELIEEEVAKLLKLKAQLGPDESKQKFVLKTPK GTRDYSPRQMAVREKVFDVI | | 71 |
| | 21-80 | KQQKASAELIEEEVAKLLKLKAQLGPDESKQKFVLKTPKG TRDYSPRQMAVREKVFDVI | | 72 |
| | 22-80 | QQKASAELIEEEVAKLLKLKAQLGPDESKQKFVLKTPKGT RDYSPRQMAVREKVFDVI | | 73 |
| | 23-80 | QKASAELIEEEVAKLLKLKAQLGPDESKQKFVLKTPKGTR DYSPRQMAVREKVFDVI | | 74 |
| | 24-80 | KASAELIEEEVAKLLKLKAQLGPDESKQKFVLKTPKGTRD YSPRQMAVREKVFDVI | | 75 |
| | 25-80 | ASAELIEEEVAKLLKLKAQLGPDESKQKFVLKTPKGTRDY SPRQMAVREKVFDVI | | 76 |
| | 26-80 | SAELIEEEVAKLLKLKAQLGPDESKQKFVLKTPKGTRDYS PRQMAVREKVFDVI | | 77 |
| | 27-80 | AELIEEEVAKLLKLKAQLGPDESKQKFVLKTPKGTRDYSP RQMAVREKVFDVI | | 78 |
| | 28-80 | ELIEEEVAKLLKLKAQLGPDESKQKFVLKTPKGTRDYSPR QMAVREKVFDVI | | 79 |
| | 29-80 | LIEEEVAKLLKLKAQLGPDESKQKFVLKTPKGTRDYSPRQ MAVREKVFDVI | | 80 |
| | 30-80 | IEEEVAKLLKLKAQLGPDESKQKFVLKTPKGTRDYSPRQM AVREKVFDVI | | 81 |
| | 31-80 | EEEVAKLLKLKAQLGPDESKQKFVLKTPKGTRDYSPRQMA VREKVFDVI | | 82 |
| | 32-80 | EEVAKLLKLKAQLGPDESKQKFVLKTPKGTRDYSPRQMAV REKVFDVI | | 83 |
| | 33-80 | EVAKLLKLKAQLGPDESKQKFVLKTPKGTRDYSPRQMAVR EKVFDVI | | 84 |
| | 34-80 | VAKLLKLKAQLGPDESKQKFVLKTPKGTRDYSPRQMAVRE KVFDVI | | 85 |
| | 35-80 | AKLLKLKAQLGPDESKQKFVLKTPKGTRDYSPRQMAVREK VFDVI | | 86 |
| | 36-80 | KLLKLKAQLGPDESKQKFVLKTPKGTRDYSPRQMAVREKV FDVI | | 87 |
| | 37-80 | LLKLKAQLGPDESKQKFVLKTPKGTRDYSPRQMAVREKVF DVI | | 88 |
| | 38-80 | LK LKAQLGPDESKQKFVLKTPKGTRDYSPRQMAVREKVFDVI | | 89 |
| | 39-80 | K LKAQLGPDESKQKFVLKTPKGTRDYSPRQMAVREKVFDVI | | 90 |
| | 40-80 | LKAQLGPDESKQKFVLKTPKGTRDYSPRQMAVREKVFDVI | | 91 |
| HisRS1^{N1} | 1-141 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAM | | 92 |
| HisRS1^{N2} | 1-408 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTE | | 93 |
| HisRS1^{N3} | 1-113 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKL | | 94 |
| HisRS1^{N4} | 1-60 | MAERAALEELVKLQGERVRGLKQQKASAELIEEEVAKLLK LKAQLGPDESKQKFVLKTPK | | 95 |
| HisRS1^{N5} | 1-243 + 27aa | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVGYPWWNSCSRILNYPKTSRPWRAWET | | 96 |
| HisRS1^{C1} | 405-509 | RTTETQVLVASAQKKLLEERLKLVSELWDAGIKAELLYKK NPKLLNQLQYCEEAGIPLVAIIGEQELKDGVIKLRSVTSR EEVDVRREDLVEEIKRRTGQPLCIC | | 97 |
| HisRS1^{C2} | 1-60 + 175-509 | MAERAALEELVKLQGERVRGLKQQKASAELIEEEVAKLLK LKAQLGPDESKQKFVLKTPKDFDIAGNFDPMIPDAECLKI MCEILSSLQIGDFLVKVNDRRILDGMFAICGVSDSKFRTI CSSVDKLDKVSWEEVKNEMVGEKGLAPEVADRIGDYVQQH GGVSLVEQLLQDPKLSQNKQALEGLGDLKLLFEYLTLFGI DDKISFDLSLARGLDYYTGVIYEAVLLQTPAQAGEEPLGV GSVAAGGRYDGLVGMFDPKGRKVPCVGLSIGVERIFSIVE QRLEALEEKIRTTETQVLVASAQKKLLEERLKLVSELWDA GIKAELLYKKNPKLLNQLQYCEEAGIPLVAIIGEQELKDG VIKLRSVTSREEVDVRREDLVEEIKRRTGQPLCIC | | 98 |
| HisRS1^{C3} | 1-60 + 211-509 | MAERAALEELVKLQGERVRGLKQQKASAELIEEEVAKLLK LKAQLGPDESKQKFVLKTPKVNDRRILDGMFAICGVSDSK FRTICSSVDKLDKVSWEEVKNEMVGEKGLAPEVADRIGDY VQQHGGVSLVEQLLQDPKLSQNKQALEGLGDLKLLFEYLT LFGIDDKISFDLSLARGLDYYTGVIYEAVLLQTPAQAGEE PLGVGSVAAGGRYDGLVGMFDPKGRKVPCVGLSIGVERIF SIVEQRLEALEEKIRTTETQVLVASAQKKLLEERLKLVSE LWDAGIKAELLYKKNPKLLNQLQYCEEAGIPLVAIIGEQE LKDGVIKLRSVTSREEVDVRREDLVEEIKRRTGQPLCIC | | 99 |
| HisRS1^{C4} | 1-100 + 211-509 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKVNDRRILDGMFAICGVSDSK FRTICSSVDKLDKVSWEEVKNEMVGEKGLAPEVADRIGDY VQQHGGVSLVEQLLQDPKLSQNKQALEGLGDLKLLFEYLT LFGIDDKISFDLSLARGLDYYTGVIYEAVLLQTPAQAGEE PLGVGSVAAGGRYDGLVGMFDPKGRKVPCVGLSIGVERIF SIVEQRLEALEEKIRTTETQVLVASAQKKLLEERLKLVSE LWDAGIKAELLYKKNPKLLNQLQYCEEAGIPLVAIIGEQE LKDGVIKLRSVTSREEVDVRREDLVEEIKRRTGQPLCIC | | 100 |
| HisRS1^{C5} | 1-174 + 211-509 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKETLMGKYGEDSKLIYDLKDQ GGELLSLRYDLTVPFARYLAMNKLTNIKRYHIAKVYRRDN PAMTRGRYREFYQCVNDRRILDGMFAICGVSDSKFRTICS SVDKLDKVSWEEVKNEMVGEKGLAPEVADRIGDYVQQHGG VSLVEQLLQDPKLSQNKQALEGLGDLKLLFEYLTLFGIDD KISFDLSLARGLDYYTGVIYEAVLLQTPAQAGEEPLGVGS VAAGGRYDGLVGMFDPKGRKVPCVGLSIGVERIFSIVEQR LEALEEKIRTTETQVLVASAQKKLLEERLKLVSELWDAGI KAELLYKKNPKLLNQLQYCEEAGIPLVAIIGEQELKDGVI KLRSVTSREEVDVRREDLVEEIKRRTGQPLCIC | | 101 |
| HisRS1^{C6} | 1-60 + 101-509 | MAERAALEELVKLQGERVRGLKQQKASAELIEEEVAKLLK LKAQLGPDESKQKFVLKTPKETLMGKYGEDSKLIYDLKDQ GGELLSLRYDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEMVGEKGLAPEVADRIGDYVQQHGGVSLV EQLLQDPKLSQNKQALEGLGDLKLLFEYLTLFGIDDKISF DLSLARGLDYYTGVIYEAVLLQTPAQAGEEPLGVGSVAAG GRYDGLVGMFDPKGRKVPCVGLSIGVERIFSIVEQRLEAL EEKIRTTETQVLVASAQKKLLEERLKLVSELWDAGIKAEL LYKKNPKLLNQLQYCEEAGIPLVAIIGEQELKDGVIKLRS VTSREEVDVRREDLVEEIKRRTGQPLCIC | | 102 |
| HisRS1^{C7} | P1-100 + 175-509 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKDFDIAGNFDPMIPDAECLKI MCEILSSLQIGDFLVKVNDRRILDGMFAICGVSDSKFRTI CSSVDKLDKVSWEEVKNEMVGEKGLAPEVADRIGDYVQQH GGVSLVEQLLQDPKLSQNKQALEGLGDLKLLFEYLTLFGI DDKISFDLSLARGLDYYTGVIYEAVLLQTPAQAGEEPLGV GSVAAGGRYDGLVGMFDPKGRKVPCVGLSIGVERIFSIVE QRLEALEEKIRTTETQVLVASAQKKLLEERLKLVSELWDA GIKAELLYKKNPKLLNQLQYCEEAGIPLVAIIGEQELKDG VIKLRSVTSREEVDVRREDLVEEIKRRTGQPLCIC | | 103 |
| HisRS1^{C8} | 1-60 + 399-509 | MAERAALEELVKLQGERVRGLKQQKASAELIEEEVAKLLK LKAQLGPDESKQKFVLKTPKALEEKIRTTETQVLVASAQK KLLEERLKLVSELWDAGIKAELLYKKNPKLLNQLQYCEEA GIPLVAIIGEQELKDGVIKLRSVTSREEVDVRREDLVEEI KRRTGQPLCIC | | 104 |
| HisRS1^{C9} | 1-100 + 399-509 | MAERAALEELVKLQGERVRGLKQQKASAELIEEEVAKLLK LKAQLGPDESKQKFVLKTPKGTRDYSPRQMAVREKVFDVI IRCFKRHGAEVIDTPVFELKALEEKIRTTETQVLVASAQK KLLEERLKLVSELWDAGIKAELLYKKNPKLLNQLQYCEEA GIPLVAIIGEQELKDGVIKLRSVTSREEVDVRREDLVEEI KRRTGQPLCIC | | 105 |
| HisRS1^{C10} | 369-509 | | | 106 |
| HisRS1^{I1} | 191-333 | CLKIMCEILSSLQIGDFLVKVNDRRILDGMFAICGVSDSK FRTICSSVDKLDKVSWEEVKNEMVGEKGLAPEVADRIGDY VQQHGGVSLVEQLLQDPKLSQNKQALEGLGDLKLLFEYLT LFGIDDKISFDLSLARGLDYYTG | | 107 |
| FL mito. wild type | 1-506 | | | 108 |
| *Mus musculus* | FL | MADRAALEELVRLQGAHVRGLKEQKASAEQIEEEVTKLLK LKAQLGQDEGKQKFVLKTPKGTRDYSPRQMAVREKVFDVI IRCFKRHGAEVIDTPVFELKETLTGKYGEDSKLIYDLKDQ GGELLSLRYDLTVPFARYLAMNKLTNIKRYHIAKVYRRDN PAMTRGRYREFYQCDFDIAGQFDPMIPDAECLKIMCEILS SLQIGNFLVKVNDRRILDGMFAVCGVPDSKFRTICSSVDK LDKVSWEEVKNEMVGEKGLAPEVADRIGDYVQQHGGVSLV EQLLQDPKLSQNKQAVEGLGDLKLLFEYLILFGIDDKISF DLSLARGLDYYTGVIYEAVLLQMPTQAGEEPLGVGSIAAG GRYDGLVGMFDPKGRKVPCVGLSIGVERIFSIVEQRLEAS EEKVRTTETQVLVASAQKKLLEERLKLVSELWDAGIKAEL LYKKNPKLLNQLQYWEEAGIPLVAIIGEQELRDGVIKLRS VASREEVDVRREDLVEEIRRRTNQPLSTC | | 109 |
| *Canis lupus familiaris* | FL | MAERAALEELVRLQGERVRGLKQQKASAEQIEEEVAKLLK LKAQLGPDEGKQKFVLKTPKGTRDYSPRQMAVREKVFDVI ISCFKRHGAEVIDTPVFELKETLTGKYGEDSKLIYDLKDQ GGELLSLRYDLTVPFARYLAMNKLTNIKRYHIAKVYRRDN PAMTRGRYREFYQCDFDIAGQFDPMIPDAECLEIMCEILR SLQIGDFLVKVNDRRILDGMFAICGVPDSKFRTICSSVDK LDKVSWEEVKNEMVGEKGLAPEVADHIGDYVQQHGGISLV EQLLQDPELSQNKQALEGLGDLKLLFEYLTLFGIADKISF DLSLARGLDYYTGVIYEAVLLQTPVQAGEEPLGVGSVAAG GRYDGLVGMFDPKGRKVPCVGLSIGVERIFSIVEQRLEAT EEKVRTTETQVLVASAQKKLLEERLKLVSELWNAGIKAEL LYKKNPKLLNQLQYCEEAGIPLVAIIGEQELKDGVIKLRS VASREEVDVPREDLVEEIKRRTSQPFCIC | | 110 |
| *Bos taurus* | FL | MADRAALEDLVRVQGERVRGLKQQKASAEQIEEEVAKLLK LKAQLGPDEGKPKFVLKTPKGTRDYSPRQMAVREKVFDVI ISCFKRHGAEVIDTPVFELKETLTGKYGEDSKLIYDLKDQ GGELLSLRYDLTVPFARYLAMNKLTNIKRYHIAKVYRRDN PAMTRGRYREFYQCDFDIAGQFDPMLPDAECLKIMCEILS SLQIGDFLVKVNDRRILDGMFAICGVPDSKFRTICSSVDK LDKVSWEEVKNEMVGEKGLAPEVADRIGDYVQQHGGVSLV EQLLQDPKLSQNKQALEGLGDLKLLFEYLTLFGIADKISF DLSLARGLDYYTGVIYEAVLLQPPARAGEEPLGVGSVAAG GRYDGLVGMFDPKGRKVPCVGLSIGVERIFSIVEQRLEAL EEKVRTTETQVLVASAQKKLLEERLKLISELWDAGIKAEL LYKKNPKLLNQLQYCEETGIPLVAIIGEQELKDGVIKLRS VASREEVDVR REDLVEEIKR RTSQPLCIC | | 111 |
| *Rattus norvegicus* | FL | MADRAALEELVRLQGAHVRGLKEQKASAEQIEEEVTKLLK LKAQLGHDEGKQKFVLKTPKGTRDYSPRQMAVREKVFDVI IRCFKRHGAEVIDTPVFELKETLTGKYGEDSKLIYDLKDQ GGELLSLRYDLTVPFARYLAMNKLTNIKRYHIAKVYRRDN PAMTRGRYREFYQCDFDIAGQFDPMIPDAECLKIMCEILS SLQIGNFQVKVNDRRILDGMFAVCGVPDSKFRTICSSVDK LDKVSWEEVKNEMVGEKGLAPEVADRIGDYVQQHGGVSLV EQLLQDPKLSQNKQAVEGLGDLKLLFEYLTLFGIDDKISF DLSLARGLDYYTGVIYEAVLLQMPTQAGEEPLGVGSIAAG GRYDGLVGMFDPKGRKVPCVGLSIGVERIFSIVEQKLEAS EEKVRTTETQVLVASAQKKLLEERLKLISELWDAGIKAEL LYKKNPKLLNQLQYCEEAGI PLVAIIGEQE LKDGVIKLRSVTSREEVDVR REDLVEEIRR RTSQPLSM | | 112 |
| *Gallus gallus* | FL | MADEAAVRQQAEVVRRLKQDKAEPDEIAKEVAKLLEMKAH LGGDEGKHKFVLKTPKGTRDYGPKQMAIRERVFSAIIACF KRHGAEVIDTPVFELKETLTGKYGEDSKLIYDLKDQGGEL LSLRYDLTVPFARYLAMNKITNIKRYHIAKVYRRDNPAMT RGRYREFYQCDFDIAGQFDPMIPDAECLKIVQEILSDLQL GDFLIKVNDRRILDGMFAVCGVPDSKFRTICSSVDKLDKM PWEEVRNEMVGEKGLSPEAADRIGEYVQLHGGMDLIEQLL QDPKLSQNKLVKEGLGDMKLLFEYLTLFGITGKISFDLSL ARGLDYYTGVIYEAVLLQQNDHGEESVSVGSVAGGGRYDG LVGMFDPKGR KVPCVGISIGIERIFSILEQRVEASEEKIR TTETQVLVASAQKKLLEERLKLISELWDAGIKAEVLYKKN PKLLNQLQYCEDTGIPLVAIVGEQELKDGVVKLRVVATGE EVNIRRESLVEEIRRRTNQL | | 113 |
| *Danio rerio* | FL | MAALGLVSMRLCAGLMGRRSAVRLHSLRVCSGMTISQIDE EVARLLQLKAQLGGDEGKHVFVLKTAKGTRDYNPKQMAIR EKVFNIIINCFKRHGAETIDSPVFELKETLTGKYGEDSKL IYDLKDQGGELLSLRYDLTVPFARYLAMNKITNIKRYHIA KVYRRDNPAMTRGRYREFYQCDFDIAGQYDAMIPDAECLK LVYEILSELDLGDFRIKVNDRRILDGMFAICGVPDEKFRT ICSTVDKLDKLAWEEVKKEMVNEKGLSEEVADRIRDYVSM QGGKDLAERLLQDPKLSQSKQACAGITDMKLLFSYLELFQ ITDKVVFDLSLARGLDYYTGVIYEAILTQANPAPASTPAE QNGAEDAGVSVGSVAGGGRYDGLVGMFDPKAGKCPVWGSA LALRGSSPSWSRRQSCLQRRCAPLKLKCLWLQHRRTF | | 114 |
| *Macaca fascicular is* | FL | MAERAALEELVKLQGERVRGLKQQKASAELIEEEVGKLLK LKAQLGPDESKQKFVLKTPKGTRDYSPRQMAVREKVFDVI IRCFKRHGAEVIDTPVFELKETLMGKYGEDSKLIYDLKDQ GGELLSLRHDLTVPFARYLAMNKLTNIKRYHIAKVYRRDN PAMTRGRYREFYQCDFDIAGNFDPMIPDAECLKIMCEILS SLQIGDFLVKVNDRRILDGMFAICGVSDSKFRTICSSVDK LDKVSWEEVKNEAVLLQTPAQAGEEPLGVGSVAAGGRYDG LVGMFDPKGRKVPCVGLSIGVERIFSIVEQ RLEALEEKVRTTETQVLVASAQKKLLEERLKLVSELWDAG IKAELLYKKNPKLLNQLQYCEEAGIPLVAIIGEQELKDGV IKLRSVTSREEVNVRREDLKTGQNGDFNFYYGYFIDYYWQ KWPDTTPFSYKALGA | | 115 |
| HRS WHEP consensus | | X_{A}-L-X_{B}-Q-G-X-X-V-R-X-L-K-X-X-K-A-X_{C}-V-X- X-L-L-X-L-K-X_{D} | | 116 |
| | | Where: | | |
| | | | X is any amino acid | |
| | | | X_{A} is 0-50 amino acids | |
| | | | X_{B} is about 5-7 amino acids, preferably 6 amino acids | |
| | | | X_{C} is about 7-9 amino acids, preferably 8 amino acids | |
| | | | X_{D} is 0-50 amino acids | |

As noted herein, a HRS polypeptide may be altered in various ways including amino acid substitutions, deletions, truncations, additions, and insertions. Methods for such manipulations are generally known in the art. For example, amino acid sequence variants of a HRS reference polypeptide can be prepared by mutations in the DNA. Methods for mutagenesis and nucleotide sequence alterations are well known in the art. See, for example, Kunkel (1985, Proc. Natl. Acad. Sci. USA. 82: 488-492), Kunkel et al., (1987, Methods in Enzymol, 154: 367-382), U.S. Pat. No. 4,873,192, Watson, J. D. et al., ("Molecular Biology of the Gene", Fourth Edition, Benjamin/Cummings, Menlo Park, Calif., 1987) and the references cited therein. Guidance as to appropriate amino acid substitutions that do not affect biological activity of the protein of interest may be found in the model of Dayhoff et al., (1978) Atlas of Protein Sequence and Structure (Natl. Biomed. Res. Found., Washington, D.C.).

Biologically active truncated and/or variant HRS polypeptides may contain conservative amino acid substitutions at various locations along their sequence, relative to a reference HRS amino acid residue. A "conservative amino acid substitution" is one in which the amino acid residue is replaced with an amino acid residue having a similar side chain. Families of amino acid residues having similar side chains have been defined in the art, which can be generally sub-classified as follows:
Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.

Basic: The residue has a positive charge due to association with H ion at physiological pH or within one or two pH units thereof (e.g., histidine) and the residue is attracted by aqueous solution so as to seek the surface positions in the conformation of a peptide in which it is contained when the peptide is in aqueous medium at physiological pH. Amino acids having a basic side chain include arginine, lysine and histidine.

Charged: The residues are charged at physiological pH and, therefore, include amino acids having acidic or basic side chains (i.e., glutamic acid, aspartic acid, arginine, lysine and histidine).

Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.

Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterizes certain amino acids as "small" since their side chains are not sufficiently large, even if polar groups are lacking, to confer hydrophobicity. With the exception of proline, "small" amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the α-amino group, as well as the α-carbon. Several amino acid similarity matrices are known in the art (see e.g., PAM120 matrix and PAM250 matrix as disclosed for example by Dayhoff et al., 1978, A model of evolutionary change in proteins). Matrices for determining distance relationships In M. O. Dayhoff, (ed.), Atlas of protein sequence and structure, Vol. 5, pp. 345-358, National Biomedical Research Foundation, Washington DC; and by Gonnet et al., (Science, 256: 14430-1445, 1992), however, include proline in the same group as glycine, serine, alanine and threonine. Accordingly, proline is classified as a "small" amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behavior.

Amino acid residues can be further sub-classified as cyclic or non-cyclic, and aromatic or non-aromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always non-aromatic. Dependent on their structural properties, amino acid residues may fall in two or more classes. For the naturally-occurring protein amino acids, sub-classification according to this scheme is presented in **Table A.**

| **Table A** | | |
|---|---|---|
| **Sub-classes** | **Amino acids** | |
| Acidic | Aspartic acid, Glutamic acid | |
| Basic | Noncyclic: Arginine, Lysine; Cyclic: Histidine | |
| Charged | Aspartic acid, Glutamic acid, Arginine, Lysine, Histidine | |
| Small | Glycine, Serine, Alanine, Threonine, Proline | |
| Polar/neutral | Asparagine, Histidine, Glutamine, Cysteine, Serine, Threonine | |
| Polar/large | Asparagine, Glutamine | |
| Hydrophobic | Tyrosine, Valine, Isoleucine, Leucine, Methionine, Phenylalanine, Tryptophan | |
| Aromatic | Tryptophan, Tyrosine, Phenylalanine | |
| Residues that influence chain orientation | Glycine and Proline | |

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional truncated and/or variant HRS polypeptide can readily be determined by assaying its non-canonical activity, as described herein. Conservative substitutions are shown in **Table B** under the heading of exemplary substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, (c) the bulk of the side chain, or (d) the biological function. After the substitutions are introduced, the variants are screened for biological activity.

| **Table B** | | |
|---|---|---|
| **Original Residue** | **Exemplary Substitutions** | **Preferred Substitutions** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser, Ala, Leu, Val | Ser, Ala |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |
| Leu | Norleu, Ile, Val, Met, Ala, Phe | Ile |
| Lys | Arg, Gln, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleu | Leu |

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G., Biochemistry, third edition, Wm.C. Brown Publishers (1993).

In some embodiments, HRS polypeptides have one or more cysteine insertions or substitutions, for example, where one or more non-cysteine residues are substituted with a cysteine residue (e.g., to alter stability, to facilitate thiol-based conjugation of an Fc fragment, to facilitate thiol-based attachment of PEG or other molecules). In some embodiments, the one or more cysteine substitutions are near the N-terminus and/or C-terminus of the HRS polypeptide, or other surface exposed regions of a HRS polypeptide. Particular embodiments include where one or more of residues within 1, 2, 3, 4, 5, 6, 7, 8, 9, 10, 11, 12, 13, 14, 15, 16, 17, 18, 19, 20, 21, 22, 23, 24, or 25 amino acids relative to the N-terminus and/or C-terminus of an HRS polypeptide are substituted with a cysteine residue. In some embodiments, cysteine residues may be added to the HRS polypeptide through the creation of N, or C-terminal fusion proteins. Such fusion proteins may be of any length, but will typically be about 1-5, or about 5-10, about 10 to 20, or about 20 to 30 amino acids in length.

Specific examples of cysteine modified proteins (not part of the invention) are shown in **Table H2,** which are based on the HRS polypeptide HRS(1-60). This approach can be applied to the HRS polypeptides of **Table H1** and other HRS polypeptides described herein.

| **Table H2. Exemplary cysteine modified HRS polypeptides (not part of the invention)** | | |
|---|---|---|
| **Name** | **Protein Sequences** | **SEQ ID NO:** |
| HRS(1-60)-M1MC- | MCAERAALEELVKLQGERVRGLKQQKASAELIEEEVAKLLKLK AQLGPDESKQKFVLKTPK | 117 |
| HRS(1-60)-A26C- | MAERAALEELVKLQGERVRGLKQQKCSAELIEEEVAKLLKLKA QLGPDESKQKFVLKTPK | 118 |
| HRS(1-60)-C61 | MAERAALEELVKLQGERVRGLKQQKASAELIEEEVAKLLKLKA QLGPDESKQKFVLKTPKC | 119 |

In some embodiments, the HRS polypeptide have mutations in which the endogenous or naturally-occurring cysteine residues are mutated to alternative amino acids, or deleted. In some embodiments, the insertion or substitution of cysteine residue(s) into the HRS polypeptide is combined with the elimination of other surface exposed reactive cysteine residues. Accordingly, in some embodiments, an HRS polypeptide comprises one or more substitutions and/or deletions at any one or more of Cys83, Cys174, Cys191, Cys196, Cys224, Cys235, Cys379, Cys455, Cys507, and/or Cys509 (as defined by SEQ ID NO: 1), for instance, to remove naturally-occurring cysteine residues, including combinations thereof.

Specific embodiments include an HRS polypeptide of **Table H1** having a mutation or deletion of any one or more of Cys83, Cys174, Cys191, Cys196, Cys224, Cys235, Cys379, Cys455, or the deletion of Cys507 and Cys509, for instance, by the deletion of the C-terminal 3 amino acids (Δ507-509). Exemplary mutations at these positions include for example the mutation of cysteine to serine, alanine, leucine, valine or glycine. In certain embodiments, amino acid residues for specific cysteine substitutions can be selected from naturally-occurring substitutions that are found in HRS orthologs from other species and organisms. Exemplary substitutions of this type are presented in Table H3.

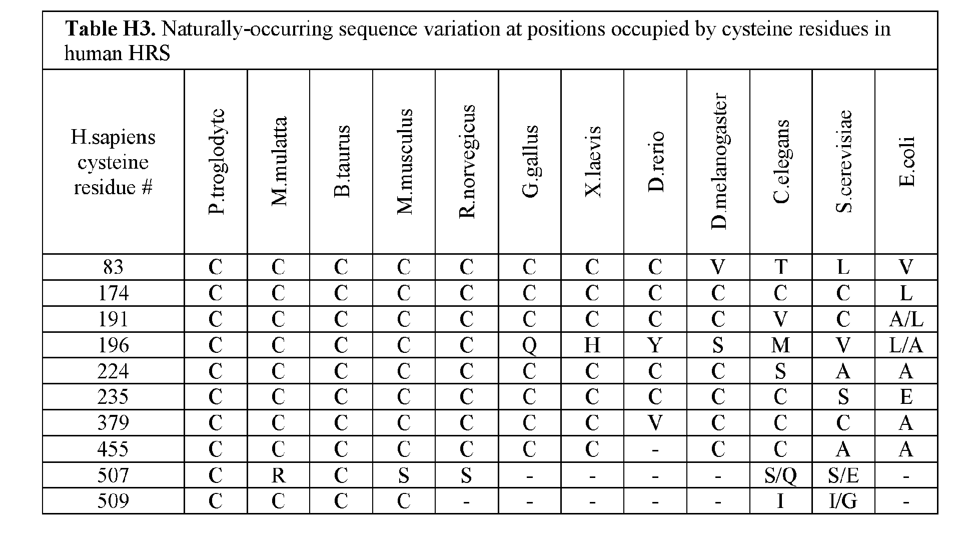

In some embodiments, the naturally-occurring cysteines selected for mutagenesis are selected based on their surface exposure. Accordingly, in one aspect the cysteine residues selected for substitution are selected from Cys224, Cys235, Cys507 and Cys509. In some embodiments, the last three (C-terminal) residues of SEQ ID NO: 1 are deleted so as to delete residues 507 to 509. In some embodiments, the cysteines are selected for mutation or deletion so as to eliminate an intramolecular cysteine pair, for example Cys174 and Cys191.

Specific examples of cysteine mutations/substitutions (indicated in bold underline) to reduce surface exposed cysteine residues include those listed below in Table H4.

| **Table H4. Exemplary HRS polypeptides with substitutions to remove surface exposed cysteines (not part of the invention)** | | |
|---|---|---|
| **Name** | **Protein Sequence** | **SEQ ID NO:** |
| HRS (1-506) C174A | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQADFDIAGNFDPMIPDAECLKIMCEILSSLQI GDFLVKVNDRRILDGMFAICGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 120 |
| HRS (1-506) C174V | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQVDFDIAGNFDPMIPDAECLKIMCEILSSLQI GDFLVKVNDRRILDGMFAICGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 121 |
| HRS (1-506) C191A | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQCDFDIAGNFDPMIPDAEALKIMCEILSSLQI GDFLVKVNDRRILDGMFAICGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 122 |
| HRS (1-506) C191S | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQCDFDIAGNFDPMIPDAESLKIMCEILSSLQI GDFLVKVNDRRILDGMFAICGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 123 |
| HRS (1-506) C191V | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQCDFDIAGNFDPMIPDAEVLKIMCEILSSLQI GDFLVKVNDRRILDGMFAICGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 124 |
| HRS (1-506) C224S | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQCDFDIAGNFDPMIPDAECLKIMCEILSSLQI GDFLVKVNDRRILDGMFAISGVSDSKFRTICSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 125 |
| HRS (1-506) C235S | MAERAALEELVKLQGERVRGLKQQKASAELIEEEVAKLLKLKAQLGPDESK QKFVLKTPKGTRDYSPRQMAVREKVFDVIIRCFKRHGAEVIDTPVFELKET LMGKYGEDSKLIYDLKDQGGELLSLRYDLTVPFARYLAMNKLTNIKRYHIA KVYRRDNPAMTRGRYREFYQCDFDIAGNFDPMIPDAECLKIMCEILSSLQI GDFLVKVNDRRILDGMFAICGVSDSKFRTISSSVDKLDKVSWEEVKNEMVG EKGLAPEVADRIGDYVQQHGGVSLVEQLLQDPKLSQNKQALEGLGDLKLLF EYLTLFGIDDKISFDLSLARGLDYYTGVIYEAVLLQTPAQAGEEPLGVGSV AAGGRYDGLVGMFDPKGRKVPCVGLSIGVERIFSIVEQRLEALEEKIRTTE TQVLVASAQKKLLEERLKLVSELWDAGIKAELLYKKNPKLLNQLQYCEEAG IPLVAIIGEQELKDGVIKLRSVTSREEVDVRREDLVEEIKRRTGQPL | 126 |

In some embodiments, such cysteine substituted mutants are modified to engineer-in, insert, or otherwise introduce a new surface exposed cysteine residue at a defined surface exposed position, where the introduced residue does not substantially interfere with the non-canonical activity of the HRS polypeptide. Specific examples include for example the insertion (or re-insertion back) of additional cysteine residues at the N- or C-terminus of any of the reduced cysteine HRS polypeptides described above. In some embodiments, the insertion of such N- or C-terminal surface exposed cysteines involves the re-insertion of the last 1, last 2, or last 3 naturally occurring C-terminal amino acids of the full length human HRS to a reduced cysteine variant of a HRS polypeptide e.g., the re-insertion of all or part of the sequence CIC (Cys Ile Cys). Exemplary reduced cysteine mutants include for example any combination of mutations (or the deletion of) at residues Cys174, Cys191, Cys224, and Cys235, and or the deletion or substitution of Cys507 and Cys509 (based on the numbering of full length human cytosolic HRS (SEQ ID NO:1) in any of the HRS polypeptides of **Table H1**.

For some types of site-specific conjugation or attachment to heterologous molecules such as Fc regions or PEG or other heterologous molecules, HRS polypeptides may have one or more glutamine substitutions, where one or more naturally-occurring (non-glutamine) residues are substituted with glutamine, for example, to facilitate transglutaminase-catalyzed attachment of the molecule(s) to the glutamine's amide group. In some embodiments, glutamine substitutions are introduced near the N-terminus and/or C-terminus of the HRS polypeptide. Particular embodiments include where one or more of residues within 1, 2, 3, 4, 5, 6, 7, 8, 9, 10, 11, 12, 13, 14, 15, 16, 17, 18, 19, 20, 21, 22, 23, 24 or 25 amino acids relative to the N-terminus and/or C-terminus of an HRS polypeptide are substituted with a glutamine residue. These and related HRS polypeptides can also include substitutions (e.g., conservative substitutions) to remove any naturally-occurring glutamine residues, if desired, and thereby regulate the degree of site-specific conjugation or attachment.

For certain types of site-specific conjugation or attachment to heterologous molecules such as Fc regions or PEG or other heterologous molecules, HRS polypeptides may have one or more lysine substitutions, where one or more naturally-occurring (non-lysine) residues are substituted with lysine, for example, to facilitate acylation or alkylation-based attachment of molecule(s) to the lysine's amino group. These methods also typically result in attachment of molecule(s) to the N-terminal residue. In some embodiments, lysine substations are near the N-terminus and/or C-terminus of the HRS polypeptide. Particular embodiments include where one or more of residues within 1, 2, 3, 4, 5, 6, 7, 8, 9, 10, 11, 12, 13, 14, 15, 16, 17, 18, 19, 20, 21, 22, 23, 24 or 25 amino acids to the N-terminus and/or C-terminus of an HRS polypeptide are substituted with a lysine residue. These and related HRS polypeptides can also include substitutions (e.g., conservative substitutions) to remove any naturally-occurring lysine residues, if desired, and thereby regulate the degree of site-specific conjugation or attachment.

Site-specific conjugation to HRS polypeptides may also be performed by substituting one or more solvent accessible surface amino acids of a HRS polypeptide. For example, suitable solvent accessible amino acids may be determined based on the predicted solvent accessibility using the SPIDDER server (http://sppider.cchmc.org/) using the published crystal structure of an exemplary HRS polypeptide *(see* Xu et al., Structure. 20:1470-7, 2012; and U.S. Application No. 61/674,639). Based on this analysis several amino acids on the surface may potentially be used as mutation sites to introduce functional groups suitable for conjugation or attachment. The surface accessibility score of amino acids based on the crystal structure can be calculated, where the higher scores represent better accessibility. In particular embodiments, higher scores (for example, >40) are preferred. Accordingly in some embodiments an amino acid position have a surface accessibility score of greater than 40 may be used to introduce a cysteine, lysine, glutamine, or other non-naturally-occurring amino acid.

In particular embodiments, a solvent accessible surface amino acid is selected from the group consisting of: alanine, glycine, and serine, and can be substituted with naturally occurring amino acids including, but not limited to, cysteine, glutamine, or lysine, or a non-naturally occurring amino acid that is optimized for site specific conjugation or attachment.

Certain embodiments include site-specific conjugation or attachment to an HRS polypeptide at any amino acid position by virtue of substituting a non-naturally-occurring amino acid comprising a functional group that will form a covalent bond with the functional group attached to a heterologous molecules such as an Fc region or PEG or other heterologous molecule. Non-natural amino acids can be inserted or substituted at, for example, one or more of residues within 1, 2, 3, 4, 5, 6, 7, 8, 9, 10, 11, 12, 13, 14, 15, 16, 17, 18, 19, 20, 21, 22, 23, 24 or 25 amino acids relative to the N-terminus and/or C-terminus, at the N-terminus and/or C-terminus, or at a solvent accessible surface amino acid residue of an HRS polypeptide described herein.

In particular embodiments, non-naturally occurring amino acids include, without limitation, any amino acid, modified amino acid, or amino acid analogue other than selenocysteine and the following twenty genetically encoded alpha-amino acids: alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine. The generic structure of an alpha-amino acid is illustrated by the following formula:

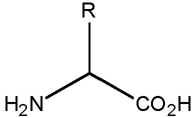

A non-natural amino acid is typically any structure having the foregoing formula wherein the R group is any substituent other than one used in the twenty natural amino acids. See, *e.g.,* biochemistry texts such as Biochemistry by L. Stryer, 3rd ed. 1988, Freeman and Company, New York, for structures of the twenty natural amino acids. Note that the non-natural amino acids disclosed herein may be naturally occurring compounds other than the twenty alpha-amino acids above. Because the non-natural amino acids disclosed herein typically differ from the natural amino acids in side chain only, the non-natural amino acids form amide bonds with other amino acids, e.g., natural or non-natural, in the same manner in which they are formed in naturally occurring proteins. However, the non-natural amino acids have side chain groups that distinguish them from the natural amino acids. For example, R in foregoing formula optionally comprises an alkyl-, aryl-, aryl halide, vinyl halide, alkyl halide, acetyl, ketone, aziridine, nitrile, nitro, halide, acyl-, keto-, azido-, hydroxyl-, hydrazine, cyano-, halo-, hydrazide, alkenyl, alkynyl, ether, thio ether, epoxide, sulfone, boronic acid, boronate ester, borane, phenylboronic acid, thiol, seleno-, sulfonyl-, borate, boronate, phospho, phosphono, phosphine, heterocyclic-, pyridyl, naphthyl, benzophenone, a constrained ring such as a cyclooctyne, thio ester, enone, imine, aldehyde, ester, thioacid, hydroxylamine, amino, carboxylic acid, alpha-keto carboxylic acid, alpha or beta unsaturated acids and amides, glyoxyl amide, or organosilane group, or the like or any combination thereof.

Specific examples of unnatural amino acids include, but are not limited to, p-acetyl-L-phenylalanine, O-methyl-L-tyrosine, an L-3-(2-naphthyl)alanine, a 3-methyl-phenylalanine, an O-4-allyl-L-tyrosine, a 4-propyl-L-tyrosine, a tri-O-acetyl-GlcNAcβ-serine, β-O-GlcNAc-L-serine, a triO-acetyl-GalNAc-α-threonine, an α-GalNAc-L-threonine, an L-Dopa, a fluorinated phenylalanine, an isopropyl-L-phenylalanine, a p-azido-L-phenylalanine, a p-acyl-L-phenylalanine, a p-benzoyl-L-phenylalanine, an L-phosphoserine, a phosphonoserine, a phosphonotyrosine, a p-iodo-phenylalanine, a p-bromophenylalanine, a p-amino-L-phenylalanine, an isopropyl-L-phenylalanine, those listed below, or elsewhere herein, and the like.

Accordingly, one may select a non-naturally occurring amino acid comprising a functional group that forms a covalent bond with any preferred functional group of a desired molecule (e.g., Fc region, PEG). Non-natural amino acids, once selected, can either be purchased from vendors, or chemically synthesized. Any number of non-natural amino acids may be incorporated into the target molecule and may vary according to the number of desired molecules that are to be attached. The molecules may be attached to all or only some of the non-natural amino acids. Further, the same or different non-natural amino acids may be incorporated into a HRS polypeptide, depending on the desired outcome. In certain embodiments, about 1, 2, 3, 4, 5, 6, 7, 8, 9, 10 or more non-natural amino acids are incorporated into a HRS polypeptide any or all of which may be conjugated to a molecule comprising a desired functional group.

In certain aspects, the use of non-natural amino acids can be utilized to modify (e.g., increase) a selected non-canonical activity of an HRS polypeptide, or to alter the *in vivo* or *in vitro* half-life of the protein. Non-natural amino acids can also be used to facilitate (selective) chemical modifications (e.g., pegylation) of an HRS polypeptide, as described herein. For instance, certain non-natural amino acids allow selective attachment of polymers such as an Fc region or PEG to a given protein, and thereby improve their pharmacokinetic properties.

Specific examples of amino acid analogs and mimetics can be found described in, for example, Roberts and Vellaccio, The Peptides: Analysis, Synthesis, Biology, Eds. Gross and Meinhofer, Vol. 5, p. 341, Academic Press, Inc., New York, N.Y. (1983). Other examples include peralkylated amino acids, particularly permethylated amino acids. See, for example, Combinatorial Chemistry, Eds. Wilson and Czamik, Ch. 11, p. 235, John Wiley & Sons Inc., New York, N.Y. (1997). Yet other examples include amino acids whose amide portion (and, therefore, the amide backbone of the resulting peptide) has been replaced, for example, by a sugar ring, steroid, benzodiazepine or carbo cycle. See, for instance, Burger's Medicinal Chemistry and Drug Discovery, Ed. Manfred E. Wolff, Ch. 15, pp. 619-620, John Wiley & Sons Inc., New York, N.Y. (1995). Methods for synthesizing peptides, polypeptides, peptidomimetics and proteins are well known in the art (see, for example, U.S. Pat. No. 5,420,109; M. Bodanzsky, Principles of Peptide Synthesis (1st ed. & 2d rev. ed.), Springer-Verlag, New York, N.Y. (1984 & 1993), see Chapter 7; Stewart and Young, Solid Phase Peptide Synthesis, (2d ed.), Pierce Chemical Co., Rockford, Ill. (1984)). Accordingly, the HRS polypeptides can be composed of naturally occurring and non-naturally occurring amino acids as well as amino acid analogs and mimetics.

The HRS polypeptide possesses at least one non-canonical activity, for example, an anti-inflammatory activity or binding to a neuropilin polypeptide, examples of which are described herein. Assays to determine anti-inflammatory activity, or neuropilin polypeptide or receptor binding, including routine measurements of cytokine release from in vitro cell based, and animal studies are well established in the art (see, for example, Wittmann et al., J Vis Exp. (65):e4203. doi: 10.3791/4203, 2012; Feldman et al., Mol Cell. 47:585-95, 2012; Clutterbuck et al., J Proteomics. 74:704-15, 2011, Giddings and Maitra, J Biomol Screen. 15:1204-10, 2010; Wijnhoven et al., Glycoconj J. 25:177-85, 2008; and Frow et al., Med Res Rev. 24:276-98, 2004) and can be readily used to profile and optimize anti-inflammatory activity. An exemplary in vivo experimental system is also described in the accompanying Examples.

It will be appreciated that in any of the HRS polypeptides, the N-terminal acid of the HRS polypeptide (for example, the N-terminal Met) may be deleted or replaced with a different amino acid.

In some embodiments, fusion proteins of HRS polypeptide to other (non HARS) proteins (e.g. heterologous proteins or polypeptides) are also included, and these fusion proteins may modulate the HRS polypeptide's biological activity, secretion, antigenicity, targeting, biological life, ability to penetrate cellular membranes, or the blood brain barrier, or pharmacokinetic properties. Examples of fusion proteins that improve pharmacokinetic properties ("PK modifiers") include without limitation, fusions to human albumin (Osborn et al.: Eur. J. Pharmacol. 456(1-3): 149-158, (2002)), antibody Fc domains, poly Glu or poly Asp sequences, and transferrin. Additionally, fusion with conformationally disordered polypeptide sequences composed of the amino acids Pro, Ala, and Ser ('PASylation') or hydroxyethyl starch (sold under the trademark HESYLATION^{®}) provides a simple way to increase the hydrodynamic volume of the HRS polypeptide. This additional extension adopts a bulky random structure, which significantly increases the size of the resulting fusion protein. By this means the typically rapid clearance of smaller HRS polypeptides via kidney filtration is retarded by several orders of magnitude. Additionally use of Ig G fusion proteins has also been shown to enable some fusion protein proteins to penetrate the blood brain barrier (Fu et al., (2010) Brain Res. 1352:208-13).

Examples of fusion proteins that modulate the antigenicity, or other properties of the HRS polypeptide, include fusions to T cell binding ligands, including for example, MHC Class I and II proteins, b-2 microglobulin, portions of LFA-3, portions of the Fc region of the heavy chain, and conjugates and derivatives thereof. Examples of such fusion proteins are described EP 1 964 854; U.S. Patent Nos. 5,468,481; 5,130,297; 5,635,363; and 6,451,314; and U.S. Application No. 2009/0280135.

In some embodiments, the HRS polypeptide can include synthetic, or naturally occurring secretion signal sequences, derived from other well characterized secreted proteins. In some embodiments such proteins, may be processed by proteolytic cleavage to form the HRS polypeptide in situ. In some embodiments the HRS polypeptide can comprise heterologous proteolytic cleavage sites, to enable the in situ expression, and production of the HRS polypeptide either at an intracellular, or an extracellular location. Other fusions proteins may also include for example fusions of HRS polypeptide to ubiquitin to provide a new N-terminal amino acid, or the use of a secretion signal to mediate high level secretion of the HRS polypeptide into the extracellular medium, or N, or C-terminal epitope tags to improve purification or detection, and fusions to cell penetrating peptides.

In certain aspects, the use of non-natural amino acids can be utilized to modify (e.g., increase) a selected non-canonical activity of a HRS polypeptide, or to alter the *in vivo* or *in vitro* half-life of the protein. Non-natural amino acids can also be used to facilitate (selective) chemical modifications (e.g., pegylation) of a HRS protein, as described elsewhere herein. For instance, certain non-natural amino acids allow selective attachment of polymers such as PEG to a given protein, and thereby improve their pharmacokinetic properties.

Certain embodiments include HRS-Fc conjugates, which comprise at least one Fc region that is covalently attached to one or more HRS polypeptides. Examples of HRS-Fc conjugates include fusion proteins and various forms of chemically cross-linked proteins. A wide variety of Fc region sequences may be employed in the HRS-Fc conjugates, including wild-type sequences from any number of species, as well as variants, fragments, hybrids, and chemically modified forms thereof. The HRS-Fc polypeptides may also (optionally) comprise one or more linkers, which typically separate the Fc region(s) from the HRS polypeptide(s), including peptide linkers and chemical linkers, as described herein and known in the art. It will be appreciated that in any of these HRS-Fc conjugates the native N or C terminal amino acid of the HRS polypeptides, or native N or C- amino acid in the Fc domain, may be deleted and/or replaced with non-native amino acid(s), for example, to facilitate expression and or cloning or to serve as a linker sequence between the two proteins.

HRS-Fc conjugate polypeptides can provide a variety of advantages relative to un-conjugated or unmodified HRS polypeptides, e.g., corresponding HRS polypeptides of the same or similar sequence having no Fc region(s) attached thereto. Merely by way of illustration, the covalent attachment of one or more Fc regions can alter (e.g., increase, decrease) the HRS polypeptide's solubility, half-life *(e.g.,* in serum, in a selected tissue, in a test tube under storage conditions, for example, at room temperature or under refrigeration), dimerization or multimerization properties, biological activity or activities, for instance, by providing Fc-region-associated effector functions (e.g., activation of the classical complement cascade, interaction with immune effector cells via the Fc receptor (FcR), compartmentalization of immunoglobulins), cellular uptake, intracellular transport, tissue distribution, and/or bioavailability, relative to an unmodified HRS polypeptide having the same or similar sequence. In certain aspects, Fc regions can confer effector functions relating to complement-dependent cytotoxicity (CDC), antibody-dependent cell-mediated cytotoxicity (ADCC), and/or antibody-dependent cell-mediated phagocytocis (ADCP), which are believed to play a role in clearing specific target cells such as tumor cells and infected cells.

Certain embodiments employ HRS-Fc fusion proteins. "Fusion proteins" are defined elsewhere herein and well known in the art, as are methods of making fusion proteins *(see, e.g.,* U.S. Patent Nos. 5,116,964; 5,428,130; 5,455,165; 5,514,582; 6,406,697; 6,291,212; and 6,300,099 for general disclosure and methods related to Fc fusion proteins). In a HRS-Fc fusion protein, the Fc region can be fused to the N-terminus of the HRS polypeptide, the C-terminus, or both. In some embodiments, one or more Fc regions can be fused internally relative to HRS sequences, for instance, by placing an Fc region between a first HRS sequence (e.g., domain) and a second HRS sequence (e.g., domain), where the first HRS sequence is fused to the N-terminus of the Fc region and the second HRS sequence is fused to the C-terminus of the Fc region. In specific embodiments, the first and second HRS sequences are identical. In some embodiments, the first and second HRS sequences are different (e.g., they include different functional domains of the HRS polypeptide). Certain HRS-Fc fusion proteins can also include additional heterologous protein sequences, that is, non-Fc region and non-HRS polypeptide sequences.

The term "HRS-Fc" can indicate, but does not necessarily indicate, the N-terminal or C-terminal attachment of the Fc region to the HRS polypeptide. For instance, in certain instances the term "Fc-HRS" indicates fusion of the Fc region to the N-terminus of the HRS polypeptide, and the term "HRS-Fc" indicates fusion of the Fc region to the C-terminus of the HRS polypeptide. However, either term can be used more generally to refer to any fusion protein or conjugate of an Fc region and a HRS polypeptide.

In some embodiments the HRS-Fc fusion proteins may comprise tandemly repeated copies of the HRS polypeptide coupled to a single Fc domain, optionally separated by linker peptides. Exemplary tandemly repeated HRS-Fc fusion proteins are provided in Table H5. The preparation and sequences for specific tandemly repeated HRS-Fc conjugates are illustrated in the Examples.

| **Table H5. Exemplary Tandem HRS-Fc conjugates** |
|---|
| HRS polypeptide-L-HRS-polypeptide-L-Fc |
| HRS-polypeptide-L-HRS-polypeptide-L-HRS-polypeptide-L-Fc |
| HRS-polypeptide-L-HRS-polypeptide-L-HRS-polypeptide-L-HRS-polypeptide-L-Fc |
| Fc-L-HRS-polypeptide-L-HRS-polypeptide |
| Fc-L-HRS-polypeptide-L-HRS-L-HRS-polypeptide |
| Fc-L-HRS-polypeptide-L-HRS-L-HRS-L-HRS-polypeptide |
| Where: |
| "Fc" is an Fc domain as described herein |
| "HRS-polypeptide" is an HRS polypeptide as described herein |
| "L" is an optional peptide linker |

Certain embodiments relate to HRS-Fc conjugates, where, for instance, one or more Fc regions are chemically conjugated or cross-linked to the HRS polypeptide(s). In these and related aspects, the Fc region can be conjugated to the HRS polypeptide at the N-terminal region (e.g., within the first 10, 20, 30, 40, 50, 60, 70, 80, 90, 100 or so amino acids), the internal region (between the N-terminal and C-terminal regions), and/or the C-terminal region (e.g., within the last 10, 20, 30, 40, 50, 60, 70, 80, 90, 100 or so amino acids). Polypeptides can be conjugated or cross-linked to other polypeptides according to a variety of routine techniques in the art. For instance, certain techniques employ the carboxyl-reactive carbodiimide crosslinker EDC (or EDAC), which covalently attaches via D, E, and C-terminal carboxyl groups. Other techniques employ activated EDC, which covalently attaches via K and N-terminal amino groups). Still other techniques employ m-maleimidobenzoyl-N-hydoxysuccinimide ester (MBS) or Sulfo-MBS, which covalently attach via the thiol group of a cysteine residue *(see* also U.S. Application No. 2007/0092940 for cysteine engineered Ig regions that can be used for thiol conjugation). Such cross-linked proteins can also comprise linkers, including cleavable or otherwise releasable linkers (e.g., enzymatically cleavable linkers, hydrolysable linkers), and non-cleavable linkers *(i.e.,* physiologically-stable linkers). Certain embodiments may employ non-peptide polymers (e.g., PEG polymers; HRS-N-PEG-N-Fc conjugate) as a cross-linker between the Fc region(s) and the HRS polypeptide(s), as described, for example, in U.S. Application No. 2006/0269553. *See also* US Application No. 2007/0269369 for exemplary descriptions of Fc region conjugation sites.

In certain embodiments, discussed in greater detail below, variant or otherwise modified Fc regions can be employed, including those having altered properties or biological activities relative to wild-type Fc region(s). Examples of modified Fc regions include those having mutated sequences, for instance, by substitution, insertion, deletion, or truncation of one or more amino acids relative to a wild-type sequence, hybrid Fc polypeptides composed of domains from different immunoglobulin classes/subclasses, Fc polypeptides having altered glycosylation/sialylation patterns, and Fc polypeptides that are modified or derivatized, for example, by biotinylation *(see, e.g.,* US Application No. 2010/0209424), phosphorylation, sulfation, etc., or any combination of the foregoing. Such modifications can be employed to alter (e.g., increase, decrease) the binding properties of the Fc region to one or more particular FcRs (e.g., FcyRI, FcγRIIa, FcγRIIb, FcγRIIc, FcγRIIIa, FcγRIIIb, FcRn), its pharmacokinetic properties (e.g., stability or half-life, bioavailability, tissue distribution, volume of distribution, concentration, elimination rate constant, elimination rate, area under the curve (AUC), clearance, Cₘₐₓ, tₘₐₓ, Cₘᵢₙ, fluctuation), its immunogenicity, its complement fixation or activation, and/or the CDC/ADCC/ADCP-related activities of the Fc region, among other properties described herein, relative to a corresponding wild-type Fc sequence.

The "Fc region" of a HRS-Fc conjugate provided herein is usually derived from the heavy chain of an immunoglobulin (Ig) molecule. A typical Ig molecule is composed of two heavy chains and two light chains. The heavy chains can be divided into at least three functional regions: the Fd region, the Fc region (fragment crystallizable region), and the hinge region, the latter being found only in IgG, IgA, and IgD immunoglobulins. The Fd region comprises the variable (V_{H}) and constant (CH₁) domains of the heavy chains, and together with the variable (V_{L}) and constant (C_{L}) domains of the light chains forms the antigen-binding fragment or Fab region.

The Fc region of IgG, IgA, and IgD immunoglobulins comprises the heavy chain constant domains 2 and 3, designated respectively as CH₂ and CH₃ regions; and the Fc region of IgE and IgM immunoglobulins comprises the heavy chain constant domains 2, 3, and 4, designated respectively as CH₂, CH₃, and CH₄ regions. The Fc region is mainly responsible for the immunoglobulin effector functions, which include, for example, complement fixation and binding to cognate Fc receptors of effector cells.

The hinge region (found in IgG, IgA, and IgD) acts as a flexible spacer that allows the Fab portion to move freely in space relative to the Fc region. In contrast to the constant regions, the hinge regions are structurally diverse, varying in both sequence and length among immunoglobulin classes and subclasses. The hinge region may also contain one or more glycosylation site(s), which include a number of structurally distinct types of sites for carbohydrate attachment. For example, IgA1 contains five glycosylation sites within a 17 amino acid segment of the hinge region, conferring significant resistance of the hinge region polypeptide to intestinal proteases. Residues in the hinge proximal region of the CH₂ domain can also influence the specificity of the interaction between an immunoglobulin and its respective Fc receptor(s) *(see, e.g.,* Shin et al., Intern. Rev. Immunol. 10:177-186, 1993).

The term "Fc region" or "Fc fragment" or "Fc" as used herein, thus refers to a protein that contains one or more of a CH₂ region, a CH₃ region, and/or a CH₄ region from one or more selected immunoglobulin(s), including fragments and variants and combinations thereof. An "Fc region" may also include one or more hinge region(s) of the heavy chain constant region of an immunoglobulin. In certain embodiments, the Fc region does not contain one or more of the CH₁, C_{L}, V_{L}, and/or V_{H} regions of an immunoglobulin.

The Fc region can be derived from the CH₂ region, CH₃ region, CH₄ region, and/or hinge region(s) of any one or more immunoglobulin classes, including but not limited to IgA, IgD, IgE, IgG, IgM, including subclasses and combinations thereof. In some embodiments, the Fc region is derived from an IgA immunoglobulin, including subclasses IgA1 and/or IgA2. In certain embodiments, the Fc region is derived from an IgD immunoglobulin. In particular embodiments, the Fc region is derived from an IgE immunoglobulin. In some embodiments, the Fc region is derived from an IgG immunoglobulin, including subclasses IgG1, IgG2, IgG2, IgG3, and/or IgG4. In certain embodiments, the Fc region is derived from an IgM immunoglobulin.

Certain Fc regions demonstrate specific binding for one or more Fc-receptors (FcRs). Examples of classes of Fc receptors include Fcγ receptors (FcγR), Fcα receptors (FcαR), Fcε receptors (FcεR), and the neonatal Fc receptor (FcRn). For instance, certain Fc regions have increased binding to (or affinity for) one or more FcγRs, relative to FcαRs, FcεRs, and/or FcRn. In some embodiments, Fc regions have increased binding to FcαRs, relative to one or more FcγRs, FcεRs, and/or FcRn. In some embodiments, Fc regions have increased binding to FcεRs (*e.g.,* FcαRI), relative to one or more FcγRs, FcαRs, and/or FcRn. In particular embodiments, Fc regions have increased binding to FcRn, relative to one or more FcγRs, FcαRs, and/or FcεRs. In certain embodiments, the binding (or affinity) of an Fc region to one or more selected FcR(s) is increased relative to its binding to (or affinity for) one or more different FcR(s), typically by about 1.5x, 2x, 2.5x, 3x, 3.5x, 4x, 4.5x, 5x, 6x, 7x, 8x, 9x, 10x, 15x, 20x, 25x, 30x, 40x, 50x, 60x, 70x, 80x, 90x, 100x, 200x, 300x, 400x, 500x, 600x, 700x, 800x, 900x, 1000x or more (including all integers in between).

Examples of FcγRs include FcyRI, FcγRIIa, FcγRIIb, FcyRIIc, FcγRIIIa, and FcγRIIIb. FcγRI (CD64) is expressed on macrophages and dendritic cells and plays a role in phagocytosis, respiratory burst, cytokine stimulation, and dendritic cell endocytic transport. Expression of FcγRI is upregulated by both GM-CSF and γ-interferon (γ-IFN) and downregulated by interleukin-4 (IL-4). FcγRIIa is expressed on polymorphonuclear leukocytes (PMN), macrophages, dendritic cells, and mast cells. FcγRIIa plays a role in phagocytosis, respiratory burst, and cytokine stimulation. Expression of FcγRIIa is upregulated by GM-CSF and γ-IFN, and decreased by IL-4. FcγIIb is expressed on B cells, PMN, macrophages, and mast cells. FcγIIb inhibits immunoreceptor tyrosine-based activation motif (ITAM) mediated responses, and is thus an inhibitory receptor. Expression of FcγRIIc is upregulated by intravenous immunoglobulin (IVIG) and IL-4 and decreased by γ-IFN. FcγRIIc is expressed on NK cells. FcγRIIIa is expressed on natural killer (NK) cells, macrophages, mast cells, and platelets. This receptor participates in phagocytosis, respiratory burst, cytokine stimulation, platelet aggregation and degranulation, and NK-mediated ADCC. Expression of FcyRIII is upregulated by C5a, TGF-β, and γ-IFN and downregulated by IL-4. Fc y RIIIb is a GPI-linked receptor expressed on PMN.

Certain Fc regions have increased binding to FcyRI, relative to FcγRIIa, FcγRIIb, FcγRIIc, FcγRIIIa, and/or FcγRIIIb. Some embodiments have increased binding to FcγRIIa, relative to FcyRI, FcγRIIb, FcyRIIc, FcγRIIIa, and/or FcγRIIIb. Particular Fc regions have increased binding to FcγRIIb, relative to FcyRI, FcγRIIa, FcyRIIc, FcγRIIIa, and/or FcγRIIIb. Certain Fc regions have increased binding to FcγRIIc, relative to FcyRI, FcγRIIa, FcγRIIb, FcγRIIIa, and/or FcγRIIIb. Some Fc regions have increased binding to FcγRIIIa, relative to FcyRI, FcγRIIa, FcγRIIb, FcyRIIc, and/or FcγRIIIb. Specific Fc regions have increased binding to FcyRIIIb, relative to FcyRI, FcγRIIa, FcγRIIb, FcyRIIc, and/or FcγRIIIa.

FcαRs include FcαRI (CD89). FcαRI is found on the surface of neutrophils, eosinophils, monocytes, certain macrophages (e.g., Kupffer cells), and certain dendritic cells. FcαRI is composed of two extracellular Ig-like domains, is a member of both the immunoglobulin superfamily and the multi-chain immune recognition receptor (MIRR) family, and signals by associating with two FcRy signaling chains.

FcεRs include FcεRI and FcεRII. The high-affinity receptor FcεRI is a member of the immunoglobulin superfamily, is expressed on epidermal Langerhans cells, eosinophils, mast cells and basophils, and plays a major role in controlling allergic responses. FcεRI is also expressed on antigen-presenting cells, and regulates the production pro-inflammatory cytokines. The low-affinity receptor FcεRII (CD23) is a C-type lectin that can function as a membrane-bound or soluble receptor. FcεRII regulates B cell growth and differentiation, and blocks IgE-binding of eosinophils, monocytes, and basophils. Certain Fc regions have increased binding to FcεRI, relative to FcεRII. Other Fc regions have increased binding to FcεRII, relative to FcεRI.

**Table H6** below summarizes the characteristics of certain FcRs.

| **Table H6. Exemplary Fc-Receptors** | | | | |
|---|---|---|---|---|
| **Receptor** | **Primary Antibody Ligand** | **Ligand Affinity** | **Cell Distribution** | **Exemplary Effects Following Binding to Fc Ligand** |
| **FcγRI (CD64)** | IgG1 and IgG3 | High (Kd ~ 10⁻⁹ M) | Macrophages | Phagocytosis |
| | | | Neutrophils | Cell activation |
| | | | Eosinophils | Activation of respiratory burst |
| | | | Dendritic cells | Induction of microbe killing |
| **FcγRIIa (CD32)** | IgG | Low (Kd > 10⁻⁷ M) | Macrophages | Phagocytosis |
| | | | Neutrophils | Degranulation (eosinophils) |
| | | | Eosinophils | |
| | | | Platelets | |
| | | | Langerhans cells | |
| **FcγRIIb1 (CD32)** | IgG | Low (Kd > 10⁻⁷ M) | B Cells | No phagocytosis |
| | | | Mast cells | Inhibition of cell activity |
| **FcγRIIb2 (CD32)** | IgG | Low (Kd > 10⁻⁷ M) | Macrophages | Phagocytosis |
| | | | Neutrophils | Inhibition of cell activity |
| | | | Eosinophils | |
| **FcγRIIIa (CD16a)** | IgG | Low (Kd > 10⁻⁶ M) | NK cells | Induction of antibody-dependent cell-mediated cytotoxicity (ADCC) |
| | | | Macrophages (certain tissues) | |
| | | | | Induction of cytokine release by macrophages |
| **FcγRIIIb (CD16b)** | IgG | Low (Kd > 10⁻⁶ M) | Eosinophils | Induction of microbe killing |
| | | | Macrophages | |
| | | | Neutrophils | |
| | | | Mast cells | |
| | | | Follicular dendritic cells | |
| **FcεRI** | IgE | High (Kd ~ 10⁻¹⁰ M) | Mast cells | Degranulation |
| | | | Eosinophils | |
| | | | Basophils | |
| | | | Langerhans cells | |
| **FcεRII (CD23)** | IgE | Low (Kd > 10⁻⁷ M) | B cells | Possible adhesion molecule |
| | | | Eosinophils | |
| | | | Langerhans cells | |
| **FcαRI (CD89)** | IgA | Low (Kd > 10⁻⁶ M) | Monocytes | Phagocytosis |
| | | | Macrophages | Induction of microbe killing |
| | | | Neutrophils | |
| | | | Eosinophils | |
| **Fcα/µR** | IgA and IgM | High for IgM, Moderate for IgA | B cells | Endocytosis |
| | | | Mesangial cells | Induction of microbe killing |
| | | | Macrophages | |
| **FcRn** | IgG | | Monocytes | Transfers IgG from a mother to fetus through the placenta |
| | | | Macrophages | |
| | | | Dendrite cells | Transfers IgG from a mother to infant in milk |
| | | | Epithelial cells | |
| | | | Endothelial cells | Protects IgG from degradation |
| | | | Hepatocytes | |

Fc regions can be derived from the immunoglobulin molecules of any animal, including vertebrates such as mammals such cows, goats, swine, dogs, mice, rabbits, hamsters, rats, guinea pigs, non-human primates, and humans. The amino acid sequences of CH₂, CH₃, CH₄, and hinge regions from exemplary, wild-type human IgA1, IgA2, IgD, IgE, IgG1, IgG2, IgG3, IgG4, and IgM immunoglobulins are shown in **Table H7.**

| **Table H7. Exemplary Fc sequences** | | |
|---|---|---|
| Name | Sequence | SEQ ID NO: |
| IgA1 hinge | VPSTPPTPSPSTPPTPSPS | 127 |
| IgA1 CH2 | CCHPRLSLHRPALEDLLLGSEANLTCTLTGLRDASGVTFTWTPSSGKSAVQ GPPERDLCGCYSVSSVLPGCAEPWNHGKTFTCTAAYPESKTPLTATLSKS | 128 |
| IgA1 CH3 | GNTFRPEVHLLPPPSEELALNELVTLTCLARGFSPKDVLVRWLQGSQELPR EKYLTWASRQEPSQGTTTFAVTSILRVAAEDWKKGDTFSCMVGHEALPLAF TQKTIDRLAGKPTHVNVSVVMAEVDGTCY | 129 |
| IgA2 hinge | VPPPPP | 130 |
| IgA2 CH2 | CCHPRLSLHRPALEDLLLGSEANLTCTLTGLRDASGATFTWTPSSGKSAVQ GPPERDLCGCYSVSSVLPGCAQPWNHGETFTCTAAHPELKTPLTANITKS | 131 |
| IgA2 CH3 | GNTFRPEVHLLPPPSEELALNELVTLTCLARGFSPKDVLVRWLQGSQELPR EKYLTWASRQEPSQGTTTFAVTSILRVAAEDWKKGDTFSCMVGHEALPLAF TQKTIDRLAGKPTHVNVSVVMAEVDGTCY | 132 |
| IgD hinge | ESPKAQASSVPTAQPQAEGSLAKATTAPATTRNTGRGGEEKKKEKEKEEQE ERETKTP | 133 |
| IgD CH2 | ECPSHTQPLGVYLLTPAVQDLWLRDKATFTCFVVGSDLKDAHLTWEVAGKV PTGGVEEGLLERHSNGSQSQHSRLTLPRSLWNAGTSVTCTLNHPSLPPQRL MALREP | 134 |
| IgD CH3 | AAQAPVKLSLNLLASSDPPEAASWLLCEVSGFSPPNILLMWLEDQREVNTS GFAPARPPPQPRSTTFWAWSVLRVPAPPSPQPATYTCVVSHEDSRTLLNAS RSLEVSYVTDHGPMK | 135 |
| IgE CH2 | VCSRDFTPPTVKILQSSCDGGGHFPPTIQLLCLVSGYTPGTINITWLEDGQ VMDVDLSTASTTQEGELASTQSELTLSQKHWLSDRTYTCQVTYQGHTFEDS TKKCA | 136 |
| IgE CH3 | DSNPRGVSAYLSRPSPFDLFIRKSPTITCLVVDLAPSKGTVNLTWSRASGK PVNHSTRKEEKQRNGTLTVTSTLPVGTRDWIEGETYQCRVTHPHLPRALMR STTKTS | 137 |
| IgE CH4 | GPRAAPEVYAFATPEWPGSRDKRTLACLIQNFMPEDISVQWLHNEVQLPDA RHSTTQPRKTKGSGFFVFSRLEVTRAEWEQKDEFICRAVHEAASPSQTVQR AVSVNPGK | 138 |
| IgG1 hinge | EPKSCDKTHTCPPCP | 139 |
| modified human IgG1 hinge | SDKTHTCPPCP | 140 |
| IgG1 CH2 | APELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPI EKTISKAK | 141 |
| IgG1 CH3 | GQPREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKSLS LSPGK | 142 |
| IgG1 heavy chain | MSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKG FYPSDIAVEWESNGQPENNYKTTPPVLDSDGSFFLYSKLTVDKSRWQQGNV FSCSVMHEALHNHYTQKSLSLSPGK | 143 |
| IgG2 hinge | ERKCCVECPPCP | 144 |
| IgG2 CH2 | APPVAGPSVFLFPPKPKDTLMISRTPEVTCVVVDVSHEDPEVQFNWYVDGV EVHNAKTKPREEQFNSTFRVVSVLTVVHQDWLNGKEYKCKVSNKGLPAPIE KTISKTK | 145 |
| IgG2 CH3 | GQPREPQVYTLPPSREEMTKNQVSLTCLVKGFYPSDIAVEWESNGQPENNY KTTPPMLDSDGSFFLYSKLTVDKSRWQQGNVFSCSVMHEALHNHYTQKSLS LSPGK | 146 |
| IgG3 hinge | ELKTPLGDTTHTCPRCPEPKSCDTPPPCPRCPEPKSCDTPPPCPRCPEPKS CDTPPPCPRCP | 147 |
| IgG3 CH2 | APELLGGPSVFLFPPKPKDTLMISRTPEVTCVVVDVSHEDPEVQFKWYVDG VEVHNAKTKPREEQYNSTFRVVSVLTVLHQDWLNGKEYKCKVSNKALPAPI EKTISKTK | 148 |
| IgG3 CH3 | GQPREPQVYTLPPSREEMTKNQVSLTCLVKGFYPSDIAVEWESSGQPENNY NTTPPMLDSDGSFFLYSKLTVDKSRWQQGNIFSCSVMHEALHNRFTQKSLS LSPGK | 149 |
| IgG4 hinge | ESKYGPPCPSCP | 150 |
| IgG4 CH2 | APEFLGGPSVFLFPPKPKDTLMISRTPEVTCVVVDVSQEDPEVQFNWYVDG VEVHNAKTKPREEQFNSTYRVVSVLTVLHQDWLNGKEYKCKVSNKGLPSSI EKTISKAK | 151 |
| IgG4 CH3 | GQPREPQVYTLPPSQEEMTKNQVSLTCLVKGFYPSDIAVEWESNGQPENNY KTTPPVLDSDGSFFLYSRLTVDKSRWQEGNVFSCSVMHEALHNHYTQKSLS LSLGK | 152 |
| IgM CH2 | VIAELPPKVSVFVPPRDGFFGNPRKSKLICQATGFSPRQIQVSWLREGKQV GSGVTTDQVQAEAKESGPTTYKVTSTLTIKESDWLGQSMFTCRVDHRGLTF QQNASSMCVP | 153 |
| IgM CH3 | DQDTAIRVFAIPPSFASIFLTKSTKLTCLVTDLTTYDSVTISWTRQNGEAV KTHTNISESHPNATFSAVGEASICEDDWNSGERFTCTVTHTDLPSPLKQTI SRPK | 154 |
| IgM CH4 | GVALHRPDVYLLPPAREQLNLRESATITCLVTGFSPADVFVQWMQRGQPLS PEKYVTSAPMPEPQAPGRYFAHSILTVSEEEWNTGETYTCVVAHEALPNRV TERTVDKSTGKPTLYNVSLVMSDTAGTCY | 155 |

An Fc region of an HRS-Fc conjugate can thus comprise, consist of, or consist essentially of one or more of the human Fc region amino acid sequences of **Table H7,** including variants, fragments, homologs, orthologs, paralogs, and combinations thereof. Certain illustrative embodiments comprise an Fc region that ranges in size from about 20-50, 20-100, 20-150, 20-200, 20-250, 20-300, 20-400, 50-100, 50-150, 50-200, 50-250, 50-300, 50-400, 100-150, 100-200, 100-250, 100-300, 100-350, 100-400, 200-250, 200-300, 200-350, or 200-400 amino acids in length, and optionally comprises, consists of, or consists essentially of any one or more of the sequences in **Table H7.** Certain embodiments comprise an Fc region of up to about 50, 60, 70, 80, 90, 100, 110, 120, 130, 140, 150, 160, 170, 180, 190, 200, 210, 220, 230, 240, 250, 300, 350, 400 or more amino acids, which optionally comprises, consists of, or consists essentially of any one or more of the amino acid sequences of **Table H7.**

Certain Fc regions comprise, consist of, or consist essentially of human IgA1 sequences of **Table H7,** in any order reading from N-terminus to C-terminus, including combinations thereof, and variants and fragments thereof. Certain Fc regions comprise, consist of, or consist essentially of human the IgA1 sequence of **Table H7.** Certain Fc regions comprise, consist of, or consist essentially of the human IgA1 sequence of **Table H7.** Certain Fc regions comprise, consist of, or consist essentially of the human IgA1 sequence of **Table H7.**

Some Fc regions comprise, consist of, or consist essentially of human IgA2 sequences of **Table H7, in** any order reading from N-terminus to C-terminus, including combinations thereof, and variants and fragments thereof. Certain Fc regions comprise, consist of, or consist essentially of human the IgA2 sequence of **Table H7.** Certain Fc regions comprise, consist of, or consist essentially of the human IgA2 sequence of **Table H7.** Certain Fc regions comprise, consist of, or consist essentially of the human IgA2 sequence of **Table H7.**

Certain Fc regions comprise, consist of, or consist essentially of human IgD sequences of **Table H7,** in any order reading from N-terminus to C-terminus, including combinations thereof, and variants and fragments of these sequences and combinations. Certain Fc regions comprise, consist of, or consist essentially of human IgE sequences of **Table H7,** in any order reading from N-terminus to C-terminus, including combinations thereof, and variants and fragments of these sequences and combinations. Certain Fc regions comprise, consist of, or consist essentially of human IgG1 sequences of **Table H7,** in any order reading from N-terminus to C-terminus, including combinations thereof, and variants and fragments of these sequences and combinations. Certain Fc regions comprise, consist of, or consist essentially of human IgG2 sequences of **Table H7,** in any order reading from N-terminus to C-terminus, including combinations thereof. Certain Fc regions comprise, consist of, or consist essentially of human IgG3 sequences of **Table H7,** in any order reading from N-terminus to C-terminus, including combinations thereof. Certain Fc regions comprise, consist of, or consist essentially of human IgG4 sequences of **Table H7,** in any order reading from N-terminus to C-terminus, including combinations thereof Certain Fc regions comprise, consist of, or consist essentially of human IgM sequences of **Table H7,** in any order reading from N-terminus to C-terminus, including combinations thereof, and variants and fragments of these sequences and combinations.

Exemplary HRS-Fc fusion conjugates are provided in **Table H8** below.

| **Table H8. Exemplary HRS-Fc fusion proteins** | | |
|---|---|---|
| Name | Sequence | SEQ ID NO: |
| Fc-HRS (2-60) **HRS^{FC1}** | MDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLN GKEYKCKVSNKALPAPIEKTISKAKGQPREPQVYTLPPSRDELTKNQV SLTCLVKGFYPSDIAVEWESNGQPENNYKTTPPVLDSDGSFFLYSKLT VDKSRWQQGNVFSCSVMHEALHNHYTQKSLSLSPGKAERAALEELVKL QGERVRGLKQQKASAELIEEEVAKLLKLKAQLGPDESKQKFVLKTPK | 156 |
| Fc-HRS(2-60) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDESKQKFVLKTPK | 157 |
| HRS (1-60) -Fc | MAERAALEELVKLQGERVRGLKQQKASAELIEEEVAKLLKLKAQLGPD ESKQKFVLKTPKSDKTHTCPPCPAPELLGGPSVFLFPPKPKDTLMISR TPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTL PPSREEMTKNQVSLTCLVKGFYPSDIAVEWESNGQPENNYKTTPPVLD SDGSFFLYSKLTVDKSRWQQGNVFSCSVMHEALHNHYTQKSLSLSPGK | 158 |
| Fc-HRS(2-60) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDESKQKFVLKTPK | 159 |
| HRS (1-60) -Fc | MAERAALEELVKLQGERVRGLKQQKASAELIEEEVAKLLKLKAQLGPD ESKQKFVLKTPKSDKTHTCPPCPAPELLGGPSVFLFPPKPKDTLMISR TPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTL PPSREEMTKNQVSLTCLVKGFYPSDIAVEWESNGQPENNYKTTPPVLD SDGSFFLYSKLTVDKSRWQQGNVFSCSVMHEALHNHYTQKSLSLSPGK | 160 |
| Fc-HRS (2-40) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLK | 161 |
| Fc-HRS (2-45) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQL | 162 |
| Fc-HRS (2-50) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDES | 163 |
| Fc-HRS (2-55) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDESKQKFV | 164 |
| Fc-HRS (2-66) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDESKQKFVLKTPK GTRDYS | 165 |
| HRS (1-40) -Fc | MAERAALEELVKLQGERVRGLKQQKASAELIEEEVAKLLKSDKTHTCP PCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKV SNKALPAPIEKTISKAKGQPREPQVYTLPPSREEMTKNQVSLTCLVKG FYPSDIAVEWESNGQPENNYKTTPPVLDSDGSFFLYSKLTVDKSRWQQ GNVFSCSVMHEALHNHYTQKSLSLSPGK | 166 |
| HRS (1-45) -Fc | MAERAALEELVKLQGERVRGLKQQKASAELIEEEVAKLLKLKAQLSDK THTCPPCPAPELLGGPSVFLFPPKPKDTLMISRTPEVTCVVVDVSHED PEVKFNWYVDGVEVHNAKTKPREEQYNSTYRVVSVLTVLHQDWLNGKE YKCKVSNKALPAPIEKTISKAKGQPREPQVYTLPPSREEMTKNQVSLT CLVKGFYPSDIAVEWESNGQPENNYKTTPPVLDSDGSFFLYSKLTVDK SRWQQGNVFSCSVMHEALHNHYTQKSLSLSPGK | 167 |
| HRS (1-50) -Fc | MAERAALEELVKLQGERVRGLKQQKASAELIEEEVAKLLKLKAQLGPD ESSDKTHTCPPCPAPELLGGPSVFLFPPKPKDTLMISRTPEVTCVVVD VSHEDPEVKFNWYVDGVEVHNAKTKPREEQYNSTYRVVSVLTVLHQDW LNGKEYKCKVSNKALPAPIEKTISKAKGQPREPQVYTLPPSREEMTKN QVSLTCLVKGFYPSDIAVEWESNGQPENNYKTTPPVLDSDGSFFLYSK LTVDKSRWQQGNVFSCSVMHEALHNHYTQKSLSLSPGK | 167 |
| HRS (1-55) -Fc | MAERAALEELVKLQGERVRGLKQQKASAELIEEEVAKLLKLKAQLGPD ESKQKFVSDKTHTCPPCPAPELLGGPSVFLFPPKPKDTLMISRTPEVT CVVVDVSHEDPEVKFNWYVDGVEVHNAKTKPREEQYNSTYRVVSVLTV LHQDWLNGKEYKCKVSNKALPAPIEKTISKAKGQPREPQVYTLPPSRE EMTKNQVSLTCLVKGFYPSDIAVEWESNGQPENNYKTTPPVLDSDGSF FLYSKLTVDKSRWQQGNVFSCSVMHEALHNHYTQKSLSLSPGK | 169 |
| HRS (1-66) -Fc | MAERAALEELVKLQGERVRGLKQQKASAELIEEEVAKLLKLKAQLGPD ESKQKFVLKTPKGTRDYSSDKTHTCPPCPAPELLGGPSVFLFPPKPKD TLMISRTPEVTCVVVDVSHEDPEVKFNWYVDGVEVHNAKTKPREEQYN STYRVVSVLTVLHQDWLNGKEYKCKVSNKALPAPIEKTISKAKGQPRE PQVYTLPPSREEMTKNQVSLTCLVKGFYPSDIAVEWESNGQPENNYKT TPPVLDSDGSFFLYSKLTVDKSRWQQGNVFSCSVMHEALHNHYTQKSL SLSPGK | 170 |
| Fc-HRS(2-60) HRS(2-60) | MSDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGKAERAALEELVK LQGERVRGLKQQKASAELIEEEVAKLLKLKAQLGPDESKQKFVLKTPK AERAALEELVKLQGERVRGLKQQKASAELIEEEVAKLLKLKAQLGPDE SKQKFVLKTPK | 171 |

Accordingly, in certain embodiments, the HRS polypeptide is fused or otherwise conjugated to an Fc region and comprises, consists, or consists essentially of an amino acid sequence that is at least 90%, 95%, 96%, 97%, 98%, 99%, or 100% identical to a sequence in **Table H8** (SEQ ID NO: 156-171).

As noted above, certain embodiments employ variants, fragments, hybrids, and/or otherwise modified forms an Fc region described herein and known in the art. Included are variants having one or more amino acid substitutions, insertions, deletions, and/or truncations relative to a reference sequence, such as any one or more of the reference sequences of **Table H7** or **Table H8.** Polypeptide variants are described elsewhere herein.

Also included are hybrid Fc regions, for example, Fc regions that comprise a combination of Fc domains (e.g., hinge, CH₂, CH₃, CH₄) from immunoglobulins of different species, different Ig classes, and/or different Ig subclasses. General examples include hybrid Fc regions that comprise, consist of, or consist essentially of the following combination of CH₂/CH₃ domains: IgA1/IgA1, IgA1/IgA2, IgA1/IgD, IgA1/IgE, IgA1/IgG1, IgA1/IgG2, IgA1/IgG3, IgA1/IgG4, IgA1/IgM, IgA2/IgA1, IgA2/IgA2, IgA2/IgD, IgA2/IgE, IgA2/IgG1, IgA2/IgG2, IgA2/IgG3, IgA2/IgG4, IgA2/IgM, IgD/IgA1, IgD/IgA2, IgD/IgD, IgD/IgE, IgD/IgG1, IgD/IgG2, IgD/IgG3, IgD/IgG4, IgD/IgM, IgE/IgA1, IgE/IgA2, IgE/IgD, IgE/IgE, IgE/IgG1, IgE/IgG2, IgE/IgG3, IgE/IgG4, IgE/IgM, IgG1/IgA1, IgG1/IgA2, IgG1/IgD, IgG1/IgE, IgG1/IgG1, IgG1/IgG2, IgG1/IgG3, IgG1/IgG4, IgG1/IgM, IgG2/IgA1, IgG2/IgA2, IgG2/IgD, IgG2/IgE, IgG2/IgG1, IgG2/IgG2, IgG2/IgG3, IgG2/IgG4, IgG2/IgM, IgG3/IgA1, IgG3/IgA2, IgG3/IgD, IgG3/IgE, IgG3/IgG1, IgG3/IgG2, IgG3/IgG3, IgG3/IgG4, IgG3/IgM, IgG4/IgA1, IgG4/IgA2, IgG4/IgD, IgG4/IgE, IgG4/IgG1, IgG4/IgG2, IgG4/IgG3, IgG4/IgG4, IgG4/IgM, IgM/IgA1, IgM/IgA2, IgM/IgD, IgM/IgE, IgM/IgG1, IgM/IgG2, IgM/IgG3, IgM/IgG4, IgM/IgM (or fragments or variants thereof), and optionally include a hinge from one or more of IgA1, IgA2, IgD, IgG1, IgG2, IgG3, or IgG4, and/or a CH₄ domain from IgE and/or IgM. In specific embodiments, the hinge, CH₂, CH₃, and CH₄ domains are from human Ig.

Additional examples include hybrid Fc regions that comprise, consist of, or consist essentially of the following combination of CH₂/CH₄ domains: IgA1/IgE, IgA2/IgE, IgD/IgE, IgE/IgE, IgG1/IgE, IgG2/IgE, IgG3/IgE, IgG4/IgE, IgM/IgE, IgA1/IgM, IgA2/IgM, IgD/IgM, IgE/IgM, IgG1/IgM, IgG2/IgM, IgG3/IgM, IgG4/IgM, IgM/IgM (or fragments or variants thereof), and optionally include a hinge from one or more of IgA1, IgA2, IgD, IgG1, IgG2, IgG3, IgG4, and/or a CH₃ domain from one or more of IgA1, IgA2, IgD, IgE, IgG1, IgG2, IgG3, IgG4, or IgM. In specific embodiments, the hinge, CH₂, CH₃, and CH₄ domains are from human Ig.

Certain examples include hybrid Fc regions that comprise, consist of, or consist essentially of the following combination of CH₃/CH₄ domains: IgA1/IgE, IgA2/IgE, IgD/IgE, IgE/IgE, IgG1/IgE, IgG2/IgE, IgG3/IgE, IgG4/IgE, IgM/IgE, IgA1/IgM, IgA2/IgM, IgD/IgM, IgE/IgM, IgG1/IgM, IgG2/IgM, IgG3/IgM, IgG4/IgM, IgM/IgM (or fragments or variants thereof), and optionally include a hinge from one or more of IgA1, IgA2, IgD, IgG1, IgG2, IgG3, IgG4, and/or a CH₂ domain from one or more of IgA1, IgA2, IgD, IgE, IgG1, IgG2, IgG3, IgG4, or IgM. In specific embodiments, the hinge, CH₂, CH₃, and CH₄ domains are from human Ig.

Particular examples include hybrid Fc regions that comprise, consist of, or consist essentially of the following combination of hinge/CH₂ domains: IgA1/IgA1, IgA1/IgA2, IgA1/IgD, IgA1/IgE, IgA1/IgG1, IgA1/IgG2, IgA1/IgG3, IgA1/IgG4, IgA1/IgM, IgA2/IgA1, IgA2/IgA2, IgA2/IgD, IgA2/IgE, IgA2/IgG1, IgA2/IgG2, IgA2/IgG3, IgA2/IgG4, IgA2/IgM, IgD/IgA1, IgD/IgA2, IgD/IgD, IgD/IgE, IgD/IgG1, IgD/IgG2, IgD/IgG3, IgD/IgG4, IgD/IgM, IgG1/IgA1, IgG1/IgA2, IgG1/IgD, IgG1/IgE, IgG1/IgG1, IgG1/IgG2, IgG1/IgG3, IgG1/IgG4, IgG1/IgM, IgG2/IgA1, IgG2/IgA2, IgG2/IgD, IgG2/IgE, IgG2/IgG1, IgG2/IgG2, IgG2/IgG3, IgG2/IgG4, IgG2/IgM, IgG3/IgA1, IgG3/IgA2, IgG3/IgD, IgG3/IgE, IgG3/IgG1, IgG3/IgG2, IgG3/IgG3, IgG3/IgG4, IgG3/IgM, IgG4/IgA1, IgG4/IgA2, IgG4/IgD, IgG4/IgE, IgG4/IgG1, IgG4/IgG2, IgG4/IgG3, IgG4/IgG4, IgG4/IgM (or fragments or variants thereof), and optionally include a CH₃ domain from one or more of IgA1, IgA2, IgD, IgE, IgG1, IgG2, IgG3, IgG4, or IgM, and/or a CH₄ domain from IgE and/or IgM. In specific embodiments, the hinge, CH₂, CH₃, and CH₄ domains are from human Ig.

Certain examples include hybrid Fc regions that comprise, consist of, or consist essentially of the following combination of hinge/CH₃ domains: IgA1/IgA1, IgA1/IgA2, IgA1/IgD, IgA1/IgE, IgA1/IgG1, IgA1/IgG2, IgA1/IgG3, IgA1/IgG4, IgA1/IgM, IgA2/IgA1, IgA2/IgA2, IgA2/IgD, IgA2/IgE, IgA2/IgG1, IgA2/IgG2, IgA2/IgG3, IgA2/IgG4, IgA2/IgM, IgD/IgA1, IgD/IgA2, IgD/IgD, IgD/IgE, IgD/IgG1, IgD/IgG2, IgD/IgG3, IgD/IgG4, IgD/IgM, IgG1/IgA1, IgG1/IgA2, IgG1/IgD, IgG1/IgE, IgG1/IgG1, IgG1/IgG2, IgG1/IgG3, IgG1/IgG4, IgG1/IgM, IgG2/IgA1, IgG2/IgA2, IgG2/IgD, IgG2/IgE, IgG2/IgG1, IgG2/IgG2, IgG2/IgG3, IgG2/IgG4, IgG2/IgM, IgG3/IgA1, IgG3/IgA2, IgG3/IgD, IgG3/IgE, IgG3/IgG1, IgG3/IgG2, IgG3/IgG3, IgG3/IgG4, IgG3/IgM, IgG4/IgA1, IgG4/IgA2, IgG4/IgD, IgG4/IgE, IgG4/IgG1, IgG4/IgG2, IgG4/IgG3, IgG4/IgG4, IgG4/IgM (or fragments or variants thereof), and optionally include a CH₂ domain from one or more of IgA1, IgA2, IgD, IgE, IgG1, IgG2, IgG3, IgG4, or IgM, and/or a CH₄ domain from IgE and/or IgM. In specific embodiments, the hinge, CH₂, CH₃, and CH₄ domains are from human Ig.

Some examples include hybrid Fc regions that comprise, consist of, or consist essentially of the following combination of hinge/CH₄ domains: IgA1/IgE, IgA1/IgM, IgA2/IgE, IgA2/IgM, IgD/IgE, IgD/IgM, IgG1/IgE, IgG1/IgM, IgG2/IgE, IgG2/IgM, IgG3/IgE, IgG3/IgM, IgG4/IgE, IgG4/IgM (or fragments or variants thereof), and optionally include a CH₂ domain from one or more of IgA1, IgA2, IgD, IgE, IgG1, IgG2, IgG3, IgG4, or IgM, and/or a CH₃ domain from one or more of IgA1, IgA2, IgD, IgE, IgG1, IgG2, IgG3, IgG4, or IgM.

Specific examples of hybrid Fc regions can be found, for example, in WO 2008/147143, which are derived from combinations of IgG subclasses or combinations of human IgD and IgG.

Also included are derivatized or otherwise modified Fc regions. In certain aspects, the Fc region may be modified by phosphorylation, sulfation, acrylation, glycosylation, methylation, farnesylation, acetylation, amidation, and the like, for instance, relative to a wild-type or naturally-occurring Fc region. In certain embodiments, the Fc region may comprise wild-type or native glycosylation patterns, or alternatively, it may comprise increased glycosylation relative to a native form, decreased glycosylation relative to a native form, or it may be entirely deglycosylated. As one example of a modified Fc glycoform, decreased glycosylation of an Fc region reduces binding to the C1q region of the first complement component C1, a decrease in ADCC-related activity, and/or a decrease in CDC-related activity. Certain embodiments thus employ a deglycosylated or aglycosylated Fc region. *See, e.g.,* WO 2005/047337 for the production of exemplary aglycosylated Fc regions. Another example of an Fc region glycoform can be generated by substituting the Q295 position with a cysteine residue *(see, e.g.,* U.S. Application No. 2010/0080794), according to the Kabat *et al.* numbering system. Certain embodiments may include Fc regions where about 80-100% of the glycoprotein in Fc region comprises a mature core carbohydrate structure that lacks fructose *(see, e.g.,* U.S. Application No. 2010/0255013). Some embodiments may include Fc regions that are optimized by substitution or deletion to reduce the level of fucosylation, for instance, to increase affinity for FcyRI, FcγRIa, or FcγRIIIa, and/or to improve phagocytosis by FcγRIIa-expressing cells *(see* U.S. Application Nos. 2010/0249382 and 2007/0148170).

As another example of a modified Fc glycoform, an Fc region may comprise oligomannose-type N-glycans, and optionally have one or more of the following: increased ADCC activity, increased binding affinity for FcγRIIIA (and certain other FcRs), similar or increased binding specificity for the target of the HRS polypeptide, similar or higher binding affinity for the target of the HRS polypeptide, and/or similar or lower binding affinity for mannose receptor, relative to a corresponding Fc region or HRS-Fc conjugate that contains complex-type N-glycans *(see, e.g.,* U.S. Application No. 2007/0092521 and U.S. Patent No. 7,700,321). As another example, enhanced affinity of Fc regions for FcγRs has been achieved using engineered glycoforms generated by expression of antibodies in engineered or variant cell lines *(see, e.g.,* Umana et al., Nat Biotechnol. 17:176-180, 1999; Davies et al., Biotechnol Bioeng. 74:288-294, 2001; Shields et al., J Biol Chem. 277:26733-26740, 2002; Shinkawa et al., 2003, J Biol Chem. 278:3466-3473, 2003; and U.S. Application No. 2007/0111281). Certain Fc region glycoforms comprise an increased proportion of N-glycoside bond type complex sugar chains, which do not have the 1-position of fucose bound to the 6-position of N-acetylglucosamine at the reducing end of the sugar chain *(see, e.g.,* U.S. Application No. 2010/0092997). Particular embodiments may include IgG Fc region that is glycosylated with at least one galactose moiety connected to a respective terminal sialic acid moiety by an α-2,6 linkage, optionally where the Fc region has a higher anti-inflammatory activity relative to a corresponding, wild-type Fc region *(see* U.S. Application No. 2008/0206246). Certain of these and related altered glycosylation approaches have generated substantial enhancements of the capacity of Fc regions to selectively bind FcRs such as FcyRIII, to mediate ADCC, and to alter other properties of Fc regions, as described herein.

Certain variant, fragment, hybrid, or otherwise modified Fc regions may have altered binding to one or more FcRs, relative to a corresponding, wild-type Fc sequence (e.g., same species, same Ig class, same Ig subclass). For instance, such Fc regions may have increased binding to one or more of Fcγ receptors, Fcα receptors, Fcε receptors, and/or the neonatal Fc receptor, relative to a corresponding, wild-type Fc sequence. In some embodiments, variant, fragment, hybrid, or modified Fc regions may have decreased binding to one or more of Fcγ receptors, Fcα receptors, Fcε receptors, and/or the neonatal Fc receptor, relative to a corresponding, wild-type Fc sequence. Specific FcRs are described elsewhere herein.

Specific examples of Fc variants having altered (e.g., increased, decreased) FcR binding can be found, for example, in U.S. Pat. Nos. 5,624,821 and 7,425,619; U.S. Application Nos. 2009/0017023, 2009/0010921, and 2010/0203046; and WO 2000/42072 and WO 2004/016750. Certain examples include human Fc regions having a one or more substitutions at position 298, 333, and/or 334, for example, S298A, E333A, and/or K334A (based on the numbering of the EU index of Kabat *et al*.), which have been shown to increase binding to the activating receptor FcγRIIIa and reduce binding to the inhibitory receptor FcγRIIb. These mutations can be combined to obtain double and triple mutation variants that have further improvements in binding to FcRs. Certain embodiments include a S298A/E333A/K334A triple mutant, which has increased binding to FcγRIIIa, decreased binding to FcγRIIb, and increased ADCC *(see, e.g.,* Shields et al., J Biol Chem. 276:6591-6604, 2001; and Presta et al., Biochem Soc Trans. 30:487-490, 2002). *See* also engineered Fc glycoforms that have increased binding to FcRs, as disclosed in Umana *et al., supra;* and U.S. Patent No. 7,662,925. Some embodiments include Fc regions that comprise one or more substitutions selected from 434S, 252Y/428L, 252Y/434S, and 428L/434S *(see* U.S. Application Nos. 2009/0163699 and 20060173170), based on the EU index of Kabat *et al.*

Certain variant, fragment, hybrid, or modified Fc regions may have altered effector functions, relative to a corresponding, wild-type Fc sequence. For example, such Fc regions may have increased complement fixation or activation, increased Clq binding affinity, increased CDC-related activity, increased ADCC-related activity, and/or increased ADCP-related activity, relative to a corresponding, wild-type Fc sequence. In some embodiments, such Fc regions may have decreased complement fixation or activation, decreased Clq binding affinity, decreased CDC-related activity, decreased ADCC-related activity, and/or decreased ADCP-related activity, relative to a corresponding, wild-type Fc sequence. As merely one illustrative example, an Fc region may comprise a deletion or substitution in a complement-binding site, such as a C1q-binding site, and/or a deletion or substitution in an ADCC site. Examples of such deletions/substitutions are described, for example, in U.S. Patent No. 7,030,226. Many Fc effector functions, such as ADCC, can be assayed according to routine techniques in the art. *(see, e.g.,* Zuckerman et al., CRC Crit Rev Microbiol. 7:1-26, 1978). Useful effector cells for such assays includes, but are not limited to, natural killer (NK) cells, macrophages, and other peripheral blood mononuclear cells (PBMC). Alternatively, or additionally, certain Fc effector functions may be assessed *in vivo,* for example, by employing an animal model described in Clynes et al. PNAS. 95:652-656, 1998.

Certain variant hybrid, or modified Fc regions may have altered stability or half-life relative to a corresponding, wild-type Fc sequence. In certain embodiments, such Fc regions may have increased half-life relative to a corresponding, wild-type Fc sequence. In some embodiments, variant hybrid, or modified Fc regions may have decreased half-life relative to a corresponding, wild-type Fc sequence. Half-life can be measured *in vitro (e.g.,* under physiological conditions) or *in vivo,* according to routine techniques in the art, such as radiolabeling, ELISA, or other methods. *In vivo* measurements of stability or half-life can be measured in one or more bodily fluids, including blood, serum, plasma, urine, or cerebrospinal fluid, or a given tissue, such as the liver, kidneys, muscle, central nervous system tissues, bone, etc. As one example, modifications to an Fc region that alter its ability to bind the FcRn can alter its half-life *in vivo.* Assays for measuring the *in vivo* pharmacokinetic properties *(e.g., in vivo* mean elimination half-life) and non-limiting examples of Fc modifications that alter its binding to the FcRn are described, for example, in U.S. Pat. Nos. 7,217,797 and 7,732,570; and U.S. Application Nos. US 2010/0143254 and 2010/0143254.

Additional non-limiting examples of modifications to alter stability or half-life include substitutions/deletions at one or more of amino acid residues selected from 251-256, 285-290, and 308-314 in the CH₂ domain, and 385-389 and 428-436 in the CH₃ domain, according to the numbering system of Kabat et al. See U.S. Application No. 2003/0190311. Specific examples include substitution with leucine at position 251, substitution with tyrosine, tryptophan or phenylalanine at position 252, substitution with threonine or serine at position 254, substitution with arginine at position 255, substitution with glutamine, arginine, serine, threonine, or glutamate at position 256, substitution with threonine at position 308, substitution with proline at position 309, substitution with serine at position 311, substitution with aspartate at position 312, substitution with leucine at position 314, substitution with arginine, aspartate or serine at position 385, substitution with threonine or proline at position 386, substitution with arginine or proline at position 387, substitution with proline, asparagine or serine at position 389, substitution with methionine or threonine at position 428, substitution with tyrosine or phenylalanine at position 434, substitution with histidine, arginine, lysine or serine at position 433, and/or substitution with histidine, tyrosine, arginine or threonine at position 436, including any combination thereof. Such modifications optionally increase affinity of the Fc region for the FcRn and thereby increase half-life, relative to a corresponding, wild-type Fc region.

Certain variant hybrid, or modified Fc regions may have altered solubility relative to a corresponding, wild-type Fc sequence. In certain embodiments, such Fc regions may have increased solubility relative to a corresponding, wild-type Fc sequence. In some embodiments, variant hybrid, or modified Fc regions may have decreased solubility relative to a corresponding, wild-type Fc sequence. Solubility can be measured, for example, *in vitro (e.g.,* under physiological conditions) according to routine techniques in the art. Exemplary solubility measurements are described elsewhere herein.

Additional examples of variants include IgG Fc regions having conservative or non-conservative substitutions (as described elsewhere herein) at one or more of positions 250, 314, or 428 of the heavy chain, or in any combination thereof, such as at positions 250 and 428, or at positions 250 and 314, or at positions 314 and 428, or at positions 250, 314, and 428 *(see, e.g.,* U.S. Application No. 2011/0183412). In specific embodiments, the residue at position 250 is substituted with glutamic acid or glutamine, and/or the residue at position 428 is substituted with leucine or phenylalanine. As another illustrative example of an IgG Fc variant, any one or more of the amino acid residues at positions 214 to 238, 297 to 299, 318 to 322, and/or 327 to 331 may be used as a suitable target for modification (e.g., conservative or non-conservative substitution, deletion). In particular embodiments, the IgG Fc variant CH₂ domain contains amino acid substitutions at positions 228, 234, 235, and/or 331 *(e.g.,* human IgG4 with Ser228Pro and Leu235Ala mutations) to attenuate the effector functions of the Fc region *(see* U.S. Patent No. 7,030,226). Here, the numbering of the residues in the heavy chain is that of the EU index *(see* Kabat et al., "Sequences of Proteins of Immunological Interest," 5th Ed., National Institutes of Health, Bethesda, Md. (1991)). Certain of these and related embodiments have altered (e.g., increased, decreased) FcRn binding and/or serum half-life, optionally without reduced effector functions such as ADCC or CDC-related activities.

Additional examples include variant Fc regions that comprise one or more amino acid substitutions at positions 279, 341, 343 or 373 of a wild-type Fc region, or any combination thereof *(see, e.g.,* U.S. Application No. 2007/0224188). The wild-type amino acid residues at these positions for human IgG are valine (279), glycine (341), proline (343) and tyrosine (373). The substation(s) can be conservative or non-conservative, or can include non-naturally occurring amino acids or mimetics, as described herein. Alone or in combination with these substitutions, certain embodiments may also employ a variant Fc region that comprises at least 1, 2, 3, 4, 5, 6, 7, 8, 9, 10 or more amino acid substitutions selected from the following: 235G, 235R, 236F, 236R, 236Y, 237K, 237N, 237R, 238E, 238G, 238H, 238I, 238L, 238V, 238W, 238Y, 244L, 245R, 247A, 247D, 247E, 247F, 247M, 247N, 247Q, 247R, 247S, 247T, 247W, 247Y, 248F, 248P, 248Q, 248W, 249L, 249M, 249N, 249P, 249Y, 251H, 251I, 251W, 254D, 254E, 254F, 254G, 254H, 254I, 254K, 254L, 254M, 254N, 254P, 254Q, 254R, 254V, 254W, 254Y, 255K, 255N, 256H, 256I, 256K, 256L, 256V, 256W, 256Y, 257A, 257I, 257M, 257N, 257S, 258D, 260S, 262L, 264S, 265K, 265S, 267H, 267I, 267K, 268K, 269N, 269Q, 271T, 272H, 272K, 272L, 272R, 279A, 279D, 279F, 279G, 279H, 279I, 279K, 279L, 279M, 279N, 279Q, 279R, 279S, 279T, 279W, 279Y, 280T, 283F, 283G, 283H, 283I, 283K, 283L, 283M, 283P, 283R, 283T, 283W, 283Y, 285N, 286F, 288N, 288P, 292E, 292F, 292G, 292I, 292L, 293S, 293V, 301W, 304E, 307E, 307M, 312P, 315F, 315K, 315L, 315P, 315R, 316F, 316K, 317P, 317T, 318N, 318P, 318T, 332F, 332G, 332L, 332M, 332S, 332V, 332W, 339D, 339E, 339F, 339G, 339H, 339I, 339K, 339L, 339M, 339N, 339Q, 339R, 339S, 339W, 339Y, 341D, 341E, 341F, 341H, 341I, 341K, 341L, 341M, 341N, 341P, 341Q, 341R, 341S, 341T, 341V, 341W, 341Y, 343A, 343D, 343E, 343F, 343G, 343H, 343I, 343K, 343L, 343M, 343N, 343Q, 343R, 343S, 343T, 343V, 343W, 343Y, 373D, 373E, 373F, 373G, 373H, 373I, 373K, 373L, 373M, 373N, 373Q, 373R, 373S, 373T, 373V, 373W, 375R, 376E, 376F, 376G, 376H, 376I, 376L, 376M, 376N, 376P, 376Q, 376R, 376S, 376T, 376V, 376W, 376Y, 377G, 377K, 377P, 378N, 379N, 379Q, 379S, 379T, 380D, 380N, 380S, 380T, 382D, 382F, 382H, 382I, 382K, 382L, 382M, 382N, 382P, 382Q, 382R, 382S, 382T, 382V, 382W, 382Y, 385E, 385P, 386K, 423N, 424H, 424M, 424V, 426D, 426L, 427N, 429A, 429F, 429M, 430A, 430D, 430F, 430G, 430H, 430I, 430K, 430L, 430M, 430N, 430P, 430Q, 430R, 430S, 430T, 430V, 430W, 430Y, 431H, 431K, 431P, 432R, 432S, 438G, 438K, 438L, 438T, 438W, 439E, 439H, 439Q, 440D, 440E, 440F, 440G, 440H, 440I, 440K, 440L, 440M, 440Q, 440T, 440V or 442K. As above, the numbering of the residues in the heavy chain is that of the EU index *(see* Kabat *et al., supra).* Such variant Fc regions typically confer an altered effector function or altered serum half-life upon HRS polypeptide to which the variant Fc region is operably attached. Preferably the altered effector function is an increase in ADCC, a decrease in ADCC, an increase in CDC, a decrease in CDC, an increase in Clq binding affinity, a decrease in Clq binding affinity, an increase in FcR (preferably FcRn) binding affinity or a decrease in FcR (preferably FcRn) binding affinity as compared to a corresponding Fc region that lacks such amino acid substitution(s).

Additional examples include variant Fc regions that comprise an amino acid substitution at one or more of position(s) 221, 222, 224, 227, 228, 230, 231, 223, 233, 234, 235, 236, 237, 238, 239, 240, 241, 243, 244, 245, 246, 247, 249, 250, 258, 262, 263, 264, 265, 266, 267, 268, 269, 270, 271, 272, 273, 274, 275, 276, 278, 280, 281, 283, 285, 286, 288, 290, 291, 293, 294, 295, 296, 297, 298, 299, 300, 302, 313, 317, 318, 320, 322, 323, 324, 325, 326, 327, 328, 329, 330, 331, 332, 333, 334, 335 336 and/or 428 *(see, e.g.,* U.S. Patent No. 7,662,925). In specific embodiments, the variant Fc region comprises at least one amino acid substitution selected from the group consisting of: P230A, E233D, L234E, L234Y, L234I, L235D, L235S, L235Y, L235I, S239D, S239E, S239N, S239Q, S239T, V240I, V240M, F243L, V264I, V264T, V264Y, V266I, E272Y, K274T, K274E, K274R, K274L, K274Y, F275W, N276L, Y278T, V302I, E318R, S324D, S324I, S324V, N325T, K326I, K326T, L328M, L328I, L328Q, L328D, L328V, L328T, A330Y, A330L, A330I, I332D, I332E, I332N, I332Q, T335D, T335R, and T335Y. In other specific embodiments, the variant Fc region comprises at least one amino acid substitution selected from the group consisting of: V264I, F243L/V264I, L328M, I332E, L328M/I332E, V264I/I332E, S298A/I332E, S239E/I332E, S239Q/I332E, S239E, A330Y, I332D, L328I/I332E, L328Q/I332E, V264T, V240I, V266I, S239D, S239D/I332D, S239D/I332E, S239D/I332N, S239D/I332Q, S239E/I332D, S239E/I332N, S239E/I332Q, S239N/I332D, S239N/I332E, S239Q/I332D, A330Y/I332E, V264I/A330Y/I332E, A330L/I332E, V264I/A330L/I332E, L234E, L234Y, L234I, L235D, L235S, L235Y, L235I, S239T, V240M, V264Y, A330I, N325T, L328D/I332E, L328V/I332E, L328T/I332E, L328I/I332E, S239E/V264I/I332E, S239Q/V264I/I332E, S239E/V264I/A330Y/I332E, S239D/A330Y/I332E, S239N/A330Y/I332E, S239D/A330L/I332E, S239N/A330L/I332E, V264I/S298A/I332E, S239D/S298A/I332E, S239N/S298A/I332E, S239D/V264I/I332E, S239D/V264I/S298A/I332E, S239D/V264I/A330L/I332E, S239D/I332E/A330I, P230A, P230A/E233D/I332E, E272Y, K274T, K274E, K274R, K274L, K274Y, F275W, N276L, Y278T, V302I, E318R, S324D, S324I, S324V, K326I, K326T, T335D, T335R, T335Y, V240I/V266I, S239D/A330Y/I332E/L234I, S239D/A330Y/I332E/L235D, S239D/A330Y/I332E/V240I, S239D/A330Y/I332E/V264T, S239D/A330Y/I332E/K326E, and S239D/A330Y/I332E/K326T, In more specific embodiments, the variant Fc region comprises a series of substitutions selected from the group consisting of: N297D/I332E, F241Y/F243Y/V262T/V264T/N297D/I332E, S239D/N297D/I332E, S239E/N297D/I332E, S239D/D265Y/N297D/I332E, S239D/D265H/N297D/I332E, V264E/N297D/I332E, Y296N/N297D/I332E, N297D/A330Y/I332E, S239D/D265V/N297D/I332E, S239D/D265I/N297D/I332E, and N297D/S298A/A330Y/I332E. In specific embodiments, the variant Fc region comprises an amino acid substitution at position 332 (using the numbering of the EU index, Kabat *et al., supra).* Examples of substitutions include 332A, 332D, 332E, 332F, 332G, 332H, 332K, 332L, 332M, 332N, 332P, 332Q, 332R, 332S, 332T, 332V, 332W and 332Y. The numbering of the residues in the Fc region is that of the EU index of Kabat *et al.* Among other properties described herein, such variant Fc regions may have increased affinity for an FcyR, increased stability, and/or increased solubility, relative to a corresponding, wild-type Fc region.

Further examples include variant Fc regions that comprise one or more of the following amino acid substitutions: 224N/Y, 225A, 228L, 230S, 239P, 240A, 241L, 243S/L/G/H/I, 244L, 246E, 247L/A, 252T, 254T/P, 258K, 261Y, 265V, 266A, 267G/N, 268N, 269K/G, 273A, 276D, 278H, 279M, 280N, 283G, 285R, 288R, 289A, 290E, 291L, 292Q, 297D, 299A, 300H, 301C, 304G, 305A, 306I/F, 311R, 312N, 315D/K/S, 320R, 322E, 323A, 324T, 325S, 326E/R, 332T, 333D/G, 335I, 338R, 339T, 340Q, 341E, 342R, 344Q, 347R, 351S, 352A, 354A, 355W, 356G, 358T, 361D/Y, 362L, 364C, 365Q/P, 370R, 372L, 377V, 378T, 383N, 389S, 390D, 391C, 393A, 394A, 399G, 404S, 408G, 409R, 411I, 412A, 414M, 421S, 422I, 426F/P, 428T, 430K, 431S, 432P, 433P, 438L, 439E/R, 440G, 441F, 442T, 445R, 446A, 447E, optionally where the variant has altered recognition of an Fc ligand and/or altered effector function compared with a parent Fc polypeptide, and wherein the numbering of the residues is that of the EU index as in Kabat *et al.* Specific examples of these and related embodiments include variant Fc regions that comprise or consist of the following sets of substitutions: (1) N276D, R292Q, V305A, I377V, T394A, V412A and K439E; (2) P244L, K246E, D399G and K409R; (3) S304G, K320R, S324T, K326E and M358T; (4) F243S, P247L, D265V, V266A, S383N and T411I; (5) H224N, F243L, T393A and H433P; (6) V240A, S267G, G341E and E356G; (7) M252T, P291L, P352A, R355W, N390D, S408G, S426F and A431S; (8) P228L, T289A, L365Q, N389S and 5440G; (9) F241L, V273A, K340Q and L441F; (10) F241L, T299A, I332T and M428T; (11) E269K, Y300H, Q342R, V422I and G446A; (12) T225A, R301c, S304G, D312N, N315D, L351S and N421S; (13) S254T, L306I, K326R and Q362L; (14) H224Y, P230S, V323A, E333D, K338R and S364C; (15) T335I, K414M and P445R; (16) T335I and K414M; (17) P247A, E258K, D280N, K288R, N297D, T299A, K322E, Q342R, S354A and L365P; (18) H268N, V279M, A339T, N361D and S426P; (19) C261Y, K290E, L306F, Q311R, E333G and Q438L; (20) E283G, N315K, E333G, R344Q, L365P and S442T; (21) Q347R, N361Y and K439R; (22) S239P, S254P, S267N, H285R, N315S, F372L, A378T, N390D, Y391C, F404S, E430K, L432P and K447E; and (23) E269G, Y278H, N325S and K370R, wherein the numbering of the residues is that of the EU index as in Kabat et al. (see, e.g., U.S. Application No. 2010/0184959).

Another specific example of an Fc variant comprises an Fc sequence of Table H7, wherein Xaa at position 1 is Ala or absent; Xaa at position 16 is Pro or Glu; Xaa at position 17 is Phe, Val, or Ala; Xaa at position 18 is Leu, Glu, or Ala; Xaa at position 80 is Asn or Ala; and/or Xaa at position 230 is Lys or is absent *(see, e.g.,* U.S. Application No. 2007/0253966). Certain of these Fc regions, and related HRS-Fc conjugates, have increased half-life, reduced effector activity, and/or are significantly less immunogenic than wild-type Fc sequences.

Variant Fc regions can also have one or more mutated hinge regions, as described, for example, in U.S. Application No. 2003/0118592. For instance, one or more cysteines in a hinge region can be deleted or substituted with a different amino acid. The mutated hinge region can comprise no cysteine residues, or it can comprise 1, 2, or 3 fewer cysteine residues than a corresponding, wild-type hinge region. In some embodiments, an Fc region having a mutated hinge region of this type exhibits a reduced ability to dimerize, relative to a wild-type Ig hinge region.

As noted above, HRS-Fc conjugates such as HRS-Fc fusion proteins typically have altered (e.g., improved, increased, decreased) pharmacokinetic properties relative to corresponding HRS polypeptides. Examples of pharmacokinetic properties include stability or half-life, bioavailability (the fraction of a drug that is absorbed), tissue distribution, volume of distribution (apparent volume in which a drug is distributed immediately after it has been injected intravenously and equilibrated between plasma and the surrounding tissues), concentration (initial or steady-state concentration of drug in plasma), elimination rate constant (rate at which drugs are removed from the body), elimination rate (rate of infusion required to balance elimination), area under the curve (AUC or exposure; integral of the concentration-time curve, after a single dose or in steady state), clearance (volume of plasma cleared of the drug per unit time), Cₘₐₓ (peak plasma concentration of a drug after oral administration), tₘₐₓ (time to reach Cₘₐₓ), Cₘᵢₙ (lowest concentration that a drug reaches before the next dose is administered), and fluctuation (peak trough fluctuation within one dosing interval at steady state). In some aspects, these improved properties are achieved without significantly altering the secondary structure and/or reducing the non-canonical biological activity of the HRS polypeptide. Indeed, some HRS-Fc conjugates have increased non-canonical biological activity.

Hence, in some embodiments, the HRS-Fc conjugate or HRS-Fc fusion polypeptide has a plasma or sera pharmacokinetic AUC profile at least 2, 3, 4, 5, 6, 7, 8, 9, 10, 11, 12, 13, 14, 15, 16, 17, 18, 19, 20, 50, 100, 200, 300, 400, or 500-fold greater than a corresponding unmodified or differently modified HRS polypeptide when administered to a mammal under the same or comparable conditions. In certain embodiments, the HRS-Fc conjugate or HRS-Fc fusion polypeptide has a stability (e.g., as measured by half-life) which is at least 10%, 20%, 30%, 40%, 50%, 60%, 70%, 80%, 90%, 100%, 200%, 300%, 400%, or 500% greater than a corresponding unmodified or differently modified HRS polypeptide when compared under similar conditions at room temperature, for example, in PBS at pH 7.4 for about 1, 2, 3, 4, 5, 6, 7, 8, 9, 10, 11, 12, 13, 14 days, or 1, 2, 3, 4 weeks or so.

In particular embodiments, a HRS-Fc conjugate or HRS-Fc fusion polypeptide has a biological half life at pH 7.4, 25°C, *e.g.,* a physiological pH, human body temperature *(e.g., in vivo,* in serum, in a given tissue, in a given species such as rat, mouse, monkey, or human), of about or at least about 30 minutes, about 1 hour, about 2 hour, about 3 hours, about 4 hours, about 5 hours, about 6 hours, about 12 hours, about 18 hours, about 20 hours, about 24 hours, about 30 hours, about 36 hours, about 40 hours, about 48 hours, about 50 hours, about 60 hours, about 70 hours, about 72 hours, about 80 hours, about 84 hours, about 90 hours, about 96 hours, about 120 hours, or about 144 hours or more or any intervening half-life.

In certain embodiments, the HRS-Fc conjugate or HRS-Fc fusion polypeptide has greater bioavailability after subcutaneous (SC) administration compared to a corresponding unmodified HRS-polypeptide. In certain embodiments, the HRS-Fc conjugate or HRS-Fc fusion polypeptide has at least about 20%, at least about 30%, at least about 40%, at least about 50%, at least about 60%, at least about 70%, at least about 80%, at least about 90%, or at least about 100 %, or more bioavailability compared to the corresponding unmodified HRS polypeptide.

In certain embodiments, the HRS-Fc fusion polypeptide has substantially the same secondary structure as a corresponding unmodified or differently modified HRS polypeptide, as determined via UV circular dichroism analysis. In certain embodiments, the HRS-Fc fusion polypeptide has substantially the same activity of a corresponding unmodified or differently modified HRS polypeptide in an assay of anti-inflammatory activity. In some embodiments, the HRS-Fc fusion polypeptide has greater than 2, 3, 4, 5, 6, 7, 8, 9, 10, 11, 12, 13, 14, 15, 16, 17, 18, 19, or 20-fold the activity of a corresponding unmodified or differently modified HRS polypeptide in an assay of anti-inflammatory activity.

Examples of HRS fusion proteins that modulate the oligomerization of the HRS polypeptide include fusions of HRS to the cartilage oligomeric matrix protein (COMP) (not part of the invention). Thus, fusion proteins may comprise an HRS polypeptide described herein fused to COMP, for example, the pentamerization domain from COMP, which is composed of approximately residues 28-73 of COMP (see, for example, Prodeus et al., JCI Insight. 2017;2(18):e94308; and Kim et al., Biochim Biophys Acta. 2009 May;1793(5):772-80). Exemplary HRS-COMP fusion proteins are provided in **Table H9** below.

| **Table H9. Exemplary HRS-COMP fusion proteins (not part of the invention)** | | |
|---|---|---|
| Name | Sequence | SEQ ID NO: |
| SPARC-HRS (2-60) - COMP-MycHIS | MRAWIFFLLCLAGRALAAERAALEELVKLQGERVRGLKQQKASAELIEEEVAKLL KLKAQLGPDESKQKFVLKTPKGGGGSGGGGSSDLGPQMLRELQETNAALQDVREL LRQQVREITFLKNTVMECDACGGGGGSEQKLISEEDLNMHTGHHHHHH | 173 |
| HRS (2-60) - COMP-MycHIS | AERAALEELVKLQGERVRGLKQQKASAELIEEEVAKLLKLKAQLGPDESKQKFVL KTPKGGGGSGGGGSSDLGPQMLRELQETNAALQDVRELLRQQVREITFLKNTVME CDACGGGGGSEQKLISEEDLNMHTGHHHHHH | 174 |
| HRS (2-60) - COMP | ERAALEELVKLQGERVRGLKQQKASAELIEEEVAKLLKLKAQLGPDESKQKFVLK TPKGGGGSGGGGSSDLGPQMLRELQETNAALQDVRELLRQQVREITFLKNTVMEC DAC | 175 |
| Underlined text represents the signal peptide. | | |

In certain embodiments, a peptide linker sequence may be employed to separate the HRS polypeptide(s) and the Fc region(s) or PEG(s) or other fusion partner(s) by a distance sufficient to ensure that each polypeptide folds into its desired secondary and tertiary structures. Such a peptide linker sequence can be incorporated into the conjugate or fusion protein using standard techniques well known in the art.

Certain peptide linker sequences may be chosen based on the following exemplary factors: (1) their ability to adopt a flexible extended conformation; (2) their inability to adopt a secondary structure that could interact with functional epitopes on the first and second polypeptides; (3) their physiological stability; and (4) the lack of hydrophobic or charged residues that might react with the polypeptide functional epitopes, or other features. *See, e.g.,* George and Heringa, J Protein Eng. 15:871-879, 2002.

The linker sequence may generally be from 1 to about 200 amino acids in length. Particular linkers can have an overall amino acid length of about 1-200 amino acids, 1-150 amino acids, 1-100 amino acids, 1-90 amino acids, 1-80 amino acids, 1-70 amino acids, 1-60 amino acids, 1-50 amino acids, 1-40 amino acids, 1-30 amino acids, 1-20 amino acids, 1-10 amino acids, 1-5 amino acids, 1-4 amino acids, 1-3 amino acids, or about 1, 2, 3, 4, 5, 6, 7, 8, 9, 10, 11, 12, 13, 14, 15, 16 ,17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 60, 70, 80, 90, 100 or more amino acids.

A peptide linker may employ any one or more naturally-occurring amino acids, non-naturally occurring amino acid(s), amino acid analogs, and/or amino acid mimetics as described elsewhere herein and known in the art. Certain amino acid sequences which may be usefully employed as linkers include those disclosed in Maratea et al., Gene 40:39-46, 1985; Murphy et al., PNAS USA. 83:8258-8262, 1986; U.S. Pat. No. 4,935,233 and U.S. Pat. No. 4,751,180. Particular peptide linker sequences contain Gly, Ser, and/or Asn residues. Other near neutral amino acids, such as Thr and Ala may also be employed in the peptide linker sequence, if desired.

Certain exemplary linkers include Gly, Ser and/or Asn-containing linkers, as follows: [G]ₓ, [S]ₓ, [N]ₓ, [GS]ₓ, [GGS]ₓ, [GSS]ₓ, [GSGS]ₓ (SEQ ID NO:201), [GGSG]ₓ (SEQ ID NO: 202), [GGGS]ₓ (SEQ ID NO: 203), [GGGGS]ₓ (SEQ ID NO: 204), [GN]ₓ, [GGN]ₓ, [GNN]ₓ, [GNGN]ₓ (SEQ ID NO: 205), [GGNG]ₓ (SEQ ID NO: 206), [GGGN]ₓ (SEQ ID NO: 207), [GGGGN]ₓ (SEQ ID NO: 208) linkers, where ₓ is 1, 2, 3, 4, 5, 6, 7, 8, 9, 10, 11, 12, 13, 14, 15, 16, 17, 18, 19, or 20 or more. Other combinations of these and related amino acids will be apparent to persons skilled in the art.

Additional examples of linker peptides include, but are not limited to the following amino acid sequences: Gly-Gly-Gly-Gly-Ser-Gly-Gly-Gly-Gly-Ser-Gly-Gly-Gly-Gly-Ser-(SEQ ID NO: 209); Gly-Ser-Gly-Gly-Gly-Gly-Ser-Gly-Gly-Gly-Gly-Ser-Gly-Gly-Gly-Gly-Ser-Gly-Gly-Gly-Gly-Ser-(SEQ ID NO: 210); Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-Gly-Gly-Gly-Gly-Ser-(SEQ ID NO: 211); Asp-Ala-Ala-Ala-Lys-Glu-Ala-Ala-Ala-Lys-Asp-Ala-Ala-Ala-Arg-Glu-Ala-Ala-Ala-Arg-Asp-Ala-Ala-Ala-Lys-(SEQ ID NO: 212); and Asn-Val-Asp-His-Lys-Pro-Ser-Asn-Thr-Lys-Val-Asp-Lys-Arg-(SEQ ID NO: 213).

Further non-limiting examples of linker peptides include DGGGS (SEQ ID NO: 214); TGEKP (SEQ ID NO: 215) (see, *e.g.,* Liu et al., PNAS. 94:5525-5530, 1997); GGRR (SEQ ID NO: 216) (Pomerantz *et al.* 1995); (GGGGS)ₙ (SEQ ID NO: 204) (Kim et al., PNAS. 93:1156-1160, 1996); EGKSSGSGSESKVD (SEQ ID NO: 217) (Chaudhary et al., PNAS. 87:1066-1070, 1990); KESGSVSSEQLAQFRSLD (SEQ ID NO: 218) (Bird et al., Science. 242:423-426, 1988), GGRRGGGS (SEQ ID NO: 219); LRQRDGERP (SEQ ID NO: 220); LRQKDGGGSERP (SEQ ID NO: 221); LRQKd(GGGS)₂ ERP (SEQ ID NO: 222). In specific embodiments, the linker sequence comprises a Gly3 linker sequence, which includes three glycine residues. In particular embodiments, flexible linkers can be rationally designed using a computer program capable of modeling both DNA-binding sites and the peptides themselves (Desjarlais & Berg, PNAS. 90:2256-2260, 1993; and PNAS. 91:11099-11103, 1994) or by phage display methods.

The peptide linkers may be physiologically stable or may include a releasable linker such as a physiologically degradable or enzymatically cleavable linker (e.g., proteolytically cleavable linker). In certain embodiments, one or more releasable linkers can result in a shorter half-life and more rapid clearance of the conjugate. These and related embodiments can be used, for example, to enhance the solubility and blood circulation lifetime of HRS polypeptides in the bloodstream, while also delivering a HRS polypeptide into the bloodstream that, subsequent to linker degradation, is substantially free of the Fc region(s). These aspects are especially useful in those cases where HRS polypeptides, when permanently conjugated to an Fc region, demonstrate reduced activity. By using the linkers as provided herein, such HRS polypeptides can maintain their therapeutic activity when in conjugated form. As another example, a large and relatively inert HRS-Fc conjugate polypeptide may be administered, which is then degraded *in vivo* (via the degradable linker) to generate a bioactive HRS polypeptide possessing a portion of the Fc region or lacking the Fc region entirely. In these and other ways, the properties of the HRS-Fc conjugate polypeptide can be more effectively tailored to balance the bioactivity and circulating half-life of the HRS polypeptide over time.

In particular embodiments, the linker peptide comprises an autocatalytic or self-cleaving peptide cleavage site. In a particular embodiment, self-cleaving peptides include those polypeptide sequences obtained from potyvirus and cardiovirus 2A peptides, FMDV (foot-and-mouth disease virus), equine rhinitis A virus, Thosea asigna virus and porcine teschovirus. In certain embodiments, the self-cleaving polypeptide site comprises a 2A or 2A-like site, sequence or domain (Donnelly et al., J. Gen. Virol. 82:1027-1041, 2001). Exemplary 2A sites include the following sequences: LLNFDLLKLAGDVESNPGP (SEQ ID NO: 223); TLNFDLLKLAGDVESNPGP (SEQ ID NO: 224); LLKLAGDVESNPGP (SEQ ID NO: 225); NFDLLKLAGDVESNPGP (SEQ ID NO: 226); QLLNFDLLKLAGDVESNPGP (SEQ ID NO: 227); APVKQTLNFDLLKLAGDVESNPGP (SEQ ID NO: 228); VTELLYRMKRAETYCPRPLLAIHPTEARHKQKIVAPVKQT (SEQ ID NO: 229); LNFDLLKLAGDVESNPGP (SEQ ID NO: 230); LLAIHPTEARHKQKIVAPVKQTLNFDLLKLAGDVESNPGP (SEQ ID NO: 231); and EARHKQKIVAPVKQTLNFDLLKLAGDVESNPGP (SEQ ID NO: 232). In some embodiments, the autocatalytic peptide cleavage site comprises a translational 2A signal sequence, such as, *e.g.,* the 2A region of the aphthovirus foot-and-mouth disease virus (FMDV) polyprotein, which is an18 amino acid sequence. Additional examples of 2A-like sequences that may be used include insect virus polyproteins, the NS34 protein of type C rotaviruses, and repeated sequences in *Trypanosoma* spp., as described, for example, in Donnelly et al., Journal of General Virology. 82:1027-1041, 2001.

Suitable protease cleavages sites and self-cleaving peptides are known to the skilled person *(see, e.g.,* Ryan et al., J. Gener. Virol. 78:699-722, 1997; and Scymczak et al., Nature Biotech. 5:589-594, 2004). Exemplary protease cleavage sites include, but are not limited to the cleavage sites of potyvirus NIa proteases (e.g., tobacco etch virus protease), potyvirus HC proteases, potyvirus P1 (P35) proteases, byovirus NIa proteases, byovirus RNA-2-encoded proteases, aphthovirus L proteases, enterovirus 2A proteases, rhinovirus 2A proteases, picorna 3C proteases, comovirus 24K proteases, nepovirus 24K proteases, RTSV (rice tungro spherical virus) 3C-like protease, PYVF (parsnip yellow fleck virus) 3C-like protease, heparin, thrombin, factor Xa and enterokinase. Due to its high cleavage stringency, TEV (tobacco etch virus) protease cleavage sites are included in some embodiments, e.g., EXXYXQ(G/S) (SEQ ID NO: 233), for example, ENLYFQG (SEQ ID NO: 234) and ENLYFQS (SEQ ID NO: 235), wherein X represents any amino acid (cleavage by TEV occurs between Q and G or Q and S).

Further examples of enzymatically degradable linkers suitable for use in particular embodiments include, but are not limited to: an amino acid sequence cleaved by a serine protease such as thrombin, chymotrypsin, trypsin, elastase, kallikrein, or substilisin. Illustrative examples of thrombin-cleavable amino acid sequences include, but are not limited to: -Gly-Arg-Gly-Asp-(SEQ ID NO: 236), -Gly-Gly-Arg-, -Gly- Arg-Gly-Asp-Asn-Pro-(SEQ ID NO: 237), -Gly-Arg-Gly-Asp-Ser-(SEQ ID NO: 238), -Gly-Arg-Gly-Asp-Ser-Pro-Lys-(SEQ ID NO: 239), -Gly-Pro- Arg-, -Val-Pro-Arg-, and -Phe- Val -Arg-. Illustrative examples of elastase-cleavable amino acid sequences include, but are not limited to: -Ala-Ala-Ala-, -Ala-Ala-Pro-Val-(SEQ ID NO: 240), -Ala-Ala-Pro-Leu-(SEQ ID NO: 241), -Ala-Ala-Pro-Phe-(SEQ ID NO:242), -Ala-Ala-Pro-Ala-(SEQ ID NO: 243), and -Ala-Tyr-Leu-Val-(SEQ ID NO: 244).

Enzymatically degradable linkers also include amino acid sequences that can be cleaved by a matrix metalloproteinase such as collagenase, stromelysin, and gelatinase. Illustrative examples of matrix metalloproteinase-cleavable amino acid sequences include, but are not limited to: -Gly-Pro-Y-Gly-Pro-Z-(SEQ ID NO: 245), -Gly-Pro-, Leu-Gly-Pro-Z-(SEQ ID NO: 246), -Gly-Pro-Ile-Gly-Pro-Z-(SEQ ID NO: 247), and -Ala-Pro-Gly-Leu-Z-(SEQ ID NO: 248), where Y and Z are amino acids. Illustrative examples of collagenase-cleavable amino acid sequences include, but are not limited to: - Pro-Leu-Gly-Pro-D-Arg-Z-(SEQ ID NO: 249), -Pro- Leu-Gly-Leu-Leu-Gly-Z-(SEQ ID NO: 250), - Pro-Gln-Gly-Ile-Ala-Gly-Trp-(SEQ ID NO: 251), -Pro-Leu-Gly-Cys(Me)-His-(SEQ ID NO: 252), - Pro-Leu-Gly-Leu-Tyr-Ala-(SEQ ID NO: 253), -Pro-Leu-Ala-Leu-Trp-Ala-Arg-(SEQ ID NO: 254), and -Pro-Leu-Ala-Tyr-Trp-Ala-Arg-(SEQ ID NO: 255), where Z is an amino acid. An illustrative example of a stromelysin-cleavable amino acid sequence is -Pro-Tyr-Ala-Tyr-Tyr-Met-Arg-(SEQ ID NO: 256); and an example of a gelatinase-cleavable amino acid sequence is -Pro-Leu-Gly-Met-Tyr-Ser-Arg-(SEQ ID NO: 257).

Enzymatically degradable linkers suitable for use in particular embodiments include amino acid sequences that can be cleaved by an angiotensin converting enzyme, such as, for example, -Asp-Lys-Pro-, -Gly-Asp-Lys-Pro-(SEQ ID NO: 258), and -Gly-Ser-Asp-Lys-Pro-(SEQ ID NO: 259).

Enzymatically degradable linkers suitable for use in particular embodiments include amino acid sequences that can be degraded by cathepsin B, such as, for example, Val-Cit, Ala-Leu-Ala-Leu-(SEQ ID NO: 260), Gly-Phe-Leu-Gly-(SEQ ID NO: 261) and Phe-Lys.

In particular embodiments, a releasable linker has a half life at pH 7.4, 25°C, *e.g.,* a physiological pH, human body temperature *(e.g., in vivo,* in serum, in a given tissue), of about 30 minutes, about 1 hour, about 2 hour, about 3 hours, about 4 hours, about 5 hours, about 6 hours, about 12 hours, about 18 hours, about 24 hours, about 36 hours, about 48 hours, about 72 hours, or about 96 hours or more or any intervening half-life. One having skill in the art would appreciate that the half life of a HRS-Fc conjugate polypeptide can be finely tailored by using a particular releasable linker.

In certain embodiments, however, any one or more of the peptide linkers are optional. For instance, linker sequences may not required when the first and second polypeptides have non-essential N-terminal and/or C-terminal amino acid regions that can be used to separate the functional domains and prevent steric interference.

The HRS polypeptides can be used in any of the compositions described herein.

### Neuropilin-2 Polypeptides and NRP2 Ligands

Embodiments of the present disclosure relate to the discovery that human histidyl-tRNA synthetase (HRS) polypeptides have unexpected biological properties which are relevant to treating a broad range of diseases and conditions, and that certain of these properties relate to the interactions between HRS and human neuropilin 2 (NRP2). Accordingly, HRS polypeptides can be used as standalone therapies in the treatment of diseases, for example, NRP2-associated disease, and/or in combination with other agents to address a spectrum of diseases and conditions associated with neuropilin-2 biology.

NRP2 is a single transmembrane receptor with a predominant extracellular region containing two CUB domains (a1/a2 combined domain), two Factor V/VIII homology domains (b1/b2 combined domain), and a MAM domain (c domain) (see Figures 1A-1B). The a1a2 combined domain interacts with the sema region of the semaphorins, and the b1 domain interacts with the semaphorin PSI and Ig-like domains. NRP2 has a higher affinity for SEMA3F and 3G; in contrast, SEMAs 3A, 3B and 3E preferentially interact with NRP1. Both NRP1 and NRP2 have similar affinity for SEMA 3C. The b1b2 combined domain interacts with several growth factors containing heparin-binding domains, including VEGF C & D, placenta growth factor (PIGF)-2, fibroblast growth factor (FGF), galectin, hepatocyte growth factor (HGF), platelet derived growth factor (PDGF), and transforming growth factor (TGF)-beta (see, e.g., Prud'homme et al., Oncotarget 3:921-939, 2012). NRP2 also interacts with various growth factor-specific receptors, and interactions with these receptors occur independently of binding to SEMAs. In this context, integrins and growth factor receptors like VEGF receptor, TGF-beta receptor, c-Met, EGFR, FGFR, and PDGFR have been shown to interact with NRPs and in general appear to increase the affinity of each ligand for its receptor and to modulate downstream signaling. The c domain (Mam) domain does not appear to be required for ligand binding, but appears essential for signaling.

NRP2 can form homodimers as well as heterodimers with other cell surface receptors and co-receptors, and is heavily glycosylated. NRP2 also exists in the form of different splice variants which are between about 551 and 926 amino acids long. Two major variants for NRP2 are categorized as NRP2a and NRP2b. These differ in their intracellular C terminal part (see Figures 1A-1B) in which for NRP2a, the c-terminal domain comprises 42 amino acids and a PDZ-binding domain with the C-terminal SEA amino acid sequence. By contrast, NRP2b comprises a 46 amino acid C terminal domain which shares about 11% of the intracellular and transmembrane sequence of NRP2a. Between the MAM domain and the transmembrane domain, additional splicing can occur and 5 additional amino acids (GENFK) can be added to either the NRP2a, or NRP2b forms - these variants are named based on the number additional amino acids added through alternative splicing. Thus the two additional variants of NRP2 are named NRP2a(17) and NRP2a(22) and the two different transmembrane variants for NRP2b are named NRP2b(0) and NRP2b(5). Additionally, a soluble form called sNRP2b can be generated (see Figure 2). The terms "Neuropilin-2", or "NRP2" or "NRP2 polypeptides" refers to all isoforms, splice variants and naturally occurring fragments of NRP2, unless the context clearly points to a different specific meaning. Exemplary NRP2 polypeptide sequences are provided in Table **N1** below.

| **Table N1. Exemplary Human NRP2 Polypeptides** | | | |
|---|---|---|---|
| **Name** | **Residues** | **Sequence** | **SEQ ID NO:** |
| Human full length NRP2 | 1-931 | MDMFPLTWVFLALYFSRHQVRGQPDPPCGGRLNSKDAGYITS PGYPQDYPSHQNCEWIVYAPEPNQKIVLNFNPHFEIEKHDCK YDFIEIRDGDSESADLLGKHCGNIAPPTIISSGSMLYIRFTS DYARQGAGFSLRYEIFKTGSEDCSKNFTSPNGTIESPGFPEK YPHNLDCTFTILAKPKMEIILQFLIFDLEHDPLQVGEGDCKY DWLDIWDGIPHVGPLIGKYCGTKTPSELRSSTGILSLTFHTD MAVAKDGFSARYYLVHQEPLENFQCNVPLGMESGRIANEQIS ASSTYSDGRWTPQQSRLHGDDNGWTPNLDSNKEYLQVDLRFL TMLTAIATQGAISRETQNGYYVKSYKLEVSTNGEDWMVYRHG KNHKVFQANNDATEVVLNKLHAPLLTRFVRIRPQTWHSGIAL RLELFGCRVTDAPCSNMLGMLSGLIADSQISASSTQEYLWSP SAARLVSSRSGWFPRIPQAQPGEEWLQVDLGTPKTVKGVIIQ GARGGDSITAVEARAFVRKFKVSYSLNGKDWEYIQDPRTQQP KLFEGNMHYDTPDIRRFDPIPAQYVRVYPERWSPAGIGMRLE VLGCDWTDSKPTVETLGPTVKSEETTTPYPTEEEATECGENC SFEDDKDLQLPSGFNCNFDFLEEPCGWMYDHAKWLRTTWASS SSPNDRTFPDDRNFLRLQSDSQREGQYARLISPPVHLPRSPV CMEFQYQATGGRGVALQVVREASQESKLLWVIREDQGGEWKH GRIILPSYDMEYQIVFEGVIGKGRSGEIAIDDIRISTDVPLE NCMEPISAFAGENFKVDIPEIHEREGYEDEIDDEYEVDWSNS SSATSGSGAPSTDKEKSWLYTLDPILITIIAMSSLGVLLGAT CAGLLLYCTCSYSGLSSRSCTTLENYNFELYDGLKHKVKMNH QKCCSEA | 176 |
| Variant 1 | | | |
| NRP2a(22) | | | |
| Human NRP2 | 1-926 | MDMFPLTWVFLALYFSRHQVRGQPDPPCGGRLNSKDAGYITS PGYPQDYPSHQNCEWIVYAPEPNQKIVLNFNPHFEIEKHDCK YDFIEIRDGDSESADLLGKHCGNIAPPTIISSGSMLYIKFTS DYARQGAGFSLRYEIFKTGSEDCSKNFTSPNGTIESPGFPEK YPHNLDCTFTILAKPKMEIILQFLIFDLEHDPLQVGEGDCKY DWLDIWDGIPHVGPLIGKYCGTKTPSELRSSTGILSLTFHTD MAVAKDGFSARYYLVHQEPLENFQCNVPLGMESGRIANEQIS ASSTYSDGRWTPQQSRLHGDDNGWTPNLDSNKEYLQVDLRFL TMLTAIATQGAISRETQNGYYVKSYKLEVSTNGEDWMVYRHG KNHKVFQANNDATEVVLNKLHAPLLTRFVRIRPQTWHSGIAL RLELFGCRVTDAPCSNMLGMLSGLIADSQISASSTQEYLWSP SAARLVSSRSGWFPRIPQAQPGEEWLQVDLGTPKTVKGVIIQ GARGGDSITAVEARAFVRKFKVSYSLNGKDWEYIQDPRTQQP KLFEGNMHYDTPDIRRFDPIPAQYVRVYPERWSPAGIGMRLE VLGCDWTDSKPTVETLGPTVKSEETTTPYPTEEEATECGENC SFEDDKDLQLPSGFNCNFDFLEEPCGWMYDHAKWLRTTWASS SSPNDRTFPDDRNFLRLQSDSQREGQYARLISPPVHLPRSPV CMEFQYQATGGRGVALQVVREASQESKLLWVIREDQGGEWKH GRIILPSYDMEYQIVFEGVIGKGRSGEIAIDDIRISTDVPLE NCMEPISAFAVDIPEIHEREGYEDEIDDEYEVDWSNSSSATS GSGAPSTDKEKSWLYTLDPILITIIAMSSLGVLLGATCAGLL LYCTCSYSGLSSRSCTTLENYNFELYDGLKHKVKMNHQKCCS EA | 177 |
| Variant 2 precursor | | | |
| NRP2a(17) | | | |
| Human NRP2 | 1-909 | MDMFPLTWVFLALYFSRHQVRGQPDPPCGGRLNSKDAGYI TSPGYPQDYPSHQNCEWIVYAPEPNQKIVLNFNPHFEIEK HDCKYDFIEIRDGDSESADLLGKHCGNIAPPTIISSGSMLYI KFTSDYARQGAGFSLRYEIFKTGSEDCSKNFTSPNGTIESPG FPEKYPHNLDCTFTILAKPKMEIILQFLIFDLEHDPLQVGEG DCKYDWLDIWDGIPHVGPLIGKYCGTKTPSELRSSTGILSLT FHTDMAVAKDGFSARYYLVHQEPLENFQCNVPLGMESGRIAN EQISASSTYSDGRWTPQQSRLHGDDNGWTPNLDSNKEYLQVD LRFLTMLTAIATQGAISRETQNGYYVKSYKLEVSTNGEDWMV YRHGKNHKVFQANNDATEVVLNKLHAPLLTRFVRIRPQTWHS GIALRLELFGCRVTDAPCSNMLGMLSGLIADSQISASSTQEY LWSPSAARLVSSRSGWFPRIPQAQPGEEWLQVDLGTPKTVKG VIIQGARGGD SITAVEARAFVRKFKVSYSLNGKDWEYIQDPRTQQPKLFEGN MHYDTPDIRRFDPIPAQYVRVYPERWSPAGIGMRLEVLGCDW TDSKPTVETLGPTVKSEETTTPYPTEEEATECGENCSFEDDK DLQLPSGFNCNFDFLEEPCGWMYDHAKWLRTTWASSSSPNDR TFPDDRNFLRLQSDSQREGQYARLISPPVHLPRSPVCMEFQY QATGGRGVALQVVREASQESKLLWVIREDQGGEWKHGRIILP SYDMEYQIVFEGVIGKGRSGEIAIDDIRISTDVPLENCMEPI SAFADEYEVDWSNSSSATSGSGAPSTDKEKSWLYTLDPILIT IIAMSSLGVLLGATCAGLLLYCTCSYSGLSSRSCTTLENYNF ELYDGLKHKVKMNHQKCCSEA | 178 |
| Variant 3 precursor | | | |
| NRP2a(0) | | | |
| Human NRP2 | 1-906 | MDMFPLTWVFLALYFSRHQVRGQPDPPCGGRLNSKDAGYITS PGYPQDYPSHQNCEWIVYAPEPNQKIVLNFNPHFEIEKHDCK YDFIEIRDGDSESADLLGKHCGNIAPPTIISSGSMLYIKFTS | 179 |
| Variant 4 precursor | | DYARQGAGFSLRYEIFKTGSEDCSKNFTSPNGTIESPGFPEK YPHNLDCTFTILAKPKMEIILQFLIFDLEHDPLQVGEGDCKY DWLDIWDGIPHVGPLIGKYCGTKTPSELRSSTGILSLTFHTD MAVAKDGFSARYYLVHQEPLENFQCNVPLGMESGRIANEQIS ASSTYSDGRWTPQQSRLHGDDNGWTPNLDSNKEYLQVDLRFL TMLTAIATQGAISRETQNGYYVKSYKLEVSTNGEDWMVYRHG KNHKVFQANNDATEVVLNKLHAPLLTRFVRIRPQTWHSGIAL RLELFGCRVTDAPCSNMLGMLSGLIADSQISASSTQEYLWSP SAARLVSSRSGWFPRIPQAQPGEEWLQVDLGTPKTVKGVIIQ GARGGDSITAVEARAFVRKFKVSYSLNGKDWEYIQDPRTQQP KLFEGNMHYDTPDIRRFDPIPAQYVRVYPERWSPAGIGMRLE VLGCDWTDSKPTVETLGPTVKSEETTTPYPTEEEATECGENC SFEDDKDLQLPSGFNCNFDFLEEPCGWMYDHAKWLRTTWASS SSPNDRTFPDDRNFLRLQSDSQREGQYARLISPPVHLPRSPV CMEFQYQATGGRGVALQVVREASQESKLLWVIREDQGGEWKH GRIILPSYDMEYQIVFEGVIGKGRSGEIAIDDIRISTDVPLE NCMEPISAFAGENFKGGTLLPGTEPTVDTVPMQPIPAYWYYV MAAGGAVLVLVSVALALVLHYHRFRYAAKKTDHSITYKTSHY TNGAPLAVEPTLTIKLEQDRGSHC | |
| NRP2b (5) | | | |
| Human NRP2 | 1-901 | MDMFPLTWVFLALYFSRHQVRGQPDPPCGGRLNSKDAGYI TSPGYPQDYPSHQNCEWIVYAPEPNQKIVLNFNPHFEIEK HDCKYDFIEIRDGDSESADLLGKHCGNIAPPTIISSGSMLYI KFTSDYARQGAGFSLRYEIFKTGSEDCSKNFTSPNGTIESPG FPEKYPHNLDCTFTILAKPKMEIILQFLIFDLEHDPLQVGEG DCKYDWLDIWDGIPHVGPLIGKYCGTKTPSELRSSTGILSLT FHTDMAVAKDGFSARYYLVHQEPLENFQCNVPLGMESGRIAN EQISASSTYSDGRWTPQQSRLHGDDNGWTPNLDSNKEYLQVD LRFLTMLTAIATQGAISRETQNGYYVKSYKLEVSTNGEDWMV YRHGKNHKVFQANNDATEVVLNKLHAPLLTRFVRIRPQTWHS GIALRLELFGCRVTDAPCSNMLGMLSGLIADSQISASSTQEY LWSPSAARLVSSRSGWFPRIPQAQPGEEWLQVDLGTPKTVKG VIIQGARGGD SITAVEARAFVRKFKVSYSLNGKDWEYIQDPRTQQPKLFEGN MHYDTPDIRRFDPIPAQYVRVYPERWSPAGIGMRLEVLGCDW TDSKPTVETLGPTVKSEETTTPYPTEEEATECGENCSFEDDK DLQLPSGFNCNFDFLEEPCGWMYDHAKWLRTTWASSSSPNDR TFPDDRNFLRLQSDSQREGQYARLISPPVHLPRSPVCMEFQY QATGGRGVALQVVREASQESKLLWVIREDQGGEWKHGRIILP SYDMEYQIVFEGVIGKGRSGEIAIDDIRISTDVPLENCMEPI SAFAGGTLLPGTEPTVDTVPMQPIPAYWYYVMAAGGAVLVLV SVALALVLHYHRFRYAAKKTDHSITYKTSHYTNGAPLAVEPT LTIKLEQDRGSHC | 180 |
| Variant 5 precursor | | | |
| NRP2b (0) | | | |
| Human NRP2 | 1-555 | MDMFPLTWVFLALYFSRHQVRGQPDPPCGGRLNSKDAGYITS PGYPQDYPSHQNCEWIVYAPEPNQKIVLNFNPHFEIEKHDCK YDFIEIRDGDSESADLLGKHCGNIAPPTIISSGSMLYIKFTS DYARQGAGFSLRYEIFKTGSEDCSKNFTSPNGTIESPGFPEK YPHNLDCTFTILAKPKMEIILQFLIFDLEHDPLQVGEGDCKY DWLDIWDGIPHVGPLIGKYCGTKTPSELRSSTGILSLTFHTD MAVAKDGFSARYYLVHQEPLENFQCNVPLGMESGRIANEQIS ASSTYSDGRWTPQQSRLHGDDNGWTPNLDSNKEYLQVDLRFL TMLTAIATQGAISRETQNGYYVKSYKLEVSTNGEDWMVYRHG KNHKVFQANNDATEVVLNKLHAPLLTRFVRIRPQTWHSGIAL RLELFGCRVTDAPCSNMLGMLSGLIADSQISASSTQEYLWSP SAARLVSSRSGWFPRIPQAQPGEEWLQVDLGTPKTVKGVIIQ GARGGDSITAVEARAFVRKFKVSYSLNGKDWEYIQDPRTQQP KVGCSWRPL | 181 |
| Variant 6 precursor S9NRP2b | | | |
| Soluble NRP2 | | | |
| Human NRP2 | 23-926 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFGCRVTDAPCSNMLGMLS GLIADSQISASSTQEYLWSPSAARLVSSRSGWFPRIPQAQPG EEWLQVDLGTPKTVKGVIIQGARGGDSITAVEARAFVRKFKV SYSLNGKDWEYIQDPRTQQPKLFEGNMHYDTPDIRRFDPIPA QYVRVYPERWSPAGIGMRLEVLGCDWTDSKPTVETLGPTVKS EETTTPYPTEEEATECGENCSFEDDKDLQLPSGFNCNFDFLE EPCGWMYDHAKWLRTTWASSSSPNDRTFPDDRNFLRLQSDSQ REGQYARLISPPVHLPRSPVCMEFQYQATGGRGVALQVVREA SQESKLLWVIREDQGGEWKHGRIILPSYDMEYQIVFEGVIGK GRSGEIAIDDIRISTDVPLENCMEPISAFAVDIPEIHEREGY EDEIDDEYEVDWSNSSSATSGSGAPSTDKEKSWLYTLDPILI TIIAMSSLGVLLGATCAGLLLYCTCSYSGLSSRSCTTLENYN FELYDGLKHKVKMNHQKCCSEA | 182 |
| Variant 2 | | | |
| NRP2a(17) | | | |
| NRP2 splice variant 5 | 23-901 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFGCRVTDAPCSNMLGMLS GLIADSQISASSTQEYLWSPSAARLVSSRSGWFPRIPQAQPG EEWLQVDLGTPKTVKGVIIQGARGGDSITAVEARAFVRKFKV SYSLNGKDWEYIQDPRTQQPKLFEGNMHYDTPDIRRFDPIPA QYVRVYPERWSPAGIGMRLEVLGCDWTDSKPTVETLGPTVKS EETTTPYPTEEEATECGENCSFEDDKDLQLPSGFNCNFDFLE EPCGWMYDHAKWLRTTWASSSSPNDRTFPDDRNFLRLQSDSQ REGQYARLISPPVHLPRSPVCMEFQYQATGGRGVALQVVREA SQESKLLWVIREDQGGEWKHGRIILPSYDMEYQIVFEGVIGK GRSGEIAIDDIRISTDVPLENCMEPISAFAGGTLLPGTEPTV DTVPMQPIPAYWYYVMAAGGAVLVLVSVALALVLHYHRFRYA AKKTDHSITYKTSHYTNGAPLAVEPTLTIKLEQDRGSHC | 183 |
| NRP2b (0) | | | |
| Soluble NRP2 | 23-555 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFGCRVTDAPCSNMLGMLS GLIADSQISASSTQEYLWSPSAARLVSSRSGWFPRIPQAQPG EEWLQVDLGTPKTVKGVIIQGARGGDSITAVEARAFVRKFKV SYSLNGKDWEYIQDPRTQQPKVGCSWRPL | 184 |
| S9Nrp-2b | | | |
| NRP2 A1 domain | 28-141 | CGGRLNSKDAGYITSPGYPQDYPSHQNCEWIVYAPEPNQKIV LNFNPHFEIEKHDCKYDFIEIRDGDSESADLLGKHCGNIAPP TIISSGSMLYIKFTSDYARQGAGFSLRYEI | 185 |
| NRP2 A2 domain | 149-265 | CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYY | 186 |
| NRP2 B1 domain | 280-426 | PLGMESGRIANEQISASSTYSDGRWTPQQSRLHGDDNGWTPN LDSNKEYLQVDLRFLTMLTAIATQGAISRETQNGYYVKSYKL EVSTNGEDWMVYRHGKNHKVFQANNDATEVVLNKLHAPLLTR FVRIRPQTWHSGIALRLELFG | 187 |
| NRP2 B2 domain | 438-591 | LGMLSGLIADSQISASSTQEYLWSPSAARLVSSRSGWFPRIP QAQPGEEWLQVDLGTPKTVKGVIIQGARGGDSITAVEARAFV RKFKVSYSLNGKDWEYIQDPRTQQPKLFEGNMHYDTPDIRRF DPIPAQYVRVYPERWSPAGIGMRLEVLG | 188 |
| NRP2 C domain | 641-794 | PSGFNCNFDFLEEPCGWMYDHAKWLRTTWASSSSPNDRTFPD DRNFLRLQSDSQREGQYARLISPPVHLPRSPVCMEFQYQATG GRGVALQVVREASQESKLLWVIREDQGGEWKHGRIILPSYDM EYQIVFEGVIGKGRSGEIAIDDIRISTD | 189 |
| NRP2 combined | 23-265 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYY | 190 |
| A1A2 domains | | | |
| NRP2 combined | 149-426 | CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFG | 191 |
| A2B1 domains | | | |
| NRP2 combined | 23-426 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFG | 192 |
| A1A2B1 domains | | | |
| NRP2 combined A1A2B1B2 domains | 23-595 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFGCRVTDAPCSNMLGMLS GLIADSQISASSTQEYLWSPSAARLVSSRSGWFPRIPQAQPG EEWLQVDLGTPKTVKGVIIQGARGGDSITAVEARAFVRKFKV SYSLNGKDWEYIQDPRTQQPKLFEGNMHYDTPDIRRFDPIPA QYVRVYPERWSPAGIGMRLEVLGCDWT | 193 |
| NRP2 combined A2B1B2 domains | 145-595 | GSEDCSKNFTSPNGTIESPGFPEKYPHNLDCTFTILAKPKME IILQFLIFDLEHDPLQVGEGDCKYDWLDIWDGIPHVGPLIGK YCGTKTPSELRSSTGILSLTFHTDMAVAKDGFSARYYLVHQE PLENFQCNVPLGMESGRIANEQISASSTYSDGRWTPQQSRLH GDDNGWTPNLDSNKEYLQVDLRFLTMLTAIATQGAISRETQN GYYVKSYKLEVSTNGEDWMVYRHGKNHKVFQANNDATEVVLN KLHAPLLTRFVRIRPQTWHSGIALRLELFGCRVTDAPCSNML GMLSGLIADSQISASSTQEYLWSPSAARLVSSRSGWFPRIPQ AQPGEEWLQVDLGTPKTVKGVIIQGARGGDSITAVEARAFVR KFKVSYSLNGKDWEYIQDPRTQQPKLFEGNMHYDTPDIRRFD PIPAQYVRVYPERWSPAGIGMRLEVLGCDWT | 194 |
| NRP2 combined B1B2 domains | 276-595 | QCNVPLGMESGRIANEQISASSTYSDGRWTPQQSRLHGDDNG WTPNLDSNKEYLQVDLRFLTMLTAIATQGAISRETQNGYYVK SYKLEVSTNGEDWMVYRHGKNHKVFQANNDATEVVLNKLHAP LLTRFVRIRPQTWHSGIALRLELFGCRVTDAPCSNMLGMLSG LIADSQISASSTQEYLWSPSAARLVSSRSGWFPRIPQAQPGE EWLQVDLGTPKTVKGVIIQGARGGDSITAVEARAFVRKFKVS YSLNGKDWEYIQDPRTQQPKLFEGNMHYDTPDIRRFDPIPAQ YVRVYPERWSPAGIGMRLEVLGCDWT | 195 |
| NRP2 v2 - Fc fusion protein | 23-855 | QPDPPCGGRLNSKDAGYITSPGYPQDYPSHQNCEWIVYAPEP NQKIVLNFNPHFEIEKHDCKYDFIEIRDGDSESADLLGKHCG NIAPPTIISSGSMLYIKFTSDYARQGAGFSLRYEIFKTGSED CSKNFTSPNGTIESPGFPEKYPHNLDCTFTILAKPKMEIILQ FLIFDLEHDPLQVGEGDCKYDWLDIWDGIPHVGPLIGKYCGT KTPSELRSSTGILSLTFHTDMAVAKDGFSARYYLVHQEPLEN FQCNVPLGMESGRIANEQISASSTYSDGRWTPQQSRLHGDDN GWTPNLDSNKEYLQVDLRFLTMLTAIATQGAISRETQNGYYV KSYKLEVSTNGEDWMVYRHGKNHKVFQANNDATEVVLNKLHA PLLTRFVRIRPQTWHSGIALRLELFGCRVTDAPCSNMLGMLS GLIADSQISASSTQEYLWSPSAARLVSSRSGWFPRIPQAQPG EEWLQVDLGTPKTVKGVIIQGARGGDSITAVEARAFVRKFKV SYSLNGKDWEYIQDPRTQQPKLFEGNMHYDTPDIRRFDPIPA QYVRVYPERWSPAGIGMRLEVLGCDWTDSKPTVETLGPTVKS EETTTPYPTEEEATECGENCSFEDDKDLQLPSGFNCNFDFLE EPCGWMYDHAKWLRTTWASSSSPNDRTFPDDRNFLRLQSDSQ REGQYARLISPPVHLPRSPVCMEFQYQATGGRGVALQVVREA SQESKLLWVIREDQGGEWKHGRIILPSYDMEYQIVFEGVIGK GRSGEIAIDDIRISTDVPLENCMEPISAFAVDIPEIHEREGY EDEIDDEYEVDWSNSSSATSGSGAPSTDKEKSWLYDKTHTCP PCPAPELLGGPSVFLFPPKPKDTLMISRTPEVTCVVVDVSHE DPEVKFNWYVDGVEVHNAKTKPREEQYNSTYRVVSVLTVLHQ DWLNGKEYKCKVSNKALPAPIEKTISKAKGQPREPQVYTLPP SRDELTKNQVSLTCLVKGFYPSDIAVEWESNGQPENNYKTTP PVLDSDGSFFLYSKLTVDKSRWQQGNVFSCSVMHEALHNHYT QKSLSLSPGK | 196 |
| NRP2 A2B1B2-Fc | 145-595 | GSEDCSKNFTSPNGTIESPGFPEKYPHNLDCTFTILAKPKME IILQFLIFDLEHDPLQVGEGDCKYDWLDIWDGIPHVGPLIGK YCGTKTPSELRSSTGILSLTFHTDMAVAKDGFSARYYLVHQE PLENFQCNVPLGMESGRIANEQISASSTYSDGRWTPQQSRLH GDDNGWTPNLDSNKEYLQVDLRFLTMLTAIATQGAISRETQN GYYVKSYKLEVSTNGEDWMVYRHGKNHKVFQANNDATEVVLN KLHAPLLTRFVRIRPQTWHSGIALRLELFGCRVTDAPCSNML GMLSGLIADSQISASSTQEYLWSPSAARLVSSRSGWFPRIPQ AQPGEEWLQVDLGTPKTVKGVIIQGARGGDSITAVEARAFVR KFKVSYSLNGKDWEYIQDPRTQQPKLFEGNMHYDTPDIRRFD PIPAQYVRVYPERWSPAGIGMRLEVLGCDWTDKTHTCPPCPA PELLGGPSVFLFPPKPKDTLMISRTPEVTCVVVDVSHEDPEV KFNWYVDGVEVHNAKTKPREEQYNSTYRVVSVLTVLHQDWLN GKEYKCKVSNKALPAPIEKTISKAKGQPREPQVYTLPPSRDE LTKNQVSLTCLVKGFYPSDIAVEWESNGQPENNYKTTPPVLD SDGSFFLYSKLTVDKSRWQQGNVFSCSVMHEALHNHYTQKSL SLSPGK | 197 |
| NRP2 combined B2C domains | 438-794 | LGMLSGLIADSQISASSTQEYLWSPSAARLVSSRSGWFPRIP QAQPGEEWLQVDLGTPKTVKGVIIQGARGGDSITAVEARAFV RKFKVSYSLNGKDWEYIQDPRTQQPKLFEGNMHYDTPDIRRF DPIPAQYVRVYPERWSPAGIGMRLEVLGCDWTDSKPTVETLG PTVKSEETTTPYPTEEEATECGENCSFEDDKDLQLPSGFNCN FDFLEEPCGWMYDHAKWLRTTWASSSSPNDRTFPDDRNFLRL QSDSQREGQYARLISPPVHLPRSPVCMEFQYQATGGRGVALQ VVREASQESKLLWVIREDQGGEWKHGRIILPSYDMEYQIVFE GVIGKGRSGEIAIDDIRISTD | 198 |
| NRP2 combined B1B2C domains | 276-794 | QCNVPLGMESGRIANEQISASSTYSDGRWTPQQSRLHGDDNG WTPNLDSNKEYLQVDLRFLTMLTAIATQGAISRETQNGYYVK SYKLEVSTNGEDWMVYRHGKNHKVFQANNDATEVVLNKLHAP LLTRFVRIRPQTWHSGIALRLELFGCRVTDAPCSNMLGMLSG LIADSQISASSTQEYLWSPSAARLVSSRSGWFPRIPQAQPGE EWLQVDLGTPKTVKGVIIQGARGGDSITAVEARAFVRKFKVS YSLNGKDWEYIQDPRTQQPKLFEGNMHYDTPDIRRFDPIPAQ YVRVYPERWSPAGIGMRLEVLGCDWTDSKPTVETLGPTVKSE ETTTPYPTEEEATECGENCSFEDDKDLQLPSGFNCNFDFLEE PCGWMYDHAKWLRTTWASSSSPNDRTFPDDRNFLRLQSDSQR EGQYARLISPPVHLPRSPVCMEFQYQATGGRGVALQVVREAS QESKLLWVIREDQGGEWKHGRIILPSYDMEYQIVFEGVIGKG RSGEIAIDDIRISTD | 199 |

Neuropilin-2 can respond to multiple ligands through the recruitment of diverse co-receptors to modulate a broad range of cellular functions through its roles both as an essential cell surface receptor, and co-receptor for a variety of ligands (see, e.g., Guo and Vander Kooi, J. Cell. Biol. 290 No 49: 29120-29126. 2015; Prud'homme et al., Oncotarget 3:921-939, 2012). For instance, NRP2 functions during epithelial to mesenchymal transition (EMT), for example, by promoting TGF-β1-mediated EMT in colorectal and other cancer cells (see, e.g., Grandclement et al., PLoS ONE 6(7) e20444, 2011) as well as mediating EMT, or endo-EMT in fibroblasts, myofibroblasts and endothelial cells to promote fibrosis formation (See, e.g., Pardali et al., Int. J. Mol. Sci. 18 2157 2017).

Neuropilin-2 expression also promotes lymphangiogenesis, and modulates vascular permeability (see, e.g., Doci et al., Cancer Res. 75(14) 2937-2948, 2015; Mucka, et al., Am. J. Path 186 (11) 2803-2812 2016), and single nucleotide polymorphisms (SNPs) in NRP2 are associated with lymphedema (see, e.g., Miaskowski et al., PLoS ONE 8(4) e60164, 2013). NRP2 also regulates smooth muscle contractility (see, e.g., Bielenberg et al., Amer. J. Path. 181:548-559, 2012], and is highly expressed in developing skeletal muscle (see, e.g. Meye, et al., PLOS ONE DOI.10.1371/journal.pone.0139520 2015).

Neuropilins are also multifunctional co-receptors involved in tumor initiation, growth, metastasis, lymphangiogenesis, lymphatic metastasis and tumor immuno-surveillance, thereby directly contributing to tumor initiation, survival, and metastasis (see, e.g., Goel et al., EMBO Mol. Med. 5:488-508, 2013; Cao et al., Can. Res. 73(14) 4579-4590 2013; Tu, et al., Oncol. Lett. 12 4224-4230, 2016), Samuel et al., PLoS ONE 6(10) e23208, 2011).

Neuropilin-2 is expressed in various cells of the immune system such as B cells, T cells, NK cells, neutrophils, dendritic cells and macrophages, including for example, alveolar macrophages, and plays an important role in the regulation of immune cell activation and migration (see, e.g., Mendesda-Cruz et al., PLoS ONE 9(7) e103405, 2014) including endosome maturation, the modulation of autophagy and efferocytosis, (see, e.g., Stanton et al., Cancer Res. 73:160-171, 2013, Schellenburg et al., Mol. Imm 90:239-244, 2017, Wang et al., Cancer Lett. 418 176-184 2018).

NRP2 is also expressed in endothelial and epithelial cells in the lung, and other tissues and cell types including bone osteoclasts and muscle cells [see, e.g., Bielenberg et al., Amer. J. Path. 181:548-559, 2012; Aung, et al., PLoS ONE 11(2) e0147358, 2016; and Wild et al., Int. J. Exp. Path. 93:81-103, 2012).

Neuropilin-2 also plays an active role both in neuronal development, and in the adult NRP2 is actively involved in peripheral nerve growth and remodeling, and plays a role in pain perception in inflammatory conditions such as arthritis, osteoarthritis and rheumatoid arthritis (see e.g. Hamilton, J et al., J. Bone & Min. Res. 2016 31(5) 911-924; Bannerman, P., et al., J. Neurosci. Res. 2008 86(14) 3163-3169; Malykhina, A., et al., BMC Physiology 2012, 12, 15).

It is becoming increasingly apparent that Neuropilin-2 also plays a key role in endosome development and regulates late endosomal maturation. These processes play important roles in phagocytosis and efferocytosis, which respectively play key roles in the clearance of infections and apoptotic cells (See, e.g. Diaz-Vera et al., J. Cell. Sci. 130, 697-711 2017; Dutta et al., Cancer Res. 76(2) 418-428 2016).

Neuropilin-2 is known to be a key player in the pathophysiology of many diseases ("NRP2-associated disease") and interacts with a broad array of soluble ligands including, for example, semaphorin 3F, VEGF-C and D, and TGF-beta, and including an array of cellular receptors and cofactors ("NRP2 ligands") (see, for example, Tables N2, N3 below), and Figures 1A-1B).

| **Table N2. Exemplary NRP2 Ligands** | | |
|---|---|---|
| **Ligand** | **NRP1** | **NRP2** |
| VEGF-A121 | + | |
| VEGF-A145 | | + |
| VEGF-A165 | + | + |
| VEGF-B 167 | + | |
| VEGF-C | + | + |
| VEGF-D | + | + |
| VEGF-E | + | |
| PIGF-2 | + | + |
| VEGFR | +R1 and R2 | +R1, R2, R3 |
| Heparin | + | + |
| SEMA 3A | + | |
| SEMA 3B, 3C, 3D, 3F, 3G | + | + |
| Plexins A1, A2, A3, A4, & D1 | + | + |
| TGF-B1, 2, 3, and LAP | + | + |
| TbRI and TbRII | + | + |
| FGF-1, 2, 4, 7 | + | + |
| FGF receptor 1 | + | + |
| Integrins (see **Table N3**) | + | + |
| Fibronectin | + | |
| Galectin-1 | + | + |
| Li-CAM | + | + |
| Glat-1 | + | |
| HRS polypeptides | | + |

| **Table N3. Vertebrate integrins as NRP2 ligands** | | | |
|---|---|---|---|
| **Name** | **Synonyms** | **Distribution** | **Ligands** |
| α₁β₁ | VLA-1 | Many | Collagens, laminins |
| α₂β₁ | VLA-2 | Many | Collagens, laminins |
| α₃β₁ | VLA-3 | Many | Laminin-5 |
| α₄β₁ | VLA-4 | Hematopoietic cells | Fibronectin, VCAM-1 |
| α₅β₁ | VLA-5; fibronectin receptor | widespread | fibronectin and proteinases |
| α₆β₁ | VLA-6; laminin receptor | widespread | laminins |
| α₇β₁ | | muscle, glioma | laminins |
| α_{L}β₂ | LFA-1 | T-lymphocytes | ICAM-1, ICAM-2 |
| α_{M}β₂ | Mac-1, CR3 | Neutrophils and monocytes | Serum proteins, ICAM-1 |
| α_{IIb}β₃ | Fibrinogen receptor; gpIIbIIIa | Platelets | fibrinogen, fibronectin^{[24]} |
| α_{V}β₁ | | ocular melanoma; neurological tumors | vitronectin; fibrinogen |
| α_{V}β₃ | vitronectin receptor | activated endothelial cells, melanoma, glioblastoma | vitronectin, fibronectin, fibrinogen, osteopontin, Cyr61, thyroxine, TETRAC |
| α_{V}β₅ | | widespread, esp. fibroblasts, epithelial cells | vitronectin and adenovirus |
| α_{V}β₆ | | proliferating epithelia, esp. lung and mammary gland | fibronectin; TGFβ1+3 |
| α_{V}β₈ | | neural tissue; peripheral nerve | fibronectin; TGFβ1+3 |
| α₆β₄ | | Epithelial cells | Laminin |

NRP2 is also polysialyated on dendritic cells, and actively interacts with the chemokine CCL21 to mediate immune cell migration via the CCR7 receptor, and for which single nucleotide polymorphisms in NRP2 are associated with ILD and RA have been described (see, e.g., Rey-Gallardo et al., Glycobiology 20:1139-1146, 2010 ., Stahl et al., Nat. Genet. 42:508-514, 2013., Miller et al., Arthritis Rheum. 65:3239-3247). Additionally, soluble, circulating forms of NRP2 are known (see, e.g., Parker et al., Structure 23(4) 677-687, 2015), and internal studies have confirmed the existence of circulating complexes of HRS polypeptides and NRP2 polypeptides in serum, and other fluids.

### ADDITIONAL THERAPEUTIC AGENTS AND COMPOSITIONS

***Immunotherapy Agents.*** Certain embodiments employ one or more cancer immunotherapy agents. In certain instances, an immunotherapy agent modulates the immune response of a subject, for example, to increase or maintain a cancer-related or cancer-specific immune response, and thereby results in increased immune cell inhibition or reduction of cancer cells. Exemplary immunotherapy agents include polypeptides, for example, antibodies and antigen-binding fragments thereof, ligands, and small peptides, and mixtures thereof. Also include as immunotherapy agents are small molecules, cells (e.g., immune cells such as T-cells), various cancer vaccines, gene therapy or other polynucleotide-based agents, including viral agents such as oncolytic viruses, and others known in the art. Thus, in certain embodiments, the cancer immunotherapy agent is selected from one or more of immune checkpoint modulatory agents, cancer vaccines, oncolytic viruses, cytokines, and a cell-based immunotherapies.

In certain embodiments, the cancer immunotherapy agent is an immune checkpoint modulatory agent. Particular examples include "antagonists" of one or more inhibitory immune checkpoint molecules, and "agonists" of one or more stimulatory immune checkpoint molecules. Generally, immune checkpoint molecules are components of the immune system that either turn up a signal (co-stimulatory molecules) or turn down a signal, the targeting of which has therapeutic potential in cancer because cancer cells can perturb the natural function of immune checkpoint molecules (see, e.g., Sharma and Allison, Science. 348:56-61, 2015; Topalian et al., Cancer Cell. 27:450-461, 2015; Pardoll, Nature Reviews Cancer. 12:252-264, 2012). In some embodiments, the immune checkpoint modulatory agent (e.g., antagonist, agonist) "binds" or "specifically binds" to the one or more immune checkpoint molecules, as described herein.

In particular embodiments, the immune checkpoint modulatory agent is a polypeptide or peptide. The terms "peptide" and "polypeptide" are used interchangeably herein, however, in certain instances, the term "peptide" can refer to shorter polypeptides, for example, polypeptides that consist of about 2, 3, 4, 5, 6, 7, 8, 9, 10, 11, 12, 13, 14, 15, 16, 17, 18, 19, 20, 25, 30, 35, 40, 45, or 50 amino acids, including all integers and ranges (e.g., 5-10, 8-12, 10-15) in between. Polypeptides and peptides can be composed of naturally-occurring amino acids and/or non-naturally occurring amino acids, as described herein

Antibodies are also included as polypeptides. Thus, in some embodiments, the immune checkpoint modulatory polypeptide agent is an antibody or "antigen-binding fragment thereof", as described elsewhere herein.

In some embodiments, the agent is or comprises a "ligand," for example, a natural ligand, of the immune checkpoint molecule. A "ligand" refers generally to a substance or molecule that forms a complex with a target molecule (e.g., biomolecule) to serve a biological purpose, and includes a "protein ligand," which generally produces a signal by binding to a site on a target molecule or target protein. Thus, certain agents are protein ligands that, in nature, bind to an immune checkpoint molecule and produce a signal. Also included are "modified ligands," for example, protein ligands that are fused to a pharmacokinetic modifier, for example, an Fc region derived from an immunoglobulin.

The binding properties of polypeptides can be quantified using methods well known in the art (see Davies et al., Annual Rev. Biochem. 59:439-473, 1990). In some embodiments, a polypeptide specifically binds to a target molecule, for example, an immune checkpoint molecule or an epitope thereof, with an equilibrium dissociation constant that is about or ranges from about ≤10-7 to about 10-8 M. In some embodiments, the equilibrium dissociation constant is about or ranges from about ≤10-9 M to about ≤10-10 M. In certain illustrative embodiments, the polypeptide has an affinity (Kd or EC₅₀) for a target described herein (to which it specifically binds) of about, at least about, or less than about, 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, or 50 nM.

In some embodiments, the agent is a "small molecule," which refers to an organic compound that is of synthetic or biological origin (biomolecule), but is typically not a polymer. Organic compounds refer to a large class of chemical compounds whose molecules contain carbon, typically excluding those that contain only carbonates, simple oxides of carbon, or cyanides. A "biomolecule" refers generally to an organic molecule that is produced by a living organism, including large polymeric molecules (biopolymers) such as peptides, polysaccharides, and nucleic acids as well, and small molecules such as primary secondary metabolites, lipids, phospholipids, glycolipids, sterols, glycerolipids, vitamins, and hormones. A "polymer" refers generally to a large molecule or macromolecule composed of repeating structural units, which are typically connected by covalent chemical bond.

In certain embodiments, a small molecule has a molecular weight of about or less than about 1000-2000 Daltons, typically between about 300 and 700 Daltons, and including about or less than about 50, 100, 150, 200, 250, 300, 350, 400, 450, 500, 550, 500, 650, 600, 750, 700, 850, 800, 950, 1000 or 2000 Daltons.

Certain small molecules can have the "specific binding" characteristics described for herein polypeptides such as antibodies. For instance, in some embodiments a small molecule specifically binds to a target, for example, an immune checkpoint molecule, with a binding affinity (Kd or EC₅₀) of about, at least about, or less than about, 0.01, 0.05, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, or 50 nM.

In some embodiments, the immune checkpoint modulatory agent is an antagonist or inhibitor of one or more inhibitory immune checkpoint molecules. Exemplary inhibitory immune checkpoint molecules include Programmed Death-Ligand 1 (PD-L1), Programmed Death-Ligand 2 (PD-L2), Programmed Death 1 (PD-1), Cytotoxic T-Lymphocyte-Associated protein 4 (CTLA-4), Indoleamine 2,3-dioxygenase (IDO), tryptophan 2,3-dioxygenase (TDO), T-cell Immunoglobulin domain and Mucin domain 3 (TIM-3), Lymphocyte Activation Gene-3 (LAG-3), V-domain Ig suppressor of T cell activation (VISTA), B and T Lymphocyte Attenuator (BTLA), CD160, and T-cell immunoreceptor with Ig and ITIM domains (TIGIT).

In certain embodiments, the agent is a PD-1 (receptor) antagonist or inhibitor, the targeting of which has been shown to restore immune function in the tumor environment (see, e.g., Phillips et al., Int Immunol. 27:39-46, 2015). PD-1 is a cell surface receptor that belongs to the immunoglobulin superfamily and is expressed on T cells and pro-B cells. PD-1 interacts with two ligands, PD-L1 and PD-L2. PD-1 functions as an inhibitory immune checkpoint molecule, for example, by reducing or preventing the activation of T-cells, which in turn reduces autoimmunity and promotes self-tolerance. The inhibitory effect of PD-1 is accomplished at least in part through a dual mechanism of promoting apoptosis in antigen specific T-cells in lymph nodes while also reducing apoptosis in regulatory T cells (suppressor T cells). Some examples of PD-1 antagonists or inhibitors include an antibody or antigen-binding fragment or small molecule that specifically binds to PD-1 and reduces one or more of its immune-suppressive activities, for example, its downstream signaling or its interaction with PD-L1. Specific examples of PD-1 antagonists or inhibitors include the antibodies nivolumab, pembrolizumab, PDR001, MK-3475, AMP-224, AMP-514, and pidilizumab, and antigen-binding fragments thereof (see, e.g., U.S. Patent Nos. 8,008,449; 8,993,731; 9,073,994; 9,084,776; 9,102,727; 9,102,728; 9,181,342; 9,217,034; 9,387,247; 9,492,539; 9,492,540; and U.S. Application Nos. 2012/0039906; 2015/0203579).

In some embodiments, the agent is a PD-L1 antagonist or inhibitor. As noted above, PD-L1 is one of the natural ligands for the PD-1 receptor. General examples of PD-L1 antagonists or inhibitors include an antibody or antigen-binding fragment or small molecule that specifically binds to PD-L1 and reduces one or more of its immune-suppressive activities, for example, its binding to the PD-1 receptor. Specific examples of PD-L1 antagonists include the antibodies atezolizumab (MPDL3280A), avelumab (MSB0010718C), and durvalumab (MEDI4736), and antigen-binding fragments thereof (see, e.g., U.S. Patent Nos. 9,102,725; 9,393,301; 9,402,899; 9,439,962).

In some embodiments, the agent is a PD-L2 antagonist or inhibitor. As noted above, PD-L2 is one of the natural ligands for the PD-1 receptor. General examples of PD-L2 antagonists or inhibitors include an antibody or antigen-binding fragment or small molecule that specifically binds to PD-L2 and reduces one or more of its immune-suppressive activities, for example, its binding to the PD-1 receptor.

In some embodiments, the agent is a CTLA-4 antagonist or inhibitor. CTLA4 or CTLA-4 (cytotoxic T-lymphocyte-associated protein 4), also known as CD152 (cluster of differentiation 152), is a protein receptor that functions as an inhibitory immune checkpoint molecule, for example, by transmitting inhibitory signals to T-cells when it is bound to CD80 or CD86 on the surface of antigen-presenting cells. General examples CTLA-4 antagonists or inhibitors include an antibody or antigen-binding fragment or small molecule that specifically binds to CTLA-4. Particular examples include the antibodies ipilimumab and tremelimumab, and antigen-binding fragments thereof. At least some of the activity of ipilimumab is believed to be mediated by antibody-dependent cell-mediated cytotoxicity (ADCC) killing of suppressor Tregs that express CTLA-4.

In some embodiments, the agent is an IDO antagonist or inhibitor, or a TDO antagonist or inhibitor. IDO and TDO are tryptophan catabolic enzymes with immune-inhibitory properties. For example, IDO is known to suppress T-cells and NK cells, generate and activate Tregs and myeloid-derived suppressor cells, and promote tumor angiogenesis. General examples of IDO and TDO antagonists or inhibitors include an antibody or antigen-binding fragment or small molecule that specifically binds to IDO or TDO (see, e.g., Platten et al., Front Immunol. 5: 673, 2014) and reduces or inhibits one or more immune-suppressive activities. Specific examples of IDO antagonists or inhibitors include indoximod (NLG-8189), 1-methyl-tryptophan (1MT), β-Carboline (norharmane; 9H-pyrido[3,4-b]indole), rosmarinic acid, and epacadostat (see, e.g., Sheridan, Nature Biotechnology. 33:321-322, 2015). Specific examples of TDO antagonists or inhibitors include 680C91 and LM10 (see, e.g., Pilotte et al., PNAS USA. 109:2497-2502, 2012).

In some embodiments, the agent is a TIM-3 antagonist or inhibitor. T-cell Immunoglobulin domain and Mucin domain 3 (TIM-3) is expressed on activated human CD4+ T-cells and regulates Th1 and Th17 cytokines. TIM-3 also acts as a negative regulator of Th1/Tc1 function by triggering cell death upon interaction with its ligand, galectin-9. TIM-3 contributes to the suppressive tumor microenvironment and its overexpression is associated with poor prognosis in a variety of cancers (see, e.g., Li et al., Acta Oncol. 54:1706-13, 2015). General examples of TIM-3 antagonists or inhibitors include an antibody or antigen-binding fragment or small molecule that specifically binds to TIM-3 and reduces or inhibits one or more of its immune-suppressive activities.

In some embodiments, the agent is a LAG-3 antagonist or inhibitor. Lymphocyte Activation Gene-3 (LAG-3) is expressed on activated T-cells, natural killer cells, B-cells and plasmacytoid dendritic cells. It negatively regulates cellular proliferation, activation, and homeostasis of T-cells, in a similar fashion to CTLA-4 and PD-1 (see, e.g., Workman and Vignali. European Journal of Immun. 33: 970-9, 2003; and Workman et al., Journal of Immun. 172: 5450-5, 2004), and has been reported to play a role in Treg suppressive function (see, e.g., Huang et al., Immunity. 21: 503-13, 2004). LAG3 also maintains CD8+ T-cells in a tolerogenic state and combines with PD-1 to maintain CD8 T-cell exhaustion. General examples of LAG-3 antagonists or inhibitors include an antibody or antigen-binding fragment or small molecule that specifically binds to LAG-3 and inhibits one or more of its immune-suppressive activities. Specific examples include the antibody BMS-986016, and antigen-binding fragments thereof.

In some embodiments, the agent is a VISTA antagonist or inhibitor. V-domain Ig suppressor of T cell activation (VISTA) is primarily expressed on hematopoietic cells and is an inhibitory immune checkpoint regulator that suppresses T-cell activation, induces Foxp3 expression, and is highly expressed within the tumor microenvironment where it suppresses anti-tumor T cell responses (see, e.g., Lines et al., Cancer Res. 74:1924-32, 2014). General examples of VISTA antagonists or inhibitors include an antibody or antigen-binding fragment or small molecule that specifically binds to VISTA and reduces one or more of its immune-suppressive activities.

In some embodiments, the agent is a BTLA antagonist or inhibitor. B- and T-lymphocyte attenuator (BTLA; CD272) expression is induced during activation of T-cells, and it inhibits T-cells via interaction with tumor necrosis family receptors (TNF-R) and B7 family of cell surface receptors. BTLA is a ligand for tumor necrosis factor (receptor) superfamily, member 14 (TNFRSF14), also known as herpes virus entry mediator (HVEM). BTLA-HVEM complexes negatively regulate T-cell immune responses, for example, by inhibiting the function of human CD8+ cancer-specific T-cells (see, e.g., Derré et al., J Clin Invest 120:157-67, 2009). General examples of BTLA antagonists or inhibitors include an antibody or antigen-binding fragment or small molecule that specifically binds to BTLA-4 and reduce one or more of its immune-suppressive activities.

In some embodiments, the agent is an HVEM antagonist or inhibitor, for example, an antagonist or inhibitor that specifically binds to HVEM and interferes with its interaction with BTLA or CD160. General examples of HVEM antagonists or inhibitors include an antibody or antigen-binding fragment or small molecule that specifically binds to HVEM, optionally reduces the HVEM/BTLA and/or HVEM/CD160 interaction, and thereby reduces one or more of the immune-suppressive activities of HVEM.

In some embodiments, the agent is a CD160 antagonist or inhibitor, for example, an antagonist or inhibitor that specifically binds to CD160 and interferes with its interaction with HVEM. General examples of CD160 antagonists or inhibitors include an antibody or antigen-binding fragment or small molecule that specifically binds to CD160, optionally reduces the CD160/HVEM interaction, and thereby reduces or inhibits one or more of its immune-suppressive activities.

In some embodiments, the agent is a TIGIT antagonist or inhibitor. T cell Ig and ITIM domain (TIGIT) is a co-inhibitory receptor that is found on the surface of a variety of lymphoid cells, and suppresses antitumor immunity, for example, via Tregs (Kurtulus et al., J Clin Invest. 125:4053-4062, 2015). General examples of TIGIT antagonists or inhibitors include an antibody or antigen-binding fragment or small molecule that specifically binds to TIGIT and reduce one or more of its immune-suppressive activities (see, e.g., Johnston et al., Cancer Cell. 26:923-37, 2014).

In certain embodiments, the immune checkpoint modulatory agent is an agonist of one or more stimulatory immune checkpoint molecules. Exemplary stimulatory immune checkpoint molecules include OX40, CD40, Glucocorticoid-Induced TNFR Family Related Gene (GITR), CD137 (4-1BB), CD27, CD28, CD226, and Herpes Virus Entry Mediator (HVEM).

In some embodiments, the agent is an OX40 agonist. OX40 (CD134) promotes the expansion of effector and memory T cells, and suppresses the differentiation and activity of T-regulatory cells (see, e.g., Croft et al., Immunol Rev. 229:173-91, 2009). Its ligand is OX40L (CD252). Since OX40 signaling influences both T-cell activation and survival, it plays a key role in the initiation of an anti-tumor immune response in the lymph node and in the maintenance of the anti-tumor immune response in the tumor microenvironment. General examples of OX40 agonists include an antibody or antigen-binding fragment or small molecule or ligand that specifically binds to OX40 and increases one or more of its immunostimulatory activities. Specific examples include OX86, OX-40L, Fc-OX40L, GSK3174998, MEDI0562 (a humanized OX40 agonist), MEDI6469 (murine OX4 agonist), and MEDI6383 (an OX40 agonist), and antigen-binding fragments thereof.

In some embodiments, the agent is a CD40 agonist. CD40 is expressed on antigen-presenting cells (APC) and some malignancies. Its ligand is CD40L (CD154). On APC, ligation results in upregulation of costimulatory molecules, potentially bypassing the need for T-cell assistance in an antitumor immune response. CD40 agonist therapy plays an important role in APC maturation and their migration from the tumor to the lymph nodes, resulting in elevated antigen presentation and T cell activation. Anti-CD40 agonist antibodies produce substantial responses and durable anticancer immunity in animal models, an effect mediated at least in part by cytotoxic T-cells (see, e.g., Johnson et al. Clin Cancer Res. 21: 1321-1328, 2015; and Vonderheide and Glennie, Clin Cancer Res. 19:1035-43, 2013). General examples of CD40 agonists include an antibody or antigen-binding fragment or small molecule or ligand that specifically binds to CD40 and increases one or more of its immunostimulatory activities. Specific examples include CP-870,893, dacetuzumab, Chi Lob 7/4, ADC-1013, CD40L, rhCD40L, and antigen-binding fragments thereof.

In some embodiments, the agent is a GITR agonist. Glucocorticoid-Induced TNFR family Related gene (GITR) increases T cell expansion, inhibits the suppressive activity of Tregs, and extends the survival of T-effector cells. GITR agonists have been shown to promote an anti-tumor response through loss of Treg lineage stability (see, e.g., Schaer et al., Cancer Immunol Res. 1:320-31, 2013). These diverse mechanisms show that GITR plays an important role in initiating the immune response in the lymph nodes and in maintaining the immune response in the tumor tissue. Its ligand is GITRL. General examples of GITR agonists include an antibody or antigen-binding fragment or small molecule or ligand that specifically binds to GITR and increases one or more of its immunostimulatory activities. Specific examples include GITRL, INCAGN01876, DTA-1, MEDI1873, and antigen-binding fragments thereof.

In some embodiments, the agent is a CD137 agonist. CD137 (4-1BB) is a member of the tumor necrosis factor (TNF) receptor family, and crosslinking of CD137 enhances T-cell proliferation, IL-2 secretion, survival, and cytolytic activity. CD137-mediated signaling also protects T-cells such as CD8+ T-cells from activation-induced cell death. General examples of CD137 agonists include an antibody or antigen-binding fragment or small molecule or ligand that specifically binds to CD137 and increases one or more of its immunostimulatory activities. Specific examples include the CD137 (or 4-1BB) ligand (see, e.g., Shao and Schwarz, J Leukoc Biol. 89:21-9, 2011) and the antibody utomilumab, including antigen-binding fragments thereof.

In some embodiments, the agent is a CD27 agonist. Stimulation of CD27 increases antigen-specific expansion of naive T cells and contributes to T-cell memory and long-term maintenance of T-cell immunity. Its ligand is CD70. The targeting of human CD27 with an agonist antibody stimulates T-cell activation and antitumor immunity (see, e.g., Thomas et al., Oncoimmunology. 2014;3:e27255. doi:10.4161/onci.27255; and He et al., J Immunol. 191:4174-83, 2013). General examples of CD27 agonists include an antibody or antigen-binding fragment or small molecule or ligand that specifically binds to CD27 and increases one or more of its immunostimulatory activities. Specific examples include CD70 and the antibodies varlilumab and CDX-1127 (1F5), including antigen-binding fragments thereof.

In some embodiments, the agent is a CD28 agonist. CD28 is constitutively expressed CD4+ T cells some CD8+ T cells. Its ligands include CD80 and CD86, and its stimulation increases T-cell expansion. General examples of CD28 agonists include an antibody or antigen-binding fragment or small molecule or ligand that specifically binds to CD28 and increases one or more of its immunostimulatory activities. Specific examples include CD80, CD86, the antibody TAB08, and antigen-binding fragments thereof.

In some embodiments, the agent is CD226 agonist. CD226 is a stimulating receptor that shares ligands with TIGIT, and opposite to TIGIT, engagement of CD226 enhances T-cell activation (see, e.g., Kurtulus et al., J Clin Invest. 125:4053-4062, 2015; Bottino et al., J Exp Med. 1984:557-567, 2003; and Tahara-Hanaoka et al., Int Immunol. 16:533-538, 2004). General examples of CD226 agonists include an antibody or antigen-binding fragment or small molecule or ligand (e.g., CD112, CD155) that specifically binds to CD226 and increases one or more of its immunostimulatory activities.

In some embodiments, the agent is an HVEM agonist. Herpesvirus entry mediator (HVEM), also known as tumor necrosis factor receptor superfamily member 14 (TNFRSF14), is a human cell surface receptor of the TNF-receptor superfamily. HVEM is found on a variety of cells including T-cells, APCs, and other immune cells. Unlike other receptors, HVEM is expressed at high levels on resting T-cells and down-regulated upon activation. It has been shown that HVEM signaling plays a crucial role in the early phases of T-cell activation and during the expansion of tumor-specific lymphocyte populations in the lymph nodes. General examples of HVEM agonists include an antibody or antigen-binding fragment or small molecule or ligand that specifically binds to HVEM and increases one or more of its immunostimulatory activities.

In certain embodiments, the cancer immunotherapy agent is a cancer vaccine. Exemplary cancer vaccines include Oncophage, human papillomavirus HPV vaccines such Gardasil or Cervarix, hepatitis B vaccines such as Engerix-B, Recombivax HB, or Twinrix, and sipuleucel-T (Provenge). In some embodiments, the cancer vaccine comprises or utilizes one or more cancer antigens, or cancer-associate d antigens. Exemplary cancer antigens include, without limitation, human Her2/neu, Her1/EGF receptor (EGFR), Her3, A33 antigen, B7H3, CD5, CD19, CD20, CD22, CD23 (IgE Receptor), MAGE-3, C242 antigen, 5T4, IL-6, IL-13, vascular endothelial growth factor VEGF (e.g., VEGF-A) VEGFR-1, VEGFR-2, VEGR-3, NRP2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), guanylyl cyclase C, NY-ESO-1, p53, survivin, integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PSMA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin.

In certain embodiments, the cancer immunotherapy agent is an oncolytic virus. An oncolytic virus is a virus that preferentially infects and kills cancer cells. Included are naturally-occurring and man-made or engineered oncolytic viruses. Most oncolytic viruses are engineered for tumor selectivity, although there are naturally-occurring examples such as Reovirus and the SVV-001 Seneca Valley virus. General examples of oncolytic viruses include VSV, Poliovirus, Reovirus, Senecavirus, and RIGVIR, and engineered versions thereof. Non-limiting examples of oncolytic viruses include herpes simplex virus (HSV) and engineered version thereof, talimogene laherparepvec (T-VEC), coxsackievirus A21 (CAVATAK^{™}), Oncorine (H101), pelareorep (REOLYSIN^{®}), Seneca Valley virus (NTX-010), *Senecavirus* SVV-001, ColoAd1, SEPREHVIR (HSV-1716), CGTG-102 (Ad5/3-D24-GMCSF), GL-ONC1, MV-NIS, and DNX-2401, among others.

In certain embodiments, the cancer immunotherapy agent is a cytokine. Exemplary cytokines include interferon (IFN)-α, IL-2, IL-12, IL-7, IL-21, and Granulocyte-macrophage colony-stimulating factor (GM-CSF).

In certain embodiments, the cancer immunotherapy agent is cell-based immunotherapy, for example, a T-cell based adoptive immunotherapy. In some embodiments, the cell-based immunotherapy comprises cancer antigen-specific T-cells, optionally *ex* vivo-derived T-cells. In some embodiments, the cancer antigen-specific T-cells are selected from one or more of chimeric antigen receptor (CAR)-modified T-cells, and T-cell Receptor (TCR)-modified T-cells, tumor infiltrating lymphocytes (TILs), and peptide-induced T-cells. In specific embodiments, the CAR-modified T-cell is targeted against CD-19 (see, e.g., Maude et al., Blood. 125:4017-4023, 2015).

In certain instances, the cancer to be treated associates with the cancer antigen, that is, the cancer antigen-specific T-cells are targeted against or enriched for at least one antigen that is known to associate with the cancer to be treated. In some embodiments, the cancer antigen is selected from one or more of CD19, human Her2/neu, Her1/EGF receptor (EGFR), Her3, A33 antigen, B7H3, CD5, CD20, CD22, CD23 (IgE Receptor), MAGE-3, C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), guanylyl cyclase C, NY-ESO-1, p53, survivin, integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin.

Additional exemplary cancer antigens include 5T4, 707-AP, 9D7, AFP, AlbZIP HPG1, alpha-5-beta-1 -integrin, alpha- 5-beta-6-integrin, alpha-actinin-4/m, alpha-methylacyl-coenzyme A racemase, ART-4, ARTC1/m, B7H4, BAGE-1, BCL-2, ber/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R1 7I, HLA-A1 1/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1 R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE- A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B1 6, MAGE-B1 7, MAGE-C1, MAGE-C2, MAGE-C3, MAGE-D1, MAGE- D2, MAGE-D4, MAGE-E1, MAGE-E2, MAGE-F1, MAGE-H1, MAGEL2, mammaglobin A, MART-1/melan-A, MART-2, MART-2/m, matrix protein 22, MCI R, M-CSF, ME1/m, mesothelin, MG50/PXDN, MMP1 1, MN/CA IX-antigen, MRP-3, MUC-1, MUC-2, MUM-1/m, MUM-2/m, MUM-3/m, myosin class l/m, NA88-A, N-acetylglucosaminyltransferase-V, Neo-PAP, Neo-PAP/m, NFYC/m, NGEP, NMP22, NPM/ALK, N-Ras/m, NSE, NY-ESO-B, NY- ESO-1, OA1, OFA-iLRP, OGT, OGT/m, OS-9, OS-9/m, osteocalcin, osteopontin, pi 5, p190 minor ber-abl, p53, p53/m, PAGE-4, PAI-1, PAI-2, PAP, PART-1, PATE, PDEF, Pim-1 Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD1 68, RU1, RU2, S-100, SAGE, SART-1, SART-2, SART-3, SCC, SIRT2/m, Sp1 7, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGF-beta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, and WT1. Certain preferred antigens include p53, CA125, EGFR, Her2/neu, hTERT, PAP, MAGE-A1, MAGE-A3, Mesothelin, MUC-1, GP100, MART-1, Tyrosinase, PSA, PSCA, PSMA, STEAP-1, Ras, CEA and WT1, and more preferably PAP, MAGE-A3, WT1, and MUC-1.

In some embodiments the antigen is selected from MAGE-A1 (e.g., MAGE-A1 according to accession number M77481 ), MAGE-A2, MAGE-A3, MAGE-A6 (e.g., MAGE-A6 according to accession number NM_005363), MAGE-C1, MAGE-C2, melan-A (e.g., melan-A according to accession number NM_00551 1 ), GP100 (e.g., GP100 according to accession number M77348), tyrosinase (e.g., tyrosinase according to accession number NM_000372), survivin (e.g., survivin according to accession number AF077350), CEA (e.g., CEA according to accession number NM_004363), Her-2/neu (e.g., Her-2/neu according to accession number M1 1 730), WT1 (e.g., WT1 according to accession number NM_000378), PRAME (e.g., PRAME according to accession number NM_0061 15), EGFRI (epidermal growth factor receptor 1 ) (e.g., EGFRI (epidermal growth factor receptor 1 ) according to accession number AF288738), MUC1, mucin-1 (e.g., mucin-1 according to accession number NM_002456), SEC61 G (e.g., SEC61 G according to accession number NM_014302), hTERT (e.g., hTERT accession number NM_198253), 5T4 (e.g., 5T4 according to accession number NM_006670), TRP-2 (e.g., TRP-2 according to accession number NM_001 922), STEAP1 (Six-transmembrane epithelial antigen of prostate 1), PSCA, PSA, PSMA, etc.

In some embodiments, the cancer antigen is selected from PCA, PSA, PSMA, STEAP, and optionally MUC-1, including fragments, variants, and derivatives thereof. In some embodiments, the cancer antigen selected from NY-ESO-1, MAGE-C1, MAGE-C2, survivin, 5T4, and optionally MUC- 1, including fragments, variants, and derivatives thereof.

In some instances, cancer antigens encompass idiotypic antigens associated with a cancer or tumor disease, particularly lymphoma or a lymphoma associated disease, for example, wherein the idiotypic antigen is an immunoglobulin idiotype of a lymphoid blood cell or a T cell receptor idiotype of a lymphoid blood cell.

In some instances, the cancer antigen-specific T-cells are selected from one or more of chimeric antigen receptor (CAR)-modified T-cells (e.g., targeted against a cancer antigen), and T-cell Receptor (TCR)-modified T-cells, tumor infiltrating lymphocytes (TILs), and peptide-induced T-cells.

The skilled artisan will appreciate that the various cancer immunotherapy agents described herein can be combined with any one or more of the various HRS polypeptides described herein, and used according to any one or more of the methods or compositions described herein.

*Chemotherapeutic Agents.* Certain embodiments employ one or more chemotherapeutic agents, for example, small molecule chemotherapeutic agents. Non-limiting examples of chemotherapeutic agents include alkylating agents, anti-metabolites, cytotoxic antibiotics, topoisomerase inhibitors (type 1 or type II), an anti-microtubule agents, among others.

Examples of alkylating agents include nitrogen mustards (e.g., mechlorethamine, cyclophosphamide, mustine, melphalan, chlorambucil, ifosfamide , and busulfan), nitrosoureas (e.g., N-Nitroso-N-methylurea (MNU), carmustine (BCNU), lomustine (CCNU), semustine (MeCCNU), fotemustine, and streptozotocin), tetrazines (e.g., dacarbazine, mitozolomide, and temozolomide), aziridines (e.g., thiotepa, mytomycin, and diaziquone (AZQ)), cisplatins and derivatives thereof (e.g., carboplatin and oxaliplatin), and non-classical alkylating agents (optionally procarbazine and hexamethylmelamine).

Examples of anti-metabolites include anti-folates (e.g., methotrexate and pemetrexed), fluoropyrimidines (e.g., 5-fluorouracil and capecitabine), deoxynucleoside analogues (e.g., ancitabine, enocitabine, cytarabine, gemcitabine, decitabine, azacitidine, fludarabine, nelarabine, cladribine, clofarabine, fludarabine, and pentostatin), and thiopurines (e.g., thioguanine and mercaptopurine);

Examples of cytotoxic antibiotics include anthracyclines (e.g., doxorubicin, daunorubicin, epirubicin, idarubicin, pirarubicin, aclarubicin, and mitoxantrone), bleomycins, mitomycin C, mitoxantrone, and actinomycin. Examples of topoisomerase inhibitors include camptothecin, irinotecan, topotecan, etoposide, doxorubicin, mitoxantrone, teniposide, novobiocin, merbarone, and aclarubicin.

Examples of anti-microtubule agents include taxanes (e.g., paclitaxel and docetaxel) and vinca alkaloids (e.g., vinblastine, vincristine, vindesine, vinorelbine).

The skilled artisan will appreciate that the various chemotherapeutic agents described herein can be combined with any one or more of the various HRS polypeptides described herein, and used according to any one or more of the methods or compositions described herein.

***Hormonal Therapeutic Agents.*** Certain embodiments employ at least one hormonal therapeutic agent. General examples of hormonal therapeutic agents include hormonal agonists and hormonal antagonists. Particular examples of hormonal agonists include progestogen (progestin), corticosteroids (e.g., prednisolone, methylprednisolone, dexamethasone), insulin like growth factors, VEGF derived angiogenic and lymphangiogenic factors (e.g., VEGF-A, VEGF-A145, VEGF-A165, VEGF-C, VEGF-D, PIGF-2), fibroblast growth factor (FGF), galectin, hepatocyte growth factor (HGF), platelet derived growth factor (PDGF), transforming growth factor (TGF)-beta, androgens, estrogens, and somatostatin analogs. Examples of hormonal antagonists include hormone synthesis inhibitors such as aromatase inhibitors and gonadotropin-releasing hormone (GnRH)s agonists (e.g., leuprolide, goserelin, triptorelin, histrelin) including analogs thereof. Also included are hormone receptor antagonist such as selective estrogen receptor modulators (SERMs; e.g., tamoxifen, raloxifene, toremifene) and anti-androgens (e.g., flutamide, bicalutamide, nilutamide).

Also included are hormonal pathway inhibitors such as antibodies directed against hormonal receptors. Examples include inhibitors of the the IGF receptor (e.g., IGF-IR1) such as cixutumumab, dalotuzumab, figitumumab, ganitumab, istiratumab, and robatumumab; inhibitors of the vascular endothelial growth factor receptors 1, 2 or 3 (VEGFR1, VEGFR2 or VEGFR3) such as alacizumab pegol, bevacizumab, icrucumab, ramucirumab; inhibitors of the TGF-beta receptors R1, R2, and R3 such as fresolimumab and metelimumab; inhibitors of c-Met such as naxitamab; inhibitors of the EGF receptor such as cetuximab, depatuxizumab mafodotin, futuximab, imgatuzumab, laprituximab emtansine, matuzumab, modotuximab, necitumumab, nimotuzumab, panitumumab, tomuzotuximab, and zalutumumab; inhibitors of the FGF receptor such as aprutumab ixadotin and bemarituzumab; and inhibitors of the PDGF receptor such as olaratumab and tovetumab.

The skilled artisan will appreciate that the various hormonal therapeutic agents described herein can be combined with any one or more of the various HRS polypeptides described herein, and used according to any one or more of the methods or compositions described herein.

***Kinase Inhibitors.*** Certain embodiments employ at least one kinase inhibitor, including tyrosine kinase inhibitors and phosphoinositide 3 (PI3) kinase inhibitors. Examples of kinase inhibitors include, without limitation, adavosertib, afanitib, aflibercept, axitinib, bevacizumab, bosutinib, cabozantinib, cetuximab, cobimetinib, crizotinib, dasatinib, entrectinib, erdafitinib, erlotinib, fostamitinib, gefitinib, ibrutinib, imatinib, lapatinib, lenvatinib, mubritinib, nilotinib, panitumumab, pazopanib, pegaptanib, ponatinib, ranibizumab, regorafenib, ruxolitinib, sorafenib, sunitinib, SU6656, tofacitinib, trastuzumab, vandetanib, and vemuafenib. Exemplary PI3 kinase inhibitors include alpelisib, buparlisib, copanlisib, CUDC-907, dactolisib, duvelisib, GNE-477, idelasib, IPI-549, LY294002, ME-401, perifosine, PI-103, pictilisib, PWT33597, RP6503, taselisib, umbralisib, voxtalisib, wortmannin, and XL147.

The skilled artisan will appreciate that the various kinase inhibitors described herein can be combined with any one or more of the various HRS polypeptides described herein, and used according to any one or more of the methods or compositions described herein.

### HRS Polypeptides for use in the treatment of cancer

The cancer expresses or overexpresses NRP2. In some instances, the cancer displays NRP2-dependent growth, NRP2-dependent adhesion, NRP2-dependent migration, NRP2-dependent chemoresistance, and/or NRP2-dependent invasion. In some embodiments, the cancer is a primary cancer. In some embodiments, the cancer is a metastatic cancer, optionally a metastatic cancer that expresses NRP2a and/or NRP2b.

In some embodiments, the cancer is chemoresistant to a cancer therapy, for example, a cancer immunotherapy agent, a chemotherapeutic agent, a hormonal therapeutic agent, and/or a kinase inhibitor. In some embodiments, the treatment method comprises selecting a subject having a cancer that is chemoresistant to at least one cancer therapy prior to administering the HRS polypeptide. Exemplary cancer immunotherapy agents, chemotherapeutic agents, hormonal therapeutic agents, and kinase inhibitors are described herein.

In some embodiments, the HRS polypeptide modulates autophagy, phagocyte maturation, or efferocytosis in a cancer cell or a cancer associated macrophage. In specific embodiments, the HRS polypeptide modulates autophagy in a cancer cell.

In some embodiments, the cancer is selected from one or more of melanoma (e.g., metastatic melanoma), pancreatic cancer, bone cancer, prostate cancer, small cell lung cancer, non-small cell lung cancer (NSCLC), mesothelioma, leukemia (e.g., lymphocytic leukemia, chronic myelogenous leukemia, acute myeloid leukemia, relapsed acute myeloid leukemia), lymphoma, hepatoma (hepatocellular carcinoma), sarcoma, B-cell malignancy, breast cancer, ovarian cancer, colorectal cancer, glioma, glioblastoma multiforme, meningioma, pituitary adenoma, vestibular schwannoma, primary CNS lymphoma, primitive neuroectodermal tumor (medulloblastoma), kidney cancer (e.g., renal cell carcinoma), bladder cancer, uterine cancer, esophageal cancer, brain cancer, head and neck cancers, cervical cancer, testicular cancer, thyroid cancer, and stomach cancer.

In some embodiments, the metastatic cancer is selected from one or more of:
(a) a bladder cancer which has metastasized to the bone, liver, and/or lungs;
(b) a breast cancer which has metastasized to the bone, brain, liver, and/or lungs;
(c) a colorectal cancer which has metastasized to the liver, lungs, and/or peritoneum;
(d) a kidney cancer which has metastasized to the adrenal glands, bone, brain, liver, and/or lungs;
(e) a lung cancer which has metastasized to the adrenal glands, bone, brain, liver, and/or other lung sites;
(f) a melanoma which has metastasized to the bone, brain, liver, lung, and/or skin/muscle;
(g) a ovarian cancer which has metastasized to the liver, lung, and/or peritoneum;
(h) a pancreatic cancer which has metastasized to the liver, lung, and/or peritoneum;
(i) a prostate cancer which has metastasized to the adrenal glands, bone, liver, and/or lungs;
(j) a stomach cancer which has metastasized to the liver, lung, and/or peritoneum;
(l) a thyroid cancer which has metastasized to the bone, liver, and/or lungs; and
(m) a uterine cancer which has metastasized to the bone, liver, lung, peritoneum, and/or vagina.

Certain cancer therapies include combination therapies, as described herein. For instance, in certain embodiments the treatment is to include administering to the subject at least one additional agent selected from one or more of a cancer immunotherapy agent, a chemotherapeutic agent, a hormonal therapeutic agent, and a kinase inhibitor. In some embodiments, the at least one HRS polypeptide and the at least one agent are to be administered separately, as separate compositions. In some embodiments, the at least one HRS polypeptide and the at least one agent are to be administered together as part of the same therapeutic composition.

In some embodiments, the cancer immunotherapy agent is selected from one or more of an immune checkpoint modulatory agent, a cancer vaccine, an oncolytic virus, a cytokine, and a cell-based immunotherapies. In some embodiments, the immune checkpoint modulatory agent is a polypeptide, optionally an antibody or antigen-binding fragment thereof or a ligand, or a small molecule. In some embodiments, the immune checkpoint modulatory agent comprises
(a) an antagonist of a inhibitory immune checkpoint molecule; or
(b) an agonist of a stimulatory immune checkpoint molecule. for instance, wherein the immune checkpoint modulatory agent specifically binds to the immune checkpoint molecule.

In some embodiments, the inhibitory immune checkpoint molecule is selected from one or more of Programmed Death-Ligand 1 (PD-L1), Programmed Death 1 (PD-1), Programmed Death-Ligand 2 (PD-L2), Cytotoxic T-Lymphocyte-Associated protein 4 (CTLA-4), Indoleamine 2,3-dioxygenase (IDO), tryptophan 2,3-dioxygenase (TDO), T-cell Immunoglobulin domain and Mucin domain 3 (TIM-3), Lymphocyte Activation Gene-3 (LAG-3), V-domain Ig suppressor of T cell activation (VISTA), B and T Lymphocyte Attenuator (BTLA), CD160, Herpes Virus Entry Mediator (HVEM), and T-cell immunoreceptor with Ig and ITIM domains (TIGIT).

In some embodiments, the antagonist is a PD-L1 and/or PD-L2 antagonist optionally selected from one or more of an antibody or antigen-binding fragment or small molecule that specifically binds thereto, atezolizumab (MPDL3280A), avelumab (MSB0010718C), and durvalumab (MEDI4736), optionally wherein the cancer is selected from one or more of colorectal cancer, melanoma, breast cancer, non-small-cell lung carcinoma, bladder cancer, and renal cell carcinoma;
the antagonist is a PD-1 antagonist optionally selected from one or more of an antibody or antigen-binding fragment or small molecule that specifically binds thereto, nivolumab, pembrolizumab, MK-3475, AMP-224, AMP-514PDR001, and pidilizumab, optionally wherein the PD-1 antagonist is nivolumab and the cancer is optionally selected from one or more of Hodgkin's lymphoma, melanoma, non-small cell lung cancer, hepatocellular carcinoma, renal cell carcinoma, and ovarian cancer;
the PD-1 antagonist is pembrolizumab and the cancer is optionally selected from one or more of melanoma, non-small cell lung cancer, small cell lung cancer, head and neck cancer, and urothelial cancer;
the antagonist is a CTLA-4 antagonist optionally selected from one or more of an antibody or antigen-binding fragment or small molecule that specifically binds thereto, ipilimumab, tremelimumab, optionally wherein the cancer is selected from one or more of melanoma, prostate cancer, lung cancer, and bladder cancer;
the antagonist is an IDO antagonist optionally selected from one or more of an antibody or antigen-binding fragment or small molecule that specifically binds thereto, indoximod (NLG-8189), 1-methyl-tryptophan (1MT), β-Carboline (norharmane; 9H-pyrido[3,4-b]indole), rosmarinic acid, and epacadostat, and wherein the cancer is optionally selected from one or more of metastatic breast cancer and brain cancer optionally glioblastoma multiforme, glioma, gliosarcoma or malignant brain tumor;
the antagonist is a TDO antagonist optionally selected from one or more of an antibody or antigen-binding fragment or small molecule that specifically binds thereto, 680C91, and LM10;
the antagonist is a TIM-3 antagonist optionally selected from one or more of an antibody or antigen-binding fragment or small molecule that specifically binds thereto;
the antagonist is a LAG-3 antagonist optionally selected from one or more of an antibody or antigen-binding fragment or small molecule that specifically binds thereto, and BMS-986016;
the antagonist is a VISTA antagonist optionally selected from one or more of an antibody or antigen-binding fragment or small molecule that specifically binds thereto;
the antagonist is a BTLA, CD160, and/or HVEM antagonist optionally selected from one or more of an antibody or antigen-binding fragment or small molecule that specifically binds thereto;
the antagonist is a TIGIT antagonist optionally selected from one or more of an antibody or antigen-binding fragment or small molecule that specifically binds thereto.

In some embodiments, the stimulatory immune checkpoint molecule is selected from one or more of OX40, CD40, Glucocorticoid-Induced TNFR Family Related Gene (GITR), CD137 (4-1BB), CD27, CD28, CD226, and Herpes Virus Entry Mediator (HVEM).

In some embodiments, the agonist is an OX40 agonist optionally selected from one or more of an antibody or antigen-binding fragment or small molecule or ligand that specifically binds thereto, OX86, Fc-OX40L, and GSK3174998;
the agonist is a CD40 agonist optionally selected from one or more of an antibody or antigen-binding fragment or small molecule or ligand that specifically binds thereto, CP-870,893, dacetuzumab, Chi Lob 7/4, ADC-1013, and rhCD40L, and wherein the cancer is optionally selected from one or more of melanoma, pancreatic carcinoma, mesothelioma, and hematological cancers optionally lymphoma such as Non-Hodgkin's lymphoma;
the agonist is a GITR agonist optionally selected from one or more of an antibody or antigen-binding fragment or small molecule or ligand that specifically binds thereto, INCAGN01876, DTA-1, and MEDI1873;
the agonist is a CD137 agonist optionally selected from one or more of an antibody or antigen-binding fragment or small molecule or ligand that specifically binds thereto, utomilumab, and 4-1BB ligand;
the agonist is a CD27 agonist optionally selected from one or more of an antibody or antigen-binding fragment or small molecule or ligand that specifically binds thereto, varlilumab, and CDX-1127 (1F5);
the agonist is a CD28 agonist optionally selected from one or more of an antibody or antigen-binding fragment or small molecule or ligand that specifically binds thereto, and TAB08; and/or
the agonist is an HVEM agonist optionally selected from one or more of an antibody or antigen-binding fragment or small molecule or ligand that specifically binds thereto.

In some embodiments, the cancer vaccine is selected from one or more of Oncophage, a human papillomavirus HPV vaccine optionally Gardasil or Cervarix, a hepatitis B vaccine optionally Engerix-B, Recombivax HB, or Twinrix, and sipuleucel-T (Provenge), or comprises a cancer antigen selected from one or more of human Her2/neu, Her1/EGF receptor (EGFR), Her3, A33 antigen, B7H3, CD5, CD19, CD20, CD22, CD23 (IgE Receptor), MAGE-3, C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), guanylyl cyclase C, NY-ESO-1, p53, survivin, integrin αvβ3, integrin α5β1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), and mesothelin, optionally wherein the subject has or is at risk for having a cancer that comprises the corresponding cancer antigen.

In some embodiments, the oncolytic virus selected from one or more of talimogene laherparepvec (T-VEC), coxsackievirus A21 (CAVATAK^{™}), Oncorine (H101), pelareorep (REOLYSIN^{®}), Seneca Valley virus (NTX-010), Senecavirus SVV-001, ColoAd1, SEPREHVIR (HSV-1716), CGTG-102 (Ad5/3-D24-GMCSF), GL-ONC1, MV-NIS, and DNX-2401.

In some embodiments, the cytokine selected from one or more of interferon (IFN)-α, IL-2, IL-12, IL-7, IL-21, and Granulocyte-macrophage colony-stimulating factor (GM-CSF).

In some embodiments, the cell-based immunotherapy agent comprises cancer antigen-specific T-cells, optionally ex vivo-derived T-cells. In some embodiments, the cancer antigen-specific T-cells are selected from one or more of chimeric antigen receptor (CAR)-modified T-cells, and T-cell Receptor (TCR)-modified T-cells, tumor infiltrating lymphocytes (TILs), and peptide-induced T-cells.

In some embodiments, the at least one chemotherapeutic agent is selected from one or more of an alkylating agent, an anti-metabolite, a cytotoxic antibiotic, a topoisomerase inhibitor (type 1 or type II), and an anti-microtubule agent.

In some embodiments, the alkylating agent is selected from one or more of nitrogen mustards (optionally mechlorethamine, cyclophosphamide, mustine, melphalan, chlorambucil, ifosfamide , and busulfan), nitrosoureas (optionally N-Nitroso-N-methylurea (MNU), carmustine (BCNU), lomustine (CCNU), semustine (MeCCNU), fotemustine, and streptozotocin), tetrazines (optionally dacarbazine, mitozolomide, and temozolomide), aziridines (optionally thiotepa, mytomycin, and diaziquone (AZQ)), cisplatins and derivatives thereof (optionally carboplatin and oxaliplatin), and non-classical alkylating agents (optionally procarbazine and hexamethylmelamine);
the anti-metabolite is selected from one or more of anti-folates (optionally methotrexate and pemetrexed), fluoropyrimidines (optionally 5-fluorouracil and capecitabine), deoxynucleoside analogues (optionally ancitabine, enocitabine, cytarabine, gemcitabine, decitabine, azacitidine, fludarabine, nelarabine, cladribine, clofarabine, fludarabine, and pentostatin), and thiopurines (optionally thioguanine and mercaptopurine);
the cytotoxic antibiotic is selected from one or more of anthracyclines (optionally doxorubicin, daunorubicin, epirubicin, idarubicin, pirarubicin, aclarubicin, and mitoxantrone), bleomycins, mitomycin C, mitoxantrone, and actinomycin;
the topoisomerase inhibitor is selected from one or more of camptothecin, irinotecan, topotecan, etoposide, doxorubicin, mitoxantrone, teniposide, novobiocin, merbarone, and aclarubicin; and/or
the anti-microtubule agent is selected from one or more of taxanes (optionally paclitaxel and docetaxel) and vinca alkaloids (optionally vinblastine, vincristine, vindesine, vinorelbine).

In some embodiments, the at least one hormonal therapeutic agent is a hormonal agonist or a hormonal antagonist. In some embodiments, the hormonal agonist is selected from one or more of a progestogen (progestin), a corticosteroid (optionally prednisolone, methylprednisolone, or dexamethasone), insulin like growth factors, VEGF derived angiogenic and lymphangiogenic factors (optionally VEGF-A, VEGF-A145, VEGF-A165, VEGF-C, VEGF-D, PIGF-2), fibroblast growth factor (FGF), galectin, hepatocyte growth factor (HGF), platelet derived growth factor (PDGF), transforming growth factor (TGF)-beta, an androgen, an estrogen, and a somatostatin analog. In some embodiments, the hormonal antagonist is selected from one or more of a hormone synthesis inhibitor, optionally an aromatase inhibitor or a gonadotropin-releasing hormone (GnRH) or an analog thereof, and a hormone receptor antagonist, optionally a selective estrogen receptor modulator (SERM) or an anti-androgen, or an antibody directed against a hormonal receptor, optionally cixutumumab, dalotuzumab, figitumumab, ganitumab, istiratumab, robatumumab, alacizumab pegol, bevacizumab, icrucumab, ramucirumab, fresolimumab, metelimumab, naxitamab, cetuximab, depatuxizumab mafodotin, futuximab, imgatuzumab, laprituximab emtansine, matuzumab, modotuximab, necitumumab, nimotuzumab, panitumumab, tomuzotuximab, zalutumumab, aprutumab ixadotin, bemarituzumab, olaratumab, or tovetumab.

In some embodiments, the kinase inhibitor is selected from one or more of adavosertib, afanitib, aflibercept, axitinib, bevacizumab, bosutinib, cabozantinib, cetuximab, cobimetinib, crizotinib, dasatinib, entrectinib, erdafitinib, erlotinib, fostamitinib, gefitinib, ibrutinib, imatinib, lapatinib, lenvatinib, mubritinib, nilotinib, panitumumab, pazopanib, pegaptanib, ponatinib, ranibizumab, regorafenib, ruxolitinib, sorafenib, sunitinib, SU6656, tofacitinib, trastuzumab, vandetanib, and vemuafenib. In some embodiments, the kinase inhibitor is a PI3 kinase inhibitor selected from one or more of alpelisib, buparlisib, copanlisib, CUDC-907, dactolisib, duvelisib, GNE-477, idelasib, IPI-549, LY294002, ME-401, perifosine, PI-103, pictilisib, PWT33597, RP6503, taselisib, umbralisib, voxtalisib, wortmannin, and XL147

For the treatment of cancer, in some instances, an HRS polypeptide enhances the immune response to the cancer by about, or at least about, 5, 10, 15, 20, 25, 30, 35, 40, 45, 50, 60, 70, 80, 90, 100, 200, 300, 400, 500, 600, 700, 800, 900, 1000, 2000% or more, relative to an untreated control. Exemplary immune responses include increasing or enhancing immune cell invasion of a solid tumor, and increasing the biological activity against the cancer. In certain embodiments, an HRS polypeptide enhances an adaptive immune response to the cancer, and in some embodiments, an HRS polypeptide enhances an innate immune response to the cancer. In some-instances, an HRS polypeptide directly or indirectly enhances a T-cell-mediated response to the cancer. In some-instances, an HRS polypeptide enhances a B-cell-mediated or antibody-mediated response to the cancer. In some-instances, an HRS polypeptide modulates a macrophage responses to the cancer. In some-instances, an HRS polypeptide modulates immune cell, or cancer autophagy. In some-instances, an HRS polypeptide modulates immune cell phagocytosis. In some-instances, an HRS polypeptide modulates cancer cell apoptosis. In some-instances, an HRS polypeptide modulates immune cell efferocytosis and/or cancer cell autophagy.

In some embodiments, an HRS polypeptide enhances macrophage responses to the cancer. In some embodiments, an HRS polypeptide inhibits macrophage responses to the cancer. In some embodiments, an HRS polypeptide enhances autophagy. In some embodiments, an HRS polypeptide inhibits autophagy. In some embodiments, an HRS polypeptide enhances phagocytosis. In some embodiments, an HRS polypeptide inhibits phagocytosis. In some embodiments, an HRS polypeptide enhances apoptosis. In some embodiments an HRS polypeptide inhibits apoptosis. In some embodiments, an HRS polypeptide enhances efferocytosis. In some embodiments, an HRS polypeptide inhibits efferocytosis.

In some-instances, an HRS polypeptide reduces cancer initiation, cancer cell migration, adhesion, or cancer cell metastasis by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control. In some-instances, HRS polypeptide reduces cancer mediated lymphoangiogenesis by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control.

In some embodiments, an HRS polypeptide reduces the rate of *in vitro* growth of the cancer (for example, cancer cells isolated from a biopsy or other sample grown *in vitro)* by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control.

In some embodiments, an HRS polypeptide reduces the adhesiveness of the cancer (for example, cancer cells isolated from a biopsy or other sample grown *in vitro)* to a substrate by about or at least about, 5, 10, 15, 20, 25, 30, 35, 40, 45, 50, 60, 70, 80, 90, 100, 200, 300, 400, 500, 600, 700, 800, 900, 1000, 2000% or more relative to an untreated control. In some instances, the substrate comprises laminin.

In some embodiments, an HRS polypeptide reduces the invasiveness of the cancer (for example, cancer cells isolated from a biopsy or other sample grown *in vitro)* by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control.

In some embodiments, an HRS polypeptide inhibits the rate of migration or motility of the cancer or a migratory cell (for example, cancer or immune cells isolated from a biopsy or other sample grown *in vitro*) by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control.

In some embodiments, an HRS polypeptide inhibits the rate of autophagy or endosome maturation (for example, endosome acidification) of the cancer or associated immune cells by about or at least about 5, 10, 15, 20, 25, 30, 35, 40, 45, 50, 60, 70, 80, 90, 100, 200, 300, 400, 500, 600, 700, 800, 900, 1000, 2000% or more relative to an untreated control.

In some embodiments, an HRS polypeptide enhances the susceptibility of the cancer to an additional agent (for example, chemotherapeutic agent, hormonal therapeutic agent, and or kinase inhibitor) by about or at least about 5, 10, 15, 20, 25, 30, 35, 40, 45, 50, 60, 70, 80, 90, 100, 200, 300, 400, 500, 600, 700, 800, 900, 1000, 2000% or more relative to the additional agent alone. In some embodiments, an HRS polypeptide enhances an anti-tumor and/or immunostimulatory activity of a cancer immunotherapy agent by about or at least about 5, 10, 15, 20, 25, 30, 35, 40, 45, 50, 60, 70, 80, 90, 100, 200, 300, 400, 500, 600, 700, 800, 900, 1000, 2000% or more, relative to the cancer immunotherapy agent alone.

In some embodiments, the therapeutic compositions described herein increase median survival time of a subject by 4 weeks, 5 weeks, 6 weeks, 7 weeks, 8 weeks, 9 weeks, 10 weeks, 15 weeks, 20 weeks, 25 weeks, 30 weeks, 40 weeks, or longer. In certain embodiments, the methods and therapeutic compositions described herein increase median survival time of a subject by 1 year, 2 years, 3 years, or longer. In some embodiments, the methods and therapeutic compositions described herein increase progression-free survival by 2 weeks, 3 weeks, 4 weeks, 5 weeks, 6 weeks, 7 weeks, 8 weeks, 9 weeks, 10 weeks or longer. In certain embodiments, the methods or therapeutic compositions described herein increase progression-free survival by 1 year, 2 years, 3 years, or longer.

In certain embodiments, the therapeutic compositions described herein are sufficient to result in tumor regression, as indicated by a statistically significant decrease in the amount of viable tumor, for example, at least a 10%, 20%, 30%, 40%, 50% or greater decrease in tumor mass, or by altered (e.g., decreased with statistical significance) scan dimensions. In certain embodiments, the methods and therapeutic compositions described herein are sufficient to result in stable disease. In certain embodiments, the methods and therapeutic compositions described herein are sufficient to result in clinically relevant reduction in symptoms of a particular disease indication known to the skilled clinician.

In some embodiments, an HRS polypeptide increases, complements, or otherwise enhances the anti-tumor and/or immunostimulatory activity of the cancer immunotherapy agent, relative to the cancer immunotherapy agent alone. In some embodiments, an HRS polypeptide enhances the anti-tumor and/or immunostimulatory activity of the cancer immunotherapy agent by about, or at least about, 5, 10, 15, 20, 25, 30, 35, 40, 45, 50, 60, 70, 80, 90, 100, 200, 300, 400, 500, 600, 700, 800, 900, 1000, 2000% or more, relative to the cancer immunotherapy agent alone.

### Pharmaceutical Compositions and Kits

Certain embodiments include pharmaceutical compositions, therapeutic compositions, and formulations suitable for the therapeutic delivery of the HRS polypeptides/expressible polynucleotides, and optionally one or more second agents, as described herein. Some embodiments therefore include pharmaceutically-acceptable compositions that comprise a therapeutically-effective amount of one or more of the HRS polypeptides/expressible polynucleotides, and optionally one or more second agents, as described herein, formulated together with one or more pharmaceutically-acceptable carriers and/or diluents.

In some embodiments, as noted above, the second agent as selected from antimicrobial agents, antifungal agents, and antihelminthic agents, including combinations thereof. In some embodiments, the antimicrobial agent, antifungal agent, and/or antihelminthic agent is selected from one or more of aminoglycosides such as Amikacin, Gentamicin, Kanamycin, Neomycin, Netilmicin, Tobramycin, Paromomycin, Streptomycin, and Spectinomycin; carbapenems such as Ertapenem, Doripenem, Imipenem/Cilastatin, and Meropenem; cephalasporins such as Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefixime, Cefdinir, Cefditoren, Cefoperazone, Cefotaxime, Cefpodoxime, Ceftazidime, Ceftibuten, Ceftizoxime, Moxalactam, Cefepime, Ceftaroline fosamil, and Ceftobiprole; glycopeptides such as Teicoplanin, Vancomycin, Telavancin, Dalbavancin, Oritavancin; lincosamines such as Clindamycin and Lincomycin; macrolides such as Azithromycin, Clarithromycin, Erythromycin, Roxithromycin, Telithromycin, and Spiramycin; penicillins such as Amoxicillin, Ampicillin, Azlocillin, Dicloxacillin, Flucloxacillin, Mezlocillin, Methicillin, Nafcillin, Oxacillin, Penicillin G, Penicillin V, Piperacillin, Penicillin G, Temocillin, and Ticarcillin; polypeptides such as Bacitracin, Colistin, and Polymyxin B; quinolones/fluoroquinolones such as Ciprofloxacin, Enoxacin, Gatifloxacin, Gemifloxacin, Levofloxacin, Lomefloxacin, Moxifloxacin, Nadifloxacin, Nalidixic acid, Norfloxacin, Ofloxacin, Trovafloxacin, Grepafloxacin, Sparfloxacin, and Temafloxacin; sulfonamides such as Mafenide, Sulfacetamide, Sulfadiazine, Silver sulfadiazine, Sulfadimethoxine, Sulfamethizole, Sulfamethoxazole, Sulfanilimide, Sulfasalazine, Sulfisoxazole, Trimethoprim-Sulfamethoxazole (Co-trimoxazole) (TMP-SMX), and Sulfonamidochrysoidine; tetracyclines such as Demeclocycline, Doxycycline, Metacycline, Minocycline, Oxytetracycline, and Tetracycline; anti-mycobacterials such as Clofazimine, Dapsone, Capreomycin, Cycloserine, Ethambutol, Ethionamide, Isoniazid, Pyrazinamide, Rifampicin (Rifampin), Rifabutin, Rifapentine, and Streptomycin; Chloramphenicol; Metronidazole; Mupirocin; Tigecycline; Tinidazole; and antihelminthic agents such as Diethylcarbamazine and Albendazole.

In some embodiments, the second agent is selected from a cancer immunotherapy agent, a chemotherapeutic agent, a hormonal therapeutic agent, and a kinase inhibitor, as described herein.

The compositions may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; (2) parenteral administration, for example, by subcutaneous, intramuscular, intravenous or epidural injection as, for example, a sterile solution or suspension, or sustained-release formulation; (3) topical application, for example, as a cream, ointment, or a controlled-release patch or spray applied to the skin; (4) intravaginally or intrarectally, for example, as a pessary, cream or foam; (5) sublingually; (6) ocularly; (7) transdermally; (8) nasally; or (9) via bladder instillation.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject.

Some examples of materials that can serve as pharmaceutically-acceptable carriers include, without limitation: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; and (22) other non-toxic compatible substances employed in pharmaceutical formulations.

Additional non-limiting examples of agents suitable for formulation with the HRS polypeptides/expressible polynucleotides and other agents include: PEG conjugated nucleic acids, phospholipid conjugated nucleic acids, nucleic acids containing lipophilic moieties, phosphorothioates, P-glycoprotein inhibitors (such as Pluronic P85) which can enhance entry of drugs into various tissues; biodegradable polymers, such as poly (DL-lactide-coglycolide) microspheres for sustained release delivery after implantation (Emerich, D F et al., 1999, Cell Transplant, 8, 47-58) Alkermes, Inc. Cambridge, Mass.; and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999).

Also included are compositions comprising surface-modified liposomes containing poly (ethylene glycol) lipids (PEG-modified, branched and unbranched or combinations thereof, or long-circulating liposomes or stealth liposomes). HRS polypeptides/expressible polynucleotides and/or other agents can also comprise covalently attached PEG molecules of various molecular weights. These formulations offer a method for increasing the accumulation of drugs in target tissues. Long-circulating liposomes are also likely to protect drugs from nuclease degradation to a greater extent compared to cationic liposomes, based on their ability to avoid accumulation in metabolically aggressive MPS tissues such as the liver and spleen.

Also included are compositions prepared for delivery as described in U.S. Pat. Nos. 6,692,911, 7,163,695 and 7,070,807. In this regard, certain embodiments include compositions comprising copolymers of lysine and histidine (HK) as described in U.S. Pat. Nos. 7,163,695, 7,070,807, and 6,692,911 either alone or in combination with PEG (e.g., branched or unbranched PEG or a mixture of both), in combination with PEG and a targeting moiety or any of the foregoing in combination with a crosslinking agent. Some embodiments provide HRS polypeptides/expressible polynucleotides and/or other agents in compositions comprising gluconic-acid-modified polyhistidine or gluconylated-polyhistidine/transferrin-polylysine. One skilled in the art will also recognize that amino acids with properties similar to His and Lys may be substituted within the composition.

Certain agents described herein may contain a basic functional group, such as amino or alkylamino, which is capable of forming pharmaceutically-acceptable salts with pharmaceutically-acceptable acids. The term "pharmaceutically-acceptable salts" in this respect, refers to the relatively non-toxic, inorganic and organic acid addition salts of an agent. These salts can be prepared *in situ* in the administration vehicle or the dosage form manufacturing process, or by separately reacting a purified agent in its free base form with a suitable organic or inorganic acid, and isolating the salt thus formed during subsequent purification. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like.

The pharmaceutically acceptable salts of the agents described herein include the conventional nontoxic salts or quaternary ammonium salts of the compounds, e.g., from non-toxic organic or inorganic acids. For example, such conventional nontoxic salts include those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In certain embodiments, the agents described herein contain one or more acidic functional groups and, thus, are capable of forming pharmaceutically acceptable salts with pharmaceutically acceptable bases. The term "pharmaceutically-acceptable salts" in these instances refers to the relatively non-toxic, inorganic and organic base addition salts of an agent. These salts can likewise be prepared in situ in the administration vehicle or the dosage form manufacturing process, or by separately reacting the purified compound in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of include those suitable for intravenous, intramuscular, oral, nasal, pulmonary, topical (including buccal and sublingual), rectal, vaginal, and/or parenteral administration. The formulations may conveniently be presented in unit dosage form and may be prepared by any methods well known in the art of pharmacy. The amount of active ingredient that can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 0.1 percent to about ninety-nine percent of active ingredient, preferably from about 5 percent to about 70 percent, most preferably from about 10 percent to about 30 percent.

In certain embodiments, a composition or formulation comprises an excipient selected from cyclodextrins, celluloses, liposomes, micelle forming agents, e.g., bile acids, and polymeric carriers, e.g., polyesters and polyanhydrides; and an HRS polypeptide/expressible polynucleotide and any other agents.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of an HRS polypeptide/expressible polynucleotide and/or other agent as an active ingredient. The compositions or agents may also be administered as a bolus, electuary, or paste.

In solid dosage forms for oral administration (capsules, tablets, pills, dragees, powders, granules, trouches and the like), the active ingredient may be mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds and surfactants, such as poloxamer and sodium lauryl sulfate; (7) wetting agents, such as, for example, cetyl alcohol, glycerol monostearate, and non-ionic surfactants; (8) absorbents, such as kaolin and bentonite clay; (9) lubricants, such as talc, calcium stearate, magnesium stearate, solid polyethylene glycols, sodium lauryl sulfate, zinc stearate, sodium stearate, stearic acid, and mixtures thereof; (10) coloring agents; and (11) controlled release agents such as crospovidone or ethyl cellulose. In the case of capsules, tablets and pills, the compositions may also comprise buffering agents. Solid compositions of a similar type may also be employed as fillers in soft and hard-shelled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (e.g., gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be formulated for rapid release, e.g., freeze-dried. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the HRS polypeptides/expressible polynucleotides and other agents include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations or dosage forms for the topical or transdermal administration of the HRS polypeptides/expressible polynucleotides and other agents include powders, sprays, ointments, pastes, creams, lotions, gels, solutions, patches and inhalants. The active HRS polypeptides/expressible polynucleotides and/or other agents may be mixed under sterile conditions with a pharmaceutically-acceptable carrier, and with any preservatives, buffers, or propellants which may be required. The ointments, pastes, creams and gels may contain, in addition to the HRS polypeptides/expressible polynucleotides and/or other agents, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of the HRS polypeptides/expressible polynucleotides and/or other agents to the body. Such dosage forms can be made by dissolving or dispersing the agent in the proper medium. Absorption enhancers can also be used to increase the flux of the agent across the skin. The rate of such flux can be controlled by either providing a rate controlling membrane or dispersing the agent in a polymer matrix or gel, among other methods known in the art.

Pharmaceutical compositions suitable for parenteral administration may comprise one or more HRS polypeptides/expressible polynucleotides and/or other agents in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents. Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms upon the subject may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility, among other methods known in the art. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms may be made by forming microencapsule matrices of the subject HRS polypeptides/expressible polynucleotides and/or other agents in biodegradable polymers such as polylactide-polyglycolide. Depending on the ratio of agent to polymer, and the nature of the particular polymer employed, the rate of release can be controlled. Examples of other biodegradable polymers include poly(orthoesters) and poly(anhydrides). Depot injectable formulations may also prepared by entrapping the drug in liposomes or microemulsions that are compatible with body tissues.

When the HRS polypeptides/expressible polynucleotides and/or other agents are administered as pharmaceuticals, to humans and animals, they can be given per se or as a pharmaceutical composition containing, for example, 0.1 to 99% (more preferably, 10 to 30%) of active ingredient in combination with a pharmaceutically acceptable carrier.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration," "administered systemically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

Regardless of the route of administration selected, the HRS polypeptides/expressible polynucleotides and/or other agents may be formulated into pharmaceutically-acceptable dosage forms by conventional methods known to those of skill in the art. Actual dosage levels of the active ingredients in the pharmaceutical compositions may be varied so as to obtain an amount of the active ingredient which is effective to achieve the desired therapeutic response for a particular patient, composition, and mode of administration, without being unacceptably toxic to the patient.

The selected dosage level will depend upon a variety of factors including the activity of the particular HRS polypeptides/expressible polynucleotides and/or other agents employed, or the ester, salt or amide thereof, the route of administration, the time of administration, the rate of excretion or metabolism of the particular agent being employed, the rate and extent of absorption, the duration of the treatment, other drugs, compounds and/or materials used in combination with the particular agent employed, the age, sex, weight, condition, general health and prior medical history of the patient being treated, and like factors well known in the medical arts.

A physician having ordinary skill in the art can readily determine and prescribe the effective amount of the pharmaceutical composition required. For example, the physician could start doses of the HRS polypeptides/expressible polynucleotides and/or other agents employed in the pharmaceutical composition at levels lower than that required in order to achieve the desired therapeutic effect and gradually increase the dosage until the desired effect is achieved. In general, a suitable daily dose of HRS polypeptides/expressible polynucleotides and/or other agents will be that amount of the compound which is the lowest dose effective to produce a therapeutic effect. Such an effective dose will generally depend upon the factors described herein. Generally, oral, intravenous, intramuscular, intracerebroventricular and subcutaneous doses of the HRS polypeptides/expressible polynucleotides and/or other agents for a subject or patient, when used for the indicated effects, will range from about 0.0001 to about 100 mg per dosage, or about about 0.0001 to about 100 mg per kilogram of body weight per dosage.

If desired, the effective daily dose of the active agent(s) may be administered as one, two, three, four, five, six or more sub-doses administered separately at appropriate intervals throughout the day or week, for example, in unit dosage forms. In certain situations, dosing is one administration per day. In certain situations, dosing is one, two, or three administration per week. In certain embodiments, dosing is one or more administration per every 2, 3, 4, 5, 6, 7, 8, 9, 10, 11, 12, 13, 14 days, or every 1, 2, 3, 4, 5, 6, 7, 8, 9, 10, 11, 12 weeks, or every 1, 2, 3, 4, 5, 6, 7, 8, 9, 10, 11, 12 months, as needed, to treat the desired condition.

HRS polypeptides/expressible polynucleotides and/or other agents can be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres, as described herein and known in the art. In certain embodiments, microemulsification technology may be utilized to improve bioavailability of lipophilic (water insoluble) pharmaceutical agents. Examples include Trimetrine (Dordunoo, S. K., et al., Drug Development and Industrial Pharmacy, 17(12), 1685-1713, 1991 and REV 5901 (Sheen, P. C., et al., J Pharm Sci 80(7), 712-714, 1991). Among other benefits, microemulsification provides enhanced bioavailability by preferentially directing absorption to the lymphatic system instead of the circulatory system, which thereby bypasses the liver, and prevents destruction of the compounds in the hepatobiliary circulation.

In some embodiments, the compositions or formulations contain micelles which are formed from the HRS polypeptides/expressible polynucleotides and/or other agents and at least one amphiphilic carrier, in which the micelles have an average diameter of less than about 100 nm. Exemplary embodiments provide micelles having an average diameter less than about 50 nm, and even certain embodiments provide micelles having an average diameter less than about 30 nm, or even less than about 20 nm. While all suitable amphiphilic carriers are contemplated, the presently preferred carriers are generally those that have Generally-Recognized-as-Safe (GRAS) status, and that can both solubilize the active ingredient and microemulsify it at a later stage when the solution comes into a contact with a complex water phase (such as one found in human gastro-intestinal tract). Usually, amphiphilic ingredients that satisfy these requirements have HLB (hydrophilic to lipophilic balance) values of 2-20, and their structures contain straight chain aliphatic radicals in the range of C-6 to C-20. Examples are polyethylene-glycolized fatty glycerides and polyethylene glycols.

Examples of amphiphilic carriers include saturated and monounsaturated polyethyleneglycolyzed fatty acid glycerides, such as those obtained from fully or partially hydrogenated various vegetable oils. Such oils may advantageously consist of tri-, di-, and mono-fatty acid glycerides and di- and mono-polyethyleneglycol esters of the corresponding fatty acids, with a particularly preferred fatty acid composition including capric acid 4-10, capric acid 3-9, lauric acid 40-50, myristic acid 14-24, palmitic acid 4-14 and stearic acid 5-15%. Another useful class of amphiphilic carriers includes partially esterified sorbitan and/or sorbitol, with saturated or monounsaturated fatty acids (SPAN-series) or corresponding ethoxylated analogs (TWEEN-series).

Commercially available amphiphilic carriers may be particularly useful, including Gelucire-series, Labrafil, Labrasol, or Lauroglycol (all manufactured and distributed by Gattefosse Corporation, Saint Priest, France), PEG-mono-oleate, PEG-di-oleate, PEG-mono-laurate and dilaurate, Lecithin, Polysorbate 80, etc. (produced and distributed by a number of companies in USA and worldwide).

In certain embodiments, the delivery may occur by use of liposomes, nanocapsules, microparticles, microspheres, lipid particles, vesicles, and the like, for the introduction of the HRS polypeptides/expressible polynucleotides and/or other agents into suitable host cells. In particular, the compositions may be formulated for delivery either encapsulated in a lipid particle, a liposome, a vesicle, a nanosphere, a nanoparticle or the like. The formulation and use of such delivery vehicles can be carried out using known and conventional techniques.

Hydrophilic polymers suitable for use are those which are readily water-soluble, can be covalently attached to a vesicle-forming lipid, and which are tolerated in vivo without toxic effects (i.e., are biocompatible). Suitable polymers include polyethylene glycol (PEG), polylactic (also termed polylactide), polyglycolic acid (also termed polyglycolide), a polylactic-polyglycolic acid copolymer, and polyvinyl alcohol. In certain embodiments, polymers have a molecular weight of from about 100 or 120 daltons up to about 5,000 or 10,000 daltons, or from about 300 daltons to about 5,000 daltons. In some embodiments, the polymer is polyethyleneglycol having a molecular weight of from about 100 to about 5,000 daltons, or having a molecular weight of from about 300 to about 5,000 daltons. In certain embodiments, the polymer is polyethyleneglycol of 750 daltons (PEG(750)). Polymers may also be defined by the number of monomers therein.

Other hydrophilic polymers which may be suitable include polyvinylpyrrolidone, polymethoxazoline, polyethyloxazoline, polyhydroxypropyl methacrylamide, polymethacrylamide, polydimethylacrylamide, and derivatized celluloses such as hydroxymethylcellulose or hydroxyethylcellulose.

In certain embodiments, a composition or formulation comprises a biocompatible polymer selected from the group consisting of polyamides, polycarbonates, polyalkylenes, polymers of acrylic and methacrylic esters, polyvinyl polymers, polyglycolides, polysiloxanes, polyurethanes and copolymers thereof, celluloses, polypropylene, polyethylenes, polystyrene, polymers of lactic acid and glycolic acid, polyanhydrides, poly(ortho)esters, poly(butic acid), poly(valeric acid), poly(lactide-co-caprolactone), polysaccharides, proteins, polyhyaluronic acids, polycyanoacrylates, and blends, mixtures, or copolymers thereof.

Cyclodextrins are cyclic oligosaccharides, consisting of 6, 7 or 8 glucose units, designated by the Greek letter α, β, and γ, respectively. The glucose units are linked by α-1,4-glucosidic bonds. As a consequence of the chair conformation of the sugar units, all secondary hydroxyl groups (at C-2, C-3) are located on one side of the ring, while all the primary hydroxyl groups at C-6 are situated on the other side. As a result, the external faces are hydrophilic, making the cyclodextrins water-soluble. In contrast, the cavities of the cyclodextrins are hydrophobic, since they are lined by the hydrogen of atoms C-3 and C-5, and by ether-like oxygens. These matrices allow complexation with a variety of relatively hydrophobic compounds, including, for instance, steroid compounds such as 17α-estradiol (see, e.g., van Uden et al. Plant Cell Tiss. Org. Cult. 38:1-3-113 (1994)). The complexation takes place by Van der Waals interactions and by hydrogen bond formation. The physico-chemical properties of the cyclodextrin derivatives depend on the kind and the degree of substitution. For example, their solubility in water ranges from insoluble (e.g., triacetyl-beta-cyclodextrin) to 147% soluble (w/v) (G-2-beta-cyclodextrin). In addition, they are soluble in many organic solvents. The properties of the cyclodextrins enable the control over solubility of various formulation components by increasing or decreasing their solubility.

Numerous cyclodextrins and methods for their preparation have been described. For example, Parmeter (I), et al. (U.S. Pat. No. 3,453,259) and Gramera, et al. (U.S. Pat. No. 3,459,731) described electroneutral cyclodextrins. Other derivatives include cyclodextrins with cationic properties [Parmeter (II), U.S. Pat. No. 3,453,257], insoluble crosslinked cyclodextrins (Solms, U.S. Pat. No. 3,420,788), and cyclodextrins with anionic properties [Parmeter (III), U.S. Pat. No. 3,426,011]. Among the cyclodextrin derivatives with anionic properties, carboxylic acids, phosphorous acids, phosphinous acids, phosphonic acids, phosphoric acids, thiophosphonic acids, thiosulphinic acids, and sulfonic acids have been appended to the parent cyclodextrin [see, Parmeter (III), supra]. Furthermore, sulfoalkyl ether cyclodextrin derivatives have been described by Stella, et al. (U.S. Pat. No. 5,134,127).

Some embodiments relate to formulations comprising liposomes containing HRS polypeptides/expressible polynucleotides and/or other agents, where the liposome membrane is formulated to provide a liposome with increased carrying capacity. Alternatively or in addition, the active ingredients may be contained within, or adsorbed onto, the liposome bilayer of the liposome. The HRS polypeptides/expressible polynucleotides and/or other agents may be aggregated with a lipid surfactant and carried within the liposome's internal space; in these cases, the liposome membrane is formulated to resist the disruptive effects of the active agent-surfactant aggregate.

Liposomes consist of at least one lipid bilayer membrane enclosing an aqueous internal compartment. Liposomes may be characterized by membrane type and by size. Small unilamellar vesicles (SUVs) have a single membrane and typically range between 0.02 and 0.05 µm in diameter; large unilamellar vesicles (LUVS) are typically larger than 0.05 µm. Oligolamellar large vesicles and multilamellar vesicles have multiple, usually concentric, membrane layers and are typically larger than 0.1 µm. Liposomes with several nonconcentric membranes, i.e., several smaller vesicles contained within a larger vesicle, are termed multivesicular vesicles.

In some embodiments, the lipid bilayer of a liposome contains lipids derivatized with polyethylene glycol (PEG), such that the PEG chains extend from the inner surface of the lipid bilayer into the interior space encapsulated by the liposome, and extend from the exterior of the lipid bilayer into the surrounding environment.

Liposomes maybe prepared by any of a variety of techniques that are known in the art. See, e.g., U.S. Pat. No. 4,235,871; Published PCT applications WO 96/14057; New RRC, Liposomes: A practical approach, IRL Press, Oxford (1990), pages 33-104; Lasic DD, Liposomes from physics to applications, Elsevier Science Publishers BV, Amsterdam, 1993. For example, liposomes may be prepared by diffusing a lipid derivatized with a hydrophilic polymer into preformed liposomes, such as by exposing preformed liposomes to micelles composed of lipid-grafted polymers, at lipid concentrations corresponding to the final mole percent of derivatized lipid which is desired in the liposome. Liposomes containing a hydrophilic polymer can also be formed by homogenization, lipid-field hydration, or extrusion techniques, as are known in the art.

In another exemplary formulation procedure, the HRS polypeptides/expressible polynucleotides and/or other agents are first dispersed by sonication in a lysophosphatidylcholine or other low CMC surfactant (including polymer grafted lipids) that readily solubilizes hydrophobic molecules. The resulting micellar suspension of active agent is then used to rehydrate a dried lipid sample that contains a suitable mole percent of polymer-grafted lipid, or cholesterol. The lipid and active agent suspension is then formed into liposomes using extrusion techniques as are known in the art, and the resulting liposomes separated from the unencapsulated solution by standard column separation.

In one aspect, the liposomes are prepared to have substantially homogeneous sizes in a selected size range. One effective sizing method involves extruding an aqueous suspension of the liposomes through a series of polycarbonate membranes having a selected uniform pore size; the pore size of the membrane will correspond roughly with the largest sizes of liposomes produced by extrusion through that membrane. See e.g., U.S. Pat. No. 4,737,323 (Apr. 12, 1988). In certain embodiments, reagents such as DharmaFECT^{™} and Lipofectamine^{™} may be utilized to introduce polynucleotides or proteins into cells.

The release characteristics of a formulation depend on the encapsulating material, the concentration of encapsulated drug, and the presence of release modifiers. For example, release can be manipulated to be pH dependent, for example, using a pH sensitive coating that releases only at a low pH, as in the stomach, or a higher pH, as in the intestine. An enteric coating can be used to prevent release from occurring until after passage through the stomach. Multiple coatings or mixtures of cyanamide encapsulated in different materials can be used to obtain an initial release in the stomach, followed by later release in the intestine. Release can also be manipulated by inclusion of salts or pore forming agents, which can increase water uptake or release of drug by diffusion from the capsule. Excipients which modify the solubility of the drug can also be used to control the release rate. Agents which enhance degradation of the matrix or release from the matrix can also be incorporated. They can be added to the drug, added as a separate phase (i.e., as particulates), or can be co-dissolved in the polymer phase depending on the compound. In most cases the amount should be between 0.1 and thirty percent (w/w polymer). Types of degradation enhancers include inorganic salts such as ammonium sulfate and ammonium chloride, organic acids such as citric acid, benzoic acid, and ascorbic acid, inorganic bases such as sodium carbonate, potassium carbonate, calcium carbonate, zinc carbonate, and zinc hydroxide, and organic bases such as protamine sulfate, spermine, choline, ethanolamine, diethanolamine, and triethanolamine and surfactants such as Tween^{™} and Pluronic^{™}. Pore forming agents which add microstructure to the matrices (i.e., water soluble compounds such as inorganic salts and sugars) are added as particulates. The range is typically between one and thirty percent (w/w polymer).

Uptake can also be manipulated by altering residence time of the particles in the gut. This can be achieved, for example, by coating the particle with, or selecting as the encapsulating material, a mucosal adhesive polymer. Examples include most polymers with free carboxyl groups, such as chitosan, celluloses, and especially polyacrylates (as used herein, polyacrylates refers to polymers including acrylate groups and modified acrylate groups such as cyanoacrylates and methacrylates).

The HRS polypeptides/expressible polynucleotides and/or other agents may be formulated to be contained within, or, adapted to release by a surgical or medical device or implant. In certain aspects, an implant may be coated or otherwise treated with an agent. For example, hydrogels, or other polymers, such as biocompatible and/or biodegradable polymers, may be used to coat an implant with the HRS polypeptides/expressible polynucleotides and/or other agents (i.e., the composition may be adapted for use with a medical device by using a hydrogel or other polymer). Polymers and copolymers for coating medical devices with an agent are well-known in the art. Examples of implants include, but are not limited to, stents, drug-eluting stents, sutures, prosthesis, vascular catheters, dialysis catheters, vascular grafts, prosthetic heart valves, cardiac pacemakers, implantable cardioverter defibrillators, IV needles, devices for bone setting and formation, such as pins, screws, plates, and other devices, and artificial tissue matrices for wound healing. In some embodiments such coatings would serve to prevent granuloma formation around the implant.

The HRS polypeptides/expressible polynucleotides and/or other agents may be administered in any convenient vehicle which is physiologically acceptable. Such a composition may include any of a variety of standard pharmaceutically acceptable carriers employed by those of ordinary skill in the art. Examples include, but are not limited to, saline, phosphate buffered saline (PBS), water, aqueous ethanol, emulsions, such as oil/water emulsions or triglyceride emulsions, tablets and capsules. The choice of suitable physiologically acceptable carrier will vary dependent upon the chosen mode of administration.

Also included are kits, for example, patient care kits, comprising one or more containers filled with one or more of the therapeutic compositions, HRS polypeptides/expressible polynucleotides and/or other agents described herein. In some embodiments, the kits include written instructions on how to use such compositions, for example, in the treatment of one or more diseases.

Certain embodiments therefore include a patient care kit, comprising: (a) a histidyl-tRNA synthetase (HRS) polypeptide, or an expressible polynucleotide that encodes the HRS polypeptide; and (b) a second agent, for example, an antimicrobial agent, an anti-fungal agent, an antihelminthic agent, a cancer immunotherapy agent, a chemotherapeutic agent, a hormonal therapeutic agent, and/or a kinase inhibitor, as described herein. In some kits, (a) and (b) are in separate compositions, and are optionally defined as described herein. In some kits, (a) and (b) are in the same composition, optionally as a therapeutic composition as described herein.

The kits and compositions described herein may also include a one or more additional therapeutic agents or other components suitable or desired for the indication being treated. An additional therapeutic agent may be contained in a second container, if desired. Examples of additional therapeutic agents include, but are not limited to anti-inflammatory agents, anticancer agents, antibacterial agents, antiviral agents, etc.

The kits herein can also include one or more syringes (e.g., injectable syringes) or other components necessary or desired to facilitate an intended mode of delivery (e.g., stents, implantable depots, etc.).

### EXAMPLES

### Materials and Methods

### ELISA assays

**Assays to measure human and mouse endogenous HRS in circulation. ELISA detection** assays were developed to quantitate the levels of mouse or human HRS in circulation using different capture and detection antibodies to enable selective measurement of full length HRS as well as the N-terminal region.

The Human N-terminal ELISA is designed to detect the N-terminal domain of human HRS (WHEP domain) utilizing capture and detection antibodies targeting this domain (approximately amino acids 1-60 of HRS).

ELISA assays were conducted using a 96 well Multi-Array plate coated with capture antibody, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibody: | ATYR12H6, mouse monoclonal antibody |
| • | Capture antibody conc: | 1 µg/mL |
| • | Protein standard range: | 100-0.046 ng/mL |
| • | Detection antibody: | 1C8-b, biotinylated mouse monoclonal antibody, |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

The Human full-length HRS ELISA is designed to detect the multi-domain human HARS utilizing capture and detection antibodies targeting separate domains of the protein (WHEP (amino acids 1-60 of HRS) and catalytic domains (approximately amino acids 60-398 of HRS).

ELISA assays were conducted using a 96 well Multi-Array plate coated with capture antibody, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibody: | ATYR12H6, mouse monoclonal antibody |
| • | Capture antibody conc: | 1 µg/mL |
| • | Protein standard range: | 100-0.046 ng/mL |
| • | Detection antibody: | ATYR13C8-b, biotinylated mouse monoclonal antibody |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

The Mouse N-terminal ELISA is designed to detect the N-terminal domain of mouse HRS (WHEP) utilizing capture and detection antibodies targeting this domain.

ELISA assays were conducted using a 96 well Multi-Array plate coated with capture antibody, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibody: | ATYR13E9, mouse monoclonal antibody |
| • | Capture antibody conc: | 1 µg/mL |
| • | Protein standard range: | 100-0.046 ng/mL |
| • | Detection antibody: | 1C8-b, biotinylated mouse monoclonal antibody, |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

The Mouse full-length HRS ELISA designed to detect the multi-domain mouse HARS utilizing capture and detection antibodies targeting separate domains of the protein (WHEP (approximately amino acids 1-60 of HRS) and catalytic domains (approximately amino acids 60-398 of HRS).

ELISA assays were conducted using a 96 well Multi-Array plate coated with capture antibody, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibody: | ATYR13E9, mouse monoclonal |
| • | Capture antibody conc: | 1 µg/mL |
| • | Protein standard range: | 100-0.046 ng/mL |
| • | Detection antibody: | ATYR13C8-b, mouse monoclonal |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

**Assays to measure human endogenous Neuropilin 2 (NRP2, or NP2) in circulation.** An ELISA detection assay was developed to quantitate the levels of human NRP2 in circulation using capture and detection antibodies to enable selective measurement of soluble NRP2. The human NRP2 ELISA was designed to detect soluble NRP2 utilizing a monoclonal capture antibody and a polyclonal detection antibody targeting NRP2. ELISA assays were conducted using a 96 well Multi-Array plate, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibody: | NRP2 mAb Cat.#MAB2215, R&D Systems |
| • | Capture antibody conc: | 2 µg/mL |
| • | Protein standard: | NRP2 Fc Cat.#2215-N2-025, R&D Systems |
| • | Protein standard range: | 100-0.046 ng/mL |
| • | Detection antibody: | NRP2 pAb Cat.#BAF2215, R&D Systems |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

**Assays to measure human endogenous HRS & NRP-2 complexes in circulation.** An ELISA detection assay was developed to measure levels of human HRS & NRP2 complexes in circulation using capture and detection antibodies specific to each protein partner. The human HRS & NRP-2 complex ELISA is designed to detect a complex of soluble NRP2 and HRS utilizing monoclonal and polyclonal antibodies specific for these two proteins. ELISA assays were conducted using a 96 well Multi-Array plate, following standard Meso Scale Diagnostics ELISA protocols, and using the following reagents:

| | | |
|---|---|---|
| • | Block buffer: | Casein (Thermo Scientific #37528) |
| • | Wash buffer: | PBST (0.05% Tween-20 in 1X PBS; made in-house) |
| • | Diluent: | 1% BSA (diluted in PBS) and Casein |
| • | Capture antibodies: | NRP2 mAb Cat.#MAB22151, R&D Systems |
| | | HRS C-terminal mAb Clone#ATYR13C8 |
| | | HRS N-terminal mAb Clone #ATYR12H6 |
| • | Capture antibody conc: | 1 µg/mL |
| • | Detection antibodies: | NRP2 pAb Cat.#BAF2215, R&D Systems HRS C-terminal mAb Clone#13C8 |
| | | HRS N-terminal mAb Clone #12H6 |
| • | Detection antibody conc: | 0.5 µg/mL |
| • | Secondary reagent: | Streptavidin SULFO-TAG, # R32AD-1, 500 µg/mL |
| • | Secondary reagent conc: | 1 µg/mL |
| • | Substrate: | MSD Read Buffer T (4X) with Surfactant #R92TC-1 |

**Antibody characterization Studies.** Surface plasmon resonance (SPR) methods were used to characterize the binding kinetics and affinities of the antibodies towards HRS proteins. SPR experiments were conducted on a Bio-Rad ProteOn XPR36 Protein Interaction Array instrument. HRS proteins were immobilized on different channels of a ProteOn GLC sensor chip through amine coupling. Each antibody at a series of different concentrations was flowed over the immobilized proteins. The sensor chip surface was regenerated between each antibody run to remove bound antibodies. The resulting sensograms were analyzed in the ProteOn Manager Software, and fitted globally to a bivalent analyte model to obtain on-rates (*kₐ*) and off-rates (*k_{d}*). The equilibrium dissociation constant (*K*_{D}) for each antibody-protein pair is the ratio of *k_{d}*/*kₐ.*

| | | |
|---|---|---|
| • | Running buffer: | 1x PBS with 0.005% Tween-20 |
| • | Amine coupling: | ProteOn Amine Coupling Kit (Bio-Rad #1762410) |
| • | Ligand coupling buffer: | Sodium acetate pH 5.5 |
| • | Regeneration buffer: | 10 mM HCl |

**Protein-Protein Interaction studies**. Surface plasmon resonance (SPR) methods were used to demonstrate interactions between protein partners. SPR experiments were conducted on a Bio-Rad ProteOn XPR36 Protein Interaction Array instrument. Proteins were immobilized on different channels of ProteOn GLC sensor chips through amine coupling. Analyte proteins were flowed over the immobilized proteins. The sensor chip surface was regenerated between each analyte run to remove interacting proteins. Data was double referenced against an interspot (untreated chip surface) and a blank surface (activated and deactivated for amine coupling).

| | | |
|---|---|---|
| • | Running buffer: | 50 mM HEPES, 300 mM NaCl, 5 mM CaCl₂, 0.005% Tween-20, pH 7.4 |
| • | Amine coupling: | ProteOn Amine Coupling Kit (Bio-Rad #1762410) |
| • | Ligand coupling buffer: Sodium | acetate (pH 4.0, 4.5, 5.0, 5.5 depending on pI of protein) |
| • | Regeneration buffer: | 10 mM glycine pH 2.0 |

Commercial proteins reagents (proteins are derived from human sequences unless noted):
- NRP2-Fc (R&D Systems #2215-N2)
- NRP1-His (R&D Systems #3870-N1)
- Mouse PLXNA1-His (R&D Systems #4309-PA)
- SEMA3C-Fc (R&D Systems #5570-S3)
- Mouse SEMA3F-Fc (R&D Systems #3237-S3)
- Mouse NRP2-Fc (R&D Systems #7988-N2)
- Rat NRP2-Fc-His (R&D Systems #567-N2)
- VEGF-C (R&D Systems #9199-VC/CF)
- VEGF-A₁₆₃ (Peprotech #100-20)
- VEGF-A₁₄₅ (R&D Systems #7626-VE-CF)
- VEGF-A₁₂₁ (Peprotech #100-20A)
- PIGF-2 (Peprotech #100-56)
- Heparin (StemCell #07980)

### Immunofluorescence assays on cultured cells.

### Reagents:

- PE anti-human IgG Fc Antibody, clone HP6017, Biolegend cat 409304
- Mouse Anti-HRS monoclonal antibody (1-96) Clone 1C8, Abnova cat H00003035-M01
- Mouse IgG2a, Isotype control antibody (MOPC-173), Biolegend cat 400223
- Recombinant Human VEGF-C Protein, R&D Systems, cat 2179-VC-025/CF
- Formaldehyde, 16%, methanol free, Ultra Pure, Polysciences, cat 18814-10
- Hoechst 33342, Trihydrochloride, Trihydrate, ThermoFisher Scientific, cat H1399
- Fc-HRS (2-60) was prepared as described in PCT application PCT/US2014/029699
- Gibco DMEM, High Glucose, ThermoFisher Scientific, cat 11965092
- PolyJet^{™} In Vitro DNA Transfection Reagent, SignaGen, cat SL100688
- Neuropilin 2 (NRP2) (NM_003872) Human ORF Clone, Origene, cat RG220706
- Collagen Coating Solution, Cell Applications Cat 125-100
- 1X PBS containing 1% BSA, 0.9 mM CaCl₂ and 20 mM glucose
- Binding buffer containing 1% normal mouse IgG (sigma cat I8765) and 2.5% (Human Fc Receptor Binding Inhibitor, ebioscience 14-9161-73).

**Cell culture and transfection.** HEK293T cells were cultured in DMEM containing 10% FBS and 1% Penicillin/Streptomycin. Cells were seeded in 6-well plates the night before transfection. 1µg of plasmid DNA encoding a NRP2a-GFP fusion protein was pre-complexed with PolyJet transfection reagent according to the manufacturer's protocol and subsequently added to cells. Media was changed 16 hours-post transfection and transfected cells were passed to 96-well plates for staining.

**Immunofluorescence assays on cultured cells.** Binding and quantitation of Fc-HRS (2-60) to cell-expressed NRP2 was achieved using immunofluorescence microscopy. Fc-HRS (2-60) was pre-complexed for 1 hour at room temperature with PE-conjugated anti-Fc at a ratio of 2:1. HEK293T cells previously transfected with NRP2v2-GFP were passed the night before staining to 96 well Greiner Clear flat bottomed Microplates pre-coated with collagen coating solution. Supernatants were removed and cells were washed 1 time with binding buffer. Cells were then fixed with 50 µL of 3.7% methanol-free formaldehyde for 20 minutes at room temperature. Cells were washed 2X with binding buffer and then blocked with 100 µL of blocking buffer for 1 hour at room temperature. The cells were then washed one time with binding buffer and then incubated with 50 µL of staining complex overnight at 4 degrees Celsius. Cells were then washed 3 times with binding buffer, and then nuclei were counter stained with 2 µg/mL Hoechst diluted in DPBS for 10 minutes at room temperature. The Hoechst stain was replaced with 1X PBS and subsequently read on an IN Cell Analyzer 2200. 20X images were acquired and analyzed using In Cell Analyzer 1000 Workstation software. Segmentation of the cell mask was achieved using the GFP channel, and the average PE signal intensity was determined within this mask above a threshold intensity of 5000 (termed GFP Bright cells).

**Stable NRP2 expressing cell pool generation.** A plasmid (Origene Technologies Cat#RC220706) encoding the NRP2 variant 2 transcript NM_003872 fused to a Myc-DDK tag was purchased. The vector was PCR amplified using Q5 polymerase (New England Biolabs Cat#M0491) with the following primer pairs:
5'-TGAGGATGACAAAGATTTGCAGCT-3' (SEQ ID NO: 265)
5'-ACCGCGGCCGGCCGTTTATGCCTCGGAGCAGCACTT-3' (SEQ ID NO: 266)
5'-AGTGCCAAGCAAGCAACTCAAA-3' (SEQ ID NO: 267)
5'-AAGTGCTGCTCCGAGGCATAAACGGCCGGCCGCGGT-3' (SEQ ID NO: 268)

The resulting PCR products were then fused, cut with MfeI/AgeI (New England Biolabs Cat#R3589, R3552), and ligated into a vector fragment of RC220706 cut with the same enzymes. This vector, containing an untagged NRP2v2 transcript, was then linearized and re-suspended in 10mM Tris-0.1mM EDTA. Suspension Expi293 cells (ThermoFisher Cat#A14527), were grown in expression medium (ThermoFisher Cat#A1435101) at 37°C and 8% CO₂. The linearized plasmid described above was transfected into Expi293 cells using an SF Cell Line 4D-Nucleofector^{®} X Kit L (Lonza Cat#V4XC-2012) and standard protocol T-030 for suspension HEK293 cells. Cells were allowed to recover in static culture for 17 hours, transferred to suspension and recovered an additional 72 hours, and then were selected with 200-350 µg/mL G418 in 50 µg increments (ThermoFisher Cat#10131035). Cell densities and viabilities were monitored for a period of 3 weeks, with fresh media/antibiotic replacement every 2-3 days. Upon recovery of viabilities to >95%, stably transfected cell pools were re-suspended in freezing media and archived.

### Flow cytometry-based assay for Fc-HRS (2-60) binding to NRP2-expressing cells

- Immobilized TCEP Disulfide Reducing Gel (Thermo Scientific #77712)
- PBS/EDTA (0.5 M EDTA in PBS)
- EZ-Link^{™} Maleimide-PEG11-Biotin (Thermo Scientific # 21911)
- Spin Columns (Thermo Scientific # 69705)
- Zeba^{™} Spin Desalting Columns, 40K MWCO (Thermo Scientific # 87770)
- Pierce Biotin Quantitation Kit (Thermo Scientific # 28005)
- Streptavidin-PE (Thermo Scientific # 12-4317-87)
- Anti-NRP2-APC clone 257103 (R&D Systems # FAB22151A)
- Propidium iodide solution (Miltenyi Biotec # 130-093-233)

**Biotinylation of Fc-HRS (2-60).** Fc disulfide bonds in Fc-HRS (2-60) were reduced using TCEP gel equilibrated with PBS/EDTA, and the sample was separated using a spin column. Biotinylation was performed using maleimide-PEG11-biotin reagent with a 2 hour reaction at room temperature, and free reagent removed using a Zeba column. Degree of biotinylation was determined to be 3.35 biotins/molecule using the Pierce Biotin Quantitation kit according to the manufacturer's instructions.

**Flow cytometry.** Biotinylated Fc-HRS (2-60) was incubated for 1 hour on ice with streptavidin-PE at a 1:2 molar ratio to form a staining complex. The staining complex was then added to stably expressing Expi293-NRP2 cells along with titrated anti-HRS antibodies and incubated 30-60 min on ice. Final concentrations were 43.75 nM (biotinylated Fc-HRS (2-60)) and 87.5 nM (streptavidin-PE). Cells were pelleted and washed as described above, and stained with anti-NRP2-APC (1:20) and resuspended in FWB buffer along with 1 µg/mL propidium iodide for viability gating. Samples were acquired on a Cytoflex S flow cytometer (Beckman Coulter), and the percentage of streptavidin-PE+/NRP2+ cells in the viable singlet gate was determined.

**Statistical analysis.** Data are expressed as mean ± SEM or as individual data points, except where noted. In experiments in which animals were euthanized due to tumor burden or body weight loss, the terminal tumor volume was carried forward for statistical analysis. In cases where an animal was found dead but did not have a large tumor (cause of mortality unknown), the animal's data were removed prior to statistical analysis. Significance of difference over time was tested with a 2-way repeated measures ANOVA followed by Dunnett's post-hoc test. Group comparisons were conducted using 1-way ANOVA (parametric or Kruskal Wallis, as noted in the figure legends). A p value ≤ 0.05 was considered significant.

### EXAMPLE 1

### INITIAL RECEPTOR IDENTIFICATION SCREEN

To identify potential interacting partners of HRS, and related HRS polypeptides, the Retrogenix cell microarray screening technology (Retrogenix Ltd., High Peak Rd, United Kingdom) was used to evaluate binding of a HRS-Fc fusion protein construct ([Fc-HRS(2-60)] to a library of approximately 4500 membrane bound human proteins individually expressed in HEK293 cells.

In brief, HEK293 cells were plated onto glass cover slides which have been pre-treated by the application of discrete expression vectors to enable reverse transfection and expression of each of the 4500 membrane proteins, to create a cell microarray. Transfection efficiencies were assessed via ZsGreen1 expression and exceeded the minimum threshold for all of the library members screened.

Use of a smaller HRS fragment with a Fc tag provided for high sensitivity detection via the use of an AlexaFluor647 labeled anti-human IgG Fc antibody (AF647) as the detection reagent, and readily available controls to confirm specificity. The detection antibody was used at screening concentrations of 2, 5, and 20 µg/ml, as more fully described below.

Test Protein was screened at a concentration of 20 µg/ml using two different screening formats; either a sequential staining method, or a pre-incubation staining method. Sequential staining involved, in brief, the sequential addition to the test cells, of the test protein and detection reagents, while the pre-incubation staining method involved the pre-incubation of the test protein with the detection reagents (2: 1 molar ratio of test protein to detection antibody) to pre-form higher avidity complexes prior to the addition to the test cells. A background screen was completed with test protein added to slides of fixed, untransfected HEK293 cells to confirm that the test protein did not bind to untransfected cells.

Primary hits (duplicate spots) were identified by analyzing fluorescence intensity in the AlexaFluor 647 and ZsGreen1 emission channels using the ImageQuant system, following standard fluorescent methodology. Confirmation Screens were run to evaluate any screening hits identified from the primary screen, using identical fixed slides treated with 20 µg/ml test protein, or positive or negative controls, using the sequential or pre-incubation methods (n=2 slides per treatment). Additionally all vectors encoding all hits, plus control vectors, were spotted in duplicate on new slides, and used to reverse transfect human HEK293 cells as before. All transfection efficiencies exceeded the minimum threshold Hits were categorized as specific, or non-specific (i.e. it also came up with at least one of the positive or negative controls), and if specific whether the hit was strong, medium or weak binding.

Confirmatory hits using both the sequential and pre-incubation staining methods are summarized in **Table E1A and Table E1B** below.

| **Table E1A. Sequential incubation confirmatory screening result summary** | | | | | | |
|---|---|---|---|---|---|---|
| Hit No. | Gene ID: | Accession # | Fc-HRS | CTLA4-FC | RITUXIMAB | PBS |
| 1 | FCGR1A | | NON SPEC | NON SPEC | NON SPEC | NON SPEC |
| 2 | SLC13A3 | | INVERSE | NON SPEC | INVERSE | N/A |
| 3 | NRP2A | NM_003872.2 | WEAK/MED | N/A | N/A | N/A |
| 4 | IGHG3 | | STRONG | STRONG | STRONG | STRONG |
| 5 | FCGR2A | | NON SPEC | NON SPEC | NON SPEC | N/A |
| 6 | NRP2B | NM_201267.1 | WEAK/MED | N/A | N/A | N/A |
| 7 | MS4A1 | | N/A | N/A | STRONG | N/A |
| 8 | CD86 | | N/A | STRONG | N/A | N/A |
| 9 | EGFR | | N/A | N/A | N/A | N/A |
| 10 | SLC38A2 | | WEAK | N/A | N/A | N/A |
| 11 | SLC38A4 | | WEAK | N/A | N/A | N/A |
| 12 | COLEC12 | | WEAK | N/A | N/A | N/A |

| **Table E1B. Pre-incubation confirmatory screening result summary** | | | | | |
|---|---|---|---|---|---|
| Hit No. | Gene ID: | Accession # | Binding scoring | | |
| | | | Fc-HRS | CTLA4-FC | RITUXIMAB |
| 1 | FCGR1A | | NON SPEC | NON SPEC | NON SPEC |
| 2 | SLC13A3 | | INVERSE | NON SPEC | INVERSE |
| 3 | NRP2 | NM_003872.2 | MED/STRONG | N/A | N/A |
| 4 | IGHG3 | | NON SPEC | NON SPEC | NON SPEC |
| 5 | FCGR2A | | NON SPEC | NON SPEC | NON SPEC |
| 6 | NRP2 | NM_201267.1 | MED/STRONG | N/A | N/A |
| 7 | MS4A1 | | N/A | N/A | STRONG |
| 8 | CD86 | | N/A | STRONG | N/A |
| 9 | EGFR | | N/A | N/A | N/A |
| 10 | SLC38A2 | | WEAK | N/A | N/A |
| 11 | SLC38A4 | | WEAK | N/A | N/A |
| 12 | COLEC12 | | WEAK | N/A | N/A |

**Summary/Conclusions.** After screening test protein (Fc-HRS) for binding against 4500+ human plasma membrane proteins expressed in human HEK293 cells, using two incubation approaches, two neuropilin 2 (NRP2) isoforms - (Neuropilin 2A and 2B) were identified as convincing and specific binding partners (using both incubation approaches). The sequential method also identified three, weak intensity hits: SLC38A2, SLC38A4 and COLEC12. These may also be of biological relevance to HRS polypeptides in general and in particular to those comprising the N-terminal domain (1-60) of HRS. Given the broad involvement of the Neuropilin 2 in a broad range of biological processes, including for example, immune activation, immune cell migration, cancer growth, motility and metastasis, lymphogenesis, epithelial-mesenchymal transition (EMT) and nerve fiber growth guidance, these results suggest that HRS, and related HRS polypeptides have the potential to play key regulatory roles in normal and pathophysiology.

### EXAMPLE 2

### CONFIRMATION OF BINDING SPECIFICITY BY SPR ANALYSIS AND IDENTIFICATION AND USE OF SPECIFIC EPITOPES

Studies were performed to confirm the binding specificity of Neuropilin 2 (NRP2) to Fc-HRS(2-60) using orthologous methods to those used in the large scale Retrogenix screening (**Example 1**). In a series of experiments, Fc-HRS(2-60) and related proteins were immobilized on SPR chips, and NRP2 and related proteins were flowed as analytes. Upon confirmation of the NRP2:Fc-HRS(2-60) interaction, the dependence on divalent cations was tested as NRP2 is known to have Ca²⁺ binding sites. The effects of previously characterized NRP2 ligands on the NRP2: Fc-HRS(2-60) interaction was also tested to determine if these known ligands have competitive effects on the Fc-HRS(2-60) interaction.

In another series of experiments, monoclonal antibodies (mAbs) which recognize Fc-HRS(2-60) were immobilized on SPR chips. Fc-HRS(2-60) and NRP2 were pre-incubated and injected over the mAb surfaces to determine if only Fc-HRS(2-60), or a larger NRP2: Fc-HRS(2-60) complex was capable of binding to the mAbs. Additionally, co-injection experiments were carried out in which sequential analyte injections of Fc-HRS(2-60) followed by NRP2 were performed. As the different mAbs bind to different epitopes on Fc-HRS(2-60), the ability of the mAbs to bind to an NRP2: Fc-HRS(2-60) complex as opposed to only binding free Fc-HRS(2-60) gives indications as to the interaction surface between the two proteins.

**Results.** NRP2 but not the closely related NRP1 protein, nor the mouse version of the Plexin A1 co-receptor bind to immobilized Fc-HRS(2-60) (**Figure 3**). In addition to human NRP2, both mouse and rat NRP2 demonstrate binding to FC-HRS(2-60) . However, none of these NRP2 forms bind to a truncated form of Fc-HRS(2-60) with a 49 amino acid deletion at the C-terminus the fusion protein ([Fc-HRS(2-11)] which deleted the majority of the WHEP domain (**Figures 4A-3B**).

Fc-HRS(2-60) consists of a human IgG Fc region fused to the WHEP domain from histidyl-tRNA synthetase (HRS). Homologous WHEP domains are found in several other tRNA synthetases, including for example, WARS, GARS, MARS, and EPRS. While NRP2 binds to Fc-HRS(2-60), it does not bind to similar proteins consisting of Fc domain fused to the WHEP domain of either GARS or MARS (**Figures 5A-4D).** Additionally NRP2 does not bind to the WHEP domain of WARS with a V5/His tag, suggesting that this interaction with NRP-2 is specific to the HRS WHEP domain and not generally applicable to the other WHEP domains tested.

NRP2 is known to have calcium binding sites in its two CUB-domains (a1 and a2 domains). The running buffer of the SPR instrument was switched to a calcium-free buffer (50 mM HEPES, 300 mM NaCl, 0.005% Tween 20, pH 7.4), and CaCl₂, MgCl₂, ZnCl₂ or EDTA were added to the analytes prior to injection and flowed over immobilized Fc-HRS(2-60) (**Figures 6A-5B****)**. Slight binding was observed in the running buffer alone, while the addition of CaCl₂ greatly enhanced binding. Conversely, addition of ZnCl₂ or EDTA (which chelates divalent cations), resulted in no significant additional binding. Additionally, MgCl₂ up to the concentrations tested, does not appear to have any significant effects on the binding. This result suggests the involvement of the a1 or a2 domains of NRP2 in the interaction with Fc-HRS(2-60) either directly or through maintenance of the conformation of the NRP-2 molecule.

A subset of the known ligands of NRP2 appears to compete binding of Fc-HRS(2-60) to NRP2. The VEGF family of ligands that bind NRP2 appear to prevent binding of Fc-HRS(2-60), while the SEMA family of ligands do not appear to compete binding under the conditions tested (**Table E2**). In the presence of either VEGF-C, VEGF-A₁₆₅, or PlGF-2/Heparin, binding of NRP2 to Fc-HRS(2-60) is reduced or ablated. Conversely, in the presence of VEGF-A₁₄₅ (which has been reported to be an NRP2 ligand but does not bind NRP2 in our system) or VEGF-A₁₂₁ (which does not bind NRP2), binding of NRP2 to Fc-HRS(2-60), is unaffected. Although SEMA3C and mouse SEMA3F do bind to NRP2, the presence of either of these proteins also do not affect NRP2 binding to Fc-HRS(2-60) under the conditions tested. These results suggest that the Fc-HRS(2-60) binding site of NRP2 overlaps with the VEGF binding site, but not with the SEMA binding site of NRP2.

| **TABLE E2** | | |
|---|---|---|
| **Ligand** | **Binding to NRP2 observed by SPR** | **Competes with** Fc-HRS(2-60) **for NRP2 binding** |
| VEGF-C | Yes | Yes |
| VEGF-A₁₆₅ | Yes | Yes |
| VEGF-A₁₄₅ | No | No |
| VEGF-A₁₂₁ | No | No |
| PlGf-2/Heparin | Not tested | Yes |
| SEMA3C | Yes | No |
| Mouse SEMA3F | Yes | No |

In another series of experiments, different monoclonal antibodies which recognize Fc-HRS(2-60) were immobilized on SPR chips. In **Figures 7A-6B****,** the mAb clones 1C8 and 4D4 were immobilized on an SPR chip and then a mixture of Fc-HRS(2-60) and NRP2 which had been pre-incubated together was injected over the mAb surfaces.

Based on the resulting pattern of signal intensities it can be concluded that the monoclonal antibody clone 1C8 likely binds to Fc-HRS(2-60) at an epitope involved in NRP2 binding, because no larger complex binding is detected when the complex is passed over the detection surface. The lack of additional binding under these conditions suggests that the 1C8 antibody is capable of displacing Nrp-2 from the Fc-HRS(2-60):Nrp-2 complex.

In contrast, when the monoclonal antibody clone 4D4 was attached to the detection surface, a significantly larger signal intensity was observed, suggesting that it was able to bind to the Fc-HRS(2-60) moiety, without displacing Nrp-2, from the Fc-HRS(2-60):Nrp-2. This indicates that NRP2 is able to bind Fc-HRS(2-60) in the presence of the 4D4 mAb and that they bind to non-overlapping regions of Fc-HRS(2-60). Additionally, co-injection experiments were carried out in which sequential analyte injections of Fc-HRS(2-60) followed by NRP2 were performed (**Figures 8A-7D**). In these experiments, Fc-HRS(2-60) that was bound to antibody clone ATYR4D4 or monoclonal antibody clone ATYR13E9 were able to further bind NRP2.

Fc-HRS(2-60) that was bound to monoclonal antibody clone ATYR12H6 showed only slight binding of NRP2, while Fc-HRS(2-60) bound to antibody clone ATYR1C8 showed no binding to NRP2. These data together indicate that antibody clone ATYR1C8 binding is able to block NRP2 binding to Fc-HRS(2-60), while antibody clone ATYR12H6 binding is able to partially block NRP2 binding, and antibody clones ATYR4D4 and ATYR13E9 are not able to block NRP2 binding to HRS.

### EXAMPLE 3

### CONFIRMATION OF BINDING TO NRP2 EXPRESSED IN HEK293 CELLS

To directly confirm direct binding of HRS to cells expressing recombinant Neuropilin 2a or 2b, Fc-HRS(2-60) was added to HEK293 cells which had been transfected with expression vectors encoding for either Neuropilin 2a or 2b, or as their respective fusion proteins with GFP, and detected via the use of fluorescently labelled anti-Fc-PE as described in the Materials and Methods.

As shown in **Figures 9A-8B****,** dose-dependent binding of Fc-HRS (2-60) to cell-expressed NRP2a was readily detectable under these conditions.

**Figure 10** shows that pre-incubation of Fc-HRS(2-60) with the blocking antibody clone 1C8, resulted in almost complete abolition of binding, demonstrating that the binding is specific for the epitope recognized by the anti-HRS antibody. Binding specificity was further confirmed through the use of the deleted control protein, Fc-HRS(2-11), which also showed negligible specific binding.

To determine the ability of anti-HRS antibodies to block binding of Fc-HRS (2-60) to NRP2, HEK293 cells were stably transfected with NRP2 and binding of biotinylated Fc-HRS (2-60) in the presence or absence of antibodies monitored by flow cytometry as described in the Materials and Methods.

**Figure 11A-11B** shows that antibodies from the KL31 series blocked binding of Fc-HRS to NRP2 in a concentration-dependent manner, whereas other antibodies tested did not demonstrate significant blocking characteristics in this assay.

Functional interactions with other Neuropilin 2 interacting proteins was demonstrated via direct competition of Fc-HRS(2-60) by pre-incubation of cells expression NRP2 with VEGF-C (**Figures 12A-12B**).

These results confirm, and extend the Retrogenix screens and suggests that the interaction of HRS proteins such as wild type HRS, and HRS polypeptides comprising the N-terminal region play important, biologically relevant roles by binding to NRP2, and by interacting with its other natural ligands including VEGF-C.

### EXAMPLE 4

### CIRCULATING LEVELS OF SOLUBLE NEUROPILIN 2 (NRP2) IN HUMAN SERUM AND PLASMA

Serum & plasma samples from normal healthy volunteers (n=72) were tested for circulating levels of soluble NRP2. NRP2 levels were quantified with an internally developed human NRP2 ELISA (as described in the Materials and Methods).

**Summary of results.** Analysis of circulating NRP2 in both serum and plasma revealed complimentary results in both matrices. Serums levels of NRP2 averaged 16.3pM while mean plasma levels were 15.6pM. Quantification revealed that 86% of the serum samples and 83% of the plasma samples were detectable and above the lower limit of quantitation for this assay (1.5pM) (**Table E3** and **Figure 13**).

| **TABLE E3** | | |
|---|---|---|
| | Serum | Plasma |
| # of samples | 72 | 72 |
| Mean +/- SD (pM) | 16.3 +/- 24.3 | 15.6 +/- 23.3 |
| Median (pM) | 6.1 | 5.5 |
| Range (pM) | ≤1.5 - 111.6 | ≤1.5 - 115.3 |

### EXAMPLE 5

### COMPARISON OF CIRCULATING HRS & NRP2 LEVELS

Circulating serum HRS levels from 72 normal healthy donors were rank ordered from lowest to highest levels. Matching serum NRP2 levels from the exact same donors were overlaid on the same axes.

**Summary of results.** Human HRS levels from normal healthy donors spans nearly two logs (~10pM - 1000pM) in concentration. Similarly, soluble NRP2 levels also demonstrate a large distribution in circulating levels (~1pM - 100pM). Comparison of serum samples from normal healthy volunteers revealed a trend whereby people with low circulating HRS levels also have lower soluble NRP2 levels and conversely those individuals with higher HRS levels demonstrate higher circulating soluble NRP2 levels (see **Figure 14**).

### EXAMPLE 6

### N-TERMINAL HRS ASSAY INTERFERENCE

Serum samples from normal healthy volunteers were assayed in two separate ELISAs to detect circulating levels of HRS. An assay designed to detect the full length version of HARS (HARS_FL) utilized an N-terminal capture antibody and a C-terminal detection antibody. The second assay was designed to exclusively detect the N-terminal portion of HRS (HARS_NT) with both capture and detection antibodies directed to the N-terminus. Accordingly, the FL-terminal assay, is unable to detect N-terminally truncated fragments of HRS, lacking the C-terminal epitope recognized by the C-terminal detection antibody. Conversely the N-terminal assay is susceptible to interference via the binding of other factors to the N-terminal domain of HRS, which compete with antibody binding.

**Summary of results.** Individual healthy donor serum was assayed for HRS levels using both the full length and N-terminal ELISA formats. Samples with low levels of HRS detected via the full length ELISA HRS levels tended to correlate well with the N-terminal ELISA results (**Figure 15**). However, in selected donors with relatively high levels of HRS detected via the FL-ELISA, it was also observed that the HRS levels detected via the N-terminal ELISA no longer showed a close correlation, but were in certain subjects significantly lower. Without being bound by any one specific explanation, it is believed that the significantly lower apparent HRS levels in the N-terminal assay is caused by the existence of an interfering substance which binds to the N-terminal domain of HRS, thereby blocking its detection in the N-terminal ELISA assay.

### EXAMPLE 7

### CORRELATION OF HRS N-TERMINAL INTERFERENCE AND SOLUBLE NRP2

To further examine the relationship between HRS N-terminal assay interference and soluble NRP2 levels, circulating HRS and NRP-2 levels were analyzed in normal healthy volunteer serum samples. The difference in observed HRS levels between the full length ELISA and the N-terminal ELISA was calculated for each of the 72 healthy serum donor samples (N-terminal Interference Units). These same samples were additionally tested for circulating human NRP2 levels.

**Summary of results.** The interference observed between the two HRS assay formats was termed HARS N-terminal Interference Units (HARS_FL minus HARS_NT) and was plotted versus soluble NRP2 levels (**Figure 16**). The resulting graph shows a clear trend for increased N-terminal interference and increased soluble NRP2 levels suggesting a potential role for soluble NRP2 to interfere with the detection of the N-terminus of HRS.

### EXAMPLE 8

### DETECTION OF HRS:NRP2 SOLUBLE COMPLEX IN NORMAL SERUM

In an attempt to observe an endogenous circulating HRS: NRP2 soluble complex in serum, several novel ELISA formats were utilized to capture this interaction. Normal healthy human serum was isolated from internal sources (#21949, #32565, #22447, #24098, #23024) or through commercial vendors (Sigma, CELLect). These healthy serum samples were analyzed for levels of N-terminal interference (data not shown) and categorized as either low N-terminal interference or high N-terminal interference and parsed accordingly. These 7 serum samples were assayed in multiple formats of a HRS:NRP-2 complex ELISA. These assays consisted of a capture antibody directed against HRS N-terminus (HARS_NT), HARS C-terminus (HARS_CT), or NRP2. The detection antibody in these assays was directed against the alternate protein in the complex (e.g., HRS detection antibody with a NRP2 capture antibody, and NRP2 detection antibody with a HRS capture antibody).

**Summary of results.** HRS:NRP2 complex ELISAs were tested with normal serum samples that had been previously identified as either having low or high N-terminal interference. All samples with low N-terminal HRS interference showed low signals in all formats of the HRS:NRP2 complex ELISAs (**Figure 17****,** left bar graphs). In contrast, serum samples identified as containing high N-terminal assay interference all showed elevated signals in HRS and NRP-2 complex ELISAs (**Figure 17**, right bar graphs). These results were observed with multiple antibody pairings to both terminal ends of HRS, suggesting that the result is not the result of unanticipated antibody cross reactivity's between NRP2 and HRS.

### EXAMPLE 9

### CONFIRMATION OF A HRS & NRP2 SOLUBLE COMPLEX IN NORMAL SERUM

To confirm the relationship between HRS N-terminal interference and the detection of an endogenous soluble HRS:NRP2 complex, the antibody reagents utilized to originally characterize the N-terminal interference observed in human serum were tested side by side in the HRS:NRP2 complex ELISA. Healthy normal serum samples from persons identified as low or high interference (as described above) were tested in a HRS:NRP2 complex ELISA consisting of an NRP2 capture antibody followed by detection with either a non-interfering HRS N-terminal antibody (HARS_NT) or an N-terminal HRS antibody that blocks the interaction (HRS blocking antibody).

**Summary of results.** The results of the HRS:NRP2 complex ELISA show an increased signal between low and high interference samples when capturing soluble NRP2 and detecting with the HARS_NT antibody. However, when these same sample are tested in an assay format where the detection antibody against HRS is directed against the site where NRP2 is believed to bind, then the signal in this complex ELISA returns to the same levels as observed in samples without assay interference (Figure 18). The results suggest that this blocking antibody is directed against the putative NRP2 binding site on the N-terminus of HRS.

### EXAMPLE 10

### EVALUATION OF ACTIVITY OF Fc-HRS(2-60) IN A MURINE MODEL OF SCLERADERMATOUS CHRONIC GRAFT VERSUS HOST DISEASE

This study was designed to investigate the *in vivo* potential of test compounds in a murine model of sclerodermatous chronic graft vs. host disease (scl cGvHD). A H-2^{d} minor histocompatibility antigen-mismatched model was employed. The model was prepared by allogenic transplantation of bone marrow cells and splenocytes of male donor B10.D2 mice into 8 week old female Balb/c mice which had been subject to whole body irradiation with 700 cGy 6 hours prior to transplantation.

**Treatment Regimen.** Fifty-six (56) Balb/c mice, (Janvier, Le Genest St. Isle, France, female, 8 weeks) were used as recipients in this study while B10.D2 mice (Jackson Laboratory, Bar Harbor, ME, USA, male) were euthanized to provide donor cells for allogenic transplantation. The animals were assigned to study groups of 8 mice, and housed in specific pathogen-free conditions with sterile food and water. The treatment regimens are shown in **Table E4** below. In brief, animals received weekly intravenous injections of the Vehicle of Fc-HRS(2-60) (20 mM Histidine/125 mM NaCl/10 mM Methionine/3% Sucrose/0.02% PS20, pH 6.9) beginning on Day 7 post transplantation (groups 2 and 3) or Fc-HRS(2-60) (0.4 mg/kg) beginning on Day 7 (group 4) or Day 21 (group 6) post-transplantation. Daily oral dosing with nintedanib was included as a known comparator beginning on Day 7 (group 5) or Day 21 (group 7) post-transplantation. A syngeneic transplantation was included as an un-induced control (group 1). Termination for skin and lung harvest was performed on Day 21 (group 2) to provide baseline endpoints for groups that initiated treatment on Day 21 post-transplantation. Remaining groups were euthanized 8 weeks after donor cell injection. Tissues were examined histologically using common staining techniques and tissue homogenates were analyzed for hydroxyproline content as an index of collagen content. Kruskal-Wallis non-parametric ANOVA statistics followed by Dunn's multiple comparison test to Vehicle treatment (group 3) were conducted in GraphPad Prism.

| **TABLE E4. GROUP TREATMENTS** | | | | | |
|---|---|---|---|---|---|
| **Group** | **#Mice** | **Transplant (Day 0)** | **Treatment** | **Start** | **Termination** |
| 1 | 8 | Balb/c (H-2^{d}) - Balb/c (H-2^{d}) | | | |
| 2 | 8 | B10.D2 (H-2^{d}) - Balb/c (H-2^{d}) | Vehicle | Day 7 | Day 21 |
| | | | IV, QW | | |
| 3 | 8 | B10.D2 (H-2^{d}) - Balb/c (H-2^{d}) | Vehicle | Day 7 | Week 8 |
| | | | IV, QW | | |
| 4 | 8 | B10.D2 (H-2^{d}) - Balb/c (H-2^{d}) | Fc-HRS(2-60) (0.4 mg/kg) | Day 7 | Week 8 |
| | | | IV, QW | | |
| 5 | 8 | B10.D2 (H-2^{d}) - Balb/c (H-2^{d}) | Nintedanib (60 mg/kg) | Day 7 | Week 8 |
| | | | PO, QD | | |
| 6 | 8 | B10.D2 (H-2^{d}) - Balb/c (H-2^{d}) | Fc-HRS(2-60) (0.4 mg/kg) | Day 21 | Week 8 |
| | | | IV, QW | | |
| 7 | 8 | B10.D2 (H-2^{d}) - Balb/c (H-2^{d}) | Nintedanib (60 mg/kg) | Day 21 | Week 8 |
| | | | PO, QD | | |
| H-2d is a mouse MHC class I antigen | | | | | |
| IV, intravenously; QW, once a week; PO, orally; QD, once a day | | | | | |

**Summary of results.** Two mice, one each in groups 5 and 7, died during the second week post-transplantation, presumably due to cGvHD inflammation. Data obtained are shown graphically in **Figures 19** **and** **20**. In animals that survived to scheduled necropsy, successful model induction was confirmed histologically in the skin by increased dermal thickness and in the lungs by increased fibrosis (both Ashcroft score and collagen stained area). The number of myofibroblasts and collagen content measures were also significantly increased in allogenic vs. syngeneic transplantation in both skin and lung at the 8 week termination time point, further substantiating successful induction of fibrosis in the model. Note that these measures were already increased in animals terminated 3 weeks post-transplantation and although all endpoints had trends for further increase in animals terminated 8 weeks post-transplantation, comparisons between these groups did not show statistically significant differences.

Intervention with nintedanib beginning 7 days post-transplantation significantly ameliorated all measures of fibrosis in both skin and lung as expected. Similarly, nintedanib significantly improved most endpoints measured when intervention was delayed until 21 days post-transplantation. The test compound, Fc-HRS(2-60), was surprisingly effective in this model in both the lung and in the skin when intervention was initiated 7 days post-transplantation. The degree to which Fc-HRS(2-60) was effective at the early intervention timepoint was particularly pronounced in lung where the moderate dose of Fc-HRS(2-60) tested had larger amplitude effects (i.e., lower p values) that the maximal dose of nintedanib tested. FC-HRS(2-60) (0.4 mg/kg) tended to improve fibrotic endpoints when initiated 21 days post-transplantation.

These results demonstrate that HRS polypeptides such as Fc-HRS(2-60) have clear potential for therapeutic impact on fibrotic processes in multiple organs in this murine model.

### EXAMPLE 11

### EVALUATION OF ACTIVITY OF Fc-HRS(2-60) IN A MURINE MODEL OF LPS-INDUCED PULMONARY INFLAMMATION

This study was designed to investigate the *in vivo* potential of test compounds in a murine model of neutrophil accumulation in the lung induced by airway instillation of lipopolysaccharide (LPS).

**Treatment Regimen.** Sixty-four (64) C57BL/6 mice (Jackson Laboratory, Bar Harbor, ME, USA, 8 week old females) were assigned to study groups of 8 mice, and housed in specific pathogen-free conditions with sterile food and water.

The treatment regimens are shown in **Table E5** below. In brief, animals received a single intravenous injection of the vehicle of Fc-HRS(2-60) (20 mM Histidine/125 mM NaCl/10 mM Methionine/3% Sucrose/0.02% PS20, pH 6.9), Fc-HRS(2-60) (1, 3, or 10 mg/kg), or HRS(2-60)-COMP (3 mg/kg). The following day, mice were anesthetized by isoflurane inhalation and, using a gavage needle and attached syringe, 50 µL of LPS (Sigma L3024, *E. coli* 0111-B4, 10 µg/mouse) formulated in PBS, or PBS alone, was delivered to the back of the oral soft palate for inhalation. Twenty-four hours later, animals received a lethal dose of ketamine/xylazine (~300 and ~30 mg/kg, respectively) and bronchoalveolar lavage was performed by inflating lungs with 0.8 mL PBS through a cannula placed in the trachea, withdrawing the fluid into a syringe, and recording the retrieved volume. Samples with a retrieved bronchoalveolar lavage volume at or above 0.4 mL were included in the analysis (see **Table E5** for included number of samples per group). Cells obtained by bronchoalveolar lavage were collected by centrifugation at 300g, 10 min at +4°C, washed once by PBS and centrifuged again as above, and resuspended in 100 µL 1x RBC Lysis Buffer (Thermo Fisher Scientific) in order to lyze red blood cells. After an incubation period of 2-3 min, 2 mL of PBS was added to neutralize the lysis buffer, the sample was again centrifuged as above, and the resulting cells were collected.

| **TABLE E5. GROUP TREATMENTS** | | | | |
|---|---|---|---|---|
| **Group** | **# Samples included** | **IV Treatment** | | **Inhaled article** |
| | | **Test article** | **Dose (mg/kg)** | |
| 1 | 8 | None | n/a | None |
| 2 | 7 | Vehicle | n/a | PBS |
| 3 | 6 | Vehicle | n/a | LPS |
| 4 | 6 | Fc-HRS(2-60) | 1 | LPS |
| 5 | 7 | Fc-HRS(2-60) | 3 | LPS |
| 6 | 7 | Fc-HRS(2-60) | 10 | LPS |
| 7 | 5 | HRS(2-60)-COMP | 3 | LPS |
| 8 | 8 | HRS(2-60)-COMP | 3 | PBS |
| n/a, Not Applicable | | | | |

**Flow Cytometry Analysis.** Flow wash buffer (FWB, 3% fetal bovine serum in PBS) was used for all subsequent staining and washing steps. Live/dead cells were distinguished by staining with 120 µL of Zombie Yellow (BioLegend) diluted 1:400 in FWB for 20 min in the dark at room temperature. An excess of FWB was then added, and the sample was centrifuged as above; these steps are henceforth collectively referred to as "washing". Fc receptors were blocked by adding 25 µL of Mouse BD Fc Block (BD Biosciences) diluted 1:25 in FWB for 10 min in the dark at room temperature. NRP2 was detected by addition of 25 µL of rabbit anti-mouse NRP2 (Cell Signaling Technology) at 10 µg/mL in FWB for 1h in the dark on ice, and cells were then washed (data not shown). Subsequently, cells were stained for specific surface markers using 50 µL of a mixture of the antibodies listed in Table E6, along with 50 µL of AF647-labelled goat-anti-rabbit secondary antibody diluted 1:200, all in FWB for 30 min in the dark on ice. Cells were then again washed, resuspended in 200 µL of FWB, and acquired on a CytoFLEX flow cytometer (Beckman Coulter).

| **TABLE E6. FLOW CYTOMETRY STAINING ANTIBODIES** | | |
|---|---|---|
| **Marker** | **Dilution in FWB (Staining Mixture)** | **Fluorophore*** |
| Cd45 | 1:100 | BV421 |
| Cd11b | 1:200 | PE-Cy7 |
| SigLec-F | 1:200 | AF700 |
| Cd11c | 1:200 | APC-Cy7 |
| CX3CR1 | 1:100 | PE |
| GR1 | 1:200 | PerCP |
| Ly6G | 1:200 | FITC |
| Conjugated antibodies were obtained from BD Biosciences or BioLegend. | | |
| *AF, AlexaFluor; APC, allophycocyanin; FITC, Fluorescein isothiocyanate; BV, Brilliant Violet; PE, phycoerythrin; PerCP, peridinin chlorphyll protein. | | |

For gating strategy, the Cd45+CD11b+ population in single live cells was separated into SigLec-F+Cd11c+ cells (alveolar macrophages) and Siglec-F-Cd11c- cells (other). The latter population was then separated into CX3CR1+ (monocytes) and CXC3CR1- (other), which was then separated into a GR1+Ly6G+ population (neutrophils) and a small percentage of GR1+Ly6G- cells that were not further defined.

Using GraphPad Prism 7, results were analyzed by one-way ANOVA followed by Dunn's multiple comparison test using the LPS/Vehicle group as the comparator. A p < 0.05 was considered significant.

**Preparation of HRS(2-60)-COMP fusion proteins (not part of the invention).** His tagged HRS(2-60)-COMP protein was designed virtually, and comprised schematically, amino acids 1-17 of the SPARC signal peptide, coupled to amino acids 2-60 of HARS, coupled by 2 copies of a (GGGGS) linker sequence (SEQ ID NO: 204), fused to the cartilage oligomeric matrix protein (COMP) pentameric domain, consisting of amino acids 28-73 of COMP fused to a GGGGS linker (SEQ ID NO:204), which was subsequently fused to a -Myc-His tag (EQKLISEEDLNMHTGHHHHHH) (SEQ ID NO:262). Reference sequences as follows were SPARC signal peptide (NP_003109.1), HARS(P12081.2), COMP(NP_000086.2).

The amino acid sequence was codon optimized for mammalian expression and synthesized by Genewiz LLC into a pUC57 vector, and subsequently sub-cloned into a CMV expression vector pNTC7485 (Nature Technologies Corporation) using flanking enzyme sites SalI and BglII. A negative control protein lacking the HARS (aa2-60) fragment was constructed by site directed mutagenesis of the previous plasmid using PCR primers 5'-GCTGGCAGAGCTCTGGCTGGAGGAGGCGGATCCGGA-3' (SEQ ID NO: 263) and 5'-TCCGGATCCGCCTCCTCCAGCCAGAGCTCTGCCAGC-3' (SEQ ID NO: 264). Proteins were produced transiently in Expi293 cells (ThermoFisher) using the Expifectamine transfection system, following the manufacturer's instructions. The over expressed recombinant proteins were then purified over a HisTrap FF column (GEHC), eluted via linear imidazole gradient, and dialysed into 1xPBS pH7.4.

**Table H9** provides the full amino acid sequences of the SPARC-HRS(2-60)-COMP-MycHIS fusion protein; the mature, processed form of the HRS(2-60)-COMP-MycHIS fusion protein (with Myc-HIS tags); and the mature, processed form of the HRS(2-60)-COMP fusion protein (without Myc-HIS tags). The optimized (for *Homo sapiens*) nucleic acid sequence of SPARC-HRS(2-60)-COMP-MycHIS construct is provided in **Table E7** below.

| **Table E7. Optimized nucleic acid sequence** | | |
|---|---|---|
| Name | **Sequence** | **SEQ ID NO:** |
| SPARC-HRS(2-60)-COMP-MycHIS | ATGAGGGCCTGGATTTTCTTTCTGCTGTGCCTGGCTGGCAGAGCTCTGGCTG CTGAGAGAGCCGCCCTGGAGGAGCTGGTCAAGCTGCAGGGCGAGAGGGTGAG GGGCCTGAAGCAGCAGAAGGCCAGCGCCGAGCTGATCGAGGAGGAGGTGGCC AAGCTGCTGAAGCTGAAGGCCCAGCTCGGCCCTGACGAGAGCAAGCAGAAGT TCGTGCTGAAGACACCCAAGGAGGAGGCGGATCCGGAGGAGGAGGAAGCAG CGATCTGGGCCCCCAGATGCTGAGGGAGCTGCAGGAGACAAACGCCGCCCTG CAGGACGTGAGAGAGCTGCTGAGACAGCAGGTGAGGGAGATCACCTTCCTGA AGAACACCGTGATGGAGTGCGACGCCTGTGGAGGAGGAGGAGGCAGCGAGCA GAAGCTGATCAGCGAGGAGGACCTGAACATGCACACCGGCCATCACCATCAC CACCACTGA | 200 |

**Summary of results.** Data obtained from the samples where a sufficient bronchoalveolar lavage volume was collected are shown graphically in **Figures 21A-21C****.** In non-treated, non-challenged animals, virtually no neutrophils or monocytes were detected in the airways, whereas alveolar macrophages could be detected as expected. None of the treatments (LPS or test agents) altered the number of alveolar macrophages retrieved by bronchoalveolar lavage. However, LPS inhalation led to robust airway accumulation of neutrophils and monocytes. Fc-HRS(2-60) significantly inhibited LPS-induced neutrophil infiltration when administered at 3 and 10 mg/kg (21A), whereas monocyte infiltration was not altered (21B). The pentamer of HRS(2-60), HRS(2-60)-COMP, also significantly reduced the number of airway neutrophils accumulating in response to LPS (21A).

These results demonstrate that HRS polypeptides such as Fc-HRS(2-60) and HRS(2-60)-COMP have clear potential to reduce neutrophilic inflammation of the airways, and have broad potential as therapeutics in a broad range of inflammatory conditions associated with the migration and/or activation of neutrophils and related immune cells.

### EXAMPLE 12

### EVALUATION OF THE ACTIVITY OF FC-HRS(2-60) ON PHAGOSOME MATURATION IN MACROPHAGES

This study was designed to investigate the *in vivo* potential of test compounds to modulate phagocytosis in murine bone marrow derived macrophages. Phagocytosis refers to the process by which a cell engulfs a solid particle to form an internal compartment known as a phagosome. The process is homologous to eating at the level of single-celled organisms; though in multicellular animals, the process has been adapted to eliminate debris and pathogens, as opposed to taking in fuel for cellular processes.

In the immune system, phagocytosis is a major mechanism used to remove pathogens and cell debris. For example, when a macrophage ingests a pathogenic microorganism, the pathogen becomes trapped in a phagosome which then fuses with a lysosome to form a phagolysosome. Central to phagocytosis is the maturation and acidification of the phagolysosome, a process which can be readily tracked with a pH sensitive dye, as described herein. Within the phagolysosome, enzymes digest the pathogen. Bacteria, dead tissue cells, and small mineral particles are all examples of objects that may be phagocytized.

**Methods and protocol.** C57BL/6J mice (8-12 weeks of age) (Jackson Laboratory, Bar Harbor, ME, USA) were housed in an animal facility under a 12-hr light/dark cycle and were given standard chow and water *ad libitum.* Bone-marrow-derived macrophages, were isolated from dissected tibiae and femoral bones after the tissue remaining on the bones was removed. The end of each bone was cut off, and the bone marrow was expelled. Cells from bone marrow were cultured for a total of 7 days with 50 ng/ml macrophage colony-stimulating factor, before confocal analysis. Compounds Fc-HRS(2-60), or control compound (N15-(Fc-HRS(2-15) comprising a deleted, non-functional WHEP domain), were added one day after plating of cells at a concentration of either 100 nM or 200 nM. Media was replaced every other day with fresh media including the respective concentrations of test compounds. After 6 days of culture, cells were washed twice with PBS and pulse labelled with 40ug/mL of pHrodo^{™} Red E.coli BioParticles for 15 minutes. After incubation, cells were washed with PBS 3 times and media added back with M-CSF and either Fc-HRS(2-60), or N15 respectively. Cells were collected during the chase phase after 0, 45, 60 and 120 minutes chase. Cells were then washed with PBS, and then fixed with 4% paraformaldehyde and counter stained with Hoechst. Confocal images were captured in Zeiss 710 Confocal Laser Scanning Microscope equipped with 4 lasers and images were captured and analyzed using Zeiss Zen 2010 software. For quantification, the intensity of red fluorescence in each field was measured and the arithmetic mean was calculated and represented graphically.

**Summary of results.** Incubation of bone marrow derived macrophages with either 100nM or 200 nM Fc-HRS(2-60), but not the Fc control compound N15, for 5 days during monocyte differentiation resulted in a profound inhibition of phagocyte maturation, as revealed by the dramatically diminished spectral shift reported by the pH sensitive fluorescent dye pH rhodo^{™} (see **Figures 22A and 22B**). Additional studies at shorter incubations, and different concentrations demonstrated a time and dose dependent inhibition of phagocyte maturation (data not shown). Similar results were also obtained with HRS(2-60)-COMP protein, comprising the pentameric COMP domain (data not shown), confirming that this was a specific effect mediated by the N-terminal region of histidyl t-RNA synthetase (HRS2-60). Bacterial uptake was not significantly impacted by incubation of test compounds (data not shown) by using bacterial particles labelled with a non pH sensitive dye. The profound inhibition of phagocyte acidification closely mimics the phenotype observed in macrophages (Roy et al., (2018) Cancer. Res. 78(19): 5600-5617) and cancer cells in which NRP2 has been knocked out, which also demonstrate endocytic processing blockage resulting in incomplete endosome acidification (Dutta et al., (2015) Cancer. Res. 76 418-28, Neuropilin-2 regulates endosome maturation and EGFR trafficking to support cancer cell pathobiology). These results are of particular significance in cancer treatment because endocytic activity is central to maintaining a metastatic phenotype (Lanzetti & Fiore (2008) Traffic 9, 2011-21 Endocytosis and cancer: an "insider" network with dangerous liaisons).

These results demonstrate that N terminal fragments of HRS, such as the HRS polypeptides Fc-HRS(2-60) and HRS(2-60)-COMP can act, *inter alia,* to functionally block NRP2's ability to mediate endosomal maturation. Because of the importance of endocytic activity to a broad range of cellular activities, including phagocytosis, efferocytosis, autophagy and receptor recycling, these results suggest that HRS polypeptides represent a fundamentally new pharmacological approach for modulating these activities, by interacting with NRP2. Accordingly such HRS polypeptides have potential utility in a broad range of NRP2 associated diseases, and in particular in the treatment of cancer, and modulation of macrophage function.

### EXAMPLE 13

### EVALUATION OF THE ACTIVITY OF FC-HRS(2-60) ON EFFEROCYTOSIS IN MACROPHAGES

This study was designed to investigate the in vivo potential of test compounds to modulate efferocytosis in murine bone marrow derived macrophages. Efferocytosis refers to the process by which dying/dead cells (e.g. apoptotic or necrotic cells) are removed by phagocytic cells. During efferocytosis, the cell membrane of phagocytic cells engulfs the apoptotic cell, forming a large fluid-filled vesicle containing the dead cell. Efferocytosis can be performed not only by 'professional' phagocytic cells such as macrophages or dendritic cells, but also by many other cell types including epithelial cells, fibroblasts and other cells. To distinguish them from living cells, apoptotic cells carry specific 'eat me' signals, such as the presence of phosphatidyl serine (resulting from phospholipid flip-flop) or calreticulin on the outer leaflet of the cell membrane. Efferocytosis uses distinct signaling pathways for the initial recognition of apoptotic cells, but shares many mechanistic similarities to bacterial phagocytosis, endocytosis and autophagy, additionally previous studies have confirmed that NRP2 plays a key regulatory role in these processes (Dutta et al., (2015) Cancer. Res. 76 418-28 Neuropilin-2 regulates endosome maturation and EGFR trafficking to support cancer cell pathobiology). Like phagocytosis, acidification of the endosome, is a central aspect of endosome maturation, which can be readily monitored through the use of a pH sensitive probe, as described herein.

**Methods and protocol.** C57BL/6J mice (8-12 weeks of age) (Jackson Laboratory, Bar Harbor, ME, USA) were housed in an animal facility under a 12-hr light/dark cycle and were given standard chow and water *ad libitum.* Bone-marrow-derived macrophages, were isolated from dissected tibiae and femoral bones after the tissue remaining on the bones was removed. The end of each bone was cut off, and the bone marrow was expelled. Cells from bone marrow were cultured for a total of 7 days with 50 ng/ml macrophage colony-stimulating factor. Media was replaced every other day with fresh media supplemented with M-CSF. At day 5 of culture, the media was replaced with fresh media containing M-CSF and Compounds HRS(2-60)-COMP or control compound (COMP alone, at a concentration of 100 nM for 24 hours. At 6 day of culture, cells were washed twice with PBS and pulse labelled with apoptotic jurkat cells (prepared by treatment of jurkat cells with 50uM etoposide for 12 hours - which had then been pre-loaded with pHrodo^{™} Red succinimidyl ester for 1 hour). After incubation, macrophages were washed with PBS 3 times and media added back with M-CSF along with either HRS (2-60)-COMP or control compound respectively. Cells were collected during the chase phase after 0, 2, 6, 8, and 10 hours chase. Macrophages were then washed with PBS, and then fixed with 4% paraformaldehyde and counter stained with Hoechst. Confocal images were captured in Zeiss 710 Confocal Laser Scanning Microscope equipped with 4 lasers and images were captured and analyzed using Zeiss Zen 2010 software. For quantification, the intensity of red fluorescence in each field were measured and the arithmetic mean was calculated and represented graphically.

**Summary of results.** Incubation of bone marrow derived macrophages with 100nM HRS(2-60)-COMP), but not the control compound COMP, for 5 days during monocyte differentiation resulted in a profound inhibition of efferocytosis, as revealed by the dramatically diminished spectral shift reported by the pH sensitive fluorescent dye pH rhodo^{™} (**Figures 23A and 23B**). The profound inhibition of efferocytosis closely mimics the phenotype observed in macrophages in which NRP2 has been knocked out (Roy et al., (2018) Cancer. Res. 78(19): 5600-5617, Macrophage-Derived Neuropilin-2 Exhibits Novel Tumor-Promoting Functions).

These results demonstrate that N terminal fragments of HRS, such as the HRS polypeptides Fc-HRS(2-60) and HRS(2-60)-COMP have the ability, *inter alia,* to functionally block NRP2's ability to mediate endosomal maturation required for both efferocytosis and phagocytosis. Because of the importance of endocytic activity to a broad range of cellular activities, and diseases, these results suggest that HRS polypeptides represent a fundamentally new pharmacological approach for modulating NRP2 activity. Accordingly such HRS polypeptides have potential utility in a broad range of NRP2 associated diseases, and in particular in the treatment of cancer, and modulation of macrophage function.

### EXAMPLE 14

### EVALUATION OF ACTIVITY OF FC-HRS(2-60) IN A P. ACNES-INDUCED MODEL OF PULMONARY GRANULOMATOUS DISEASE

This study was designed to investigate the *in vivo* potential of test compounds in a murine model of sarcoid-like granulomatous disease. Granuloma formation and lung fibrosis was induced by sensitizing and challenging mice with heat-killed *Propionibacterium acnes* (*P. acnes)* over a four week period.

**Treatment Regimen.** 60 C57BL/6 mice separated into seven groups, housed under standard housing conditions, were used in this study. The *P. acnes* suspension needed to induce pulmonary granuloma formation was generated as follows: *P. acnes* (ATCC #6919) was grown in reinforced clostridial medium or agar for five days under anaerobic conditions at 37°C until confluency. *P. acnes* colonies were washed twice with phosphate buffered saline and resuspended in PBS. The resulting *P*. *acnes* suspension was then heat-killed by autoclaving at 121°C for 20 minutes. The protein concentration of the suspension was determined by Bradford method, and then maintained at -80°C prior to use.

Treatment groups and treatment regimens are shown in **Table E8** below. In brief, animals in group 1-2 were left untreated throughout the study. Animals in groups 3 and 6 - 7 received weekly intravenous injections of the Vehicle of Fc-HRS(2-60) (20 mM Histidine/125 mM NaCl/10 mM Methionine/3% Sucrose/0.02% PS20, pH 6.9) (group 3) or Fc-HRS(2-60) at 0.4 mg/kg (group 6 ) or 3 mg/kg (group 7). Group 4 - 5 mice were dosed with positive control Anti-mouse-TNF-α (group 5) or its vehicle control PBS (group 4) via IP route. On day 0 mice in groups 3 - 7 were first sensitized with 0.5 mg of *P. acnes* via an IP injection and then challenged with an intratracheal (IT) instillation of *P. acnes* on days 14 (0.1 mg) and 28 (0.025 mg).

| **TABLE E8. GROUP TREATMENTS** | | | | | |
|---|---|---|---|---|---|
| **Group** | **#Mice** | ***P. acnes* induction** | **Treatment** | **Start** | **Termination** |
| 1 | 5 | No | Untreated | N/A | Day 42 |
| 2 | 5 | No (PBS) | Untreated | N/A | Day 42 |
| 3 | 10 | Yes (Day 0) | Vehicle, IV QW | Day 0 | Day 42 |
| 4 | 10 | Yes (Day 0) | PBS, IP TIW | Day 0 | Day 42 |
| 5 | 10 | Yes (Day 0) | Anti-mTNF-α, 500 µg, IP TIW | Day 0 | Day 42 |
| 6 | 10 | Yes (Day 0) | Fc-HRS(2-60), 0.4 mg/kg, IV QW | Day 0 | Day 42 |
| 7 | 10 | Yes (Day 0) | Fc-HRS(2-60), 3 mg/kg, IV QW | Day 0 | Day 42 |
| IV, intravenous; IP, intraperitoneal; QW, once weekly; TIW, thrice weekly | | | | | |

**Sample Analysis.** All mice were sacrificed for tissue collection and analysis on day 42 post disease induction. A portion of the lungs was designated for histopathological analysis. Paraffin-embedded sections were stained with H&E or Masson's Trichrome using standard histology procedures and scored for the extent of inflammation and fibrosis by a board-certified veterinary pathologist. In addition, harvested lung tissue was snap-frozen for later protein analysis. Frozen lung tissues were homogenized for 15 sec in 350 µl of ice-cold lysis buffer (150 mM NaCl, 50 mM TRIS pH 7.5, 1 mM EDTA, 0.5% Triton X-100, Protease Inhibitor Cocktail) using a hand-held homogenizer. The tissue samples were being kept on ice during the homogenization procedure. After a 20 min incubation period on ice the lysate was cleared by centrifugation at 13000 rpm at 4°C for 10 min. The protein lysates were then analyzed via the MAGPIX Luminex instrument system using the following kit: MCYTOMAG-70K. One-way ANOVA with Dunn's multiple comparisons test to Vehicle treatment (group 3) was conducted in GraphPad Prism.

**Summary of Results.** A total of ten mice died throughout the study: One mouse died on day 10 and nine died after the first intratracheal dose on day 15. Two group 3 (Vehicle) animals were found dead on day 19, three group 4 (PBS) animals were found dead on day 10, 17 and 19, respectively. Another five mice were found dead in group 6 (Fc-HRS(2-60)); 0.4 mg/kg) on day 19. The mortality in the mice that died after the first IT challenge on day 14 was attributed to the initial intratracheal dosage of heat-killed *P. acnes* (0.1 mg). Based on these adverse events, the dose for the second IT challenge on day 28 was lowered to 0.025 mg and no additional mice were lost.

Successful model induction was confirmed histologically by presence of increased inflammation (H&E) and fibrosis (Masson's Trichrome) in mice that received *P. acnes* (group 3 and 4) as compared to mice that did not receive *P. acnes* (group 2) (see **Figures 24A-24B**). Although the presence of Fc-HRS(2-60) at 0.4 or 3 mg/kg did not lead to a statistically significant overall reduction in lung inflammation or fibrosis at study termination (see **Figures 25A-25B**), several profibrotic cytokines in the lung were reduced in response to Fc-HRS(2-60) treatment at 3 mg/kg (see **Figures 26A-26H**). Anti-TNF antibodies (the positive control) also failed to show significance in this study (data not shown), possibly indicating animal model variation in this study. Of the eight proteins analyzed, IL-6, MCP-1/CCL2 and IFN-γ were significantly reduced as compared to lungs of vehicle treated mice. The protein levels shown in **Figures 26A-26H** are normalized to total lung protein.

These results demonstrate that Fc-HRS(2-60) is a potent inhibitor of several pro-inflammatory lung proteins in this highly inflammatory model of pulmonary sarcoidosis. Controlling inflammation in sarcoidosis is key for managing flare-ups and inhibiting progression to fibrosis, suggesting that Fc-HRS(2-60) could be of potentially therapeutic value in sarcoidosis and other inflammatory lung diseases.

### EXAMPLE 15

### EVALUATION OF ACTIVITY OF Fc-HRS(2-60) IN A MURINE MODEL OF S. RECTIVIRGULA-INDUCED CHRONIC HYPERSENSITIVITY PNEUMONITIS

This study was designed to investigate the *in vivo* potential of Fc-HRS(2-60) in a murine model of *Saccharopolyspora rectivirgula* (S. rectivirgula, S. rec)-induced chronic hypersensitivity pneumonitis (CHP).

**Treatment Regimen.** Sixty C57BL/6 female mice were assigned to study groups of five or ten mice, and housed under standard housing conditions. *S. rectivirgula* antigen required for disease induction was prepared as follows: *S. rectivirgula* was obtained from the American Type Cultures Collection (ATCC #15347), and cultivated in 1L ATCC Medium 3 Broth at ~52°C in a shaking incubator for 4 days, centrifuged, and washed three times with water. The bacterial cell walls were disrupted with a homogenizer or sonication and the resulting *S. rectivirgula* antigen was then reconstituted in saline at a concentration of 1 mg/mL and kept at -80°C until use.

The treatment regimens are shown in **Table E9** below. In brief, animals in group 1 - 2 were left untreated throughout the study. Animals in groups 3 and 6 - 7 received weekly intravenous injections of the Vehicle of Fc-HRS(2-60) (20 mM Histidine/125 mM NaCl/10 mM Methionine/3% Sucrose/0.02% PS20, pH 6.9) (group 3) or Fc-HRS(2-60) at 0.4 mg/kg (group 6) or 3 mg/kg (group 7). Mice in group 5 were dosed with positive control CTLA-4-Fc (IP) and mice in group 4 with the corresponding vehicle control PBS (IP). The disease was induced in groups 2 - 7 via intranasal instillation of 25 µl S. *rectivirgula* antigen (1 mg/ml) or PBS on three consecutive days per week for three weeks.

| **TABLE E9. GROUP TREATMENTS** | | | | | |
|---|---|---|---|---|---|
| **Group** | **#Mice** | ***S. rec* induction** | **Treatment** | **Start** | **Termination** |
| 1 | 5 | No | Untreated | N/A | Day 20 |
| 2 | 5 | No (PBS) | Untreated | N/A | Day 20 |
| 3 | 10 | Yes (Day 0) | Vehicle, IV QW | Day -1 | Day 20 |
| 4 | 10 | Yes (Day 0) | PBS, IP TIW | Day -1 | Day 20 |
| 5 | 10 | Yes (Day 0) | CTLA-4-Fc, 50 µg, IP TIW | Day -1 | Day 20 |
| 6 | 10 | Yes (Day 0) | Fc-HRS(2-60), 0.4 mg/kg, IV QW | Day -1 | Day 20 |
| 7 | 10 | Yes (Day 0) | Fc-HRS(2-60), 3 mg/kg, IV QW | Day -1 | Day 20 |
| IV, intravenous; IP, intraperitoneal; QW, once weekly; TIW, thrice weekly | | | | | |

**Sample Analysis.** All mice were sacrificed for tissue collection and analysis on day 20 post disease induction. A portion of the lungs was designated for histopathological analysis. Paraffin-embedded sections were stained with H&E for examination by a board-certified veterinary pathologist. Presence and severity of multifocal chronic pneumonia was scored using the accepted industry scoring system described in Mann, et al. (2012) 'International Harmonization of Toxicologic Pathology Nomenclature: An Overview and Review of Basic Principles', Toxicologic Pathology, 40(4_suppl), pp. 7S-13S. doi: 10.1177/0192623312438738). H&E stained lung sections were also analyzed using the HALO platform in order to quantify bronchus-associated lymphoid tissue (BALT). In addition, a portion of harvested lung tissue was snap-frozen for later protein analysis. Frozen lung tissues were homogenized for 15 s in 350 µl of ice-cold lysis buffer (150 mM NaCl, 50 mM TRIS pH 7.5, 1 mM EDTA, 0.5% Triton X-100, Protease Inhibitor Cocktail) using a hand-held homogenizer. The tissue samples were being kept on ice during the homogenization procedure. After a 20 min incubation period on ice the lysate was cleared by centrifugation at 13000 rpm at 4°C for 10 min. The protein lysates were then analyzed via the MAGPIX Luminex instrument system using the following kits: MCYTOMAG-70K, MMMP3MAG-79K.

One-way ANOVA with Dunn's multiple comparisons test to vehicle treatment (group 3) was conducted in GraphPad Prism.

**Summary of results.** Chronic hypersensitivity pneumonitis was successfully induced with 25µg intranasal challenges of *S*. *rectivirgula* administered on three consecutive days per week for three weeks. Mice exposed to *S. rectivirgula* in control groups (Group 3 and Group 4) had a robust and consistent multifocal chronic pneumonia compared to mice exposed to PBS in Group 2 (**Figure 27A**). The mean histopathology scores as determined by a veterinary pathologist were similar among groups 3 - 7 (**Figure 27B**). However, a following in-depth analysis of the H&E stained lung tissue sections using the HALO platform showed a reduction of individual BALT area in the Fc-HRS(2-60) 3 mg/kg group (**Figure 27C****)** suggesting anti-inflammatory activity in this model. Further, lung homogenates were analyzed for levels of pro-inflammatory and profibrotic proteins. Several pro-inflammatory cytokines and chemokines (see **Figures 28A-28G****)** as well as matrix metalloproteases (MMPs) (see **Figures 29A-29E****)** were significantly reduced in presence of Fc-HRS(2-60) at both 0.4 and 3 mg/kg. These results demonstrate that Fc-HRS(2-60) negatively regulates lung proteins that are implicated in the development of fibrosis. This broad anti-inflammatory effect may be translatable to human patients suffering of similar, highly inflammatory types of interstitial lung diseases.

### EXAMPLE 16

### EVALUATION OF ACTIVITY OF Fc-HRS(2-60) IN A MURINE MODEL OF RA-ILD

This study was designed to investigate the *in vivo* activity of Fc-HRS(2-60) in a transgenic mouse model (SKG) of Rheumatoid-Arthritis Interstitial Lung Disease (RA-ILD). SKG mice, which are genetically prone to develop autoimmune arthritis, develop severe chronic arthritis after a single intraperitoneal injection of zymosan, and approximately 20% of these SKG mice also develop ILD.

**Treatment Regimen.** 70 SKG/jcl female mice were assigned to seven study groups, and housed under standard housing conditions. The study groups are described in **Table E10** below. In brief, animals in group 1 were left untreated throughout the study. Mice in groups 3 were dosed with positive control Anti-GM-CSF (BioXcell, # and mice in group 2 were dosed with the corresponding IgG control. Mice in groups 4 - 7 received weekly intravenous injections of the vehicle for Fc-HRS(2-60) (20 mM Histidine/125 mM NaCl/10 mM Methionine/3% Sucrose/0.02% PS20, pH 6.9) (group 4) or Fc-HRS(2-60) at 0.3 mg/kg (group 5), 1 mg/kg (group 6), or 3 mg/kg (group 7). Arthritis was induced in groups 2 - 7 on day 0 via IP administration of 5 mg zymosan (Sigma, #Z4250).

| **TABLE E10. GROUP TREATMENTS** | | | | | |
|---|---|---|---|---|---|
| **Group** | **#Mice** | **Disease induction** (Day 0) | **Treatment** | **Start** | **Termination** |
| 1 | 10 | No | Untreated | N/A | Day 56 |
| 2 | 10 | Zymosan | Rat IgG2a, 100 µg, IP QW | Day -1 | Day 56 |
| 3 | 10 | Zymosan | Anti-GM-CSF, 100 µg, IP QW | Day -1 | Day 56 |
| 4 | 10 | Zymosan | Vehicle, IV QW | Day -1 | Day 56 |
| 5 | 10 | Zymosan | Fc-HRS(2-60), 0.3 mg/kg, IV QW | Day -1 | Day 56 |
| 6 | 10 | Zymosan | Fc-HRS(2-60), 1 mg/kg, IV QW | Day -1 | Day 56 |
| 7 | 10 | Zymosan | Fc-HRS(2-60), 3 mg/kg, IV QW | Day -1 | Day 56 |
| IV, intravenous; IP, intraperitoneal; QW, once weekly | | | | | |

**Clinical Scoring and Sample Analysis.** Body weights and scoring for arthritis was conducted once a week. For assessing the extent of arthritis, each limb was scored separately and clinical points were assigned as follows: 0 = no joint swelling, 0.1 = swelling of one finger joint, 0.5 = mild swelling of wrist and ankle, 0.75 = moderate swelling of wrist and ankle and 1 = severe swelling of wrist and ankle. At study termination on day 56, lungs were perfused with PBS, excised, and dissociated for flow cytometric analysis using Miltenyi's lung dissociation kit (#130-095-927).

**Flow Cytometry Analysis.** Flow wash buffer (FWB, 3% fetal bovine serum in PBS) was used for all subsequent staining and washing steps. Live/dead cells were distinguished by staining with 120 µL of Zombie Yellow (BioLegend) diluted 1:400 in FWB for 20 min in the dark at room temperature. An excess of FWB was then added, and the sample was centrifuged as above; these steps are henceforth collectively referred to as "washing". Fc receptors were blocked by adding 25 µL of Mouse BD Fc Block (BD Biosciences) diluted 1:25 in FWB for 10 min in the dark at room temperature. Subsequently, cells were stained for specific surface markers using 50 µL of a mixture of the antibodies listed in **Table E11,** all in FWB for 30 min in the dark on ice. Cells were then again washed, resuspended in 200 µL of FWB, and acquired on a CytoFLEX flow cytometer (Beckman Coulter). Only three of the study groups were analyzed by flow cytometry: group 1, group 4 and group 7.

| **TABLE E11. FLOW CYTOMETRY STAINING ANTIBODIES** | | |
|---|---|---|
| **Marker** | **Dilution in FWB (Staining Mixture)** | **Fluorophore*** |
| CD3 | 1:200 | APC-Cy7 |
| CD4 | 1:200 | PerCpCy5.5 |
| CD19 | 1:200 | Alexa700 |
| CD8 | 1:200 | BV650 |
| F4/80 | 1:100 | BV510 |
| CD11b | 1:200 | PE-Cy7 |
| CD11c | 1:200 | FITC |
| NK1.1 | 1:100 | PE |
| GR-1 | 1:100 | BV421 |
| CD64 | 1:100 | PE/Dazzle594 |
| Live/Dead (Zombie Yellow) | 1:400 | BV570 |
| Conjugated antibodies were obtained from BD Biosciences or BioLegend. | | |

**Summary of results.** Inflammatory arthritis was successfully induced in SKG mice upon administration of 5 mg of zymosan, and flow cytometric analysis of lung single cell suspensions, demonstrated that Fc-HRS(2-60) can lower the number of specific immune cells in the lungs of SKG mice; most notably B cells and T cells (see **Figures 30A-30H**). Despite significant effects on immune cell infiltration, no significant effects of Fc-HRS(2-60) on overall clinical arthritis scores were observed in this experiment (see **Figures 31A-31D**). Both B and T cells are implicated in RA-ILD pathogenesis and the observed reduction of their numbers in the lung of diseased mice may provide an avenue for potential therapeutic use of Fc-HRS(2-60) in RA-ILD or other T-cell driven inflammatory diseases, either alone, or in combination with other agents.

## Claims

1. A therapeutic composition comprising a histidyl-tRNA synthetase (HRS) polypeptide for use in treating a neuropilin-2 (NRP2) associated cancer in a subject in need thereof, wherein the NRP2 associated cancer expresses or overexpresses NRP2, and wherein the HRS polypeptide comprises, consists, or consists essentially of an amino acid sequence that is at least 90%, 95%, 96%, 97%, 98%, 99%, or 100% identical to a sequence selected from SEQ ID NOs: 156-171.

2. The therapeutic composition for use according to claim 1, wherein the cancer displays NRP2-dependent growth, NRP2-dependent adhesion, NRP2-dependent migration, NRP2-dependent chemoresistance, and/or NRP2-dependent invasion.

3. The therapeutic composition for use according to claim 2, wherein the cancer is a primary cancer, or wherein the cancer is a metastatic cancer, including a metastatic cancer that expresses NRP2a and/or NRP2b.

4. The therapeutic composition for use according to claim 2 or 3, wherein the cancer is chemoresistant to at least one cancer therapy selected from an immunotherapy agent, a chemotherapeutic agent, a hormonal therapeutic agent, and a kinase inhibitor, optionally wherein the method comprises selecting a subject having a cancer that is chemoresistant prior to administering the HRS polypeptide.

5. The therapeutic composition for use according to any one of claims 2-4, wherein the HRS polypeptide modulates autophagy, efferocytosis, or phagocyte maturation in a cancer cell or cancer-associated macrophage, optionally wherein the HRS polypeptide modulates autophagy in the cancer cell.

6. The therapeutic composition for use according to any one of claims 1-5, wherein the cancer is selected from one or more of melanoma (e.g., metastatic melanoma), pancreatic cancer, bone cancer, prostate cancer, small cell lung cancer, non-small cell lung cancer (NSCLC), mesothelioma, leukemia (e.g., lymphocytic leukemia, chronic myelogenous leukemia, acute myeloid leukemia, relapsed acute myeloid leukemia), lymphoma, hepatoma (hepatocellular carcinoma), sarcoma, B-cell malignancy, breast cancer, ovarian cancer, colorectal cancer, glioma, glioblastoma multiforme, meningioma, pituitary adenoma, vestibular schwannoma, primary CNS lymphoma, primitive neuroectodermal tumor (medulloblastoma), kidney cancer (e.g., renal cell carcinoma), bladder cancer, uterine cancer, esophageal cancer, brain cancer, head and neck cancers, cervical cancer, testicular cancer, thyroid cancer, and stomach cancer.

7. The therapeutic composition for use according to claim 5 or 6, wherein the metastatic cancer is selected from one or more of:
(a) a bladder cancer which has metastasized to the bone, liver, and/or lungs;
(b) a breast cancer which has metastasized to the bone, brain, liver, and/or lungs;
(c) a colorectal cancer which has metastasized to the liver, lungs, and/or peritoneum;
(d) a kidney cancer which has metastasized to the adrenal glands, bone, brain, liver, and/or lungs;
(e) a lung cancer which has metastasized to the adrenal glands, bone, brain, liver, and/or other lung sites;
(f) a melanoma which has metastasized to the bone, brain, liver, lung, and/or skin/muscle;
(g) a ovarian cancer which has metastasized to the liver, lung, and/or peritoneum;
(h) a pancreatic cancer which has metastasized to the liver, lung, and/or peritoneum;
(i) a prostate cancer which has metastasized to the adrenal glands, bone, liver, and/or lungs;
(j) a stomach cancer which has metastasized to the liver, lung, and/or peritoneum;
(l) a thyroid cancer which has metastasized to the bone, liver, and/or lungs; and
(m) a uterine cancer which has metastasized to the bone, liver, lung, peritoneum, and/or vagina.

8. The therapeutic composition for use according to any one of claims 1-7, comprising administering to the subject at least one additional agent selected from one or more of a cancer immunotherapy agent comprising an immune checkpoint modulatory agent, comprising an antagonist of a inhibitory immune checkpoint molecule and an agonist of a stimularoty immune checkpoint molecule, a chemotherapeutic agent, a hormonal therapeutic agent, and a kinase inhibitor.

9. The therapeutic composition for use according to claim 8, wherein the inhibitory immune checkpoint molecule is selected from one or more of Programmed Death-Ligand 1 (PD-L1), Programmed Death 1 (PD-1), Programmed Death-Ligand 2 (PD-L2), Cytotoxic T-Lymphocyte-Associated protein 4 (CTLA-4), Indoleamine 2,3-dioxygenase (IDO), tryptophan 2,3-dioxygenase (TDO), T-cell Immunoglobulin domain and Mucin domain 3 (TIM-3), Lymphocyte Activation Gene-3 (LAG-3), V-domain Ig suppressor of T cell activation (VISTA), B and T Lymphocyte Attenuator (BTLA), CD160, Herpes Virus Entry Mediator (HVEM), and T-cell immunoreceptor with Ig and ITIM domains (TIGIT), including wherein
the antagonist is a PD-L1 and/or PD-L2 antagonist optionally selected from one or more of an antibody or antigen-binding fragment or small molecule that specifically binds thereto, atezolizumab (MPDL3280A), avelumab (MSB0010718C), and durvalumab (MEDI4736), optionally wherein the cancer is selected from one or more of colorectal cancer, melanoma, breast cancer, non-small-cell lung carcinoma, bladder cancer, and renal cell carcinoma;
the antagonist is a PD-1 antagonist optionally selected from one or more of an antibody or antigen-binding fragment or small molecule that specifically binds thereto, nivolumab, pembrolizumab, MK-3475, AMP-224, AMP-514PDR001, and pidilizumab, optionally wherein the PD-1 antagonist is nivolumab and the cancer is optionally selected from one or more of Hodgkin's lymphoma, melanoma, non-small cell lung cancer, hepatocellular carcinoma, renal cell carcinoma, and ovarian cancer;
the PD-1 antagonist is pembrolizumab and the cancer is optionally selected from one or more of melanoma, non-small cell lung cancer, small cell lung cancer, head and neck cancer, and urothelial cancer;
the antagonist is a CTLA-4 antagonist optionally selected from one or more of an antibody or antigen-binding fragment or small molecule that specifically binds thereto, ipilimumab, tremelimumab, optionally wherein the cancer is selected from one or more of melanoma, prostate cancer, lung cancer, and bladder cancer;
the antagonist is an IDO antagonist optionally selected from one or more of an antibody or antigen-binding fragment or small molecule that specifically binds thereto, indoximod (NLG-8189), 1-methyl-tryptophan (1MT), β-Carboline (norharmane; 9H-pyrido[3,4-b]indole), rosmarinic acid, and epacadostat, and wherein the cancer is optionally selected from one or more of metastatic breast cancer and brain cancer optionally glioblastoma multiforme, glioma, gliosarcoma or malignant brain tumor;
the antagonist is a TDO antagonist optionally selected from one or more of an antibody or antigen-binding fragment or small molecule that specifically binds thereto, 680C91, and LM10;
the antagonist is a TIM-3 antagonist optionally selected from one or more of an antibody or antigen-binding fragment or small molecule that specifically binds thereto;
the antagonist is a LAG-3 antagonist optionally selected from one or more of an antibody or antigen-binding fragment or small molecule that specifically binds thereto, and BMS-986016;
the antagonist is a VISTA antagonist optionally selected from one or more of an antibody or antigen-binding fragment or small molecule that specifically binds thereto;
the antagonist is a BTLA, CD160, and/or HVEM antagonist optionally selected from one or more of an antibody or antigen-binding fragment or small molecule that specifically binds thereto; or wherein the antagonist is a TIGIT antagonist optionally selected from one or more of an antibody or antigen-binding fragment or small molecule that specifically binds thereto.

10. The therapeutic composition for use according to claim 8, wherein the stimulatory immune checkpoint molecule is selected from one or more of OX40, CD40, Glucocorticoid-Induced TNFR Family Related Gene (GITR), CD137 (4-1BB), CD27, CD28, CD226, and Herpes Virus Entry Mediator (HVEM), including wherein
the agonist is an OX40 agonist optionally selected from one or more of an antibody or antigen-binding fragment or small molecule or ligand that specifically binds thereto, OX86, Fc-OX40L, and GSK3174998;
the agonist is a CD40 agonist optionally selected from one or more of an antibody or antigen-binding fragment or small molecule or ligand that specifically binds thereto, CP-870,893, dacetuzumab, Chi Lob 7/4, ADC-1013, and rhCD40L, and wherein the cancer is optionally selected from one or more of melanoma, pancreatic carcinoma, mesothelioma, and hematological cancers optionally lymphoma such as Non-Hodgkin's lymphoma;
the agonist is a GITR agonist optionally selected from one or more of an antibody or antigen-binding fragment or small molecule or ligand that specifically binds thereto, INCAGN01876, DTA-1, and MEDI1873;
the agonist is a CD137 agonist optionally selected from one or more of an antibody or antigen-binding fragment or small molecule or ligand that specifically binds thereto, utomilumab, and 4-1BB ligand;
the agonist is a CD27 agonist optionally selected from one or more of an antibody or antigen-binding fragment or small molecule or ligand that specifically binds thereto, varlilumab, and CDX-1127 (1F5);
the agonist is a CD28 agonist optionally selected from one or more of an antibody or antigen-binding fragment or small molecule or ligand that specifically binds thereto, and TAB08; and/or
the agonist is an HVEM agonist optionally selected from one or more of an antibody or antigen-binding fragment or small molecule or ligand that specifically binds thereto.

11. The therapeutic composition for use according to claim 8, wherein the at least one chemotherapeutic agent is selected from one or more of an alkylating agent, an anti-metabolite, a cytotoxic antibiotic, a topoisomerase inhibitor (type 1 or type II), and an anti-microtubule agent, including wherein:
the alkylating agent is selected from one or more of nitrogen mustards (optionally mechlorethamine, cyclophosphamide, mustine, melphalan, chlorambucil, ifosfamide , and busulfan), nitrosoureas (optionally N-Nitroso-N-methylurea (MNU), carmustine (BCNU), lomustine (CCNU), semustine (MeCCNU), fotemustine, and streptozotocin), tetrazines (optionally dacarbazine, mitozolomide, and temozolomide), aziridines (optionally thiotepa, mytomycin, and diaziquone (AZQ)), cisplatins and derivatives thereof (optionally carboplatin and oxaliplatin), and non-classical alkylating agents (optionally procarbazine and hexamethylmelamine);
wherein the anti-metabolite is selected from one or more of anti-folates (optionally methotrexate and pemetrexed), fluoropyrimidines (optionally 5- fluorouracil and capecitabine), deoxynucleoside analogues (optionally ancitabine, enocitabine, cytarabine, gemcitabine, decitabine, azacitidine, fludarabine, nelarabine, cladribine, clofarabine, fludarabine, and pentostatin), and thiopurines (optionally thioguanine and mercaptopurine);
wherein the cytotoxic antibiotic is selected from one or more of anthracyclines (optionally doxorubicin, daunorubicin, epirubicin, idarubicin, pirarubicin, aclarubicin, and mitoxantrone), bleomycins, mitomycin C, mitoxantrone, and actinomycin;
wherein the topoisomerase inhibitor is selected from one or more of camptothecin, irinotecan, topotecan, etoposide, doxorubicin, mitoxantrone, teniposide, novobiocin, merbarone, and aclarubicin; and whrerin the anti-microtubule agent is selected from one or more of taxanes (optionally paclitaxel and docetaxel) and vinca alkaloids (optionally vinblastine, vincristine, vindesine, vinorelbine);
wherein the at least one hormonal therapeutic agent is a hormonal agonist or hormonal antagonist; wherein the hormonal agonist is selected from one or more of a progestogen (progestin), a corticosteroid (optionally prednisolone, methylprednisolone, or dexamethasone), insulin like growth factors, VEGF derived angiogenic and lymphangiogenic factors (optionally VEGF-A, VEGF-A145, VEGF-A165, VEGF-C, VEGF-D, PIGF-2), fibroblast growth factor (FGF), galectin, hepatocyte growth factor (HGF), platelet derived growth factor (PDGF), transforming growth factor (TGF)-beta, an androgen, an estrogen, and a somatostatin analog; and wherein
the hormonal antagonist is selected from one or more of a hormone synthesis inhibitor, optionally an aromatase inhibitor or a gonadotropin-releasing hormone (GnRH) or an analog thereof, and a hormone receptor antagonist, optionally a selective estrogen receptor modulator (SERM) or an anti-androgen, or an antibody directed against a hormonal receptor, optionally cixutumumab, dalotuzumab, figitumumab, ganitumab, istiratumab, robatumumab, alacizumab pegol, bevacizumab, icrucumab, ramucirumab, fresolimumab, metelimumab, naxitamab, cetuximab, depatuxizumab mafodotin, futuximab, imgatuzumab, laprituximab emtansine, matuzumab, modotuximab, necitumumab, nimotuzumab, panitumumab, tomuzotuximab, zalutumumab, aprutumab ixadotin, bemarituzumab, olaratumab, or tovetumab; or
wherein the kinase inhibitor is selected from one or more of adavosertib, afanitib, aflibercept, axitinib, bevacizumab, bosutinib, cabozantinib, cetuximab, cobimetinib, crizotinib, dasatinib, entrectinib, erdafitinib, erlotinib, fostamitinib, gefitinib, ibrutinib, imatinib, lapatinib, lenvatinib, mubritinib, nilotinib, panitumumab, pazopanib, pegaptanib, ponatinib, ranibizumab, regorafenib, ruxolitinib, sorafenib, sunitinib, SU6656, tofacitinib, trastuzumab, vandetanib, and vemuafenib, or wherein the kinase inhibitor is a PI3 kinase inhibitor selected from one or more of alpelisib, buparlisib, copanlisib, CUDC-907, dactolisib, duvelisib, GNE-477, idelasib, IPI-549, LY294002, ME-401, perifosine, PI-103, pictilisib, PWT33597, RP6503, taselisib, umbralisib, voxtalisib, wortmannin, and XL147.

12. The therapeutic composition for use according to any one of claims 1-11, wherein the HRS polypeptide comprises, consists, or consists essentially of an amino acid sequence that is at least 95%, 96%, 97%, 98%, 99%, or 100% identical to SEQ ID NO:156 (Fc-HRS(2-60) or HRS^{FC1}).

13. The therapeutic composition for use according to claim 12, wherein the HRS polypeptide comprises SEQ ID NO: 156 (Fc-HRS(2-60) or **HRS**^{FC1}).

## Patentansprüche

1. Eine therapeutische Zusammensetzung umfassend ein Histidyl-tRNA-Synthetase- (HRS-) Polypeptid zur Verwendung in der Behandlung von Neuropilin-2- (NRP2-) assoziiertem Krebs in einem Subjekt, der dessen bedarf, wobei der NRP2-assoziierte Krebs NRP2 exprimiert oder überexprimiert, und wobei das HRS-Polypeptid eine Aminosäuresequenz umfasst, daraus besteht oder im Wesentlichen daraus besteht, die mindestens 90%, 95%, 96%, 97%, 98%, 99% oder 100% identisch zu einer Sequenz ausgewählt aus SEQ ID NOs: 156-171 ist.

2. Die therapeutische Zusammensetzung zur Verwendung gemäß Anspruch 1, wobei der Krebs NRP2-abhängiges Wachstum, NRP2-abhängige Adhäsion, NRP2-abhängige Migration, NRP2-abhängige Chemoresistenz und/oder NRP2-abhängige Invasion aufweist.

3. Die therapeutische Zusammensetzung zur Verwendung gemäß Anspruch 2, wobei der Krebs ein primärer Krebs ist oder wobei der Krebs ein metastatischer Krebs ist, einschließlich eines metastatischen Krebses, der NRP2a und/oder NRP2b exprimiert.

4. Die therapeutische Zusammensetzung zur Verwendung gemäß Anspruch 2 oder 3, wobei der Krebs gegen mindestens eine Krebstherapie ausgewählt aus einem immuntherapeutischen Agens, einem chemotherapeutischen Agens, einem hormontherapeutischen Agens und einem Kinaseinhibitor chemoresistent ist, optional wobei das Verfahren Auswählen eines Subjekts mit einem Krebs, der chemoresistent ist, vor Verabreichen des HRS-Polypeptids umfasst.

5. Die therapeutische Zusammensetzung zur Verwendung gemäß irgendeinem der Ansprüche 2-4, wobei das HRS-Polypeptid Autophagie, Efferozytose oder Phagozytenreifung in einer Krebszelle oder einem Krebs-assoziierten Makrophagen moduliert, optional wobei das HRS-Polypeptid Autophagie in der Krebszelle moduliert.

6. Die therapeutische Zusammensetzung zur Verwendung gemäß irgendeinem der Ansprüche 1-5, wobei der Krebs ausgewählt ist aus einem oder mehr von Melanom (z.B. metastatischem Melanom), Bauchspeicheldrüsenkrebs, Knochenkrebs, Prostatakrebs, kleinzelligem Lungenkrebs, nicht-kleinzelligem Lungenkrebs (NSCLC), Mesotheliom, Leukämie (z.B. lymphozytischer Leukämie, chronischer myelogener Leukämie, akuter myeloischer Leukämie, rezidivierter akuter myeloischer Leukämie), Lymphom, Hepatom (hepatozellulärem Karzinom), Sarkom, B-Zell-Malignom, Brustkrebs, Eierstockkrebs, Kolorektalkrebs, Gliom, Glioblastoma multiforme, Meningiom, Hypophysenadenom, Vestibularis-Schwannom, primärem ZNS-Lymphom, primitivem neuroektodermalem Tumor (Medulloblastom), Nierenkrebs (z.B. Nierenzellkarzinom), Blasenkrebs, Gebärmutterkrebs, Speiseröhrenkrebs, Hirnkrebs, Kopf-Hals-Krebsen, Gebärmutterhalskrebs, Hodenkrebs, Schilddrüsenkrebs und Magenkrebs.

7. Die therapeutische Zusammensetzung zur Verwendung gemäß Anspruch 5 oder 6, wobei der metastatische Krebs ausgewählt ist aus einem oder mehr von:
(a) einem Blasenkrebs, der in Knochen, Leber und/oder Lunge metastasiert hat;
(b) einem Brustkrebs, der in Knochen, Hirn, Leber und/oder Lunge metastasiert hat;
(c) einem Kolorektalkrebs, der in Leber, Lunge und/oder Peritoneum metastasiert hat;
(d) einem Nierenkrebs, der in Nebennieren, Knochen, Hirn, Leber und/oder Lunge metastasiert hat;
(e) einem Lungenkrebs, der in Nebennieren, Knochen, Hirn, Leber und/oder andere Lungenstellen metastasiert hat;
(f) einem Melanom, der in Knochen, Hirn, Leber, Lunge und/oder Haut/Muskel metastasiert hat;
(g) einem Eierstockkrebs, der in Leber, Lunge und/oder Peritoneum metastasiert hat;
(h) einem Bauchspeicheldrüsenkrebs, der in Leber, Lunge und/oder Peritoneum metastasiert hat;
(i) einem Prostatakrebs, der in Nebennieren, Knochen, Leber und/oder Lunge metastasiert hat;
(j) einem Magenkrebs, der in Leber, Lunge und/oder Peritoneum metastasiert hat;
(l) einem Schilddrüsenkrebs, der in Knochen, Leber und/oder Lunge metastasiert hat; und
(m) einem Gebärmutterkrebs, der in Knochen, Leber, Lunge, Peritoneum und/oder Scheide metastasiert hat.

8. Die therapeutische Zusammensetzung zur Verwendung gemäß irgendeinem der Ansprüche 1-7, umfassend Verabreichen an das Subjekt von mindestens einem zusätzlichen Agens ausgewählt aus einem oder mehr von einem Krebsimmuntherapieagens umfassend ein Immuncheckpointmodulierendes Agens, umfassend einen Antagonisten eines inhibitorischen Immuncheckpointmoleküls und einen Agonisten eines stimulatorischen Immuncheckpointmoleküls, einem chemotherapeutischen Agens, einem hormontherapeutischen Agens und einem Kinaseinhibitor.

9. Die therapeutische Zusammensetzung zur Verwendung gemäß Anspruch 8, wobei das inhibitorische Immuncheckpointmolekül ausgewählt ist aus einem oder mehr von Programmed Death-Ligand 1 (PD-L1), Programmed Death 1 (PD-1), Programmed Death-Ligand 2 (PD-L2), zytotoxische T-Lymphozyten-assoziiertem Protein 4 (CTLA-4), Indoleamin 2,3-Dioxygenase (IDO), Tryptophan 2,3-Dioxygenase (TDO), T-Zell-Immunglobulindomäne und Mucindomäne 3 (TIM-3), Lymphozyten-Aktivierungsgen-3 (LAG-3), V-Domäne-Ig-Suppressor der T-Zell-Aktivierung (VISTA), B- und T-Lymphozyten-Attenuator (BTLA), CD160, Herpesvirus-Eintrittsmediator (HVEM) und T-Zell-Immunrezeptor mit Ig- und ITIM-Domänen (TIGIT), einschließlich wobei
der Antagonist ein PD-L1 und/oder PD-L2-Antagonist ist, optional ausgewählt aus einem oder mehr von einem Antikörper oder antigenbindenden Fragment oder kleinem Molekül, das spezifisch daran bindet, Atezolizumab (MPDL3280A), Avelumab (MSB0010718C) und Durvalumab (MEDI4736), optional wobei der Krebs ausgewählt ist aus einem oder mehr von Kolorektalkrebs, Melanom, Brustkrebs, nicht-kleinzelligem Lungenkarzinom, Blasenkrebs und Nierenzellkarzinom;
der Antagonist ein PD-1-Antagonist ist, optional ausgewählt aus einem oder mehr von einem Antikörper oder antigenbindenden Fragment oder kleinem Molekül, das spezifisch daran bindet, Nivolumab, Pembrolizumab, MK-3475, AMP-224, AMP-514PDR001 und Pidilizumab, optional wobei der PD-1-Antagonist Nivolumab ist und der Krebs optional ausgewählt ist aus einem oder mehr von Hodgkin-Lymphom, Melanom, nicht-kleinzelligem Lungenkrebs, Leberzellkarzinom, Nierenzellkarzinom und Eierstockkrebs;
der PD-1-Antagonist Pembrolizumab ist und der Krebs optional ausgewählt ist aus einem oder mehr von Melanom, nicht-kleinzelligem Lungenkrebs, kleinzelligem Lungenkrebs, Kopf-Hals-Krebs und Urothelkrebs;
der Antagonist ein CTLA-4-Antagonist ist, optional ausgewählt aus einem oder mehr von einem Antikörper oder antigenbindenden Fragment oder kleinem Molekül, das spezifisch daran bindet, Ipilimumab, Tremelimumab, optional wobei der Krebs ausgewählt ist aus einem oder mehr von Melanom, Prostatakrebs, Lungenkrebs und Blasenkrebs;
der Antagonist ein IDO-Antagonist ist, optional ausgewählt aus einem oder mehr von einem Antikörper oder antigenbindenden Fragment oder kleinem Molekül, das spezifisch daran bindet, Indoximod (NLG-8189), 1-Methyltryptophan (1MT), β-Carbolin (Norharman; 9H-Pyrido[3,4-b]indol), Rosmarinsäure und Epacadostat, und wobei der Krebs optional ausgewählt ist aus einem oder mehr von metastatischem Brustkrebs und Hirnkrebs, optional Glioblastoma multiforme, Gliom, Gliosarkom oder bösartigem Hirntumor;
der Antagonist ein TDO-Antagonist ist, optional ausgewählt aus einem oder mehr von einem Antikörper oder antigenbindenden Fragment oder kleinem Molekül, das spezifisch daran bindet, 680C91 und LM10;
der Antagonist ein TIM-3-Antagonist ist, optional ausgewählt aus einem oder mehr von einem Antikörper oder antigenbindenden Fragment oder kleinem Molekül, das spezifisch daran bindet;
der Antagonist ein LAG-3-Antagonist ist, optional ausgewählt aus einem oder mehr von einem Antikörper oder antigenbindenden Fragment oder kleinem Molekül, das spezifisch daran bindet, und BMS-986016;
der Antagonist ein VISTA-Antagonist ist, optional ausgewählt aus einem oder mehr von einem Antikörper oder antigenbindenden Fragment oder kleinem Molekül, das spezifisch daran bindet;
der Antagonist ein BTLA-, CD160- und/oder HVEM-Antagonist ist, optional ausgewählt aus einem oder mehr von einem Antikörper oder antigenbindenden Fragment oder kleinem Molekül, das spezifisch daran bindet; oder wobei der Antagonist ein TIGIT-Antagonist ist, optional ausgewählt aus einem oder mehr von einem Antikörper oder antigenbindenden Fragment oder kleinem Molekül, das spezifisch daran bindet.

10. Die therapeutische Zusammensetzung zur Verwendung gemäß Anspruch 8, wobei das stimulatorische Immuncheckpointmolekül ausgewählt ist aus einem oder mehr von OX40, CD40, Glucokortikoid-induziertem TNFR-Familie-verwandtem Gen (GITR), CD137 (4-1BB), CD27, CD28, CD226 und Herpesvirus-Eintrittsmediator (HVEM), einschließlich wobei
der Agonist ein OX40-Agonist ist, optional ausgewählt aus einem oder mehr von einem Antikörper oder antigenbindenden Fragment oder kleinem Molekül oder Liganden, das/der spezifisch daran bindet, OX86, Fc-OX40L und GSK3174998;
der Agonist ein CD40-Agonist ist, optional ausgewählt aus einem oder mehr von einem Antikörper oder antigenbindenden Fragment oder kleinem Molekül oder Liganden, das/der spezifisch daran bindet, CP-870,893, Dacetuzumab, Chi Lob 7/4, ADC-1013 und rhCD40L, und wobei der Krebs optional ausgewählt ist aus einem oder mehr von Melanom, Bauchspeicheldrüsenkarzinom, Mesotheliom und hämatologischen Krebsen, optional Lymphom wie etwa Nicht-Hodgkin-Lymphom;
der Agonist ein GITR-Agonist ist, optional ausgewählt aus einem oder mehr von einem Antikörper oder antigenbindenden Fragment oder kleinem Molekül oder Liganden, das/der spezifisch daran bindet, INCAGN01876, DTA-1 und MEDI1873;
der Agonist ein CD137-Agonist ist, optional ausgewählt aus einem oder mehr von einem Antikörper oder antigenbindenden Fragment oder kleinem Molekül oder Liganden, das/der spezifisch daran bindet, Utomilumab und 4-1BB-Ligand;
der Agonist ein CD27-Agonist ist, optional ausgewählt aus einem oder mehr von einem Antikörper oder antigenbindenden Fragment oder kleinem Molekül oder Liganden, das/der spezifisch daran bindet, Varlilumab und CDX-1127 (1F5);
der Agonist ein CD28-Agonist ist, optional ausgewählt aus einem oder mehr von einem Antikörper oder antigenbindenden Fragment oder kleinem Molekül oder Liganden, das/der spezifisch daran bindet, und TAB08; und/oder
der Agonist ein HVEM-Agonist ist, optional ausgewählt aus einem oder mehr von einem Antikörper oder antigenbindenden Fragment oder kleinem Molekül oder Liganden, das/der spezifisch daran bindet.

11. Die therapeutische Zusammensetzung zur Verwendung gemäß Anspruch 8, wobei das mindestens eine chemotherapeutische Agens ausgewählt ist aus einem oder mehr von einem alkylierenden Agens, einem Anti-Metaboliten, einem zytotoxischem Antibiotikum, einem TZopoisomerase-Inhibitor (Type 1 oder Typ II) und einem Anti-Mikrotubulus-Agens, einschließlich wobei:
das alkylierende Agens ausgewählt ist aus einem oder mehr von Stickstoffsenfen (optional Mechlorethamin, Cyclophosphamid, Mustin, Melphalan, Chlorambucil, Ifosfamid und Busulfan), Nitrosoharnstoffen (optional N-Nitroso-N-methylharnstoff (MNU), Carmustin (BCNU), Lomustin (CCNU), Semustin (MeCCNU), Fotemustin und Streptozotocin), Tetrazinen (optional Dacarbazin, Mitozolomid und Temozolomid), Aziridinen (optional Thiotepa, Mytomycin und Diaziquon (AZQ)), Cisplatinen und Derivativen davon (optional Carboplatin und Oxaliplatin) und nicht-klassischen alkylierenden Agentien (optional Procarbazin und Hexamethylmelamin);
wobei der Anti-Metabolit ausgewählt ist aus einem oder mehr von Antifolaten (optional Methotrexat und Pemetrexed), Fluorpyrimidinen (optional 5-Fluoruracil und Capecitabin), Deoxynukleosid-Analoga (optional Ancitabin, Enocitabin, Cytarabin, Gemcitabin, Decitabin, Azacitidin, Fludarabin, Nelarabin, Cladribin, Clofarabin, Fludarabin und Pentostatin) und Thiopurinen (optional Thioguanin und Mercaptopurin);
wobei das zytotoxische Antibiotikum ausgewählt ist aus einem oder mehr von Anthracyclinen (optional Doxorubicin, Daunorubicin, Epirubicin, Idarubicin, Pirarubicin, Aclarubicin und Mitoxantron), Bleomycinen, Mitomycin C, Mitoxantron und Actinomycin;
wobei der Topoisomerase-Inhibitor ausgewählt ist aus einem oder mehr von Camptothecin, Irinotecan, Topotecan, Etoposid, Doxorubicin, Mitoxantron, Teniposid, Novobiocin, Merbaron und Aclarubicin; und wobei das Anti-Mikrotubulus-Agens ausgewählt ist aus einem oder mehr von Taxanen (optional Paclitaxel und Docetaxel) und Vinca-Alkaloiden (optional Vinblastin, Vincristin, Vindesin, Vinorelbin);
wobei das mindestens eine hormontherapeutische Agens ein hormonaler Agonist oder hormonaler Antagonist ist; wobei der hormonale Agonist ausgewählt ist aus einem oder mehr von Progestogen (Progestin), einem Kortikosteroid (optional Prednisolon, Methylprednisolon oder Dexamethason), Insulin-artigen Wachstumsfaktoren, VEGF-abgeleiteten angiogenen und lymphangiogenen Faktoren (optional VEGF-A, VEGF-A145, VEGF-A165, VEGF-C, VEGF-D, PIGF-2), Fibroblasten-Wachstumsfaktor (FGF), Galectin, Hepatozyten-Wachstumsfaktor (HGF), Plättchen-abgeleitetem Wachstumsfaktor (PDGF), transformierendem Wachstumsfaktor-(TGF)-beta, einem Androgen, einem Östrogen und einem Somatostatin-Analogon; und wobei
der hormonale Antagonist ausgewählt ist aus einem oder mehr von einem Hormonsyntheseinhibitor, optional einem Aromatase-Inhibitor oder einem Gonadotropin-freisetzendem Hormon (GnRH) oder einem Analogon davon und einem Hormonrezeptorantagonisten, optional einem selektiven Östrogenrezeptormodulator (SERM) oder einem Anti-Androgen, oder einem Antikörper gegen einen hormonalen Rezeptor, optional Cixutumumab, Dalotuzumab, Figitumumab, Ganitumab, Istiratumab, Robatumumab, Alacizumab Pegol, Bevacizumab, Icrucumab, Ramucirumab, Fresolimumab, Metelimumab, Naxitamab, Cetuximab, Depatuxizumab Mafodotin, Futuximab, Imgatuzumab, Laprituximab Emtansin, Matuzumab, Modotuximab, Necitumumab, Nimotuzumab, Panitumumab, Tomuzotuximab, Zalutumumab, Aprutumab Ixadotin, Bemarituzumab, Olaratumab oder Tovetumab; oder
wobei der Kinaseinhibitor ausgewählt ist aus einem oder mehr von Adavosertib, Afanitib, Aflibercept, Axitinib, Bevacizumab, Bosutinib, Cabozantinib, Cetuximab, Cobimetinib, Crizotinib, Dasatinib, Entrectinib, Erdafitinib, Erlotinib, Fostamitinib, Gefitinib, Ibrutinib, Imatinib, Lapatinib, Lenvatinib, Mubritinib, Nilotinib, Panitumumab, Pazopanib, Pegaptanib, Ponatinib, Ranibizumab, Regorafenib, Ruxolitinib, Sorafenib, Sunitinib, SU6656, Tofacitinib, Trastuzumab, Vandetanib und Vemuafenib, oder wobei der Kinaseinhibitor ein PI3-Kinase-Inhibitor ist ausgewählt aus einem oder mehr von Alpelisib, Buparlisib, Copanlisib, CUDC-907, Dactolisib, Duvelisib, GNE-477, Idelasib, IPI-549, LY294002, ME-401, Perifosin, PI-103, Pictilisib, PWT33597, RP6503, Taselisib, Umbralisib, Voxtalisib, Wortmannin und XL147.

12. Die therapeutische Zusammensetzung zur Verwendung gemäß irgendeinem der Ansprüche 1-11, wobei das HRS-Polypeptid eine Aminosäuresequenz umfasst, daraus besteht oder im Wesentlichen daraus besteht, die mindestens 95%, 96%, 97%, 98%, 99% oder 100% identisch ist zu SEQ ID NO:156 (Fc-HRS(2-60) oder **HRS^{FC1}**).

13. Die therapeutische Zusammensetzung zur Verwendung gemäß Anspruch 12, wobei das HRS-Polypeptid SEQ ID NO:156 (Fc-HRS(2-60) oder **HRS^{FC1}**) umfasst.

## Revendications

1. Composition thérapeutique comprenant un polypeptide histidyl-ARNt synthétase (HRS) pour une utilisation dans le traitement d'un cancer associé à la neuropiline-2 (NRP2) chez un sujet qui en a besoin, dans laquelle le cancer associé à la NRP2 exprime ou surexprime la NRP2, et dans laquelle le polypeptide HRS comprend, consiste en, ou consiste essentiellement en une séquence d'acides aminés qui est d'au moins 90 %, 95 %, 96 %, 97 %, 98 %, 99 % ou 100 % identique à une séquence choisie parmi les SEQ ID NO : 156-171.

2. Composition thérapeutique pour une utilisation selon la revendication 1, dans laquelle le cancer présente une croissance dépendante de NRP2, une adhérence dépendante de NRP2, une migration dépendante de NRP2, une chimiorésistance dépendante de NRP2 et/ou une invasion dépendante de NRP2.

3. Composition thérapeutique pour une utilisation selon la revendication 2, dans laquelle le cancer est un cancer primaire, ou dans laquelle le cancer est un cancer métastatique, y compris un cancer métastatique qui exprime NRP2a et/ou NRP2b.

4. Composition thérapeutique pour une utilisation selon la revendication 2 ou 3, dans laquelle le cancer est chimiorésistant à au moins un traitement anticancéreux choisi parmi un agent immunothérapeutique, un agent chimiothérapeutique, un agent hormonothérapeutique et un inhibiteur de kinase, facultativement dans laquelle le procédé comprend la sélection d'un sujet présentant un cancer qui est chimiorésistant avant l'administration du polypeptide HRS.

5. Composition thérapeutique pour une utilisation selon l'une quelconque des revendications 2 à 4, dans laquelle le polypeptide HRS module l'autophagie, l'éphérocytose ou la maturation phagocytaire dans une cellule cancéreuse ou un macrophage associé au cancer, facultativement dans laquelle le polypeptide HRS module l'autophagie dans la cellule cancéreuse.

6. Composition thérapeutique pour une utilisation selon l'une quelconque des revendications 1 à 5, dans laquelle le cancer est choisi parmi un ou plusieurs parmi les mélanome (par exemple, mélanome métastatique), cancer du pancréas, cancer de l'os, cancer de la prostate, cancer du poumon à petites cellules, cancer du poumon non à petites cellules (CPNPC), mésothéliome, leucémie (par exemple, leucémie lymphoïde, leucémie myéloïde chronique, leucémie myéloïde aiguë, leucémie myéloïde aiguë récidivante), lymphome, hépatome (carcinome hépatocellulaire), sarcome, malignité à lymphocytes B, cancer du sein, cancer de l'ovaire, cancer colorectal, gliome, glioblastome multiforme, méningiome, adénome hypophysaire, schwannome vestibulaire, lymphome primaire du SNC, tumeur neuroectodermique primitive (médulloblastome), cancer du rein (p. ex., carcinome à cellules rénales), cancer de la vessie, cancer de l'utérus, cancer de l'œsophage, cancer du cerveau, cancers de la tête et du cou, cancer du col de l'utérus, cancer du testicule, cancer de la thyroïde et cancer de l'estomac.

7. Composition thérapeutique pour une utilisation selon la revendication 5 ou 6, dans laquelle le cancer métastatique est choisi parmi un ou plusieurs parmi :
(a) un cancer de la vessie qui s'est métastasé et propagé à l'os, au foie et/ou aux poumons ;
(b) un cancer du sein qui s'est métastasé et propagé à l'os, au cerveau, au foie et/ou aux poumons ;
(c) un cancer colorectal qui s'est métastasé et propagé au foie, aux poumons et/ou au péritoine ;
(d) un cancer du rein qui s'est métastasé et propagé aux glandes surrénales, à l'os, au cerveau, au foie et/ou aux poumons ;
(e) un cancer du poumon qui s'est métastasé et propagé aux glandes surrénales, à l'os, au cerveau, au foie et/ou à d'autres sites pulmonaires ;
(f) un mélanome qui s'est métastasé et propagé à l'os, au cerveau, au foie, au poumon et/ou à la peau/au muscle ;
(g) un cancer de l'ovaire qui s'est métastasé et propagé au foie, au poumon et/ou au péritoine ;
(h) un cancer du pancréas qui s'est métastasé et propagé au foie, au poumon et/ou au péritoine ;
(i) un cancer de la prostate qui s'est métastasé et propagé aux glandes surrénales, à l'os, au foie et/ou aux poumons ;
(j) un cancer de l'estomac qui s'est métastasé et propagé au foie, au poumon et/ou au péritoine ;
(l) un cancer de la thyroïde qui s'est métastasé et propagé à l'os, au foie et/ou aux poumons ; et
(m) un cancer de l'utérus qui s'est métastasé et propagé à l'os, au foie, au poumon, au péritoine et/ou au vagin.

8. Composition thérapeutique pour une utilisation selon l'une quelconque des revendications 1 à 7, comprenant l'administration au sujet d'au moins un agent supplémentaire choisi parmi un ou plusieurs parmi un agent d'immunothérapie contre le cancer comprenant un agent modulateur de point de contrôle immunitaire, comprenant un antagoniste d'une molécule de point de contrôle immunitaire inhibitrice et un agoniste d'une molécule de point de contrôle immunitaire de stimulation, un agent chimiothérapeutique, un agent hormonothérapeutique et un inhibiteur de kinase.

9. Composition thérapeutique pour une utilisation selon la revendication 8, dans laquelle la molécule de point de contrôle immunitaire inhibitrice est choisie parmi un ou plusieurs parmi les ligand de mort programmée 1 (PD-L1), mort programmée 1 (PD-1), ligand de mort programmée 2 (PD-L2), protéine cytotoxique associée aux lymphocytes T 4 (CTLA-4), indoléamine 2,3-dioxygénase (IDO), tryptophane 2,3-dioxygénase (TDO), domaine des immunoglobulines des lymphocytes T et domaine de la mucine 3 (TIM-3), gène d'activation des lymphocytes 3 (LAG-3), suppresseur Ig du domaine V de l'activation des lymphocytes T (VISTA), atténuateur des lymphocytes B et T (BTLA), CD160, médiateur d'entrée du virus de l'herpès (HVEM) et immunorécepteur des lymphocytes T avec les domaines Ig et ITIM (TIGIT), y compris dans laquelle
l'antagoniste est un antagoniste de PD-L1 et/ou de PD-L2 facultativement choisi parmi un ou plusieurs parmi un anticorps ou un fragment de liaison à l'antigène ou une petite molécule qui s'y lie spécifiquement, l'atézolizumab (MPDL3280A), l'avélumab (MSB0010718C) et le durvalumab (MEDI4736), facultativement dans laquelle le cancer est choisi parmi un ou plusieurs parmi les cancer colorectal, mélanome, cancer du sein, carcinome pulmonaire non à petites cellules, cancer de la vessie et carcinome à cellules rénales ;
l'antagoniste est un antagoniste de PD-1 facultativement choisi parmi un ou plusieurs parmi un anticorps ou un fragment de liaison à l'antigène ou une petite molécule qui s'y lie spécifiquement, le nivolumab, le pembrolizumab, MK-3475, AMP-224, AMP-514PDR001 et le pidilizumab, facultativement dans laquelle l'antagoniste de PD-1 est le nivolumab et le cancer est facultativement choisi parmi un ou plusieurs parmi les lymphome de Hodgkin, mélanome, cancer du poumon non à petites cellules, carcinome hépatocellulaire, carcinome à cellules rénales, et cancer de l'ovaire ;
l'antagoniste de PD-1 est le pembrolizumab et le cancer est facultativement choisi parmi un ou plusieurs parmi les mélanome, cancer du poumon non à petites cellules, cancer du poumon à petites cellules, cancer de la tête et du cou et cancer urothélial ;
l'antagoniste est un antagoniste de CTLA-4 facultativement choisi parmi un ou plusieurs parmi un anticorps ou un fragment de liaison à l'antigène ou une petite molécule qui s'y lie spécifiquement, l'ipilimumab, le trémélimumab, facultativement dans laquelle le cancer est choisi parmi un ou plusieurs parmi les mélanome, cancer de la prostate, cancer du poumon et cancer de la vessie ;
l'antagoniste est un antagoniste de l'IDO facultativement choisi parmi un ou plusieurs parmi un anticorps ou un fragment de liaison à l'antigène ou une petite molécule qui s'y lie spécifiquement, l'indoximod (NLG-8189), le 1-méthyl-tryptophane (1MT), la β-carboline (norharmane ; 9H-pyrido[3,4-b]indole), l'acide rosmarinique et l'épacadostat, et dans laquelle le cancer est facultativement choisi parmi un ou plusieurs parmi les cancer du sein métastatique et cancer du cerveau facultativement glioblastome multiforme, gliome, gliosarcome ou tumeur cérébrale maligne ;
l'antagoniste est un antagoniste de TDO facultativement choisi parmi un ou plusieurs parmi un anticorps ou un fragment de liaison à l'antigène ou une petite molécule qui s'y lie spécifiquement, 680C91, et LM10 ;
l'antagoniste est un antagoniste de TIM-3 facultativement choisi parmi un ou plusieurs parmi un anticorps ou un fragment de liaison à l'antigène ou une petite molécule qui s'y lie spécifiquement ;
l'antagoniste est un antagoniste de LAG-3 facultativement choisi parmi un ou plusieurs parmi un anticorps ou un fragment de liaison à l'antigène ou une petite molécule qui s'y lie spécifiquement, et BMS-986016 ;
l'antagoniste est un antagoniste de VISTA facultativement choisi parmi un ou plusieurs parmi un anticorps ou un fragment de liaison à l'antigène ou une petite molécule qui s'y lie spécifiquement ;
l'antagoniste est un antagoniste de BTLA, CD160 et/ou HVEM facultativement choisi parmi un ou plusieurs parmi un anticorps ou un fragment de liaison à l'antigène ou une petite molécule qui s'y lie spécifiquement ; ou dans laquelle l'antagoniste est un antagoniste de TIGIT facultativement choisi parmi un ou plusieurs parmi un anticorps ou un fragment de liaison à l'antigène ou une petite molécule qui s'y lie spécifiquement.

10. Composition thérapeutique pour une utilisation selon la revendication 8, dans laquelle la molécule de point de contrôle immunitaire stimulant est choisie parmi un ou plusieurs parmi les OX40, CD40, gène lié à la famille TNFR induit par glucocorticoïde (GITR), CD137 (4-1BB), CD27, CD28, CD226, et médiateur d'entrée de virus de l'herpès (HVEM), y compris dans laquelle
l'agoniste est un agoniste d'OX40 facultativement choisi parmi un ou plusieurs parmi un anticorps ou un fragment de liaison à l'antigène ou une petite molécule ou un ligand qui s'y lie spécifiquement, OX86, Fc-OX40L et GSK3174998 ;
l'agoniste est un agoniste de CD40 facultativement choisi parmi un ou plusieurs parmi un anticorps ou un fragment de liaison à l'antigène ou une petite molécule ou un ligand qui s'y lie spécifiquement, CP-870,893, le dacétuzumab, Chi Lob 7/4, ADC-1013, et rhCD40L, et dans laquelle le cancer est facultativement choisi parmi un ou plusieurs parmi les mélanome, carcinome pancréatique, mésothéliome, et les cancers hématologiques, facultativement le lymphome tel que le lymphome non-hodgkinien ;
l'agoniste est un agoniste de GITR facultativement choisi parmi un ou plusieurs parmi un anticorps ou un fragment de liaison à l'antigène ou une petite molécule ou un ligand qui s'y lie spécifiquement, INCAGN01876, DTA-1 et MEDI1873 ;
l'agoniste est un agoniste de CD137 facultativement choisi parmi un ou plusieurs parmi un anticorps ou un fragment de liaison à l'antigène ou une petite molécule ou un ligand qui s'y lie spécifiquement, l'utomilumab et le ligand 4-1BB ;
l'agoniste est un agoniste de CD27 facultativement choisi parmi un ou plusieurs parmi un anticorps ou un fragment de liaison à l'antigène ou une petite molécule ou un ligand qui s'y lie spécifiquement, le varlilumab et CDX-1127 (1F5) ;
l'agoniste est un agoniste de CD28 facultativement choisi parmi un ou plusieurs parmi un anticorps ou un fragment de liaison à l'antigène ou une petite molécule ou un ligand qui s'y lie spécifiquement, et TAB08 ; et/ou
l'agoniste est un agoniste de HVEM facultativement choisi parmi un ou plusieurs parmi un anticorps ou un fragment de liaison à l'antigène ou une petite molécule ou un ligand qui s'y lie spécifiquement.

11. Composition thérapeutique pour une utilisation selon la revendication 8, dans laquelle l'au moins un agent chimiothérapeutique est choisi parmi un ou plusieurs parmi un agent alkylant, un antimétabolite, un antibiotique cytotoxique, un inhibiteur de topoisomérase (type 1 ou type II) et un agent anti-microtubules, y compris dans laquelle :
l'agent alkylant est choisi parmi un ou plusieurs parmi les moutardes azotées (facultativement méchloréthamine, cyclophosphamide, mustine, melphalan, chlorambucil, ifosfamide et busulfan), nitroso-urées (facultativement N-Nitroso-N-méthylurée (MNU), carmustine (BCNU), lomustine (CCNU), sémustine (MeCCNU), fotémustine, et streptozotocine), tétrazines (facultativement dacarbazine, mitozolomide et témozolomide), aziridines (facultativement thiotépa, mytomycine et diaziquone (AZQ)), cisplatines et leurs dérivés (facultativement le carboplatine et l'oxaliplatine), et les agents alkylants non classiques (facultativement la procarbazine et l'hexaméthylmélamine) ;
dans laquelle l'antimétabolite est choisi parmi un ou plusieurs parmi les antifolates (facultativement méthotrexate et pémétrexed), fluoropyrimidines (facultativement 5-fluorouracile et capécitabine), analogues de désoxynucléoside (facultativement ancitabine, énocitabine, cytarabine, gemcitabine, décitabine, azacitidine, fludarabine, nelarabine, cladribine, clofarabine, fludarabine et pentostatine), et thiopurines (facultativement thioguanine et mercaptopurine) ;
dans laquelle l'antibiotique cytotoxique est choisi parmi un ou plusieurs parmi les anthracyclines (facultativement doxorubicine, daunorubicine, épirubicine, idarubicine, pyrarubicine, aclarubicine et mitoxantrone), bléomycines, mitomycine C, mitoxantrone et actinomycine ;
dans laquelle l'inhibiteur de topoisomérase est choisi parmi un ou plusieurs parmi les camptothécine, irinotécan, topotécan, étoposide, doxorubicine, mitoxantrone, téniposide, novobiocine, merbarone et aclarubicine ; et dans laquelle l'agent anti-microtubules est choisi parmi un ou plusieurs parmi les taxanes (facultativement paclitaxel et docétaxel) et alcaloïdes de vinca (facultativement vinblastine, vincristine, vindésine, vinorelbine) ;
dans laquelle l'au moins un agent thérapeutique hormonal est un agoniste hormonal ou un antagoniste hormonal ; dans laquelle l'agoniste hormonal est choisi parmi un ou plusieurs parmi un progestatif (progestine), un corticostéroïde (facultativement prednisolone, méthylprednisolone ou dexaméthasone), les facteurs de croissance semblables à l'insuline, les facteurs angiogènes et lymphangiogènes dérivés du VEGF (en option VEGF-A, VEGF-A145, VEGF-A165, VEGF-C, VEGF-D, PIGF-2), le facteur de croissance des fibroblastes (FGF), la galectine, le facteur de croissance hépatocytaire (HGF), le facteur de croissance dérivé des plaquettes (PDGF), le facteur de croissance transformant (TGF)-bêta, un androgène, un œstrogène, et un analogue de somatostatine ; et dans laquelle
l'antagoniste hormonal est choisi parmi un ou plusieurs parmi un inhibiteur de synthèse hormonale, facultativement un inhibiteur d'aromatase ou une hormone libérant des gonadotrophines (GnRH) ou un analogue de celui-ci, et un antagoniste des récepteurs hormonaux, facultativement un modulateur sélectif des récepteurs des œstrogènes (SERM) ou un anti-androgène, ou un anticorps dirigé contre un récepteur hormonal, facultativement cixutumumab, dalotuzumab, figitumumab, ganitumab, istiratumab, robatumumab, alacizumab pégol, bévacizumab, icrucumab, ramucirumab, fresolimumab, métélimumab, naxitamab, cétuximab, dépatuxizumab mafodotine, futuximab, imgatuzumab, laprituximab emtansine, matuzumab, modotuximab, necitumumab, nimotuzumab, panitumumab, tomuzotuximab, zalutumumab, aprutumab ixadotine, bemarituzumab, olaratumab ou tovetumab ; ou
dans laquelle l'inhibiteur de kinase est choisi parmi un ou plusieurs parmi adavosertib, afanitib, aflibercept, axitinib, bévacizumab, bosutinib, cabozantinib, cétuximab, cobimétinib, crizotinib, dasatinib, entrectinib, erdafitinib, erlotinib, fostamitinib, géfitinib, ibrutinib, imatinib, lapatinib, lenvatinib, mubritinib, nilotinib, panitumumab, pazopanib, pegaptanib, ponatinib, ranibizumab, regorafénib, ruxolitinib, sorafénib, sunitinib, SU6656, tofacitinib, trastuzumab, vandétanib et vémuafénib, ou dans laquelle l'inhibiteur de kinase est un inhibiteur de kinase PI3 choisi parmi un ou plusieurs parmi alpelisib, buparlisib, copanlisib, CUDC-907, dactolisib, duvelisib, GNE-477, idélasib, IPI-549, LY294002, ME-401, périfosine, PI-103, pictilisib, PWT33597, RP6503, taselisib, umbralisib, voxtalisib, wortmannine, et XL147.

12. Composition thérapeutique pour une utilisation selon l'une quelconque des revendications 1 à 11, dans laquelle le polypeptide HRS comprend, consiste ou consiste essentiellement en une séquence d'acides aminés qui est d'au moins 95 %, 96 %, 97 %, 98 %, 99 %, ou 100 % identique à la SEQ ID NO : 156 (Fc-HRS(2-60) ou **HRS^{FC1}**).

13. Composition thérapeutique pour une utilisation selon la revendication 12, dans laquelle le polypeptide HRS comprend la SEQ ID NO : 156 (Fc-HRS(2-60) ou **HRS^{FC1}**).
